(19) Europäisches Patentamt
European Patent Office
Office européen des brevets

(11) EP 1 277 729 A1

(12) **EUROPEAN PATENT APPLICATION**
published in accordance with Art. 158(3) EPC

(43) Date of publication:
**22.01.2003 Bulletin 2003/04**

(21) Application number: **01925984.5**

(22) Date of filing: **26.04.2001**

(51) Int Cl.7: **C07C 233/65**, C07C 233/66, C07C 233/75, C07C 233/80, C07C 233/81, C07C 271/28, C07C 311/08, C07C 311/21, C07C 311/58, C07C 311/64

(86) International application number:
**PCT/JP01/03655**

(87) International publication number:
**WO 01/083427 (08.11.2001 Gazette 2001/45)**

(84) Designated Contracting States:
**AT BE CH CY DE DK ES FI FR GB GR IE IT LI LU MC NL PT SE TR**

(30) Priority: **28.04.2000 JP 2000129565**
**05.03.2001 JP 2001060366**

(71) Applicant: **Sankyo Company, Limited**
**Tokyo 103-8426 (JP)**

(72) Inventors:
- **AMEMIYA, Yoshiya**
**Shinagawa-ku, Tokyo 140-8710 (JP)**
- **WAKABAYASHI, Kenji**
**Shinagawa-ku, Tokyo 140-8710 (JP)**
- **TAKAISHI, Sachiko**
**Shinagawa-ku, Tokyo 140-8710 (JP)**
- **FUKUDA, Chie**
**Shinagawa-ku, Tokyo 140-8710 (JP)**

(74) Representative:
**Gibson, Christian John Robert et al**
**MARKS & CLERK,**
**57/60 Lincoln's Inn Fields**
**London WC2A 3LS (GB)**

(54) **PPAR (GAMMA) MODULATORS**

(57) [Subject]

The present invention has a purpose for providing a PPAR γ modulator which can be a therapeutic agent for retrograde osteoporosis in which excessive differentiation of adipocytes was inhibited and formation and differentiation of osteoblasts from stem cells is facilitated or that for diabetes mellitus without characteristics such as excessive adipogenesis, liver dysfunction, vascular disorders, heart diseases and the like.

[Solution]

A compound of the following formula or a pharmacologically acceptable salt thereof:

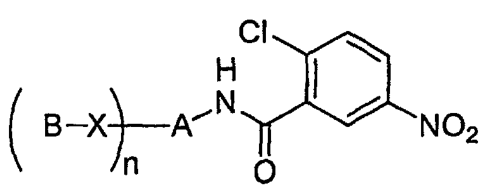

wherein A represents a phenyl group or the like, B represents an aryl group or the like, X represents an oxygen atom or the like, and n represents 0 or 1.

EP 1 277 729 A1

## Description

[Technical field of the invention]

The present invention relates to compounds having PPAR γ modulating activities and their uses.
More precisely, the present invention relates to 2-chloro-5-nitrocarboxamide derivatives and their pharmacologically acceptable salts; to preventive or therapeutic agents containing the said compounds for diseases such as senile osteoporosis, postmenopausal osteoporosis, disuse osteoporosis, method for treatment of steroid-induced osteoporosis, fracture, osteogenesis imperfecta, rachitis, senile arthrosis, obesity, emaciation, type I and type II diabetes mellitus, sclerosis of pulse, lipid metabolism disorder, pancreatitis, autoimmune diseases, glucose metabolism disorder, diabetic neuropathy, diabetic complications, hyperuricemia, leukemia, functional disorders in retinoid related receptors, liver dysfunction, anemia, cancers, inflammation, Basedow's disease, heart disease, Alzheimer's disease, eating disorders, hypertension and renal diseases.

[Background of the invention]

Peroxisome proliferator activated receptor (PPAR) is one of the nuclear receptor members.
It is known that there are generally partial agonists and antagonists, which are called as a group modulators, besides agonists and antagonists in the nuclear receptors. As examples of other nuclear receptors, raloxifene or tamoxifen are known, which are partial agonists or antagonists toward estrogen receptors. Schematic dose-response curves of a typical partial agonist or antagonist are presented in Fig. 1. As is demonstrated in Fig. 1, the partial agonist has a character to induce lower transcriptional activation when compared with the agonist. Further, when a partial antagonist is present under the presence of an agonist, it inhibits the transcriptional activation by the agonist. However, the extent of the inhibition is characteristically lower than that shown by the antagonist. Furthermore generally, the partial agonist often shows character of the partial antagonist.
In many cases, partial agonists and antagonists are developed under expectation that they may reduce undesirable tissue-specific effects which are shown by agonists or antagonists and induce desirable tissue-specific effects which are also shown by agonists or antagonists.
Meanwhile, various thiazolidinedione derivatives decrease blood sugar level in animal models of non-insulin-dependent diabetes mellitus (NIDDM), and they are expected as novel therapeutic agents for NIDDM which have a releasing effect from insulin resistance. Recent researches have disclosed that these thiazolidinedione derivatives act as PPAR γ modulators and activate PPAR γ specifically [Lehmann et al., Journal Biological Chemistry 270, 12953-12956, (1995)]. Since PPAR γ activating actions of these thiazolidinedione derivatives correlate well to blood sugar lowering effects in transmissible adiposis mice, PPAR γ is considered to be a target molecule of the pharmacological actions of thiazolidinedione derivatives [Willson et al., Journal of Medicinal Chemistry, 39, 665-668, (1996)].
From these findings, a compound acting specifically as a PPAR γ modulator is thought to be very effective as an agent for diabetes mellitus.
On the other hand, the bone salt level decreases with aging in both men and women, and osteoporosis is diagnosed when the bone salt level decreases to a level less than a certain level (less than 70% of the bone salt level of young adults). Osteoporosis is mainly divided into two large groups, primary osteoporosis without background diseases which are due to osteoporosis and secondary osteoporosis in which background diseases are clearly found. Primary osteoporosis includes post-menopausal osteoporosis in post-menopausal women and senile osteoporosis in elderly people. These two kinds of osteoporoses are called as a group retrograde osteoporosis.
It has been reported that the rate of adipose marrow in the bone tissue of retrograde osteoporosis is higher compared with those of healthy people [Burkuhardt et al., Bone 8: 157-164 (1987)]; Meunier et al., Clin. Ortyop. Re I. Res 80: 147-154 (1971)]. Moreover, similar changes are observed to occur in patients with atrophy of the bones due to immobility [Minaire et al., Calcified Tissue International 36: 338-340 (1984); Calcified Tissue International 17: 57-73 (1974)].
On the other hand, PPAR γ is considered to be a closely related factor to the differentiation of adipocytes [Tontonoz et al., Genes and Development, 8, 1224-1234, 1994; Tontonoz et al., Cell, 79, 1147-1156, 1994].
Thus PPAR γ modulators which prevent marrow cells from becoming adipocytes are promising as therapeutic agents for retrograde osteoporosis.
Active formulations of vitamin D, vitamin K, calcitonin, bisphosphonates and so forth have been used as therapeutic agents for retrograde osteoporosis. However, the major pharmacological actions of these agents are inhibitory actions against enhanced osteoclasis and there have been no compounds so far found to recover or facilitate osteogenesis which is decreased with aging.
The inventors of the present invention considered that PPAR γ modulators which inhibit excessive differentiation of adipocytes and facilitate formation and differentiation of osteoblasts differentiating from stem cells could be

therapeutic agents for retrograde osteoporosis or therapeutic agents for diabetes mellitus without characteristic actions such as excessive adipogenesis, liver dysfunction, vascular disorders, heart diseases and so forth. As a result of various studies, they found that certain compounds have PPAR γ modulating activities. Further, they completed their invention by confirming that these PPAR γ modulators were useful as preventive and therapeutic agents for osteoporosis and diabetes mellitus.

[Disclosure of the invention]

The present inventions are

(1) a compound of the following formula (I) or a pharmacologically acceptable salt thereof:

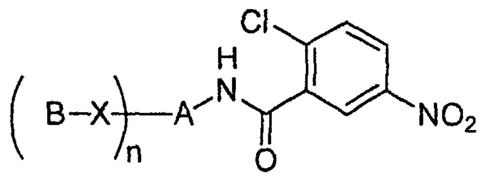

wherein

A represents a phenyl group, a naphthyl group, an acenaphthenyl group, a pyridyl group, a quinolyl group, an isoquinolyl group, a pyrimidinyl group, a furyl group, a benzofuryl group, a pyranyl group, a chromenyl group, a thienyl group, a benzothienyl group, a pyrrolyl group, an indolyl group, an isoindolyl group, an imidazolyl group, a pyrazolyl group, a pyridazinyl group, a pyrazinyl group, an oxazolyl group, an isoxazolyl group, a benzoxazolyl group, a benzisoxazolyl group, a thiazolyl group, an isothiazolyl group, a benzothiazolyl group, benzisothiazolyl or a biphenyl group (said group A is optionally substituted with one, two or more substituents which are the same or different and are selected from the substituent group α described below);
B represents an aryl group, a cycloalkyl group or a heterocyclic group (said group B is optionally substituted with one, two or more substituents which are the same or different and are selected from the substituent group α described below);
X represents a bond, an oxygen atom, a sulfur atom, a $CH_2$ group, a CO group, an NH group, an $SO_2NH$ group, an $NHSO_2$ group, a CONH group, an NHCO group or an $OCH_2$ group;
n represents 0 or 1;

Substituent group α comprises a $C_1$-$C_{20}$ alkyl group, a nitro group, a cyano group, a carboxyl group, a carboxy-$C_2$-$C_7$ alkyl group, a $C_2$-$C_7$ alkyloxycarbonyl group, a $C_3$-$C_{15}$ alkyloxycarbonylalkyl group, an amino group (said amino group is optionally substituted with one or two $C_1$-$C_6$ alkyl groups which are the same or different, or a $C_3$-$C_6$ alkenyl group), a hydroxyl group (said hydroxyl group is optionally substituted with a $C_1$-$C_6$ alkyl group or a $C_1$-$C_6$ haloalkyl group) and a mercapto group (said mercapto group is optionally substituted with a $C_1$-$C_6$ alkyl group);

Substituent group β comprises a halogen atom, a sulfonamide group, a $C_1$-$C_6$ alkylsulfonamide group, an amidinoaminosulfonyl group and a phenyl group;

(2) a compound or a pharmacologically acceptable salt thereof according to (1) wherein A is a thiazolyl group;
(3) an agent inhibiting adipocyte differentiation in the marrow containing a compound or a pharmacologically acceptable salt thereof according to (1) or (2);
(4) an agent enhancing or recovering osteogenetic function containing a compound or a pharmacologically acceptable salt thereof according to (1) or (2);
(5) an agent for treatment or prevention of osteoporosis containing a compound or a pharmacologically acceptable salt thereof according to (1) or (2);
(6) an agent for treatment or prevention of senile osteoporosis, post-menopausal osteoporosis or disuse osteoporosis containing a compound or a pharmacologically acceptable salt thereof according to (1) or (2);
(7) a PPAR γ modulator containing a compound or a pharmacologically acceptable salt thereof according to (1) or (2);

(8) a blood sugar lowering agent containing a compound or a pharmacologically acceptable salt thereof according to (1) or (2);
(9) an agent for treatment or prevention of diabetes mellitus containing a compound or a pharmacologically acceptable salt thereof according to (1) or (2);
(10) an agent for treatment or prevention of type I diabetes mellitus, type II diabetes mellitus, glucose metabolism disorder, diabetes neuropathy or diabetic complications containing a compound or a pharmacologically acceptable salt thereof according to (1) or (2);
(11) an agent for treatment or prevention of fracture, osteogenesis imperfecta, rachitis, senile arthrosis, obesity, emaciation, sclerosis of pulse, lipid metabolism disorder, pancreatitis, autoimmune diseases, hyperuricemia, leukemia, functional disorders in retinoid related receptors, liver dysfunction, anemia, cancers, inflammation, Basedow's disease, heart disease, Alzheimer's disease, eating disorders, hypertension or renal diseases containing a compound or a pharmacologically acceptable salt thereof according to (1) or (2);
(12) an agent inhibiting adipocyte differentiation in the marrow containing a PPAR $\gamma$ modulator;
(13) an agent enhancing or recovering osteogenetic function containing a PPAR $\gamma$ modulator;
(14) an agent for treatment or prevention of osteoporosis containing a PPAR $\gamma$ modulator;
(15) an agent for treatment or prevention of senile osteoporosis, post-menopausal osteoporosis or disuse osteoporosis containing a PPAR $\gamma$ modulator;
(16) a blood sugar lowering agent containing a PPAR $\gamma$ modulator;
(17) an agent for treatment or prevention of diabetes mellitus containing a PPAR $\gamma$ modulator;
(18) an agent for treatment or prevention of type I diabetes mellitus, type II diabetes mellitus, glucose metabolism disorder, diabetic neuropathy or diabetic complications containing a PPAR $\gamma$ modulator;
(19) an agent for treatment or prevention of fracture, osteogenesis imperfecta, rachitis, senile arthrosis, obesity, emaciation, sclerosis of pulse, lipid metabolism disorder, pancreatitis, autoimmune diseases, hyperuricemia, leukemia, functional disorders in retinoid related receptors, liver dysfunction, anemia, cancers, inflammation, Basedow's disease, heart disease, Alzheimer's disease, eating disorders, hypertension or renal diseases containing a PPAR $\gamma$ modulator;
(20) the use of a compound or a pharmacologically acceptable salt thereof according to (1) or (2) in preparation of an agent for treatment or prevention of osteoporosis;
(21) the use of a PPAR $\gamma$ modulator in preparation of an agent for treatment or prevention of osteoporosis,
(22) a partial antagonist of PPAR $\gamma$;
(23) an agent for treatment or prevention according to (12) to (19) wherein the PPAR $\gamma$ modulator is a partial antagonist of PPAR $\gamma$; and
(24) the use of a partial antagonist of PPAR $\gamma$ in preparation of an agent for treatment or prevention of osteoporosis.

In the present invention, the "$C_1$-$C_{20}$ alkyl group" is a straight or branched chain $C_1$-$C_{20}$ alkyl group such as methyl, ethyl, propyl, isopropyl, butyl, isobutyl, s-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl group, heptyl, octyl, nonyl, decyl, undecyl, dodecyl, tridecyl, tetradecyl, pentadecyl, hexadecyl, heptadecyl, octadecyl, nonadecyl and icosyl. In the present invention, this alkyl group is preferably a $C_1$-$C_6$ alkyl group, more preferably a $C_1$-$C_6$ alkyl group and still more preferably a methyl group or an ethyl group.

In the present invention, the "$C_2$-$C_7$ alkyloxycarbonyl group" is a group in which an oxygen atom to which the $C_1$-$C_6$ alkyl group described above is attached binds to a carbonyl group.

In the present invention, the "halogen atom" is a fluorine atom, a chlorine atom, a bromine atom or a iodine atom.

In the present invention, the "$C_3$-$C_6$ alkenyl group" is a straight or branched chain $C_3$-$C_6$ alkenyl group such as 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl and 5-hexenyl. In the present invention, this alkenyl group is preferably a $C_1$-$C_4$ alkenyl group and more preferably butenyl or pentenyl.

In the present invention, the "amino group (said amino group is optionally substituted with one or two $C_1$-$C_6$ alkyl groups which are the same or different or a $C_3$-$C_6$ alkenyl group)" is an unsubstituted amino group, an amino group substituted with a $C_1$-$C_6$ alkyl group such as methylamino, ethylamino, propylamino, pentylamino, butylamino, pentylamino, and hexylamino, an amino group substituted with two $C_1$-$C_6$ alkyl groups which are the same or different such as dimethylamino, diethylamino, dipropylamino, N-ethylmethylamino, N-methylpropylamino and N-methylhexylamino or an amino group substituted with a $C_3$-$C_6$ alkenyl group such as allylamino, butenylamino, pentenylamino

and hexenylamino. In the present invention, this amino group is preferably an unsubstituted amino group or an amino group substituted with a $C_1$-$C_6$ alkyl group and more preferably an unsubstituted amino group, a methylamino group or an ethylamino group.

In the present invention, the "hydroxyl group (said hydroxyl group is optionally substituted with a $C_1$-$C_6$ alkyl group or a $C_1$-$C_6$ haloalkyl group)" is an unsubstituted hydroxyl group, a straight or branched chain $C_1$-$C_6$ alkoxy group such as methoxy, ethoxy, propoxy, butoxy, pentyloxy and hexyloxy group or a straight or branched chain $C_1$-$C_6$ alkoxyl group substituted with one, two or more halogen atoms as described above such as trifluoromethoxy group. In the present invention, this hydroxyl group is preferably an unsubstituted hydroxyl group, a methoxy group, an ethoxy group or a trifluoromethoxy group.

In the present invention, the "mercapto group (said mercapto group is optionally substituted with a $C_1$-$C_6$ alkyl group)" is an unsubstituted mercapto group or a straight or branched chain $C_1$-$C_6$ alkylthio group such as methylthio, ethylthio, propylthio, butylthio, pentylthio and hexylthio group. In the present invention, this mercapto group is preferably an unsubstituted hydroxy group, a methylthio group or an ethylthio group.

In the present invention, the "cycloalkyl group" is a 3 to 10-membered saturated cyclic hydrocarbon group which optionally forms a fused ring such as cyclopropyl, cyclobutyl, cyclpentyl, cyclohexyl, cycloheptyl, norbornyl and adamantly and preferably a 5 to 10-membered saturated cyclic hydrocarbon group.

In the present invention, the "aryl group" is an aromatic hydrocarbon group having 5 to 14 carbons such as phenyl, indenyl, naphthyl, phenanthrenyl and anthracenyl and preferably a phenyl group.

In addition, the aryl group described above may optionally fuse to a cycloalkyl group having 3 to 10 carbons and such group is for example an 2-indanyl group.

In the present invention, the "heterocyclic group" is a 5 to 7-membered heterocyclic group having 1 to 3 hetero atoms which are a sulfur atom, an oxygen atom and/or a nitrogen atom; and is for example an aromatic heterocyclic group such as furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl and pyrazinyl or a partially or completely reduced group according to these groups such as morpholinyl, thiomorpholinyl, pyrrolidinyl, pyrrolinyl, imidazolidynyl, imidazolinyl, pyrazolidinyl, pyrazolinyl, piperidyl and piperazinyl. The preferable group is a 5 to 7-membered heterocyclic group which has at least one nitrogen atom and may optionally contain an oxygen atom or a sulfur atom; and is for example an aromatic hetrocyclic group such as pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyridyl, pyridazinyl, pyrimidinyl and pyrazinyl or a partially or completely reduced group according to these groups such as morpholinyl, thiomorpholinyl, pyrrolidinyl, pyrrolinyl, imidazolidinyl, imidazolinyl, pyrazolidinyl, pyrazolinyl, piperidyl and piperazinyl; and the more preferable group is imidazolyl, oxazolyl, isoxazolyl, thiazolyl group or a partially or completely reduced group according to these groups.

In addition, the "heterocyclic group" described above may optionally fuse to another ring group and such a group is for example isobenzofuranyl, benzoxazolyl, benzisoxazolyl, benzothiazolyl, benzisothiazolyl, chromenyl, chromanonyl, xanthenyl, phenoxathiinyl, indolizinyl, isoindolyl, indolyl, indazolyl, purinyl, quinolizinyl, isoquinolyl, quinolyl, phthalazinyl, naphthylidinyl, quinoxalinyl, quinazolinyl, carbazolyl, carbolinyl, acridinyl or isoindolinyl group.

In the present invention, the "carboxy $C_2$-$C_7$ alkyl group" is a group in which a carboxyl group binds to the $C_1$-$C_6$ alkyl group described above.

In the present invention, a "$C_3$-$C_{15}$ alkyloxycarbonylalkyl group" is a group in which the carboxyl group of a carboxy $C_2$-$C_7$ alkyl group described above and an alkyl group form an ester group.

In the present invention, when n is 1, a group of formula B-X-A- represents a B-A-group, a B-O-A- group, a B-S-A- group, a B-NH-A- group, a B-$SO_2$NH-A- group, a B-NH$SO_2$-A- group, a B-CONH-A- group, a B-NHCO-A- group or a B-O$CH_2$-A- group.

In the present invention, when n is 1, a group of formula B-X- may bind to any substitution position of the group of formula A.

In a compound of formula (I), the 2-chloro-5-nitrophenylcarbonylamino group may bind to any substitution position of the group of formula A.

In addition, when n is 1, every relative substitution position of the group of formula B-X- and the 2-chloro-5-nitrophenylcarbonylamino group may be permitted. When A is a phenyl group, the preferred substitution position is the para position. When A is a pyridyl group, preferred substitution positions are the 2-position for the group of formula B-X-and the 5-position for the 2-chloro-5-nitrophenylcarbonylamino group; or the 3-position for the group of formula B-X- and the 6-position for the 2-chloro-5-nitrophenylcarbonylamino group.

The compound of formula (I) of the present invention can be converted to a salt by any usual method and the present invention encompasses these salts of the compounds.

Such salts are for example metal salts such as alkaline metal salts, which are for example sodium salts, potassium salts or lithium salts, alkaline earth metal salts, which are for example calcium salts or magnesium salts, aluminum salts, iron salts, zinc salts, copper salts, nickel salts and cobalt salts; amine salts such as inorganic salts,

which are for example ammonium salts, and organic salts, which are for example t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-N-phenethylamine salts, piperazine salts, tetramethylammonium salts or tris(hydroxymethyl)aminomethane salts; hydrohalogenic acid salts such as hydrofluoric acid salts, hydrochloric acid salts, hydrobromic acid salts and hydroiodic acid salts; inorganic acid salts such as nitric acid salts, perchloric acid salts, sulfuric acid salts and phosphoric acid salts; organic acid salts such as lower alkanesulfonic acid salts, which are for example methanesulfonic acid salts, trifluoromethanesulfonic acid salts or ethanesulfonic acid salts, arylsulfonic acid salts, which are for example benzensulfonic acid salts or p-toluenesulfonic acid salts, acetic acid, malic acid, fumaric acid salts, succinic acid salts, citric acid salts, tartaric acid salts, oxalic acid salts and maleic acid salts; or amino-acid salts such as ornithinic acid salts, glutamic acid salts and aspartic acid salts. Preferred salts are hydrohalogenic acid salts or organic acid salts.

When a compound of formula (I) of the present invention is allowed to stand in the atmosphere or is recrystallized, it may absorb water or water may be attached to it and then it may form a hydrate. When it forms such a solvate, the present invention encompasses all the solvates.

In addition, a compound of formula (I) of the present invention may absorb another kind of solvent to form a solvate. The present invention encompasses such solvates.

In addition, the present invention also encompasses all of what are called prodrugs which can be converted to a compound of formula (I) or a pharmacologically acceptable salt thereof by metabolism in vivo.

[Brief explanation of figures]

[Figure 1] A schematic diagram of dose-response curves of a partial agonist and a partial antagonist.

In this figure, the solid line indicates transcriptional activity in the presence of an agonist and the dotted line indicates transcriptional activity in the absence of an agonist.

Transcriptional activity rate in the presence of an agonist is defined as 100% and that in vehicle alone is defined as 0%. Transcriptional activity rate of a partial agonist alone is indicated as Emax (%) and that of a partial antagonist in the presence of an agonist is indicated as Imax (%). Further, the concentration of a partial agonist showing a value of Emax/2 is defined as $EC_{50}$ and the concentration of a partial antagonist showing a value of 100-Imax/2 is defined as $IC_{50}$.

[Figure 2] A schematic diagram showing composition of a plasmid employed, pSG5-hPPARg.

[Figure 3] A schematic diagram showing composition of a plasmid employed, pGV-P2-PPRE.

[Best mode for carrying out the invention]

An amido-carboxylic acid derivative of formula (I) and a pharmacologically acceptable salt thereof can be easily prepared according to the following methods.

(Method A)

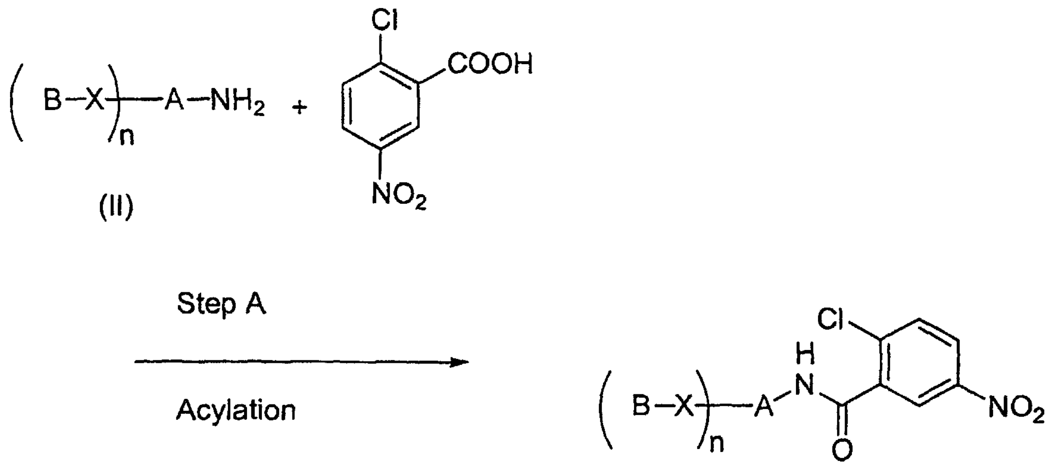


(II)

(I)

In the above formula, A, B, X and n have the same meanings as described above. Step A

Step A is a step in which the above compound of formula (I) is prepared and the compound is prepared by acylation of the above amine compound of formula (II).

The compound of formula (II), a starting material in this step, is commercially available or can be prepared according to well known methods such as a method described after Method B.

The reaction is an amide-bond forming reaction well known in the field of organic synthetic chemistry and is usually preferably carried out in the presence of a solvent.

The solvent employed is not particularly limited provided that it has no effect on the reaction. Such solvent is for example an inert solvent and preferably a halogenohydrocarbon such as dichloromethane and chloroform, an ester such as ethyl acetate, an ether such as tetrahydrofuran and dioxane or an amide such as N,N-dimethylacetamide and N,N-dimethylformamide.

The reaction is accomplished by treatment with a condensing reagent. The condensing reagent employed is for example a carbodiimide such as N,N-dicyclohexylcarbodiimide and 1-(3-dimethylaminopropyl)-3-ethylcarbodiimide hydrochloride, a phosphoryl compound such as diphenylphosphoryl azide and diethylphosphoryl cyanide, carbonyldiimidazole, triphenylphosphine - diethyl azodicarboxylate, 1-propanephosphonic acid cyclic anhydride or the like; and preferably carbonyldiimidazole or a carbodiimide. When phosphoryl compounds are used, the reaction is preferably carried out in the presence of a tertiary amine such as triethylamine and N-methylmorpholine.

The reaction can also be accomplished by; 1) forming a mixed acid anhydride by the reaction of the carboxylic acid or a salt thereof used in this reaction with a lower-alkyl chloroformate such as ethyl chloroformate, isobutyl chloroformate and the like in the presence of a tertiary amine such as triethylamine, N-methylmorpholine and the like; or 2) forming the corresponding active ester by the reaction of the carboxylic acid or salt thereof used in this reaction with N-hydroxysuccinimide, N-hydroxybenztriazole, p-nitrophenol and the like in the presence of a carbodiimide such as N,N-dicyclohexylcarbodiimide and the like; and

3) condensing the mixed acid anhydride in 1) or the active ester in 2) with the amine.

The reaction is usually preferably carried out in the presence of a solvent.

The solvent employed is not particularly limited provided that it has no effect on the reaction. Such solvent is for example an inert solvent and preferably a halogenohydrocarbon such as dichloromethane and chloroform, an ether such as tetrahydrofuran and dioxane or an aromatic hydrocarbon such as benzene and toluene.

Furthermore a compound of formula (I) can also be obtained by reaction of an acyl halide such as carbonchloride with the above amine of formula (II) in N,N-dimethylacetamide, if necessary, in the presence of a base such as pyridine, triethylamine and the like.

The reaction is usually preferably carried out in the presence of a solvent.

The solvent employed is not particularly limited provided that it has no effect on the reaction. Such solvent is for example an inert solvent and preferably a halogenohydrocarbon such as dichloromethane, an ether such as tetrahydrofuran and dioxane or an aromatic hydrocarbon such as benzene and toluene.

The reaction temperature is carried out at -20°C to 100°C, preferably at -5°C to 50°C.

The reaction time differs depending on factors such as the reagents, reaction temperature, the solvent and

the like, and it is usually from 30 minutes to 24 hours, preferably from 1 hour to 16 hours.

(Method B)

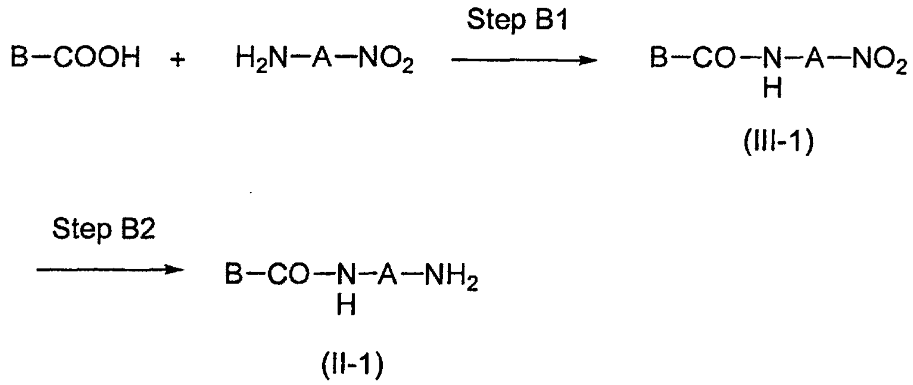

(III-1)
(II-1)

In the above formula, A and B have the same meanings as described above.

Method B is a method for preparation of a compound of formula (II-1) in which X is CONH and n is 1 in a compound of formula (II), a starting material in the Step A.

Step B1

Step B1 is a step in which a nitro-amide compound of formula (III-1) having an amide bond is prepared by condensing a carboxylic acid with an amine.

This step can be carried out according to a similar method to that described in Step A.

Step B2

Step B2 is a step in which an amino compound of formula (II-1) is prepared by reduction of a nitro-amide compound of formula (III-1).

The reaction is a catalytic hydrogenation reaction well known in the field of organic synthetic chemistry and is usually preferably carried out in the presence of a solvent.

The catalyst employed is for example palladium-carbon, palladium hydroxide-carbon, palladium black, platinum oxide, platinum black or the like and preferably palladium-carbon.

The reaction is usually preferably carried out in the presence of a solvent.

The solvent employed is not particularly limited provided that it has no effect on the reaction. Such solvent is for example a hydrocarbon such as benzene, toluene, xylene, hexane and heptane; a halogenohydrocarbon such as chloroform, methylene chloride and carbon tetrachloride; an ether such as diethyl ether, tetrahydrofuran and dioxane; an alcohol such as methanol, ethanol and isopropanol; an amide such as N,N-dimethylformamide, N,N-dimethylacetamide and hexamethyl phosphoroustriamide; a carboxylic acid such as formic acid and acetic acid; or a mixture thereof; and preferably an alcohol or an ether.

The reaction temperature is from 10°C to 140°C and preferably from 20°C to 120°C.

The reaction time differs depending on factors such as the reagents, reaction temperature and the solvent and it is usually from 30 minutes to 3 days and preferably from 1 hour to 24 hours.

In addition the reaction of this step can also be carried out using a reducing agent such as tin (IV) chloride, nickel chloride and the like and, if necessary, a reducing agent such as sodium borohydride and the like may coexist.

The reaction is usually preferably carried out in the presence of a solvent. The solvent employed is not particularly limited provided that it has no effect on the reaction. Such solvent is for example a hydrocarbon such as benzene, toluene, xylene, hexane and heptane; a halogenohydrocarbon such as chloroform, methylene chloride and carbon tetrachloride; an ether such as diethyl ether, tetrahydrofuran and dioxane; an alcohol such as methanol, ethanol, and isopropanol; an amide such as N,N-dimethylformamide, N,N-dimethylacetamide and hexamethyl phosphoroustriamide; a carboxylic acid such as formic acid and acetic acid; or a mixture thereof; and preferably an alcohol or an ether.

The reaction temperature is from 10°C to 140°C and preferably from 20°C to 120°C.

The reaction time differs depending on factors such as the reagents, reaction temperature, the solvent and the like, and it is usually from 30 minutes to 3 days and preferably from 1 hour to 24 hours.

(Method C)

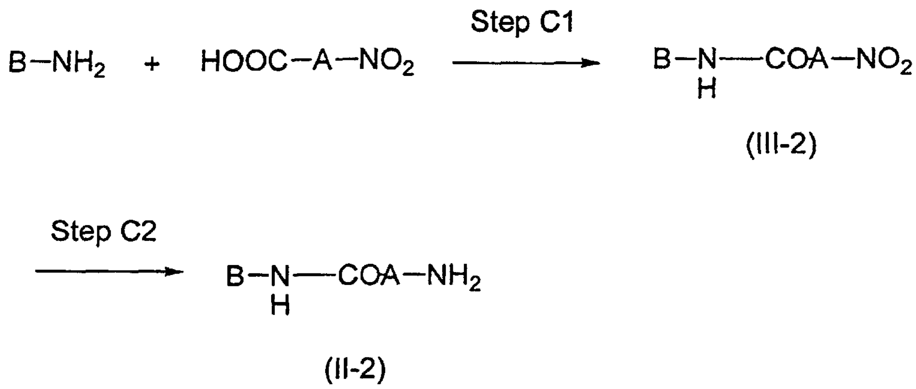


(III-2)

(II-2)

In the above formula, A and B have the same meanings as described above.

Method C is a method for preparation of a compound of formula (II-2) in which X is NHCO and n is 1 in a compound of formula (II), a starting material in the Step A.

Step C1

Step C1 is a step in which a nitro-amide compound of formula (III-2) having an amide bond is prepared by condensing an amine with a carboxylic acid.

In the above formula, A, B and X have the same meanings as described above.

This step can be carried out according to a similar method to that described in Step A.

Step C2

Step C2 is a step in which an amino compound of formula (II-2) is prepared by reducing a nitro-amide compound of formula (III-2).

This step can be carried out according to a similar method to that described in Step B2.

(Method D)

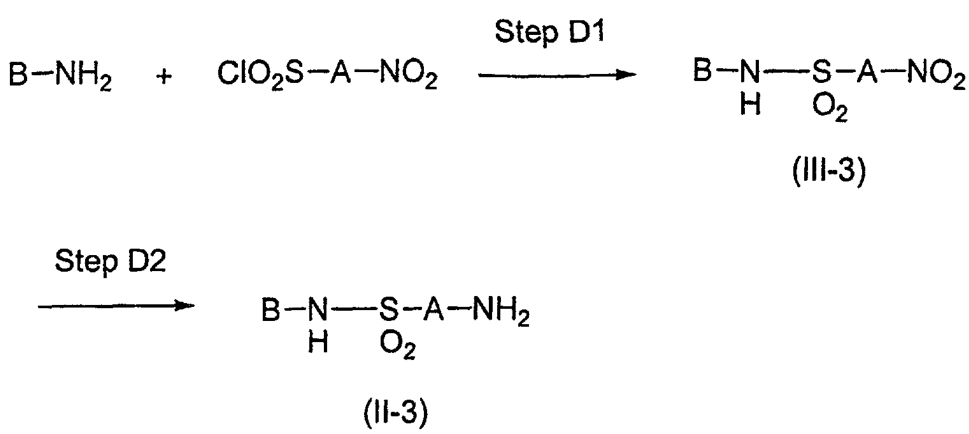


(III-3)

(II-3)

In the above formula, A and B have the same meanings as described above.

Method D is a method for preparation of a compound of formula (II-3) in which X is $NHSO_2$ and n is 1 in a compound of formula (II), a starting material in the Step A.

Step D1

Step D1 is a step in which a nitro-sulfonamide compound of formula (III-3) having an sulfonamide bond is prepared by condensing an amine with a sulfonyl chloride.
The reaction is a sulfonamide bond forming reaction generally known in the field of organic synthetic chemistry.
The reaction is usually preferably carried out in the presence of a solvent.
The solvent employed is not particularly limited provided that it has no effect on the reaction. Such solvent is for example an inert solvent and preferably a halogenohydrocarbon such as dichloromethane, an ether such as tetrahydrofuran and dioxane or an aromatic hydrocarbon such as benzene and toluene.
The reaction temperature is from -20°C to 100°C and preferably from -5°C to 50°C.
The reaction time differs depending on factors such as the reagents, reaction temperature and the solvent and it is usually from 30 minutes to 24 hours and preferably from 1 hour to 16 hours.

Step D2

Step D2 is a step in which an amino-sulfonamide compound of formula (II-3) is prepared by reducing a nitro-sulfonamide compound of formula (III-3).
This step can be carried out according to a similar method to that described in Step B2.

(Method E)

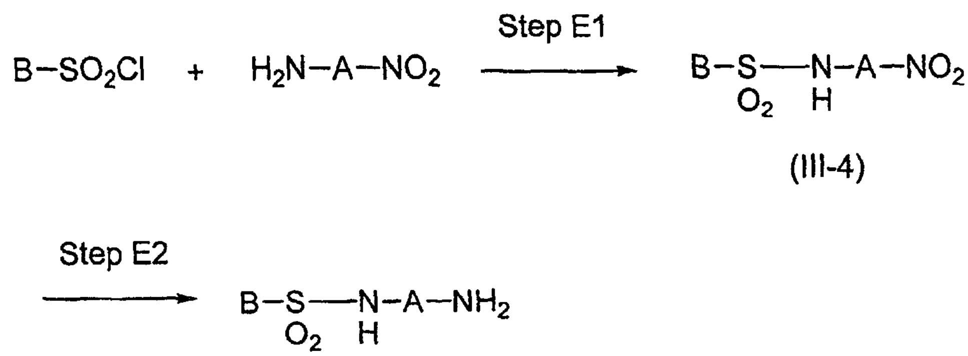


(III-4)

(II-4)

In the above formula, A and B have the same meanings as described above.
Method E is a method for preparation of a compound of formula (II-4) in which X is $SO_2NH$ and n is 1 in a compound of formula (II), a starting material in the Step A.

Step E1

Step E1 is a step in which a nitro-sulfonamide compound of formula (III-4) having a sulfonamide bond is prepared.
This step can be carried out according to a similar method to that described in Step D1.

Step E2

Step E2 is a step in which an amino-sulfonamide compound of formula (II-4) is prepared by reducing a nitro-sulfonamide compound of formula (III-4).
This step can be carried out according to a similar method to that described in Step B2.

(Method F)

A 2-thiazole-amine derivative of formula (II-4) described below, which is a starting material for preparation of a compound of this invention, can be easily prepared according to the following methods.

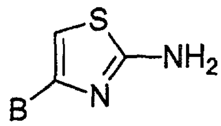

(II-4)

In the above formula, B has the same meaning as described above.

Step F1

Step F1 is a step in which a 2-thiazole-amine derivative of formula (II-4) is prepared from a methylketone compound.

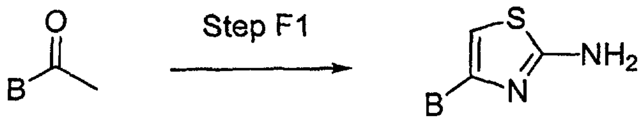


(II-4)

This step is a step in which a 2-thiazole-amine derivative of formula (II-4) is prepared by heating a mixture of a methylketone compound and thiourea in the presence of iodine or bromine according to a similar procedure to that described in J. Am. Chem. Soc. 72, 3722-3725 or Bull. Soc. Chim. Fr. 1437-1439 (1958).

Step F2

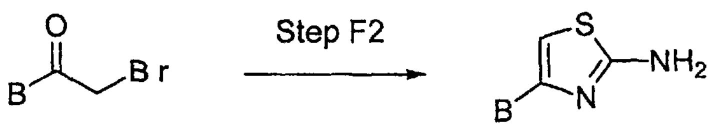


(II-4)

Step F2 is a step in which a 2-thiazole-amine derivative of formula (II-4) is prepared by the reaction of an α-bromomethylketone compound with thiourea according to a similar procedure to that described in J. Indian. Chem. Soc. 51, 1031-1034 (1974).

The reaction is usually preferably carried out in the presence of a solvent.

The solvent employed is not particularly limited provided that it has no effect on the reaction. Such solvent is for example an inert solvent and preferably a halogenohydrocarbon such as dichloromethane and chloroform, an ester such as ethyl acetate, an ether such as tetrahydrofuran and dioxane, an amide such as N,N-dimethylacetamide and N,N-dimethylformamide or a ketone such as acetone, MEK and the like.

The reaction temperature is from -20°C to 100°C and preferably from -5°C to 50°C.

The reaction time differs depending on factors such as the reagents, reaction temperature and a solvent and it is usually from 30 minutes to 24 hours and preferably from 5 hour to 16 hours.

After completion of the reactions described in methods A to F, a desired product in each reaction is isolated from the reaction mixture according to a usual method. For example, the reaction mixture is neutralized; the insoluble material is filtered off, if there is insoluble material in the reaction mixture; immiscible solvents such as water and ethyl acetate are added; an organic layer containing the desired product is separated, washed with water and the like and then dried over anhydrous magnesium sulfate, anhydrous sodium sulfate, anhydrous sodium bicarbonate or the like;

and then the desired product is afforded by removal of the solvent. The desired product afforded can, if necessary, be separated and purified by a usual method; for example, by combining a method commonly used for separation and purification of oarganic compounds such as recrystallization, reprecipitation and the like, or by applying chromatography and eluting with an appropriate eluant.

When a compound of the above formula (I) or a pharmacologically acceptable salt thereof is used as an agent for treatment or prevention of a disease, it can be administered alone, orally as a tablet, a capsule, a granule, a powder or a syrup or non-orally as an injection or a suppository by mixing with pharmacologically acceptable appropriate ingredients, diluents and the like.

These formulations are prepared by known methods using additives such as an excipient (which is for example an organic excipient or an inorganic excipient, in which an organic excipient is for example a sugar derivative such as lactose, sucrose, glucose, mannitol, and sorbitol; a starch derivative such as corn starch, potato starch, α-starch, and dextrin; a cellulose derivative such as crystalline cellulose; acacia; dextran; or pullulan and an inorganic excipient is for example a silicate derivative such as light silicic acid anhydride, synthetic aluminum silicate, calcium silicate, and magnesium metasilicate aluminate; a phosphate such as calcium hydrogenphosphate; a carbonate such as calcium carbonate; a sulfate such as calcium sulfate; or the like), a lubricant (which is for example a stearic acid; a metal stearate such as calcium stearate and magnesium stearate; talc; colloidal silica; a wax such as beeswax and spermaceti; boric acid; adipic acid; a sulfate such as sodium sulfate; glycol; fumaric acid; sodium benzoate; DL-leucine; a sodium salt of a fatty acid; a lauryl sulfate such as sodium lauryl sulfate and magnesium lauryl sulfate; a silicic acid compound such as silicic acid anhydride and silicic acid hydrate; or a starch derivative described above), a binder (which is for example a hydroxypropyl cellulose, hydroxypropylmethyl cellulose, polyvinylpyrrolidone, macrogol or a compound similar to the excipients described above), a disintegrator (which is for example a cellulose derivative such as low-substituted hydroxypropyl cellulose, carboxymethyl cellulose, calcium carboxymethyl cellulose and internally bridged sodium carboxymethyl cellulose; a chemically modified starch or cellulose such as carboxymethyl starch, sodium carboxymethyl starch and bridged polyvinylpyrrolidone), a stabilizer (which is for example a paraoxybenzoate such as methylparaben and propylparaben; an alcohol such as chlorobutanol, benzyl alcohol and phenylethyl alcohol; benzalkonium chloride; a phenol derivative such as phenol and cresol; thimerosal; dehydroacetic acid; or sorbic acid), a corrigent (which is for example a sweetening, souring or flavoring agent usually used), a diluent and the like.

The dosage differs depending on the condition and age of the patient and the method of administration. It is for example desirable to administer the active ingredient in an amount of 0.001 mg/kg body weight (preferably 0.01 mg/kg body weight) as the lowest dosage to 500 mg/kg body weight (preferably 50 mg/kg body weight) as the highest dosage in an oral administration per unit dose and in an amount of 0.001 mg/kg body weight (preferably 0.01 mg/kg body weight) as the lowest dosage to 500 mg/kg body weight (preferably 50 mg/kg body weight) in an intravenous administration per unit dose, once to several times per day according to the condition.

The following Examples, Reference examples and Test examples are intended to further illustrate the present invention in detail and the scope of the invention is not limited to these examples.

[Example]

In the following examples trimethylsilane (TMS) was used as an internal standard in measurement of NMR spectra provided that there is no special notice.

[Example 1] N-(4-Methylphenyl)-(2-chloro-5-nitrophenyl)carboxamide

2-Chloro-5-nitrobenzoyl chloride (0.754 g) was added to a solution of 4-methylaniline (0.330 g) in dimethylacetamide (DMA, 10 ml) at room temperature and the mixture was stirred for 3 hours. Ethyl acetate (5 ml) and saturated aqueous sodium bicarbonate solution (30 ml) were added to the reaction mixture. The resulting mixture was stirred for 1 hour and extracted with ethyl acetate. The organic layer was washed with aqueous sodium chloride solution, dried over anhydrous sodium sulfate, filtered, and concentrated. Crystallization with isopropyl ether (IPE) followed by filtration afforded the title compound (0.759 g).
$^1$H-NMR (400MHz, $CDCl_3$, TMS): δ(ppm) 2.36 (1H, s), 7.21 (1H, d, J=8.3Hz), 7.26 (1H, s), 7.51 (1H, d, J=8.3Hz), 7.65 (1H, d, J=8.8Hz), 7.77 (1H, s), 8.26 (1H, dd, J=8.8, 2.7Hz), 8.61 (1H, d, J=2.7Hz).

[Example 2] N-(4-Phenylphenyl)-(2-chloro-5-nitrophenyl)carboxamide

2-Chloro-5-nitrobenzoyl chloride (0.294 g) was added to a solution of 4-phenylaniline hydrochloride (0.229 g) in pyridine (3 ml) at room temperature and the mixture was stirred overnight. After ethyl acetate (5 ml) and saturated aqueous sodium bicarbonate solution (30 ml) were added to the reaction mixture, the resulting mixture was stirred for 1 hour. Water (20 ml) was further added and the mixture was stirred. The formed crystals were filtered and dried to

afford the title compound (0.270 g) as a crystalline solid.
$^1$H-NMR (400MHz,$CDCl_3$,TMS): δ(ppm) 7.34-7.81 (1H, m), 7.43-7.48 (2H, m), 7.59-7.74 (7H, m), 7.89 (1H, s), 8.28 (1H, dd, J=8.8, 2.7Hz), 8.65 (1H, d, J=2.7Hz).

[Example 3] N-(4-Methylthiazol-2-yl)-(2-chloro-5-nitrophenyl)carboxamide

A solution of 2-amino-4-methylthiazole (0.380 g), 2-chloro-5-nitrobenzoic acid (0.671 g) and 1,1'-carbonyldiimidazole (CDI, 1.079 g) in tetrahydrofuran (THF, 20 ml) was stirred under reflux for 16 hours. The reaction solution was directly purified by chromatography on a silica gel column [hexane:ethyl acetate = 3:2(v/v)]. Crystallization of the residue with IPE and filtration afforded the title compound (0.155 g) as a crystalline solid.
$^1$H-NMR (400MHz, $CDCl_3$, TMS): δ(ppm) 2.00 (3H, s), 6.59 (1H, s), 7.62 (1H, d, J=8.8Hz), 8.28 (1H, dd, J=8.8, 2.7Hz), 8.58 (1H, d, J=2.7Hz), 11.75 (1H ,bs).

[Example 4] N-(5-Methylthiazol-2-yl)-(2-chloro-5-nitrophenyl)carboxamide

The title compound (0.619 g) was obtained as a crystalline solid according to the procedure described in Example 1 using 2-amino-5-methylthiazole (0.264 g), DMA (5 ml) and 2-chloro-5-nitrobenzoyl chloride (0.560 g).
$^1$H-NMR (400MHz, $CDCl_3$, TMS): δ(ppm) 2.34 (3H, s), 6.32 (1H, s), 7.71 (1H ,d, J=8.8Hz), 8.35 (1H, dd, J=8.8, 2.7Hz), 8.57 (1H, d, J=2.7Hz)

[Example 5] N-(3-Phenylphenyl)-(2-chloro-5-nitrophenyl)carboxamide

The title compound (0.309 g) was obtained as a crystalline solid according to the procedure described in Example 1 using 3-phenylaniline hydrochloride (0.239 g), pyridine (5 ml) and 2-chloro-5-nitrobenzoyl chloride (0.307 g).
$^1$H-NMR (400MHz, $CDCl_3$, TMS): δ(ppm) 7.35-7.40 (1H, m), 7.41-7.52 (4H, m), 7.60-7.67 (4H, m), 7.86 (1H, s), 7.94 (1H, s), 8.26 (1H, dd, J=8.8, 2.7Hz), 8.63 (1H, d, J=2.7Hz).

[Example 6] N-(4-Methoxy-3-phenyl)phenyl-(2-chloro-5-nitrophenyl)carboxamide

The title compound (0.586 g) was obtained as a crystalline solid according to the procedure described in Example 2 using (4-methoxy-3-phenyl)aniline hydrochloride (0.478 g), pyridine (5 ml) and 2-chloro-5-nitrobenzoyl chloride (0.535 g).
$^1$H-NMR (400MHz, $CDCl_3$, TMS): δ(ppm) 3.95 (3H, s), 7.31-7.46 (4H, m), 7.63-7.69 (3H, m), 8.27 (1H, dd, J=8.8, 2.7Hz), 8.67 (1H, d, J=2.7Hz), 8.54 (1H, d, 2.2Hz).

[Example 7] N-(2-Phenylphenyl)-(2-chloro-5-nitrophenyl)carboxamide

The title compound (0.323 g) was obtained as a crystalline solid according to the procedure described in Example 1 using 2-phenylaniline hydrochloride (0.251 g), pyridine (5 ml) and 2-chloro-5-nitrobenzoyl chloride (0.322 g).
$^1$H-NMR (400MHz, $CDCl_3$, TMS): δ(ppm) 7.27-7.34 (2H, m), 7.39-7.54 (7H, m), 7.90 (1H, s), 8.18 (1H, dd, J=8.8, 2.7Hz), 8.45 (1H, d, 3=8.1Hz), 8.53 (1H, d, J=2.7Hz).

[Example 8] N-[4-(2-Pyridyl)phenyl)-(2-chloro-5-nitrophenyl)carboxamide

The title compound (0.388 g) was obtained as a crystalline solid according to the procedure described in Example 1 using 4-(2-pyridyl)aniline hydrochloride (0.320 g), pyridine (5 ml) and 2-chloro-5-nitrobenzoyl chloride (0.347 g).
$^1$H-NMR (400MHz, $CDCl_3$, TMS): δ(ppm) 7.22-7.26 (1H, m), 7.66 (1H, d, J=8.8Hz), 7.72-7.79 (4H, m), 8.05 (1H, d, J=8.8Hz), 8.08 (1H, s), 8.27 (1H, dd, J=8.8, 2.7Hz), 8.63 (1H, d, J=2.7Hz), 8.69 (1H, d, J=4.7Hz).

[Example 9] N-[4-(4-Nitrophenyl)phenyl)-(2-chloro-5-nitrophenyl)carboxamide

The title compound (0.625 g) was obtained as a crystalline solid according to the procedure described in Example 2 using 4-(4-nitrophenyl)aniline (0.344 g), DMA (5 ml) and 2-chloro-5-nitrobenzoyl chloride (0.424 g).
$^1$H-NMR (400MHz, $CDCl_3$, TMS): δ(ppm) 7.84-7.89 (4H, m), 7.92 (1H, d, J=8.8Hz), 7.92 (2H, d, J=8.8Hz), 8.31 (2H, d, J=8.8Hz), 8.36 (1H, dd, J=8.8, 2.7Hz), 8.52 (1H, d, J=2.7Hz), 10.93 (1H, s).

[Example 10] N-[4-(6-Methylbenzothiazol-2-yl)phenyl]-(2-chloro-5-nitrophenyl)carboxamide

The title compound (0.614 g) was obtained as a crystalline solid according to the procedure described in Example 1 using 4-(6-methylbenzothiazol-2-yl)aniline (0.353 g), DMA (5 ml) and 2-chloro-5-nitrobenzoyl chloride (0.349 g).
$^1$H-NMR (400MHz, DMSO-$d_6$, TMS): δ(ppm) 2.47 (3H, s), 7.36 (1H, d, J=8.5Hz), 7.89-7.94 (2H, m), 8.11 (1H, d, J=8.8Hz), 8.37 (1H, dd, J=8.8, 2.8Hz), 8.55 (1H, d, J=2.8Hz), 11.04 (1H, s).

[Example 11] N-(3-Methylphenyl)-(2-chloro-5-nitrophenyl)carboxamide

The title compound (0.677 g) was obtained as a crystalline solid according to the procedure described in Example 2 using m-toluidine (0.269 g), DMA (5 ml) and 2-chloro-5-nitrobenzoyl chloride (0.663 g).
$^1$H-NMR (400MHz, $CDCl_3$, TMS): δ(ppm) 2.40 (3H, s), 7.04 (1H, d, J=7.6Hz), 7.29 (1H, t, J=7.9Hz), 7.41 (1H, d, J=8.1Hz), 7.74-7.84 (1H, m), 8.27 (1H, dd, J=8.8, 2.7Hz), 8.61 (1H, d, J=2.7Hz).

[Example 12] N-(4-Ethylphenyl)-(2-chloro-5-nitrophenyl)carboxamide

The title compound (0.676 g) were obtained as a crystalline solid according to the procedure described in Example 2 using 4-ethylaniline (0.280 g), DMA (5 ml) and 2-chloro-5-nitrobenzoyl chloride (0.610 g).
$^1$H-NMR (400MHz, $CDCl_3$, TMS): δ(ppm) 1.25 (3H, t, J=7.6Hz), 2.66 (2H, q, J=7.6Hz), 7.24 (1H, d, J=8.4Hz), 7.54 (1H, d, J=8.4Hz), 7.66 (1H, d, J=8.8Hz), 8.26 (1H, dd, J=8.8, 2.7Hz), 8.61 (1H, d, J=2.7Hz).

[Example 13] N-(2-Ethylphenyl)-(2-chloro-5-nitrophenyl)carboxamide

The title compound (0.667 g) was obtained as a crystalline solid according to the procedure described in Example 2 using 2-ethylaniline (0.269 g), DMA (5 ml) and 2-chloro-5-nitrobenzoyl chloride (0.586 g).
$^1$H-NMR (400MHz, $CDCl_3$, TMS): δ(ppm) 1.28 (3H, t, J=7.6Hz), 2.69 (2H, q, J=7.6Hz), 7.21-7.33 (3H, m), 7.68 (1H, d, J=8.8Hz), 7.80 (1H, s), 8.28 (1H, dd, J=8.8, 2.7Hz), 8.67 (1H, d, J=2.7Hz).

[Example 14] N-(3-Ethylphenyl)-(2-chloro-5-nitrophenyl)carboxamide

Title compound (0.359 g) was obtained as a crystalline solid according to the procedure described in Example 2 using 3-ethylaniline (0.210 g), DMA (5 ml) and 2-chloro-5-nitrobenzoyl chloride (0.458 g).
$^1$H-NMR (400MHz, DMSO-$d_6$, TMS): δ(ppm) 1.19 (3H, t, J=7.6Hz), 2.61 (2H, q, J=7.6Hz), 7.00 (1H, d, J=7.8Hz), 7.28 (1H, t, J=7.8Hz), 7.51 (1H, d, J=7.8Hz), 7.58 (1H, s), 7.89 (1H, d, J=8.8Hz), 8.34 (1H, dd, J=8.8, 2.7Hz), 8.45 (1H, d, J=2.7Hz).

[Example 15] N-(4-Propylphenyl)-(2-chloro-5-nitrophenyl)carboxamide

The title compound (0.677 g) was obtained as a crystalline solid according to the procedure described in Example 2 using 4-propylaniline (0.340 g), DMA (5 ml) and 2-chloro-5-nitrobenzoyl chloride (0.664 g).
$^1$H-NMR (400MHz, DMSO-$d_6$, TMS): δ(ppm) 0.89 (3H, t, J=7.3Hz), 1.58 (2H, m), 2.54 (2H, t, J=7.6Hz), 7.19 (2H, d, J=8.4Hz), 7.60 (2H, d, J=8.4Hz), 7.89 (1H, d, J=8.8Hz), 8.33 (1H, dd, J=8.8, 2.7Hz), 8.44 (1H, d, J=2.7Hz), 10.62 (1H, s).

[Example 16] N-(4-Pentylphenyl)-(2-chloro-5-nitrophenyl)carboxamide

The title compound (0.514 g) was obtained as a crystalline solid according to the procedure described in Example 2 using 4-pentylaniline (0.300 g), DMA (5 ml) and 2-chloro-5-nitrobenzoyl chloride (0.485 g).
$^1$H-NMR (400MHz, $CDCl_3$, TMS): δ(ppm) 0.90 (3H, t, J=6.9Hz), 1.28-1.38 (4H, m), 1.58-1.66 (2H, m), 2.61 (2H, t, J=7.7Hz), 7.21 (2H, d, J=8.4Hz), 7.53 (2H, d, J=8.5Hz), 7.66 (1H, d, J=8.8Hz), 7.77 (1H, s), 8.26 (1H, dd, J=8.8, 2.8Hz), 8.61 (1H, d, J=2.8Hz).

[Example 17] N-(4-Butyloxyphenyl)-(2-chloro-5-nitrophenyl)carboxamide

The title compound (0.513 g) was obtained as a crystalline solid according to the procedure described in Example 2 using 4-butyloxyaniline (0.267 g), DMA (5 ml) and 2-chloro-5-nitrobenzoyl chloride (0.427 g).
$^1$H-NMR (400MHz, DMSO-$d_6$, TMS): δ(ppm) 0.94 (3H, t, J=7.4Hz), 1.39-1.48 (2H, m), 1.65-1.73 (2H, m), 3.96 (2H, t, J=6.5Hz), 6.94 (2H, d, J=9.0Hz), 7.60 (2H, d, J=9.0Hz), 7.88 (1H, d, J=8.8Hz), 8.33 (1H, dd, J=8.8, 2.8Hz), 8.43 (1H,

d, J=2.8Hz), 10.54 (1H, s).

[Example 18] N-(4-Trifluoromethyloxyphenyl)-(2-chloro-5-nitrophenyl)carboxamide

The title compound (0.541 g) was obtained as a crystalline solid according to the procedure described in Example 2 using 4-trifluoromethyloxyaniline (0.310 g), DMA (5 ml) and 2-chloro-5-nitrobenzoyl chloride (0.462 g).
$^1$H-NMR (400MHz, DMSO-$d_6$, TMS): δ(ppm) 7.40 (2H, d, J=8.5Hz), 7.81 (2H, d, J=8.5Hz), 7.90 (1H, d, J=8.8Hz), 8.35 (1H, dd, J=8.8, 2.7Hz), 8.50 (1H, d, J=2.7Hz).

[Example 19] N-(2-Isopropyl-6-methylphenyl)-(2-chloro-5-nitrophenyl)carboxamide

After treatment according to the procedure described in Example 2 using 2-isopropyl-6-methylaniline (0.309 g), DMA (5 ml) and 2-chloro-5-nitrobenzoyl chloride (0.546 g), the reaction product was purified by chromatography on a silica gel column [hexane:ethyl acetate = 2:1 (v/v)], solidified with IPE and filtered to afford the title compound (0.630 g) as a crystalline solid.
$^1$H-NMR (400MHz, DMSO-$d_6$, TMS): δ(ppm) 1.26 (6H, d, J=6.9Hz), 3.23 (1H, sept, J=6.9Hz), 7.17 (1H, m), 7.23-7.31 (2H, m), 7.45 (1H, bs), 7.69 (1H, d, J=8.8Hz), 8.28 (1H, dd, J=8.8, 2.8Hz), 8.64 (1H, d, J=2.8Hz).

[Example 20] N-(4-Cyanophenyl)-(2-chloro-5-nitrophenyl)carboxamide

The title compound (0.558 g) was obtained as a crystalline solid according to the procedure described in Example 2 using 4-cyanoaniline (0.251 g), DMA (5 ml) and 2-chloro-5-nitrobenzoyl chloride (0.559 g).
$^1$H-NMR (400MHz, $CDCl_3$, TMS): δ(ppm) 7.68-7.79 (3H, m), 7.81 (2H, d, J=8.6Hz), 8.09 (1H, s), 8.31 (1H, dd, J=8.8, 2.7Hz), 8.63 (1H, d, J=2.7Hz).

[Example 21] N-(3-Cyanophenyl)-(2-chloro-5-nitrophenyl)carboxamide

The title compound (0.616 g) was obtained as a crystalline solid according to the procedure described in Example 2 using 3-cyanoaniline (0.255 g), DMA (5 ml) and 2-chloro-5-nitrobenzoyl chloride (0.559 g).
$^1$H-NMR (400MHz, $CDCl_3$, TMS): δ(ppm) 7.50-7.56 (2H, m), 7.70 (1H, d, J=8.8Hz), 7.84-7.87 (1H, m), 8.02 (1H, s), 8.07 (1H, s), 8.31 (1H, dd, J=8.8, 2.7Hz), 8.64 (1H, d, J=2.7Hz).

[Example 22] N-(2-Cyanophenyl)-(2-chloro-5-nitrophenyl)carboxamide

The title compound (0.682 g) was obtained as a crystalline solid according to the procedure described in Example 2 using 2-cyanoaniline (0.279 g), DMA (5 ml) and 2-chloro-5-nitrobenzoyl chloride (0.623 g).
$^1$H-NMR (400MHz, $CDCl_3$, TMS): δ(ppm) 7.30 (1H, t, J=7.7Hz), 7.66-7.72 (3H, m), 8.33 (1H, dd, J=8.8, 2.7Hz), 8.50 (1H, s), 8.56 (1H, d, J=8.4Hz), 8.71 (1H, d, J=2.7Hz).

[Example 23] N-(4-Nitrophenyl)-(2-chloro-5-nitrophenyl)carboxamide

The title compound (0.619 g) was obtained as a crystalline solid according to the procedure described in Example 2 using 4-nitroaniline (0.285 g), DMA (5 ml) and 2-chloro-5-nitrobenzoyl chloride (0.545 g).
$^1$H-NMR (400MHz, DMSO-$d_6$, TMS): δ(ppm) 7.93 (1H, d, J=8.9Hz), 7.96 (2H, d, J=9.2Hz), 8.31 (2H, d, J=9.2Hz), 8.38 (1H, dd, J=8.9, 2.7Hz), 8.59 (1H, d, J=2.7Hz), 11.32 (1H, s).

[Example 24] N-(5-Nitropyridin-2-yl)-(2-chloro-5-nitrophenyl)carboxamide

The title compound (0.627 g) was obtained as a crystalline solid according to the procedure described in Example 1 using 2-amino-4-nitropyridine (0.293 g), DMA (5 ml) and 2-chloro-5-nitrobenzoyl chloride (0.556 g).
$^1$H-NMR (400MHz, DMSO-$d_6$, TMS): δ(ppm) 7.89 (1H, d, J=8.8Hz), 8.36 (1H, dd, J=8.8, 2.8Hz), 8.42 (1H, d, J=9.2Hz), 8.58 (1H, d, J=2.8Hz), 8.72 (1H, dd, J=9.2, 2.5Hz), 9.23 (1H, d, J=2.5Hz), 12.00 (1H, s).

[Example 25] N-(3-Nitrophenyl)-(2-chloro-5-nitrophenyl)carboxamide

The title compound (0.638 g) was obtained as a crystalline solid according to the procedure described in Example 2 using 3-nitroaniline (0.274 g), DMA (5 ml) and 2-chloro-5-nitrobenzoyl chloride (0.524 g).
$^1$H-NMR (400MHz, DMSO-$d_6$, TMS): δ(ppm) 7.70 (1H, t, J=8.2Hz), 7.93 (1H, d, J=8.9Hz), 8.00-8.04 (2H, m), 8.38 (1H,

dd, J=8.9, 2.7Hz), 8.58 (1H, d, J=2.7Hz), 8.74 (1H, bs), 11.20 (1H, s).

[Example 26] N-(4-Ethoxycarbonylphenyl)-(2-chloro-5-nitrophenyl)carboxamide

The title compound (20.33 g) was obtained as a crystalline solid according to the procedure described in Example 2 using ethyl 4-aminobenzoate (9.88 g), DMA (50 ml) and 2-chloro-5-nitrobenzoyl chloride (14.47 g).
$^1$H-NMR (400MHz, DMSO-$d_6$, TMS): δ(ppm) 1.33 (1H, t, J=7.1Hz), 4.31 (2H, q, J=7.1Hz), 7.85 (2H, d, J=8.7Hz), 7.91 (1H, d, J=8.8Hz), 7.99 (2H, d, 8.7Hz), 8.36 (1H, dd, J=8.9, 2.8Hz), 8.53 (1H, d, J=2.8Hz), 11.05 (1H, s).

[Example 27] 4-[(2-Chloro-5-nitrophenyl)carbonylamino]benzoic acid

Aqueous sodium hydroxide solution (1N, 84 ml) was added to a solution of N-(4-ethoxycarbonylphenyl) -(2-chloro-5-nitrophenyl)carboxamide (19.55 g) obtained in Example 26 in dioxane (100 ml) and the mixture was allowed to stand at room temperature for 3 days. The reaction solution was concentrated under reduced pressure. Water (300 ml) was added to the resulting residue and hydrochloric acid (1N, 90 ml) was added dropwise to the mixture under cooling with an ice-water bath and stirred. The formed crystals were filtered and dried to afford the title compound (17.59 g).
$^1$H-NMR (400MHz, DMSO-$d_6$, TMS): δ(ppm) 7.82 (2H, d, J=8.7Hz), 7.91 (1H, d, J=8.8Hz), 7.97 (2H, d, 8.7Hz), 8.36 (1H, dd, J=8.8, 2.8Hz), 8.52 (1H, d, J=2.8Hz), 11.01 (1H, s).

[Example 28] N-(2-Chloro-5-nitrophenyl)-(2-chloro-5-nitrophenyl)carboxamide

The title compound (0.708 g) was obtained as a crystalline solid according to the procedure described in Example 2 using 2-chloro-5-nitroaniline (0.348 g), DMA (5 ml) and 2-chloro-5-nitrobenzoyl chloride (0.532 g).
$^1$H-NMR (400MHz, DMSO-$d_6$, TMS): δ(ppm) 7.89 (1H, d, J=8.8Hz), 7.91 (1H, d, J=8.8Hz), 8.15 (1H, dd, J=8.8, 2.7Hz), 8.37 (1H, dd, J=8.8, 2.7Hz), 8.65 (1H, d, J=2.7Hz), 8.88 (1H, d, J=2.7Hz), 10.83 (1H, s).

[Example 29] N-(3,5-Di-t-butyl-4-hydroxyphenyl)-(2-chloro-5-nitrophenyl)carboxamide

The title compound (0.645 g) was obtained as a crystalline solid according to the procedure described in Example 1 using 3,5-di-t-butyl-4-hydroxyaniline (0.500 g), THF (10 ml) and 2-chloro-5-nitrobenzoyl chloride (0.538 g).
$^1$H-NMR (400MHz, $CDCl_3$, TMS): δ(ppm) 1.47 (6H, s), 5.20 (1H, s), 7.43 (2H, s), 7.66 (1H, d, J=8.8Hz), 8.26 (1H, dd, J=8.8,2.8Hz), 8.62 (1H, d, J=2.7Hz).

[Example 30] N-(3-Benzensulfonylaminophenyl)-(2-chloro-5-nitrophenyl)carboxamide

After treatment according to the procedure described in Example 1 using 3-benzenesulfonylaminoaniline (0.595 g), DMA (5 ml) and 2-chloro-5-nitrobenzoyl chloride (0.633 g), the reaction product was purified by chromatography on a silica gel column [hexane:ethyl acetate = 1:1 (v/v)], solidified with IPE and filtered to afford the title compound (0.700 g) as a crystalline solid.
$^1$H-NMR (400MHz, $CDCl_3$, TMS): δ(ppm) 6.87 (1H, d, J=8.1Hz), 7.21 (1H, t, J=8.1Hz), 7.38 (1H, d, J=8.1Hz), 7.54-7.66 (4H, m), 7.80-7.82 (2H, m), 7.87 (1H, d, J=8.8Hz), 8.33 (1H, dd, J=8.8, 2.8Hz), 8.44 (1H, d, J=2.8Hz), 10.38 (1H, s), 10.69 (1H, s).

[Example 31] N-[[(3-(Pyrrolidin-1-yl)carbonyl]phenyl]-(2-chloro-5-nitrophenyl)carboxamide

The title compound (0.319 g) was obtained as a crystalline solid according to the procedure described in Example 1 using [3-(pyrrolidin-1-yl)carbonyl]aniline hydrochloride (0.244 g), pyridine (5 ml) and 2-chloro-5-nitrobenzoyl chloride (0.300 g).
$^1$H-NMR (400MHz, $CDCl_3$, TMS): δ(ppm) 1.85-1.93 (4H, m), 3.36-3.45 (4H, m), 7.27 (1H, d, J=8.1Hz), 7.41 (1H, t, J=8.1Hz), 7.65 (1H, d, J=8.8Hz), 7.70 (1H, s), 7.91 (1H, d, J=8.1Hz), 8.26 (1H, dd,J=8.8, 2.7Hz), 8.50 (1H, d, J=2.7Hz), 9.09 (1H, s).

[Example 32] N-[4-(4-Methylbenzene)sulfonylaminophenyl]-(2-chloro-5-nitrophenyl)carboxamide

The title compound (0.516 g) was obtained as a crystalline solid according to the procedure described in Example 2 using 4-(p-toluenesulfonylamino)aniline (0.308 g), DMA (5 ml) and 2-chloro-5-nitrobenzoyl chloride (0.310 g).

$^1$H-NMR (400MHz, DMSO-$d_6$, TMS): δ(ppm) 2.34 (1H, s), 7.08 (2H, d, J=8.9Hz), 7.35 (2H, d, J=8.2Hz), 7.54 (2H, d, J=8.9Hz), 7.63 (2H, d, J=8.2Hz), 7.87 (1H, d, J=8.8Hz), 8.32 (1H, dd, J=8.8, 2.8Hz), 8.42 (1H, d, J=2.8Hz), 10.15 (1H, s), 10.64 (1H, s).

[Example 33] N-(Benzothiazol-2-yl)-(2-chloro-5-nitrophenyl)carboxamide

The title compound (0.544 g) was obtained as a crystalline solid according to the procedure described in Example 2 using 2-aminobenzothiazole (0.312 g), DMA (5 ml) and 2-chloro-5-nitrobenzoyl chloride (0.503 g).
$^1$H-NMR (400MHz, DMSO-$d_6$, TMS): δ(ppm) 7.58 (1H, t, J=7.1Hz), 7.67 (2H, d, J=7.2Hz), 7.75-7.79 (2H, m), 8.08 (1H, d, J=7.9Hz), 8.19-8.28 (2H, m), 8.77 (1H, d, J=2.8Hz).

[Example 34] N-(6-Nitrobenzothiazol-2-yl)-(2-chloro-5-nitrophenyl)carboxamide

The title compound (0.755 g) was obtained as a crystalline solid according to the procedure described in Example 2 using 2-amino-6-nitrobenzothiazole (0.421 g), DMA (5 ml) and 2-chloro-5-nitrobenzoyl chloride (0.569 g).
$^1$H-NMR (400MHz, DMSO-$d_6$, TMS): δ(ppm) 7.95 (2H, t, J=8.9Hz), 8.32 (2H, dd, J=8.9, 2.4Hz), 8.41 (1H, dd, J=8.9, 2.8Hz), 8.70 (1H, d, J=2.8Hz), 9.13 (1H, d, J=2.4Hz).

[Example 35] N-(4-Phenylthiazol-2-yl)-(2-chloro-5-nitrophenyl)carboxamide

After treatment according to a similar procedure to that described in Example 1 using 2-amino-4-phenylthiazole hydrobromide (2.53 g), pyridine (20 ml), and 2-chloro-5-nitrobenzoyl chloride (2.12 g), the reaction product was purified by chromatography on a silica gel column [hexane: ethyl acetate= 3:2 (v/v)], solidified with IPE and filtered to afford the title compound (0.240 g).
$^1$H-NMR (400MHz, $CDCl_3$, TMS): δ(ppm) 7.14-7.24 (5H, m), 7.43-7.45 (2H, m), 7.88 (1H, dd, J=8.8, 2.7Hz), 8.03 (1H, d, J=2.7Hz).

[Example 36] N-[4-(2-Thienyl)thiazol-2-yl)-(2-chloro-5-nitrophenyl)carboxamide

After treatment according to a similar procedure to that described in Example 1 using 2-amino-4-(2-thienyl) thiazole (1.03 g), THF (20 ml), CDI (1.33 g) and 2-chloro-5-nitrobenzic acid (1.10 g), the reaction product was purified by chromatography on a silica gel column [hexane:ethyl acetate= 1:1 (v/v)], solidified with IPE and filtered to afford the title compound (0.687 g).
$^1$H-NMR (400MHz, DMSO-$d_6$, TMS): δ(ppm) 7.12 (1H, d, J=5.1, 3.6Hz), 7.52 (1H, d, J=5.1Hz), 7.56 (1H, d, J=3.6Hz), 7.90 (1H, d, J=8.8Hz), 8.37 (1H, dd, J=8.8, 2.7Hz), 8.60 (1H, d, J=2.7Hz).

[Example 37] N-[4-(3-Pyridyl)thiazol-2-yl)-(2-chloro-5-nitrophenyl)carboxamide

The title compound (0.308 g) was obtained according to the procedure described in Example 1 using 2-amino-4-(3-pyridyl)thiazole (0.181 g) prepared by the procedure described in Reference example 2, DMA (5 ml) and 2-chloro-5-nitrobenzoyl chloride (0.247 g).
$^1$H-NMR (400MHz, DMSO-$d_6$, TMS): δ(ppm) 7.49 (1H, dd, J=8.0, 4.8Hz), 7.92 (1H, d, J=8.9Hz), 7.97 (1H, s), 8.27 (1H, dt, J=8.0, 2.0Hz), 8.38 (1H, dd, J=8.9, 1.6Hz), 8.61 (1H, d, J=2.8Hz), 9.15 (1H, d, J=1.6Hz).

[Example 38] N-[4-(2-Pyridyl)thiazol-2-yl)-(2-chloro-5-nitrophenyl)carboxamide

The title compound (0.491 g) was obtained according to the procedure described in Example 2 using 2-amino-4-(2-pyridyl)thiazole (0.324 g), DMA (5 ml) and 2-chloro-5-nitrobenzoyl chloride (0.442 g).
$^1$H-NMR (400MHz, DMSO-$d_6$, TMS): δ(ppm) 7.34-7.37 (1H, m), 7.87-7.97 (4H, m), 8.38 (1H, dt, J=8.9, 2.7Hz), 8.61-8.63 (2H, m).

[Example 39] N-[4-(2-Methylphenyl)thiazol-2-yl]-(2-chloro-5-nitrophenyl)carboxamide

After treatment according to the procedure described in Example 1 using 2-amino-4-(2-methylphenyl)thiazole (0.398 g), DMA (5 ml) and 2-chloro-5-nitrobenzoyl chloride (0.385 g), the reaction product was purified by chromatography on a silica gel column [hexane: ethyl acetate= 1:1 (v/v)] to afford the title compound (0.452 g).
$^1$H-NMR (400MHz, DMSO-$d_6$, TMS): δ(ppm) 2.45 (1H, s), 7.23-7.31 (3H, m), 7.42 (1H, s), 7.57-7.60 (1H, m), 7.91 (1H, dJ=8.9Hz), 8.37 (1H, dd, J=8.9, 2.8Hz), 8.59 (1H, d, J=2.8Hz).

[Example 40] N-[4-(3-Methylphenyl)thiazol-2-yl)-(2-chloro-5-nitrophenyl)carboxamide

After treatment according to a similar procedure to that described in Example 3 using 2-amino-4-(3-methylphenyl)thiazole (0.303 g), 2-chloro-5-nitrobenzoic acid (0.247 g), CDI (0.640 g) and THF (10 ml), the reaction product was purified by chromatography on a silica gel column [hexane:ethyl acetate= 1:1 (v/v)] to afford the title compound (0.105 g).
$^1$H-NMR (400MHz, $CDCl_3$,TMS): δ(ppm) 2.32 (3H, s), 6.98 (1H, d, J=7.6Hz), 7.12 (1H, t, J=7.7Hz), 7.14 (1H, s), 7.25-7.27 (2H, m), 7.30 (1H, d, J=7.7Hz), 7.93 (1H, dd, J=8.9, 2.7Hz), 8.12 (1H, d, J=2.7Hz).

[Example 41] N-[4-(1-Naphthyl)thiazol-2-yl]-(2-chloro-5-nitrophenyl)carboxamide

The title compound (0.580 g) was obtained according to the procedure described in Example 1 using 2-amino-4-(1-naphthyl)thiazole (0.378 g), DMA (5 ml) and 2-chloro-5-nitrobenzoyl chloride (0.404 g).
$^1$H-NMR (400MHz, $CDCl_3$, TMS): δ(ppm) 7.02 (1H, d, J=8.8Hz), 7.33-7.54 (5H, m), 7.69 (1H, d, J=8.2Hz), 7.81 (1H, d, J=8.1Hz), 7.84 (1H, d, J=2.7Hz), 8.16 (1H, d, J=8.6Hz).

[Example 42] N-[4-(2-Naphthyl)thiazol-2-yl)-(2-chloro-5-nitrophenyl)carboxamide

The title compound (0.566 g) was obtained according to the procedure described in Example 2 using 2-amino-4-(2-naphthyl)thiazole (0.398 g), DMA (5 ml) and 2-chloro-5-nitrobenzoyl chloride (0.464 g).
$^1$H-NMR (400MHz, DMSO-$d_6$, TMS): δ(ppm) 7.49-7.56 (2H, m), 7.87-7.99 (5H, m), 8.09 (1H, dd, J=8.6, 1.6Hz), 8.36 (1H, dd, J=8.8, 2.7Hz), 8.47 (1H, s), 8.62 (1H, d, J=2.7Hz).

[Example 43] N-[4-(3,4-Dichlorophenyl)thiazol-2-yl]-(2-chloro-5-nitrophenyl)carboxamide

The title compound (0.449 g) was obtained according to the procedure described in Example 2 using 2-amino-4-(3,4-dichlorophenyl)thiazole (0.313 g), DMA (5 ml) and 2-chloro-5-nitrobenzoyl chloride (0.337 g).
$^1$H-NMR (400MHz, DMSO-$d_6$, TMS): δ(ppm) 7.70 (1H, d, J=8.4Hz), 7.86-7.93 (3H, m), 8.17 (1H, d, J=2.0Hz), 8.34 (1H, dd, J=8.8, 2.8Hz), 8.60 (1H, d, J=2.8Hz).

[Example 44] N-[4-(4-Ethylphenyl)thiazol-2-yl]-(2-chloro-5-nitrophenyl)carboxamide

The title compound (0.393 g) was obtained according to the procedure described in Example 1 using 2-amino-4-(4-ethylphenyl)thiazole (0.339 g), DMA (5 ml) and 2-chloro-5-nitrobenzoyl chloride (0.438 g).
$^1$H-NMR (400MHz, DMSO-$d_6$, TMS): δ(ppm) 1.21 (3H, t, J=7.5Hz), 2.63 (2H, q, J=7.5Hz), 7.28 (1H, d, J=8.2Hz), 7.71 (1H, s), 7.84 (1H, d, J=8.2Hz), 7.91 (1H, d, J=8.9Hz), 8.38 (1H, dd, =8.9, 2.7Hz), 8.61 (1H, d, J=2.7Hz).

[Example 45] N-[4-(tert-Butyloxycarbonylaminophenyl)]-(2-chloro-5-nitrophenyl)carboxamide

The title compound (6.96 g, 83%) was obtained according to the procedure described in Example 2 using (4-tert-butyloxycarbonylaminophenyl)amine (4.47 g, 21.5 mmol), DMA (50 ml) and 2-chloro-5-nitrobenzoyl chloride (5.19 g, 23.6 mmol).
$^1$H NMR (DMSO, 400MHz): δ 1.48 (9H, s), 7.44 (2H, d, J=8.9Hz), 7.58 (2H, d, J=8.9Hz), 7.88 (1H,d, J=8.8Hz), 7.33 (1H, dd, J=2.8, 8.8Hz), 8.43 (1H, d, J=2.8Hz), 9.34 (1H, s), 10.58 (1H, s);
MS(FAB) m/z: 391 $M^+$;

[Example 46] N-[4-(3-Chloro-4-methylphenyl)-5-methylthiazol-2-yl]-(2-chloro-5-nitrophenyl)carboxamide

The title compound (0.510 g) was obtained according to the procedure described in Example 1 using 2-amino-4-(3-chloro-4-methylphenyl)-5-methylthiazole (0.368 g), DMA (5 ml) and 2-chloro-5-nitrobenzoyl chloride (0.407 g).
$^1$H-NMR (400MHz, DMSO-$d_6$, TMS): δ(ppm) 2.39 (3H, s), 2.52 (3H, s), 7.47-7.52 (2H, m), 7.64-7.69 (1H ,m), 7.89 (1H, d, J=8.8Hz), 8.36 (1H, dd, J=8.8, 2.8Hz), 8.56 (1H, d, J=2.8Hz).

[Example 47] N-[(4,5-Dimethyl)thiazol-2-yl]-(2-chloro-5-nitrophenyl)carboxamide

The title compound (0.281 g) was obtained according to the procedure described in Example 2 using 2-amino-4,5-dimethylthiazole hydrochloride (0.182 g), pyridine (5 ml) and 2-chloro-5-nitrobenzoyl chloride (0.304 g).
$^1$H-NMR (400MHz, DMSO-$d_6$, TMS): δ(ppm) 2.08 (3H, s), 2.31 (3H, s), 7.62 (1H, d, J=8.8Hz), 8.27 (1H, dd, J=8.8,

2.7Hz), 8.63 (1H, d, J=2.7Hz).

[Example 48] N-(5-Bromothiazol-2-yl)-(2-chloro-5-nitrophenyl)carboxamide

The title compound (0.428 g) was obtained according to the procedure described in Example 1 using 2-amino-5-bromothiazole hydrobromide (0.677 g), pyridine (5 ml) and 2-chloro-5-nitrobenzoyl chloride (0.680 g).
$^1$H-NMR (400MHz, $CDCl_3$, TMS): δ(ppm) 7.00 (1H, s), 7.74 (1H, d, J=8.8Hz), 8.38 (1H, dd, J=8.8, 2.7Hz), 8.68 (1H, d, J=2.7Hz).

[Example 49] N-(4-Pyridyl)-(2-chloro-5-nitrophenyl)carboxamide

The title compound (0.788 g) was obtained according to the procedure described in Example 1 using 4-aminopyridine (0.421 g), DMA (10 ml) and 2-chloro-5-nitrobenzoyl chloride (1.08 g).
$^1$H-NMR (400MHz, DMSO-$d_6$, TMS): δ(ppm) 6.27 (2H, d, J=6.2Hz), 7.92 (1H, d, J=8.8Hz), 8.37 (1H, dd, J=8.8, 2.7Hz), 8.52 (2H, d, J=6.2Hz), 8.55 (1H, d, J=2.7Hz), 11.10 (1H, s).

[Example 50] N-(3-Pyridyl)-(2-chloro-5-nitrophenyl)carboxamide

The title compound (0.820 g) was obtained according to the procedure described in Example 1 using 3-aminopyridine (0.321 g), DMA (5 ml) and 2-chloro-5-nitrobenzoyl chloride (0.825 g).
$^1$H-NMR (400MHz, $CDCl_3$, TMS): δ(ppm) 7.38 (1H, dd, J=4.8, 8.3Hz), 7.69 (1H, d, J=8.8Hz), 8.19 (1H, s), 8.28-8.31 (2H, m), 8.44 (1H, dd, 4.8, 1.4Hz), 8.63 (1H, d, J=2.7Hz), 8.68 (1H, d, J=2.5Hz).

[Example 51] N-(6-Methyl-pyridin-2-yl)-(2-chloro-5-nitrophenyl)carboxamide

The title compound (0.632 g) was obtained according to the procedure described in Example 1 using 2-amino-6-methylpyridine (0.314 g), DMA (5 ml) and 2-chloro-5-nitrobenzoyl chloride (0.703 g).
$^1$H-NMR (400MHz, DMSO-$d_6$, TMS): δ(ppm) 2.43 (3H, s), 7.07 (1H, d, J=7.8Hz), 7.76 (1H, d, J=7.8Hz), 8.01 (1H, d, J=7.8Hz), 8.31(1H, dd, J=8.8, 2.7Hz), 8.42 (1H, d, J=2.7Hz), 11.21 (1H, s).

[Example 52] N-(5-Methyl-pyridin-2-yl)-(2-chloro-5-nitrophenyl)carboxamide

After treatment according to a similar procedure to that described in Example 1 using 2-amino-5-methylpyridine (0.367 g), DMA (5 ml) and 2-chloro-5-nitrobenzoyl chloride (0.896 g), the reaction product was purified by chromatography on a silica gel column [hexane:ethyl acetate= 2:1 (v/v)] to afford 0.506 g.
$^1$H-NMR (400MHz, $CDCl_3$, TMS): δ(ppm) 2.32 (3H, s), 7.61 (1H, dd, J=8.5, 1.9Hz), 7.66 (1H, d, J=8.8Hz), 8.05 (1H, bs), 8.24 (1H, d, J=8.5Hz), 8.28 (1H, dd, J=8.8, 2.7Hz), 8.61 (1H, d, J=2.7Hz), 8.76 (1H, s).

[Example 53] N-[4-(4-Dimethylaminophenyl)phenyl]-(2-chloro-5-nitrophenyl)carboxamide

The title compound (0.389 g) was obtained as a crystal according to the procedure described in Example 1 using 4-(4-dimethylaminophenyl)aniline (0.244 g), DMA (5 ml) and 2-chloro-5-nitrobenzoyl chloride (0.279 g).
$^1$H-NMR (400MHz, DMSO-$d_6$, TMS): δ(ppm) 2.94 (6H, s), 6.80 (2H, d, J=8.9Hz), 7.52 (2H, d, J=8.9Hz), 7.61 (2H, d, J=8.7Hz), 7.73 (2H, d, J=8.7Hz), 7.90 (1H, d, J=8.8), 8.35 (1H, dd, J=8.8, 2.7Hz), 8.47 (1H, d, J=2.7Hz), 10.72 (1H, s).

[Example 54] N-(Acenaphthen-5-yl)-(2-chloro-5-nitrophenyl)carboxamide

The title compound (0.335 g) was obtained by a similar procedure to that described in Example 2 using 5-aminoacenaphthene (0.326 g), DMA (5 ml) and 2-chloro-5-nitrobenzoyl chloride (0.267 g).
$^1$H-NMR (400MHz, DMSO-$d_6$, TMS): δ(ppm) 3.35-3.42 (4H, m), 7.34-7.37 (2H, m), 7.49-7.54 (1H, m), 7.78-7.83 (2H, m), 7.92 (1H, d, J=8.8Hz), 8.36 (1H, dd, J=8.8, 2.7Hz), 8.56 (1H, d, J=2.7Hz), 10.63 (1H, s).

[Example 55] N-(3-Quinolinyl)-(2-chloro-5-nitrophenyl)carboxamide

The title compound (0.555 g) was obtained by a similar procedure to that described in Example 2 using 3-aminoquinoline (0.377 g), DMA (5 ml) and 2-chloro-5-nitrobenzoyl chloride (0.633 g).
$^1$H-NMR (400MHz, DMSO-$d_6$, TMS): δ(ppm) 7.62 (1H, d, J=8.0Hz), 7.71 (1H, dd, 6.8, 8.3Hz), 7.94 (1H, d, J=8.8Hz), 8.02 (2H, dd, J=8.8, 8.0Hz), 8.39 (1H, dd, J=8.8, 2.7Hz), 8.61 (1H, d, J=2.7Hz), 8.86 (1H, d, J=2.4Hz), 9.00 (1H, d,

J=2.4Hz), 11.22 (1H, s).

[Example 56] N-(5-Quinolinyl)-(2-chloro-5-nitrophenyl)carboxamide

The title compound (0.704 g) was obtained by a similar procedure to that described in Example 2 using 5-aminoquinoline (0.326 g), DMA (5 ml) and 2-chloro-5-nitrobenzoyl chloride (0.547 g).
$^1$H-NMR (400MHz, DMSO-$d_6$, TMS): δ(ppm) 7.62 (1H, dd, J=8.6, 4.1Hz), 7.81-7.99 (3H, m), 8.38 (1H, dd ,J=8.8, 2.7Hz), 8.59 (1H, d, J=8.6Hz), 8.67 (1H, d, J=2.6Hz), 8.96 (2H, d, J=4.1Hz), 10.88 (1H, s).

[Example 57] N-(8-Quinolinyl)-(2-chloro-5-nitrophenyl)carboxamide

The title compound (0.634 g) was obtained by a similar procedure to that described in Example 2 using 8-aminoquinoline (0.326 g), DMA (5 ml) and 2-chloro-5-nitrobenzoyl chloride (0.525 g).
$^1$H-NMR (400MHz, DMSO-$d_6$, TMS): δ(ppm) 7.64-7.71 (2H, m), 7.81(1H, d, J=7.1Hz), 7.91 (1H, d, J=8.8Hz), 8.86 (1H, dd, J=8.8, 2.7Hz), 8.46 (1H, d, J=8.3Hz), 8.55 (1H, d, J=2.7Hz), 8.74 (1H, d, J=7.5Hz), 8.93 (1H, d, 4.2Hz), 10.85 (1H,s).

[Example 58] N-(4-Aminophenyl)-(2-chloro-5-nitrophenyl)carboxamide mono-hydrochloride

The title compound (4.62 g, yield 99%) was obtained according to the procedure described in Example 8 using N-[4-(tert-butyloxycarbonylaminophenyl)]-(2-chloro-5-nitrophenyl)carboxamide (5.60 g, 14.3 mmol) prepared in Example 45, dioxane (65 ml) and 4N-hydrogen chloride/dioxane solution (10 ml).
$^1$H NMR (DMSO, 400MHz): δ 7.34 (2H, d, J=8.8Hz), 7.78 (2H, d, J=8.8Hz), 7.99 (1H, d, J=8.8Hz), 8.35 (1H, dd, J=2.8, 8.8Hz), 8.48 (1H, d, J=2.7Hz), 9.91 (1H ,s), 10.89 (1H, s);

[Example 59] N-(Isoquinolin-1-yl)-(2-chloro-5-nitrophenyl)carboxamide

The title compound (0.599 g) was obtained by a similar procedure to that described in Example 2 using 1 -aminoisoquinoline (0.394 g), DMA (5 ml) and 2-chloro-5-nitrobenzoyl chloride (0.752 g).
$^1$H-NMR (400MHz, DMSO-$d_6$, TMS): δ(ppm) 7.80 (1H, d, J=8.8Hz), 7.89-7.97 (2H, m), 8.03-8.06 (1H, m), 8.22 (1H, dd, J=8.8, 2.7Hz), 8.37 (1H, d, J=5.6Hz), 8.56-8.59 (1H, m), 8.64-8.65 (1H, m).

[Example 60] N-(2-Methoxycarbonylpyrazin-3-yl)-(2-chloro-5-nitrophenyl)carboxamide

The title compound (0.617 g) was obtained by a similar procedure to that described in Example 2 using methyl 3-aminopyrazin-2-carboxylate (0.338 g), DMA (5 ml) and 2-chloro-5-nitrobenzoyl chloride (0.583 g).
$^1$H-NMR (400MHz, $CDCl_3$, TMS): δ(ppm) 4.07 (3H, s), 7.67 (1H, d, J=8.8Hz), 8.30 (1H, dd, J=8.8, 2.7Hz), 8.49 (1H, d, J=2.3Hz), 8.53 (1H, d, J=2.7Hz), 8.59 (1H, d, J=2.3Hz), 11.32 (1H, s).

[Example 61] N-[4-(3-Nitrophenyl)thiazol-2-yl]-(2-chloro-5-nitrophenyl)carboxamide

The title compound (0.728 g) was obtained as a crystalline solid according to the procedure described in Example 2 using 2-amino-4-(3-nitrophenyl)thiazole (0.439 g), DMA (5 ml) and 2-chloro-5-nitrobenzoyl chloride (0.524 g).
$^1$H-NMR (400MHz, DMSO-$d_6$, TMS): δ(ppm) 7.76 (1H, t, d=8.8Hz), 7.92 (1H, d, J=8.8Hz), 8.11 (1H, s), 8.20 (1H, dd, J=1.2, 8.8Hz), 8.39 (2H, dd, J=2.8, 8.8Hz), 8.63 (1H, d, J=2.8Hz), 8.76 (1H, t, J=2.8Hz).

[Example 62] N-(6-Chloropyridin-3-yl)-(2-chloro-5-nitrophenyl)carboxamide

The title compound (0.554 g) was obtained as a crystalline solid according to the procedure described in Example 2 using 5-amino-2-chloropyridine (0.257 g), DMA (5 ml) and 2-chloro-5-nitrobenzoyl chloride (0.528 g).
$^1$H-NMR (400MHz, DMSO-$d_6$, TMS): δ(ppm) 7.57 (1H, d, J=8.8Hz), 7.92 (1H, d, J=8.8Hz), 8.20 (1H, dd, J=2.9, 8.8Hz), 8.37 (1H, dd, J=2.9, 8.8Hz), 8.56 (1H, d, J=2.9Hz), 8.71 (1H, d, J=2.9Hz).

[Example 63] N-(4-Methyl-3-nitropyridin-2-yl)-(2-chloro-5-nitrophenyl)carboxamide

The title compound (0.482 g) was obtained as a crystalline solid according to the procedure described in Example 2 using 2-amino-4-methyl-3-nitropyridine (0.306 g), DMA (5 ml) and 2-chloro-5-nitrobenzoyl chloride (0.528 g).
$^1$H-NMR (400MHz, DMSO-$d_6$, TMS): δ(ppm) 7.51 (1H, d, J=5.0Hz), 7.90 (1H, d, J=8.8Hz), 8.26 (1H, d, J=2.8Hz), 8.38

(1H, dd, J=2.8, 8.8Hz), 8.56 (1H, d, J=5.0Hz).

[Example 64] 2,5-bis(2-Chloro-5-nitrobenzoylamino)pyridine

The title compound (0.254 g) was obtained as a crystalline solid according to the procedure described in Example 2 using 2,5-diaminopyridine di-hydrochloride (0.182 g), triethylamine (0.335 ml), DMA (5 ml) and 2-chloro-5-nitrobenzoyl chloride (0.528 g).
$^1$H-NMR (400MHz, DMSO-$d_6$, TMS): δ(ppm) 7.87 (1H, d, J=8.8Hz), 7.92 (1H, d, J=8.8Hz), 8.17 (1H, dd, J=3.0, 8.8Hz), 8.25 (1H, d, J=8.8Hz), 8.33 (1H, dd, J=3.0, 8.8Hz), 8.36 (1H, dd, J=3.0, 8.8Hz), 8.48 (1H, d, J=3.0Hz), 8.54 (1H, d, J=3.0Hz), 8.71 (1H, s), 10.96 (1H, s), 11.30 (1H, s).

[Example 65] N-(4-Methylpyrimidin-2-yl)-(2-chloro-5-nitrophenyl)carboxamide

The title compound (0.341 g) was obtained as a crystalline solid according to the procedure described in Example 2 using 2-amino-4-methylpyrimidine (0.218 g), DMA (5 ml) and 2-chloro-5-nitrobenzoyl chloride (0.528 g).
$^1$H-NMR (400MHz, DMSO-$d_6$, TMS): δ(ppm) 7.10 (1H, s), 7.84 (1H, dd, J=3.0, 8.8Hz), 8.30 (1H, dd, J=3.0, 8.8Hz), 8.45 (1H, d, J=5.2Hz), 11.39 (1H, s).

[Example 66] N-(2-Methoxycarbonylthiophen-3-yl)-(2-chloro-5-nitrophenyl)carboxamide

The title compound (0.590 g) was obtained as a crystalline solid according to the procedure described in Example 2 using methyl 3-aminothiophen-2-carboxylate (0.314 g), DMA (5 ml) and 2-chloro-5-nitrobenzoyl chloride (0.528 g).
$^1$H-NMR (400MHz, DMSO-$d_6$, TMS): δ(ppm) 7.93 (1H, d, J=8.6Hz), 7.96-8.03 (2H, m), 8.39 (1H, dd, J=3.0, 8.6Hz), 8.56 (1H, d, J=3.0Hz), 10.67 (1H, s).

[Example 67] N-(6-Methoxybenzothiazol-2-yl)-(2-chloro-5-nitrophenyl)carboxamide

The title compound (0.577 g) was obtained as a crystalline solid according to the procedure described in Example 2 using 2-amino-6-methoxybenzothiazole (0.360 g), DMA (5 ml) and 2-chloro-5-nitrobenzoyl chloride (0.528 g).
$^1$H-NMR (400MHz, DMSO-$d_6$, TMS): δ(ppm) 7.08 (1H, dd, J=2.8, 8.8Hz), 7.65 (1H ,d, J=2.8Hz), 7.71 (1H, d, J=8.8Hz), 7.92 (1H, d, J=8.8Hz), 8.39 (1H, dd, J=2.8, 8.8Hz), 8.64 (1H, d, J=2.8Hz).

[Example 68] N-(6-Chlorobenzothiazol-2-yl)-(2-chloro-5-nitrophenyl)carboxamide

The title compound (0.751 g) was obtained as a crystalline solid according to the procedure described in Example 2 using 2-amino-6-chlorobenzothiazole (0.368 g), DMA (5 ml) and 2-chloro-5-nitrobenzoyl chloride (0.528 g).
$^1$H-NMR (400MHz DMSO-$d_6$, TMS): δ(ppm) 7.52 (1H, dd, J=2.8, 8.8Hz), 7.81 (1H, d, J=8.8Hz), 7.94 (1H, d, J=8.8Hz), 8.22 (1H, d, J=2.8Hz), 8.40 (1H, dd, J=3.0, 8.8Hz), 8.66 (1H, d, J=3.0Hz).

[Example 69] N-(4-Chlorobenzothiazol-2-yl)-(2-chloro-5-nitrophenyl)carboxamide

The title compound (0.464 g) was obtained according to the procedure described in Example 2 using 2-amino-4-chlorobenzothiazole (0.368 g), DMA (5 ml) and 2-chloro-5-nitrobenzoyl chloride (0.528 g).
$^1$H-NMR (400MHz, DMSO-$d_6$, TMS): δ(ppm) 7.38 (1H, t, J=7.8Hz), 7.59 (1H, d, J=7.8Hz), 7.93 (1H, d, J=8.6Hz), 8.06 (1H, d, J=7.8Hz), 8.40 (1H, dd, J=3.0, 8.6Hz), 8.67 (1H, d, J=3.0Hz).

[Example 70] N-(6-Fluorobenzothiazol-2-yl)-(2-chloro-5-nitrophenyl)carboxamide

The title compound (0.512 g) was obtained as a crystalline solid according to the procedure described in Example 2 using 2-amino-6-fluorobenzothiazole (0.336 g), DMA (5 ml) and 2-chloro-5-nitrobenzoyl chloride (0.528 g).
$^1$H-NMR (400MHz, DMSO-$d_6$, TMS): δ(ppm) 7.31-7.39 (1H, m), 7.80-7.88 (1H, m), 7.94 (1H, d, J=8.8Hz), 7.96 (1H, dd, J=3.0, 8.8Hz), 8.40 (1H, dd, J=3.0, 8.8Hz), 8.66 (1H, d, J=3.0Hz).

[Example 71] N-(4-Ethoxycarbonylpyrazol-3-yl)-(2-chloro-5-nitrophenyl)carboxamide

The title compound (0.579 g) was obtained as a crystalline solid according to the procedure described in

Example 2 using 3-amino-4-ethoxycarbonylpyrazole (0.310 g), DMA (5 ml) and 2-chloro-5-nitrobenzoyl chloride (0.528 g).
$^1$H-NMR (400MHz, DMSO-$d_6$, TMS): δ(ppm) 1.29 (3H, t, J=7.2Hz), 4.28 (2H, q, J=7.2Hz), 6.11 (1H, s), 7.92 (1H, d, J=8.8Hz), 8.38 (1H, dd, J=2.9, 8.8Hz), 8.66 (1H, d, J=2.9Hz).

[Example 72] N-(5-Phenylpyrazol-3-yl)-(2-chloro-5-nitrophenyl)carboxamide

The title compound (0.636 g) was obtained as a crystalline solid according to the procedure described in Example 2 using 3-amino-5-phenylpyrazole (0.318 g), DMA (5 ml) and 2-chloro-5-nitrobenzoyl chloride (0.528 g).
$^1$H-NMR (400MHz, DMSO-$d_6$, TMS): δ(ppm) 7.06 (1H, s), 7.34-7.52 (3H, m), 7.87 (1H, d, J=8.8Hz), 7.78 (2H, d, J=7.8Hz), 8.32 (1H, dd, J=3.0, 8.8Hz), 8.41 (1H, s), 11.28 (1H, s).

[Example 73] N-(3,5-Dimethoxypyrimidin-2-yl)-(2-chloro-5-nitrophenyl)carboxamide

The title compound (0.498 g) was obtained as a crystalline solid according to the procedure described in Example 2 using 2-amino-3,5-dimethoxypyrimidine (0.310 g), DMA (5 ml) and 2-chloro-5-nitrobenzoyl chloride (0.528 g).
$^1$H-NMR (400MHz, DMSO-$d_6$, TMS): δ(ppm) 3.58 (6H, s), 5.90 (1H, s), 7.83 (1H, d, J=8.8Hz), 8.26 (1H, dd, J=2.7, 8.8Hz), 8.36 (1H, d, J=2.7Hz), 11.38 (1H, s).

[Example 74] N-(3,5-Dimethylpyrimidin-2-yl)-(2-chloro-5-nitrophenyl)carboxamide

The title compound (0.159 g) was obtained as a crystalline solid according to the procedure described in Example 2 using 2-amino-3,5-dimethylpyrimidine (0.246 g), DMA (5 ml) and 2-chloro-5-nitrobenzoyl chloride (0.528 g).
$^1$H-NMR (400MHz, DMSO-$d_6$, TMS): δ(ppm) 2.25 (6H, s), 6.86 (1H, s), 7.80 (1H, d, J=8.8Hz), 8.27-8.33 (2H, m), 11.28 (1H, s).

[Example 75] N-(5-Chlorobenzoxazol-2-yl)-(2-chloro-5-nitrophenyl)carboxamide

The title compound (0.583 g) was obtained as a crystalline solid according to the procedure described in Example 2 using 2-amino-5-chlorobenzoxazole (0.336 g), DMA (5 ml) and 2-chloro-5-nitrobenzoyl chloride (0.528 g).
$^1$H-NMR (400MHz, DMSO-$d_6$, TMS): δ(ppm) 7.35 (1H, dd, J=2.2, 8.8Hz), 7.69 (1H, s), 7.70 (1H, d, J=8.8Hz), 7.91 (1H, d, J=8.8Hz), 8.37 (1H, dd, J=2.2, 8.8Hz), 8.61 (1H, d, J=2.2Hz).

[Example 76] N-(4-Methoxycarbonylthiophen-3-yl)-(2-chloro-5-nitrophenyl)carboxamide

The title compound (0.546 g) was obtained as a crystalline solid according to the procedure described in Example 2 using 3-amino-4-methoxycarbonylthiophene hydrochloride (0.386 g), triethylamine (0.335 ml), DMA (5 ml) and 2-chloro-5-nitrobenzoyl chloride (0.528 g).
$^1$H-NMR (400MHz, DMSO-$d_6$, TMS): δ(ppm) 3.84 (3H, s), 7.92 (1H, d, J=8.7Hz), 8.08(1H, d, J=2.9Hz), 8.38 (1H, dd, J=2.9, 8.7Hz), 8.44 (1H, d, J=2.9Hz), 8.53 (1H, d, J=2.9Hz), 10.54 (1H, s).

[Example 77] N-(5-Bromopyrimidin-2-yl)-(2-chloro-5-nitrophenyl)carboxamide

The title compound (0.541 g) was obtained as a crystalline solid according to the procedure described in Example 2 using 2-amino-5-bromopyrimidine (0.348 g), DMA (5 ml) and 2-chloro-5-nitrobenzoyl chloride (0.528 g).
$^1$H-NMR (400MHz, DMSO-$d_6$, TMS): δ(ppm) 7.84 (1H, d, J=8.8Hz), 8.32 (1H, dd, J=2.9, 8.8Hz), 8.41 (1H, d, J=2.9Hz), 8.83 (2H, s), 10.65 (1H, s).

[Example 78] N-(4-Chloro-6-methylpyrimidin-2-yl)-(2-chloro-5-nitrophenyl)carboxamide

The title compound (0.336 g) was obtained as a crystalline solid according to the procedure described in Example 2 using 2-amino-4-chloro-6-methylpyrimidine (0.287 g), DMA (5 ml) and 2-chloro-5-nitrobenzoyl chloride (0.528 g).
$^1$H-NMR (400MHz, DMSO-$d_6$, TMS): δ(ppm) 2.34 (3H, s), 7.31 (1H, s), 7.83 (1H, d, J=8.8Hz), 8.31 (1H, dd, J=2.9, 8.8Hz), 8.39 (1H, d, J=2.9Hz), 10.67 (1H, s).

[Example 79] N-[3-Carboethoxy-4,5,6,7-tetrahydrobenzo[b]thiophen-2-yl]-(2-chloro-5-nitrophenyl)carboxamide

The title compound (0.544 g) was obtained as a compound according to the procedure described in Example 2 using 2-amino-3-carboethoxy-4,5,6,7-tetrahydrobenzo[b]thiophene (0.451 g), DMA (5 ml) and 2-chloro-5-nitrobenzoyl chloride (0.528 g).
$^1$H-NMR (400MHz, DMSO-$d_6$, TMS): δ(ppm) 1.29 (3H, t, J=7.3Hz), 1.70-1.82 (4H, m), 2.63-2.80 (4H, m), 4.26 (2H, q, J=7.3Hz), 7.94 (1H, d, J=8.8Hz), 8.40 (1H, dd, J=2.2, 8.8Hz), 8.56 (1H, d, J=2.2Hz), 10.65 (1H, s).

[Example 80] N-(3-Nitropyridin-2-yl)-(2-chloro-5-nitrophenyl)carboxamide

The title compound (0.405 g) was obtained as a crystalline solid according to the procedure described in Example 2 using 2-amino-3-nitropyridine (0.278 g), DMA (5 ml) and 2-chloro-5-nitrobenzoyl chloride (0.528 g).
$^1$H-NMR (400MHz, DMSO-$d_6$, TMS): δ(ppm) 7.59 (1H, dd, J=4.8, 8.1Hz), 7.92 (1H, d, J=8.8Hz), 8.32 (1H, d, J=2.9Hz), 8.39 (1H, dd, J=2.9, 8.8Hz), 8.51 (1H, d, J=8.1Hz), 8.77 (1H, dd, J=1.5, 4.8Hz), 11.97 (1H, s).

[Example 81] N-(4,6-Dichloropyrimidin-5-yl)-(2-chloro-5-nitrophenyl)carboxamide

The title compound (0.478 g) was obtained as a compound according to the procedure described in Example 2 using 5-amino-4,6-dichloropyrimidine (0.326 g), DMA (5 ml) and 2-chloro-5-nitrobenzoyl chloride (0.528 g).
$^1$H-NMR (400MHz, DMSO-$d_6$, TMS): δ(ppm) 7.96 (1H, d, J=8.8Hz), 8.35 (1H, d, J=2.9Hz), 8.41 (1H, dd, J=2.9, 8.8Hz), 8.96 (1H, s), 11.32 (1H, s).

[Example 82] N-(1-Methylbenzimidazol-2-yl)-(2-chloro-5-nitrophenyl)carboxamide

2-Chloro-5-nitrobenzoyl chloride (0.528 g, 2.4 mmol) was added to a solution of 2-amino-1-methylbenzimidazole (a product of Aldrich, 0.294 g, 2.0 mmol) in DMA (5 ml) and the mixture was stirred at room temperature for 2.5 hours. Saturated aqueous sodium bicarbonate solution (5 ml) and water (20 ml) were added to the reaction mixture. The solid thus formed was filtered, washed with water and diisopropyl ether and dried in vacuo to give the crude title compound (0.438 g). The obtained crude product (0.438 g) was purified by chromatography on a silica gel column [hexane:ethyl acetate=2:1 (v/v)] to afford the title compound (0.043 g, yield 7%).
Rf 0.40 [hexane:ethyl acetate= 1:1 (V/V)];
$^1$H-NMR (400MHz, DMSO-$d_6$, TMS): δ 3.68 (3H, s), 7.24-7.34 (2H, m), 7.53-7.59 (2H, m), 7.80 (1H ,d, J=8.8Hz), 8.25 (1H, dd, J=2.9, 8.8 Hz), 8.66 (1H, d, J=2.9Hz);
MS(EI) m/z: 330 $M^+$;

[Example 83] N-(4,6-Dichloropyrimidin-2-yl)-(2-chloro-5-nitrophenyl)carboxamide

The reaction was carried out according to the procedure described in Example 1 using 2-amino-4,6-dichloropyrimidine (a product of Aldrich, 0.328 g, 20 mmol), DMA (5 ml) and 2-chloro-5-nitrobenzoyl chloride (0.528 g, 2.4 mmol). Saturated aqueous sodium bicarbonate solution(5 ml) and water (20 ml) was added to the reaction mixture. The formed solid was filtered, washed with diisopropyl ether and dried in vacuo to afford the title compound (0.363 g, yield 52%).
Rf 0.61 [hexane:ethyl acetate= 1:1(v/v)];
$^1$H-NMR (400MHz, DMSO-$d_6$, TMS): δ 7.71 (1H, s), 7.85 (1H, d, J=8.8Hz), 8.34 (1H, dd, J=2.9, 8.8Hz), 8.45 (1H, d, J=2.9Hz), 12.02 (1H, s);
MS(EI) m/z: 347 $(M + H)^+$;

[Example 84] N-(5-Bromo-3-nitropyridin-2-yl)-(2-chloro-5-nitrophenyl)carboxamide

The title compound (0.477 g, yield 59%) was obtained according to the procedure described in Example 2 using 2-amino-5-bromo-3-nitropyridine (a product of Aldrich, 0.436 g, 2.0 mmol), DMA (5 ml) and 2-chloro-5-nitrobenzoyl chloride (0.660 g, 3.0 mmol).
Rf 0.20 [hexane:ethyl acetate = 2:1(v/v)];
$^1$H-NMR (400MHz, DMSO-$d_6$, TMS): δ 7.92 (1H, d, J=8.8Hz), 8.33 (1H, d, J=2.9Hz), 8.39 (1H, dd, J=2.9, 8.8Hz), 8.80 (1H, d, J=2.2Hz), 8.95 (1H, d, J=2.2Hz), 12.10 (1H, s);
MS(EI) m/z: 400 $M^+$;

[Example 85] N-(1,3,4-Thiadiazo-2-yl)-(2-chloro-5-nitrophenyl)carboxamide

The title compound (0.489 g, yield 86%) was obtained according to the procedure described in Example 2 using 2-amino-1,3,4-thiadiazole (a product of Tokyou-kasei, 0.202 g, 2.0 mmol), DMA (5 ml) and 2-chloro-5-nitrobenzoyl chloride (0.528 g, 2.4 mmol).
$^1$H-NMR (400MHz, DMSO-$d_6$, TMS): δ 7.92 (1H, d, J=8.8 Hz), 8.39 (1H, dd, J=2.9, 8.8Hz), 8.65 (1H, d, J=2.9Hz), 9.31 (1H, s);
MS(EI) m/z: 284 $M^+$;

[Example 86] N-(2,1,3-Benzothiazol-4-yl)-(2-chloro-5-nitrophenyl)carboxamide

The title compound (0.561 g, yield 84%) was obtained according to the procedure described in Example 2 using 4-amino-2,1,3-benzothiazole (a product of Tokyou-kasei, 0.302 g, 2.0 mmol), DMA (5 ml) and 2-chloro-5-nitrobenzoyl chloride (0.528 g, 2.4 mmol).
$^1$H NMR ($CDCl_3$, 400MHz,TMS): δ 7.70 (1H, t, J=8.8Hz), 7.73 (1H, d, J=8.8Hz), 7.80 (1H, d, J=8.8Hz), 8.33 (1H, dd, J=2.9, 8.8Hz), 8.66 (1H, d, J=8.8Hz), 8.76 (1H, d, J=2.9Hz), 9.39 (1H, br);
MS(EI) m/z: 334 $M^+$;

[Example 87] N-[4-[4-(tert-Butoxycarbonylamino)phenyl]phenyl]-(2-chloro-5-nitrophenyl)carboxamide

The title compound (0.335 g, yield 72%) was obtained according to the procedure described in Example 2 using 4-[4-(tert-butoxycarbonylamino)phenyl]aniline (Synth. Commun., 28, 963(1998))(0.284 g, 1.0 mmol), DMA (5 ml) and 2-chloro-5-nitrobenzoyl chloride (0.264 g, 1.2 mmol).
$^1$H-NMR (400MHz, DMSO-$d_6$, TMS): δ 1.49 (9H, s), 7.52-7.62 (4H, m), 7.65 (2H, d, J=8.8Hz), 7.77 (2H, d, J=8.8Hz), 7.90 (1H, d, J=8.8Hz), 8.35 (1H, dd, J=2.2, 8.8Hz), 8.49 (1H, d, J=2.2Hz), 9.43 (1H, s), 10.77 (1H, s);
MS(EI) m/z: 467 $(M + H)^+$;

[Example 88] N-[4-(4-Aminophenyl)phenyl]-(2-chloro-5-nitrophenyl)carboxamide mono-hydrochloride

N-[4-[4-(tert-Butoxycarbonylamino)phenyl]phenyl]-(2-chloro-5-nitrophenyl)carboxamide (0.200 g, 0.427 mmol) was suspended in 1N hydrogen chloride/1,4-dioxane solution (2 ml) and the mixture was stirred for 1 week. The reaction mixture was diluted with ethyl ether and the solid thus formed was filtered. The solid was washed with 1,4-dioxane and ethyl ether and then dried in vacuo to afford the title compound (0.131 g, yield 76%).
$^1$H-NMR (400MHz, DMSO-$d_6$, TMS): δ 7.32 (2H, d, J=8.1Hz), 7.70 (2H, d, J=8.8Hz), 7.73 (2H, d, J=8.1Hz), 7.81 (2H, d, J=8.8Hz), 7.91 (1H, d, J=8.8Hz), 8.36 (1H, dd, J=2.9, 8.8Hz), 8.49 (1H, d, J=2.9Hz), 10.85 (1H, s);
MS(EI) m/z: 367$(M - HCl)^+$;

[Example 89] N-(6-Chloropyridazin-2-yl)-(2-chloro-5-nitrophenyl)carboxamide

The title compound (0.513 g, yield 82%) was obtained according to the procedure described in Example 2 using 3-amino-6-chloropyridazine (a product of Lancaster, 0.259 g, 2.0 mmol), DMA (5 ml) and 2-chloro-5-nitrobenzoyl chloride (0.528 g, 2.4 mmol).
Rf 0.46 [hexane:ethyl acetate = 2:1(v/v)];
$^1$H-NMR (400MHz, DMSO-$d_6$, TMS): δ 7.90 (1H, d, J=8.8Hz), 8.00 (1H, d, J=8.8Hz), 8.36 (1H, dd, J=2.9, 8.8Hz), 8.50 (1H, d, J=8.8Hz), 8.58 (1H, d, J=2.9Hz), 12.10 (1H, s);
MS(FAB) m/z: 313$(M + H)^+$;

[Example 90] N-[6-(4-Fluorobenzyl)benzothiazol-2-yl]-(2-chloro-5-nitrophenyl)carboxamide

The title compound (0.315 g, yield 36%) was obtained according to the procedure described in Example 2 using 6-(4-fluorobenzyl)-2-aminobenzothiazole (Chem. Pharm. Bull., 40, 2055 (1992)) (0.258 g, 1.0 mmol), DMA (5 ml) and 2-chloro-5-nitrobenzoyl chloride (0.264 g, 1.2 mmol).
Rf 0.45 [hexane:ethyl acetate = 2:1 (v/v)];
$^1$H-NMR (400MHz, DMSO-$d_6$, TMS): δ 4.07 (2H,s), 7.12 (1H, d, J=8.8Hz), 7.14 (1H, d, J=8.8Hz), 7.30-7.36 (3H, m), 7.72 (1H, d, J=8.1Hz), 7.92 (1H, d, J=8.8Hz), 8.39 (1H, dd, J=2.9, 8.8Hz), 8.64 (1H, d, J=2.9Hz);
MS(FAB) m/z: 442$(M + H)^+$;

[Example 91] N-[4-(6-Acetoxy-2,5,7,8-tetramethyl-4-oxochroman-2-ylmethoxy)phenyl]-(2-chloro-5-nitrophenyl) carboxamide

A crude desired product (1.17 g) was afforded according to the procedure described in Example 2 using 4-(6-acetoxy-2,5,7,8-tetramethyl-4-oxochroman-2-ylmethoxy)aniline (0.787 g, 2.0 mmol), DMA (5 ml) and 2-chloro-5-nitrobenzoyl chloride (0.528 g, 2.4 mmol). The obtained crude product (0.612 g) was suspended in methanol (5 ml) for 1.5 hours. The solid thus formed was filtered, washed with methanol and then dried in vacuo to afford the title compound (0.486 g, yield 43%).
Rf 0.14 [hexane:ethyl acetate = 2:1 (v/v)];
$^1$H NMR ($CDCl_3$, 400MHz,TMS): δ 1.52 (3H, s), 2.09 (3H, s), 2.13 (3H, s), 2.36 (3H, s), 2.42 (3H, s), 2.71 (1H, d, J=16.0Hz), 3.10 (d, 1H, J=16.0Hz), 4.03 (d, 1H, J=10.3Hz), 4.14(1H, d, J=10.3Hz), 6.93 (2H, d, J=8.8Hz), 7.53 (2H, d, J=8.8Hz), 7.65 (1H, d, J=8.8Hz), 7.79 (1H, br), 8.26 (1H, dd, J=2.9, 8.8Hz), 8.62 (1H, d, J=2.9Hz);
MS(FAB) m/z: 567(M + H)$^+$;

[Example 92] N-[4-(6-Hydroxy-2,5,7,8-tetramethyl-4-oxochroman-2-ylmethoxy)phenyl]-(2-chloro-5-nitrophenyl) carboxamide

A small amount of a solution of sodium methoxide/methanol solution (25 wt%) was added to a suspension of N-[4-(6-acetoxy-2,5,7,8-tetramethyl-4-oxochroman-2-ylmethoxy)phenyl]-(2-chloro-5-nitrophenyl)carboxamide prepared in Example 91 (0.342 g, 0.603 mmol) in methanol (6 ml). The mixture was stirred for 21 hours. Acetic acid was added to the reaction mixture and the mixture was diluted with ethyl acetate. The organic layer was washed with water and saturated aqueous sodium chloride solution, dried over anhydrous sodium sulfate and the solvent was removed under reduced pressure. The resulting residue was purified by chromatography on a silica gel column [hexane: ethyl acetate = 1:1 (v/v)] and the product was dried in vacuo to afford the title compound (0.153 g, yield 48%).
Rf 0.48 [hexane:ethyl acetate = 1:1 (v/v)];
$^1$H-NMR (400MHz, DMSO-$d_6$, TMS): δ 1.40 (3H, s), 2.05 (3H, s), 2.15 (3H, s), 2.44 (3H, s), 2.69 (1H, d, J=16.1Hz), 3.00 (1H, d, J=16.1Hz), 4.09 (1H, d, J=10.3Hz), 4.13 (1H, d, J=10.3Hz), 6.97 (2H, d, J=8.8Hz), 7.60 (2H, d, J=8.8Hz), 7.88 (1H, d, J=8.8Hz), 7.92 (1H, br), 8.33 (1H, dd, J=2.9, 8.8 Hz), 8.44 (1H, d, J=2.9Hz), 10.57 (1H, s);
MS(FAB) m/z: 525(M + H)$^+$;

[Example 93] N-[4-[4-(Methanesulfonylamino)phenyl]phenyl]-(2-chloro-5-nitrophenyl)carboxamide

Triethylamine (0.279 ml, 2.0 mmol) and methanesulfonylchloride (0.116 ml, 1.5 mmol) were added to a solution of N-[4-(4-aminophenyl)phenyl]-(2-chloro-5-nitrophenyl)carboxamide mono-hydrochloride (0.404 g, 1.0 mmol) prepared in Example 88 in DMA (5 ml). The mixture was stirred at room temperature for 5 hours. Saturated aqueous sodium bicarbonate solution (4 ml), water (20 ml) and ethyl acetate (20 ml) were added to the reaction mixture. The insoluble material thus formed was filtered, washed with water and diisopropyl ether and then dried in vacuo to afford the title compound (0.238 g, 53%).
Rf 0.51 [methylene chloride:methanol = 7:1 (v/v)];
$^1$H-NMR (400MHz, DMSO-$d_6$, TMS): δ 3.02 (3H,s), 7.30 (2H, d, J=8.8Hz), 7.66 (2H, d, J=8.8Hz), 7.68 (2H, d, J=8.8Hz), 7.79 (2H, d, J=8.8Hz), 7.91 (1H, d, J=8.8Hz), 8.35 (1H, dd, J=2.9, 8.8Hz), 8.49 (1H, d, J=2.9 Hz), 9.83 (1H, s), 10.80 (1H, s);
MS(FAB) m/z: 446 (M + H)$^+$;

[Example 94] N-[4-[4-(4-Toluenesulfonylamino)phenyl]phenyl]-(2-chloro-5-nitrophenyl)carboxamide

Triethylamine (0.279 ml, 2.0 mmol) and 4-toluenesulfonylchloride (0.286 g, 1.5 mmol) were added to a solution of N-[4-[4-(4-aminophenyl)phenyl]-(2-chloro-5-nitrophenyl)carboxamide mono-hydrochloride (0.404 g, 1.0 mmol) prepared in Example 88 in DMA (5 ml). The mixture was stirred at room temperature for 4.5 hours. After an addition of saturated aqueous sodium bicarbonate solution (4 ml), water (20 ml) and ethyl acetate (20 ml), the reaction mixture was partitioned. The organic layer was dried over anhydrous sodium sulfate, and the solvent was removed under reduced pressure. The resulting residue was filtered, washed with water and diisopropyl ether and then dried in vacuo to afford the title compound (0.365 g, 70%).
$^1$H-NMR (400MHz, DMSO-$d_6$, TMS): δ 2.34 (3H, s), 7.17 (2H, d, J=8.8Hz), 7.36 (2H, d, J=8.8Hz), 7.55 (2H, d, J=8.8Hz), 7.61 (2H, d, J=8.8 Hz), 7.69 (2H, d, J=8.8Hz), 7.75 (2H, d, J=8.8Hz), 7.90 (1H, d, J=8.8Hz), 8.35 (1H, dd, J=2.9, 8.8Hz), 8.48 (1H, d, J=2.9Hz), 10.34 (1H, s), 10.77 (1H, s);
MS(FAB) m/z: 522(M + H)$^+$;

[Example 95] N-[4-[(Pyrimidin-2-yl)aminosulfonyl]phenyl]-(2-chloro-5-nitrophenyl)carboxamide

The title compound (0.208 g, yield 48%) was obtained according to the procedure described in Example 2 using sulfadiazine (a product of Aldrich, 0.250 g, 1.0 mmol), DMA (9 ml) and 2-chloro-5-nitrobenzoyl chloride (0.264 g, 1.2 mmol).
Rf 0.07 [hexane:ethyl acetate = 1:1 (v/v)];
$^1$H-NMR (400MHz, DMSO-$d_6$, TMS): δ 7.06 (1H, t, J=5.1Hz), 7.87 (2H, d, J=8.8Hz), 7.90 (2H, d, J=8.8Hz), 8.01 (2H, d, J=8.8Hz), 8.36 (1H, dd, J=2.2, 8.8Hz), 8.51 (2H, d, J=5.1Hz), 8.52 (1H, m), 11.09 (1H, br), 11.76 (1H, br);
MS(FAB) m/z: 434 $(M + H)^+$;

[Example 96] N-[4-(Thiazol-2-yl)aminosulfonyl]phenyl-(2-chloro-5-nitrophenyl)carboxamide

The title compound (0.843 g, yield 97%) was obtained according to the procedure described in Example 2 using 4-[(thiazol-2-yl)aminosulfonyl]phenylamine (a product of Merck, 0.510 g, 2.0 mmol), DMA (5 ml) and 2-chloro-5-nitrobenzoyl chloride (0.528 g, 2.4 mmol).
Rf 0.58 [methylene chloride:methanol = 7:1 (v/v)];
$^1$H-NMR (400MHz, DMSO-$d_6$, TMS): δ 6.84 (1H, d, J=4.4Hz), 7.26 (1H, d, J=4.4Hz), 7.84 (4H, m), 7.90 (1H, d, J=8.8Hz), 8.35 (1H, dd, J=2.9, 8.8Hz), 8.51 (1H, d, J=2.9Hz), 11.04 (1H, s);
MS(FAB) m/z: 439$(M + H)^+$;

[Example 97] N-[4-(4,5-Dimethyloxazol-2-yl)aminosulfonyl]phenyl-(2-chloro-5-nitrophenyl)carboxamide

The title compound (0.763 g, yield 85%) was obtained according to the procedure described in Example 2 using 4-[(4,5-dimethyloxazol-2-yl)aminosulfonyl]aniline (a product of Sigma, 0.534 g, 2.0 mmol), DMA (5 ml) and 2-chloro-5-nitrobenzoyl chloride (0.528 g, 2.4 mmol).
Rf 0.68 [methylene chloride:methanol = 7:1 (v/v)];
$^1$H-NMR (400MHz, DMSO-$d_6$, TMS): δ 1.95 (3H, s), 2.06 (3H, s), 7.83 (2H, d, J=8.8Hz), 7.87 (2H, d, J=8.8Hz), 7.91 (1H, d, J=8.8Hz), 8.36 (1H, dd, J=2.9, 8.8Hz), 8.52 (1H, d, J=2.9Hz), 11.02 (1H, s), 11.78 (1H, s);
MS(FAB) m/z: 451 $(M + H)^+$;

[Example 98] N-[[4-(2,6-Dimethylpyrimidin-4-yl)aminosulfonyl]phenyl]-(2-chloro-5-nitrophenyl)carboxamide

The title compound (0.054 g, yield 12%) was obtained according to the procedure described in Example 2 using sulfisomidine (a product of Wako-junyaku, 0.278 g, 1.0 mmol), DMA (2.5 ml) and 2-chloro-5-nitrobenzoyl chloride (0.264 g, 1.2 mmol).
Rf 0.55 [methylene chloride:methanol = 7:1 (v/v)];
$^1$H-NMR (400MHz, DMSO-$d_6$, TMS): δ 2.28 (3H, s), 2.36 (3H, s), 7.82-7.94 (6H, m), 8.35 (1H, dd, J=2.9, 8.8Hz), 8.51 (1H, d, J=2.9Hz), 11.03 (1H, s);
MS(FAB) m/z: 462$(M + H)^+$;

[Example 99] N-[[(5-Methylisoxazol-3-yl)aminosulfonyl]phenyl]-(2-chloro-5-nitrophenyl)carboxamide

The title compound (0.325 g, yield 74%) was obtained according to the procedure described in Example 2 using sulfamethoxazole (a product of Tokyo-kasei 0.253 g, 1.0 mmol), DMA (2.5 ml) and 2-chloro-5-nitrobenzoyl chloride (0.264 g, 1.2 mmol).
Rf 0.70 [methylene chloride:methanol = 7:1 (v/v)];
$^1$H-NMR (400MHz, DMSO-$d_6$, TMS): δ 2.30 (3H, s), 6.16 (1H, s), 7.85-7.92 (5H, m), 7.91 (1H, d, J=8.8Hz), 8.36 (1H, dd, J=2.9, 8.8Hz), 8.53 (1H, d, J=2.9Hz), 11.14 (1H, s), 11.40(1H,s);
MS(FAB) m/z: 437 $(M + H)^+$;

[Example 100] N-[4-[(Pyridin-2-yl)aminosulfonyl]phenyl]-(2-chloro-5-nitrophenyl)carboxamide

The title compound (0.408 g, yield 94%) was obtained according to the procedure described in Example 2 using sulfisomidine (a product of Tokyo-kasei, 0.278 g, 1.0 mmol), DMA (2.5 ml) and 2-chloro-5-nitrobenzoyl chloride (0.264 g, 1.2 mmol).
Rf 0.57 [methylene chloride:methanol = 7:1 (v/v)];
$^1$H-NMR (400MHz, DMSO-$d_6$, TMS): δ 6.88 (1H, m), 7.15 (1H, d, J=8.8Hz), 7.72 (1H, ddd, J=1.5, 8.8, 8.8Hz), 7.84 (2H, d, J=8.8Hz), 7.90 (2H, d, J=8.8Hz), 7.90 (1H, d, J=8.8Hz), 8.03 (1H, m), 8.35 (1H, dd, J=2.9, 8.8Hz), 8.51 (1H,

d, J=2.9Hz), 11.03 (1H, s);
MS(FAB) m/z: 433 $(M + H)^+$;

[Example 101] N-[4-[(5-Methyl-[1,3,4]thiadiazol-2-yl)aminosulfonyl]phenyl]-(2-chloro-5-nitrophenyl)carboxamide

The title compound (0.060 g, yield 13%) was obtained according to the procedure described in Example 2 using sulfamethyzole (a product of Tokyo-kasei, 0.270 g, 1.0 mmol), DMA (2.5 ml) and 2-chloro-5-nitrobenzoyl chloride (0.264 g, 1.2 mmol).
Rf 0.47 [methylene chloride:methanol=7:1 (v/v)];
$^1$H-NMR (400MHz, DMSO-$d_6$,TMS): δ 2.46 (3H, s), 7.81 (2H, d, J=8.8Hz), 7.86 (2H, d, J=8.8Hz), 7.91 (1H, d, J=8.8Hz), 8.36 (1H, dd, J=2.9, 8.8Hz), 8.51 (1H, d, J=2.9Hz), 11.07 (1H, s);
MS(FAB) m/z: 454 $(M + H)^+$;

[Example 102] N-[4-[(6-Chloropyridazin-3-yl)aminosulfonyl]phenyl]-(2-chloro-5-nitrophenyl)carboxamide

The title compound (0.454 g, yield 97%) was obtained according to the procedure described in Example 2 using sulfachloropyridazine (a product of Shigma, 0.284 g, 1.0 mmol), DMA (2.5 ml) and 2-chloro-5-nitrobenzoyl chloride (0.264 g, 1.2 mmol).
Rf 0.54 [methylene chloride:methanol=7:1 (v/v)];
$^1$H-NMR (400MHz, DMSO-$d_6$, TMS): δ 7.77-7.97 (7H, m, J=8.8Hz), 8.36 (1H, dd, J=2.9, 8.8Hz), 8.51 (1H, d, J=2.9Hz), 11.11 (1H, s);
MS(FAB) m/z: 468 $(M + H)^+$;

[Example 103] N-[4-(1H-Indazol-6-yl)aminosulfonyl]phenyl]-(2-chloro-5-nitrophenyl)carboxamide

The title compound (0.454 g, yield 97%) was obtained according to the procedure described in Example 2 using 4-[(6-indazoyl)aminosulfonyl]aniline (a product of Aldrich, 0.288 g, 1.0 mmol), DMA (2.5 ml) and 2-chloro-5-nitrobenzoyl chloride (0.264 g, 1.2 mmol).
Rf 0.71 [methylene chloride:methanol=7:1 (v/v)];
$^1$H-NMR (400MHz, DMSO-$d_6$, TMS): δ 6.90 (1H, dd, J=2.2, 8.8Hz), 7.27 (1H, d, J=2.2Hz), 7.61 (1H, d, J=8.8Hz), 7.79 (2H, d, J=8.8Hz), 7.82 (2H, d, J=8.8Hz), 7.88 (1H, d, J=8.8Hz), 7.94 (1H, s), 8.34 (1H, dd, J=2.9, 8.8Hz), 8.50 (1H, d, J=2.9Hz), 10.38 (1H, s), 11.05 (1H, s);
MS(FAB) m/z: 472 $(M + H)^+$;

[Example 104] N-[4-[(3,4-Dimethylisoxazol-6-yl)aminosulfonyl]phenyl]-(2-chloro-5-nitrophenyl)carboxamide

The title compound (0.364 g, yield 81%) was obtained according to the procedure described in Example 2 using sulfaisoxazole (a product of Sigma, 0.267 g, 1.0 mmol), DMA (2.5 ml) and 2-chloro-5-nitrobenzoyl chloride (0.264 g, 1.2 mmol).
Rf 0.41 [methylene chloride:methanol=7:1 (v/v)];
$^1$H-NMR (400MHz, DMSO-$d_6$, TMS): δ 1.68 (3H, s), 2.10 (3H, s), 7.79 (2H, d, J=8.8Hz), 7.91 (2H, d, J=8.8Hz), 7.91 (1H, d, J=8.8Hz), 8.36 (1H, dd, J=2.9, 8.8Hz), 8.56 (1H, d, J=2.9Hz), 11.15 (1H, s);
MS(FAB) m/z: 451 $(M + H)^+$;

[Example 105] N-[4-[(5-Methoxypyrimidin-2-yl)aminosulfonyl]phenyl]-(2-chloro-5-nitrophenyl)carboxamide

The title compound (0.377 g, yield 97%) was obtained according to the procedure described in Example 2 using sulfameter (a product of Sigma, 0.280 g, 1.0 mmol), DMA (2.5 ml) and 2-chloro-5-nitrobenzoyl chloride (0.264 g, 1.2 mmol).
Rf 0.71 [methylenchloride:methanol=7:1 (v/v)];
$^1$H-NMR (400MHz,DMSO-$d_6$, TMS): δ 3.80 (3H, s), 7.87 (2H, d, J=8.8Hz), 7.91 (1H, d, J=8.8Hz), 7.98 (2H, d, J=8.8Hz), 8.30 (2H, s), 8.36 (1H, dd, J=2.2, 8.8Hz), 8.52 (1H, d, J=2.2Hz), 11.09 (1H, s), 11.46 (1H, br);
MS(FAB) m/z: 464 $(M + H)^+$;

[Example 106] N-[4-(Amidinoaminosulfonyl)phenyl]-(2-chloro-5-nitrophenyl)carboxamide

The title compound (0.280 g, yield 70%) was obtained according to the procedure described in Example 2 using sulfaguanidine (a product of Sigma, 0.214 g, 1.0 mmol), DMA (2.5 ml) and 2-chloro-5-nitrobenzoyl chloride (0.242

g, 1.1 mmol).
Rf 0.24 [methylenchloride:methanol=7:1 (v/v)];
$^1$H-NMR (400MHz, DMSO-$d_6$, TMS): δ 6.70 (4H, br), 7.77 (2H, d, J=8.8Hz), 7.81 (2H, d, J=8.8Hz), 7.91 (1H, d, J=8.8Hz), 8.36 (1H, dd, J=2.2, 8.8Hz), 8.51 (1H, d, J=2.2Hz), 10.98 (1H, s);
MS(FAB) m/z: 398 $(M + H)^+$;

[Example 107] N-[4-(Butylaminocarbonylaminosulfonyl)phenyl]-(2-chloro-5-nitrophenyl)carboxamide

The title compound (0.346 g, yield 76%) was obtained according to the procedure described in Example 2 using 1-butyl-3-sulfanilylurea (0.271 g, 1.0 mmol, a product of Aldrich), DMA (2.5 ml) and 2-chloro-5-nitrobenzoyl chloride (0.264 g, 1.2 mmol).
Rf 0.24 [methylene chloride:methanol=7:1 (v/v)];
$^1$H-NMR (400MHz, DMSO-$d_6$, TMS): δ 0.82 (3H, t, J=7.3Hz), 1.18 (1H, m), 1.31 (1H, m), 2.94 (1H, m), 6.44 (1H, m), 7.90-7.93 (5H, m), 8.36 (1H, dd, J=2.8, 8.8Hz), 8.54 (1H, d, J=2.8Hz), 10.48 (1H, br), 11.12 (1H, s);
MS(FAB) m/z: 455 $(M + H)^+$;

[Example 108] N-[4-[(2-Phenyl-(2H)-pyrazol-3-yl)aminosulfonyl]phenyl]-(2-chloro-5-nitrophenyl)carboxamide

The title compound (0.428 g, yield 86%) was obtained according to the procedure described in Example 2 using sulfaphenazole (0.314 g, 1.0 mmol, a product of Tokyoksei), DMA (2.5 ml) and 2-chloro-5-nitrobenzoyl chloride (0.264 g, 1.2 mmol).
Rf 0.63 [methylene chloride:methanol=7:1 (v/v)];
$^1$H-NMR (400MHz, DMSO-$d_6$,TMS): δ 5.89 (1H, d, J=1.8Hz), 7.40 (1H, m), 7.48 (4H, m), 7.59 (1H, d, J=1.8Hz), 7.69 (2H, d, J=8.8Hz), 7.85 (2H, d, J=8.8Hz), 7.93 (1H, d, J=8.8Hz), 8.37 (1H, dd, J=2.9, 8.8Hz), 8.56 (1H, d, J=2.9Hz), 11.13 (1H, s);
MS(FAB) m/z: 498 $(M + H)^+$;

[Example 109] N-(4-Phenyl-5-tetradecylthiazol-2-yl)-(2-chloro-5-nitrophenyl)carboxamide

The title compound (0.311 g, yield 56%) was obtained according to the procedure described in Example 2 using 2-amino-4-phenyl-5-tetradecylthiazole (0.373 g, 1.0 mmol, a product of Aldrich), DMA (2.5 ml) and 2-chloro-5-nitrobenzoyl chloride (0.264 g, 1.2 mmol).
Rf 0.59 [hexane:ethyl acetate=3:1 (v/v)];
$^1$H NMR ($CDCl_3$, 400MHz, TMS): δ 0.88 (3H, J=7.3Hz), 1.10-1.47 (10H, m), 1.71 (2H, m), 2.85 (2H, t, J=7.8Hz), 7.16-7.28 (5H, m), 7.33 (1H, d, J=8.6Hz), 8.05 (1H, dd, J=2.7, 8.6Hz), 8.07 (1H, d, J=2.7Hz);
MS(FAB) m/z: 566 $(M + H)^+$;

[Example 110] N-(5-Phenyl-[1,3,4]thiadiazol-2-yl)-(2-chloro-5-nitrophenyl)carboxamide

The title compound (0.141 g, yield 39%) was obtained according to the procedure described in Example 2 using 2-amino-5-phenyl-1,3,4-thiadiazole sulfate (a product of Aldrich, 0.177 g, 1.0 mmol), DMA (2.5 ml) and 2-chloro-5-nitrobenzoyl chloride (0.264 g, 1.2 mmol).
Rf 0.59 [hexane:ethyl acetate=3:1 (v/v)];
$^1$H-NMR (400MHz, DMSO-$d_6$, TMS): δ 7.56 (3H, m), 7.93 (1H, d, J=8.8Hz), 8.01 (2H, m), 8.40 (1H, dd, J=2.9, 8.8Hz), 8.68 (1H, d, J=2.9Hz);
MS(EI) m/z: 360 $M^+$;

[Example 111] N-[4-(3,4-Difluorophenyl)thiazol-2-yl]-(2-chloro-5-nitrophenyl)carboxamide

The title compound (0.361 g, yield 91%) was obtained according to the procedure described in Example 2 using 2-amino-4-(3,4-difluorophenyl)thiazole (a product of Maybridge, 0.212 g, 1.0 mmol), DMA (2.5 ml) and 2-chloro-5-nitrobenzoyl chloride (0.264 g, 1.2 mmol).
Rf 0.43 [hexane:ethyl acetate=3:1 (v/v)];
$^1$H-NMR (400MHz,DMSO-$d_6$, TMS): δ 7.52 (1H, ddd, J=2.2, 8.8, 8.8Hz), 7.79 (1H, m), 7.88 (1H, s), 7.91 (1H, d, J=8.8Hz), 7.94 (1H, ddd, J=2.2, 2.2, 8.8Hz), 8.38 (1H, dd, J=2.9, 8.8Hz), 8.61 (1H, d, J=2.9Hz);
MS(EI) m/z: 395 $M^+$;

[Example 112] N-[3-(2-Methylpyrimidin-4-yl)phenyl]-(2-chloro-5-nitrophenyl)carboxamide

The title compound (0.198 g, yield 52%) was obtained according to the procedure described in Example 2 using 4-(3-aminophenyl)-2-methylpyrimidine (a product of Maybridge, 0.185 g, 1.0 mmol), DMA (2.5 ml) and 2-chloro-5-nitrobenzoyl chloride (0.264 g, 1.2 mmol).
Rf 0.06 [hexane:ethyl acetate=3:1 (v/v)];
$^1$H-NMR(400MHz, DMSO-$d_6$, TMS): δ 2.69 (3H, s), 7.57 (1H, dd, J=8.1, 8.1Hz), 7.84 (1H, d, J=5.1Hz), 7.90-7.96 (3H, m), 8.36 (1H, dd, J=2.9, 8.8Hz), 8.53 (1H, d, J=2.9Hz), 8.54 (1H, m), 8.78 (1H, d, J=5.9Hz), 10.94 (1H, s);
MS(EI) m/z: 368 $M^+$;

[Example 113] N-[4-(Morpholin-4-yl)phenyl]-(2-chloro-5-nitrophenyl)carboxamide

The title compound (0.288 g, yield 80%) was obtained according to the procedure described in Example 2 using N-(4-aminophenyl)morpholine (a product of Maybridge, 0.179 g, 1.0 mmol), DMA (2.5 ml) and 2-chloro-5-nitrobenzoyl chloride (0.264 g, 1.2 mmol).
Rf 0.06 [hexane:ethyl acetate=3:1 (v/v)];
$^1$H-NMR (400MHz, DMSO-$d_6$, TMS): δ 3.08 (4H, t, J=4.8Hz), 3.74 (4H, t, J=4.8Hz), 6.96 (2H, d, J=8.8Hz), 7.57 (2H, d, J=8.8Hz), 7.88 (1H, d, J=8.8Hz), 8.32 (1H, dd, J=2.9, 8.8Hz), 8.42 (1H, d, J=2.9Hz), 10.49 (1H, s);
MS(EI) m/z: 361 $M^+$;

[Example 114] N-[4-(Piperidin-1-yl)phenyl]-(2-chloro-5-nitrophenyl)carboxamide

The title compound (0.249 g, yield 69%) was obtained according to the procedure described in Example 2 using N-(4-aminophenyl)piperizine (a product of Maybridge, 0.176 g, 1.0 mmol), DMA (2.5 ml) and 2-chloro-5-nitrobenzoyl chloride (0.264 g, 1.2 mmol).
Rf 0.06 [hexane:ethyl acetate=3:1 (v/v)];
$^1$H-NMR (400MHz, DMSO-$d_6$, TMS): δ 1.55 (2H, m), 1.62 (4H, m), 3.10 (4H, t, J=5.6Hz), 6.94 (2H, d, J=9.1Hz), 7.54 (2H, d, J=9.1Hz), 7.88 (1H, d, J=8.8Hz), 8.33 (1H, dd, J=2.8, 8.8Hz), 8.41 (1H, d, J=2.8Hz), 10.42 (1H, s);
MS(EI) m/z: 359 $M^+$;

[Example 115] N-(4-Ethylaminophenyl)-(2-chloro-5-nitrophenyl)carboxamide mono-hydrochloride

Sodium cyanoborohydride (251 mg, 4.0 mmol) and acetaldehyde (0.224 ml, 4.0 mmol) were added to a suspension of N-(4-aminophenyl)-(2-chloro-5-nitrophenyl)carboxamide mono-hydrochloride (0.583 g, 2.0 mmol) prepared in Example 58 in methanol (10 ml). The mixture was stirred at 0°C for 30 minutes. Water (20 ml) and saturated aqueous sodium bicarbonate solution (1 ml) were added to the reaction mixture. The insoluble material thus formed was filtered, washed with water and dissolved in ethyl acetate. The solution was dried over anhydrous sodium sulfate and the solvent was removed. The resulting residue was purified by chromatography on a silica gel column [hexane:ethyl acetate=3:1 to 1:1 (v/v)] to afford the title compound (0.263 g, yield 41%).
Rf 0.38 [hexane:ethyl acetate=1:1 (v/v)];
$^1$H NMR ($CDCl_3$, 400MHz, TMS): δ 1.27 (3H, t, J=7.3Hz), 3.17 (2H, q, J=7.3Hz), 6.62 (2H, d, J=8.8Hz), 7.41 (2H, d, J=8.8Hz), 7.64 (1H, d, J=8.8Hz), 7.66 (1H, br), 8.24 (1H, dd, J=2.2, 8.8Hz), 8.60 (1H, d, J=2.2Hz);
MS(FAB) m/z: 319 $M^+$;

[Example 116] N-[4-(4-Toluenesulfonylamino)phenyl]-(2-chloro-5-nitrophenyl)carboxamide

The title compound (0.249 g, yield 29%) was obtained according to the procedure described in Example 36 using N-(4-amiophenyl)-(2-chloro-5-nitrophenyl)carboxamide mono-hydrochloride (0.291 g, 1.0 mmol) prepared in Example 58, DMA (5 ml), triethylamine (0.209 ml, 1.5 mmol) and 4-toluenesulfonyl chloride (0.286 g, 1.5 mmol).
Rf 0.64 [methylene chloride:methanol=7:1 (v/v)];
$^1$H-NMR (400MHz, DMSO-$d_6$, TMS): δ 2.35 (3H, s), 7.08 (2H, d, J=8.8Hz), 7.35 (2H, d, J=8.8Hz), 7.54 (2H, d, J=8.8Hz), 7.63 (2H, d, J=8.8Hz), 7.87 (1H, d, J=8.8Hz), 8.32 (1H, dd, J=2.9, 8.8Hz), 8.42 (1H, d, J=2.9Hz), 10.14 (1H, s), 10.63 (1H, s);
MS(FAB) m/z: 446 $(M + H)^+$;

[Example 117] N-(4-Acetylaminophenyl)-(2-chloro-5-nitrophenyl)carboxamide

The title compound (0.255 g, yield 76%) was obtained according to the procedure described in Example 2

using N-(4-aminophenyl)-(2-chloro-5-nitrophenyl)carboxamide mono-hydrochloride (0.291 g, 1.0 mmol) prepared in Example 58, DMA (3 ml) and acetyl chloride (0.078 ml, 1.1 mmol).
Rf 0.54 [methylene chloride:methanol=7:1 (v/v)];
$^1$H-NMR (400MHz, DMSO-$d_6$, TMS): δ 2.04 (3H, s), 7.57 (2H, d, J=8.8Hz), 7.62 (2H, d, J=8.8Hz), 7.89 (1H, d, J=8.8Hz), 8.33 (1H, dd, J=2.9, 8.8Hz), 8.44 (1H, d, J=2.9Hz), 9.95 (1H, s), 10.63 (1H, s);
MS(FAB) m/z: 334 $(M + H)^+$;

[Example 118] N-[4-(4-Acetylaminophenyl)phenyl]-(2-chloro-5-nitrophenyl)carboxamide

The title compound (0.263 g, yield 86%) was obtained according to the procedure described in Example 2 using N-[4-(4-aminophenyl)phenyl-(2-chloro-5-nitrophenyl)carboxamide mono-hydrochloride (0.303 g, 0.75 mmol) prepared in Example 88, DMA (4 ml), triethylamine (0.627 ml, 4.5 mmol) and acetyl chloride (0.117 ml, 1.65 mmol).
Rf 0.56 [methylene chloride:methanol=7:1 (v/v)];
$^1$H-NMR (400MHz, DMSO-$d_6$, TMS): δ 2.07 (3H, s), 7.62 (2H, d, J=8.8Hz), 7.67 (2H, d, J=8.8Hz), 7.67 (2H, d, J=8.8Hz), 7.78 (2H, d, J=8.8Hz), 7.91 (1H, d, J=8.8Hz), 8.35 (1H, dd, J=2.9, 8.8Hz), 8.49 (1H, d, J=2.9Hz), 10.02 (1H, s), 10.78 (1H, s);
MS(FAB) m/z: 410 $(M + H)^+$;

[Example 119] N-(4-Benzoylaminophenyl)-(2-chloro-5-nitrophenyl)carboxamide

The title compound (0.317 g, yield 80%) was obtained according to the procedure described in Example 2 using N-(4-aminophenyl)-(2-chloro-5-nitrophenyl)carboxamide mono-hydrochloride (0.291 g, 1.0 mmol) prepared in Example 58, DMA (3 ml) and benzoyl chloride (0.128 ml, 1.1 mmol).
Rf 0.67 [methylene chloride:methanol=7:1 (v/v)];
$^1$H-NMR (400MHz, DMSO-$d_6$, TMS): δ 7.50-7.63 (3H, m), 7.69 (2H, d, J=8.8Hz), 7.79 (2H, d, J=8.8Hz), 7.90 (1H, d, J=8.8Hz), 7.97 (2H, d, J=8.8Hz), 8.34 (1H, dd, J=2.9, 8.8Hz), 8.47 (1H, d, J=2.9Hz), 10.28 (1H, s), 10.70 (1H, s);
MS(FAB) m/z: 396 $(M + H)^+$;

[Example 120] N-[4-(4-Benzoylaminophenyl)phenyl]-(2-chloro-5-nitrophenyl)carboxamide

The title compound (0.312 g, yield 88%) was obtained according to the procedure described in Example 2 using N-[4-(4-aminophenyl)phenyl-(2-chloro-5-nitrophenyl)carboxamide mono-hydrochloride (0.303 g, 0.75 mmol) prepared in Example 88, DMA (3 ml), triethylamine (0.314 ml, 2.25 mmol) and benzoyl chloride (0.096 ml, 0.83 mmol).
Rf 0.73 [methylene chloride:methanol=7:1 (v/v)];
$^1$H-NMR (400MHz, DMSO-$d_6$, TMS): δ 7.52-7.62 (3H, m), 7.69 (4H, m), 7.80 (2H, d, J=8.7Hz), 7.90 (3H, m), 7.98 (2H, m), 8.36 (1H, dd, J=2.9, 8.8Hz), 8.49 (1H, d, J=2.9Hz), 10.35 (1H, s), 10.80 (1H, s);
MS(FAB) m/z: 472 $(M + H)^+$;

[Example 121] N-[4-(4-Methylbenzoyl)aminophenyl]-(2-chloro-5-nitrophenyl)carboxamide

The title compound (0.302 g, yield 74%) was obtained according to the procedure described in Example 2 using N-(4-aminophenyl)-(2-chloro-5-nitrophenyl)carboxamide mono-hydrochloride (0.291 g, 1.0 mmol) prepared in Example 115, DMA (3 ml), and 4-methylbenzoyl chloride (0.145 ml, 1.1 mmol).
Rf 0.55 [methylene chloride:methanol=7:1 (v/v)];
$^1$H-NMR (400MHz, DMSO-$d_6$, TMS): δ 2.40 (3H, s), 7.34 (2H, d, J=8.1Hz), 7.68 (2H, d, J=8.8Hz), 7.78 (2H, d, J=8.8Hz), 7.89 (2H, d, J=8.1Hz), 7.90 (1H, d, J=8.8Hz), 8.34 (1H, dd, J=2.9, 8.8Hz), 8.47 (1H ,d, J=2.9 Hz), 10.19 (1H, s), 10.69 (1H, s);
MS(FAB) m/z: 410 $(M + H)^+$;

[Example 122] N-[4-(4-Methylbenzoylamino)phenyl]phenyl]-(2-chloro-5-nitrophenyl)carboxamide

The title compound (0.309 g, yield 85%) was obtained according to the procedure described in Example 2 using N-[4-(4-aminophenyl)phenyl]-(2-chloro-5-nitrophenyl)carboxamide mono-hydrochloride (0.303 g, 0.75 mmol) prepared in Example 88, DMA (3 ml), triethylamine (0.314 ml, 2.25 mmol) and 4-methylbenzoyl chloride (0.109 ml, 0.83 mmol).
Rf 0.72 [methylene chloride:methanol=7:1 (v/v)];
$^1$H-NMR (400MHz, DMSO-$d_6$, TMS): δ 2.40 (3H, s), 7.35 (2H, d, J=8.8Hz), 7.68 (2H, d, J=8.8Hz), 7.71 (2H, d, J=8.8Hz), 7.80 (2H, d, J=8.8Hz), 7.87-7.93 (5H, m), 8.36 (1H, dd, J=2.9, 8.8Hz), 8.49 (1H, d, J=2.9Hz), 10.25 (1H, s), 10.79 (1H, s);

MS(FAB) m/z: 468 $(M + H)^+$;

[Example 123] N-[4-(Pyridin-3-yl-carbonylamino)phenyl]-(2-chloro-5-nitrophenyl)carboxamide

The title compound (0.149 g, yield 38%) was obtained according to the procedure described in Example 2 using N-(4-aminophenyl)-(2-chloro-5-nitrophenyl)carboxamide mono-hydrochloride (0.291 g, 1.0 mmol) prepared in Example 58, DMA (3 ml) and nicotinoyl chloride hydrochloride (0.336 g, 1.9 mmol).
Rf 0.29 [methylene chloride:methanol=7:1 (v/v)];
$^1$H-NMR (400MHz, DMSO-$d_6$, TMS): δ 7.58 (1H, dd, J=2.9, 8.0Hz), 7.71 (2H, d, J=8.8Hz), 7.78 (2H, d, J=8.8Hz), 7.90 (1H, d, J=8.8Hz), 8.31 (1H, ddd, J=1.5, 2.2, 8.0Hz), 8.35 (1H, dd, J=2.9, 8.8Hz), 8.48 (1H, d, J=2.9Hz), 8.77 (1H, dd, J=1.5, 5.1Hz), 9.12 (1H, d, J=2.2Hz), 10.48 (1H, s), 10.73 (1H, s);
MS(EI) m/z: 396 $M^+$;

[Example 124] N-[4-[4-(Pyridin-3-yl-carbonylamino)phenyl]phenyl]-(2-chloro-5-nitrophenyl)carboxamide

The title compound (0.263 g, yield 74%) was obtained according to the procedure described in Example 2 using N-[4-(4-aminophenyl)phenyl]-(2-chloro-5-nitrophenyl)carboxamide mono-hydrochloride (0.303 g, 0.75 mmol) prepared in Example 88, DMA (3 ml), triethylamine (0.314 ml, 2.25 mmol) and nicotinoyl chloride hydrochloride (0.294 g, 1.65 mmol).
Rf 0.55 [methylene chloride:methanol=7:1 (v/v)];
$^1$H-NMR (400MHz, DMSO-$d_6$, TMS): δ 7.59 (1H, dd, J=2.9, 7.3Hz), 7.70-7.74 (4H, m), 7.80 (2H, d, J=8.8Hz), 7.89 (2H, d, J=8.8Hz), 7.91 (1H, d, J=8.8Hz), 8.32 (1H, ddd, J=2.2, 2.2, 8.1Hz), 8.36 (1H, dd, J=2.9, 8.8Hz), 8.50 (1H, d, J=2.9Hz), 8.78 (1H, m), 9.14 (1H, d, J=1.5Hz), 10.54 (1H, s), 10.81 (1H, s);
MS(EI) m/z: 472 $(M + H)^+$;

[Example 125] N-[4-(Pyridin-4-yl-carbonylamino)phenyl]-(2-chloro-5-nitrophenyl)carboxamide

The title compound (0.216 g, yield 59%) was obtained according to the procedure described in Example 2 using N-(4-aminophenyl)-(2-chloro-5-nitrophenyl)carboxamide mono-hydrochloride (0.291 g, 1.0 mmol) prepared in Example 58, DMA (3 ml) and isonicotinoyl chloride hydrochloride (0.356 g, 2.0 mmol).
Rf 0.48 [methylene chloride:methanol=7:1 (v/v)];
$^1$H-NMR (400MHz, DMSO-$d_6$, TMS): δ 7.71 (2H, d, J=8.8Hz), 7.79 (2H, d, J=8.8Hz), 7.88 (2H, d, J=5.9Hz), 7.90 (1H, d, J=8.8Hz), 8.34 (1H, dd, J=2.9, 8.8Hz), 8.48 (1H, d, J=2.9Hz), 8.79 (1H, d, J=5.9Hz), 10.55 (1H, s), 10.74 (1H, s);
MS(EI) m/z: 396 $M^+$;

[Example 126] N-[4-[4-(Pyridin-4-yl-carbonylamino)phenyl]phenyl]-(2-chloro-5-nitrophenyl)carboxamide

The title compound (0.256 g, yield 72%) was obtained according to the procedure described in Example 2 using N-[4-(4-aminophenyl)phenyl]-4-yl)-(2-chloro-5-nitrophenyl)carboxamide mono-hydrochloride (0.303 g, 0.75 mmol) prepared in Example 88, DMA (3 ml), triethylamine (0.314 ml, 2.25 mmol) and isonicotinoyl chloride hydrochloride (0.267 g, 1.5 mmol).
Rf 0.53 [methylene chloride:methanol=7:1 (v/v)];
$^1$H-NMR (400MHz, DMSO-$d_6$, TMS): δ 7.70-7.74 (4H, m), 7.81 (2H, d, J=8.8Hz), 7.87-7.93 (5H, m), 8.36 (1H, dd, J=2.9, 8.8Hz), 8.50 (1H, d, J=2.9Hz), 8.81 (2H, d, J=6.6Hz), 10.60 (1H, s), 10.81 (1H, s);
MS(EI) m/z: 472 $M^+$;

[Example 127] N-(4-Benzenesulfonylaminophenyl)-(2-chloro-5-nitrophenyl)carboxamide

The title compound (0.139 g, yield 32%) was obtained according to the procedure described in Example 2 using N-(4-aminophenyl)-(2-chloro-5-nitrophenyl)carboxamide mono-hydrochloride (0.291 g, 1.0 mmol) prepared in Example 58, DMA (3 ml), triethylamine (0.279 ml, 2.0 mmol) and benzenesulfonyl chloride (0.153 ml, 1.2 mmol).
Rf 0.68 [methylene chloride:methanol=7:1 (v/v)];
$^1$H-NMR (400MHz, DMSO-$d_6$, TMS): δ 7.09 (2H, d, J=8.8Hz), 7.53-7.62 (5H, m), 7.75 (2H, d, J=7.3Hz), 7.87 (1H, d, J=8.8Hz), 8.32 (1H, dd, J=2.9, 8.8Hz), 8.42 (1H, d, J=2.9Hz), 10.21 (1H, s), 10.64 (1H, s);
MS(FAB) m/z: 432 $(M + H)^+$;

[Example 128] N-[4-(Benzenesulfonylaminophenyl)phenyl]-(2-chloro-5-nitrophenyl)carboxamide

The title compound (0.308 g, yield 75%) was obtained according to the procedure described in Example 2 using N-[4-(4-aminophenyl)phenyl]-(2-chloro-5-nitrophenyl)carboxamide mono-hydrochloride (0.303 g, 0.75 mmol) prepared in Example 88, DMA (3 ml), triethylamine (0.209 ml, 1.5 mmol) and benzenesulfonyl chloride (0.119 ml, 0.9 mmol).
Rf 0.70 [methylene chloride:methanol=7:1 (v/v)];
$^1$H-NMR (400MHz, DMSO-$d_6$, TMS): δ 7.18 (2H, d, J=8.8Hz), 7.53-7.95 (12H, m), 7.89 (1H, d, J=8.8Hz), 8.35 (1H, dd, J=2.9, 8.8Hz), 8.48 (1H, d, J=2.9Hz), 9.40 (1H, br), 10.76 (1H, s);
MS(FAB) m/z: 508 $(M + H)^+$;

[Example 129] N-(4-Ethanesulfonylaminophenyl)-(2-chloro-5-nitrophenyl)carboxamide

Ethanesulfonyl chloride (0.153 ml, 1.2 mmol) was added to a solution ofN-(4-aminophenyl)-(2-chloro-5-nitrophenyl)carboxamide mono-hydrochloride (0.291 g, 1.0 mmol) prepared in Example 58 in pyridine (3 ml). The mixture was stirred at room temperature for 6 hours. Saturated aqueous sodium bicarbonate solution (3 ml), water (20 ml) and ethyl acetate (1 ml) were added to the reaction mixture. The insoluble material thus formed was filtered, washed with water and diisopropyl ether and then dried in vacuo to afford the title compound (0.254 g, yield 66%).
Rf 0.34 [methylene chloride:methanol=20:1 (v/v)];
$^1$H-NMR (400MHz, DMSO-$d_6$, TMS): δ 1.20 (3H, t, J=7.3Hz), 3.06 (2H, q, J=7.3Hz), 7.23 (2H, d, J=8.8Hz), 7.65 (2H, d, J=8.8Hz), 7.89 (1H, d, J=8.8Hz), 8.34 (1H, dd, J=2.9, 8.8Hz), 8.45 (1H, d, J=2.9Hz), 9.73 (1H, s), 10.69 (1H, s);
MS(FAB) m/z: 384 $(M + H)^+$;

[Example 130] N-[4-(4-Ethanesulfonylaminophenyl)phenyl]-(2-chloro-5-nitrophenyl)carboxamide

The title compound (0.267 g, yield 77%) was obtained according to the procedure described in Example 50 using N-[4-(4-aminophenyl)phenyl]-(2-chloro-5-nitrophenyl)carboxamide mono-hydrochloride (0.303 g, 0.75 mmol) prepared in Example 88, DMA (3 ml), and ethanesulfonyl chloride (0.085 ml, 0.90 mmol).
Rf 0.32 [methylene chloride:methanol=20:1 (v/v)];
$^1$H-NMR (400MHz, DMSO-$d_6$, TMS): δ 1.21 (3H, t, J=7.3Hz), 3.12 (2H, q, J=7.3Hz), 7.30 (2H, d, J=8.8Hz), 7.65 (2H, d, J=8.8Hz), 7.67 (2H, d, J=8.8Hz), 7.79 (2H, d, J=8.8Hz), 7.91 (1H, d, J=8.8Hz), 8.35 (1H, dd, J=2.9, 8.8Hz), 8.49 (1H, d, J=2.9Hz), 9.88 (1H, s), 10.80 (1H, s);
MS(FAB) m/z: 460 $(M + H)^+$;

[Example 131] N-[4-(Diethylamino)-2-methylphenyl)-(2-chloro-5-nitrophenyl)carboxamide

The title compound (0.431 g, yield 60%) was obtained according to the procedure described in Example 2 using 2-amino-5-(diethylamino)toluene hydrochloride (a product of Tokyo-kasei, 0.429 g, 2.0 mmol), DMA (5 ml), triethylamine (0.335 ml, 2.4 mmol) and 2-chloro-5-nitrobenzoyl chloride (0.528 g, 2.4 mmol).
$^1$H-NMR (400MHz, DMSO-$d_6$, TMS): δ 1.09 (6H, t, J=7.0Hz), 2.22 (3H, s), 3.33 (4H, m), 6.53 (2H, m), 7.17 (1H, d, J=8.8Hz), 7.87 (1H, d, J=8.8Hz), 8.32 (1H, dd, J=2.9, 8.8Hz), 8.39 (1H, d, J=2.9Hz), 9.86 (1H, s);
MS(FAB) m/z: 361 $M^+$;

[Example 132] N-[4-(4-tert-Butoxycarbonylpiperazin-1-yl)phenyl]-(2-chloro-5-nitrophenyl)carboxamide

The title compound (7.62 g, yield 90%) was obtained according to the procedure described in Example 2 using tert-butyl 4-(4-aminophenyl)piperazin-1-carboxylate [Tetrahedron Lett., 41, 385 (2000)] (5.08 g, 18.3 mmol), DMA (50 ml), and 2-chloro-5-nitrobenzoyl chloride (4.84 g, 22.0 mmol).
Rf 0.56 [hexane:ethyl acetate=1:1 (v/v)];
$^1$H NMR($CDCl_3$, 400MHz,TMS): δ 1.49 (9H, s), 3.13 (4H, t, J=5.1Hz), 3.59 (4H, t, J=5.1Hz), 6.94 (2H, d, J=8.8Hz), 7.53 (2H, d, J=8.8Hz), 7.64 (1H, d, J=8.8Hz), 7.86 (1H, br), 8.24 (1H, dd, J=2.9, 8.8Hz), 8.59 (1H, d, J=2.9Hz);
MS(FAB) m/z: 460 $M^+$;

[Example 133] N-[4-(Piperazin-1-yl)phenyl]-(2-chloro-5-nitrophenyl)carboxamide

A suspension of N-[4-(4-tert-butoxycarbonylpiperazin-1-yl)phenyl]-{2-chloro-5-nitrophenyl)carboxamide (6.15 g, 13.3 mmol) prepared in Example 132 in 2N hydrogen chloride/1,4-dioxane solution (40 ml) was stirred for 20 hours. The reaction mixture was diluted with ethyl ether and insoluble material was filtered, washed with 1,4-dioxane

and ethyl ether. The obtained solid was dried in vacuo to afford the title compound (5.17 g, yield 90%).
$^1$H-NMR (400MHz, DMSO-$d_6$, TMS): δ 3.23 (4H, m), 3.37 (4H, m), 7.04 (2H, d, J=8.8Hz), 7.62 (2H, d, J=8.8Hz), 7.88 (1H, d, J=8.8Hz), 8.33 (1H, dd, J=2.9, 8.8Hz), 8.42 (1H, d, J=2.9Hz), 9.40 (1H, m), 10.62 (1H, s);
MS(FAB) m/z: 361 $(M + H)^+$;

[Example 134] N-[4-(4-Acetoxyphenyl)phenyl]-(2-chloro-5-nitrophenyl)carboxamide [134a] 4-(4-Acetoxyphenyl)aniline

A 5% palladium on carbon catalyst (0.18 g) was added to a solution of 4-(4-acetoxyphenyl)nitrobenzene [J. Am. Chem. Soc., 66, 1245 (1944)] (0.860 g, 3.34 mmol) in ethanol (9 ml). The mixture was stirred at room temperature for 2 hours. The catalyst was filtered off and the filtrate was concentrated under reduced pressure. The resulting residue was purified by chromatography on a silica gel column [hexane:ethyl acetate=1:1] to afford the title compound (0.699 g, yield 92 %).
Rf 0.11 [hexane:ethyl acetate=3:1 (v/v)]
$^1$H-NMR (400MHz, DMSO-$d_6$, TMS): δ 2.32 (3H, s), 3.73 (2H, br), 6.75 (2H, d, J=8.6Hz), 7.11 (2H, d, J=8.7Hz), 7.38 (2H, d, J=8.6Hz), 7.52 (2H, d, J=8.7Hz); [134b] N-[4-(4-Acetoxyphenyl)phenyl]-(2-chloro-5-nitrophenyl)carboxamide
The title compound (1.03 g, yield 82%) was obtained according to the procedure described in Example 2 using 4-(4-acetoxyphenyl)aniline (0.694 g, 3.05 mmol) prepared in Example 134a, DMA (7 ml), and 2-chloro-5-nitrobenzoyl chloride (0.873 g, 3.97 mmol).
Rf 0.16 [hexane:ethyl acetate=2:1 (v/v)];
$^1$H-NMR (400MHz, DMSO-$d_6$/TMS): δ 2.30 (3H, s), 7.22 (2H, d, J=8.8Hz), 7.71 (2H, d, J=8.8Hz), 7.71 (2H, d, J=8.8Hz), 7.81 (2H, d, J=8.8Hz), 7.91 (1H, d, J=8.8Hz), 8.36 (1H, dd, J=2.9, 8.8Hz), 8.49 (1H, d, J=2.9Hz), 10.82 (1H, s);
MS(FAB) m/z: 411 $(M + H)^+$;

[Example 135] N-[4-(4-Hydroxyphenyl)phenyl]-2-(chloro-5-nitrophenyl)carboxamide

A small amount of a solution of sodium methoxide (25wt%) in methanol was added to a suspension of N-[4-(4-acetoxyphenyl)phenyl]-(2-chloro-5-nitrophenyl)carboxamide (0.882 g, 2.14 mmol) in methanol (18 ml). The mixture was stirred for 22 hours. The reaction mixture was adjusted to pH 4 with the addition of acetic acid. The material thus formed was filtered, washed with methanol and then dried in vacuo to afford the crude title compound (0.728 g). The crude title compound was dissolved in ethanol by heating and the solution was allowed to stand for 3 days. The solid thus formed was filtered off and the solvent of the filtrate was removed. The residue was dried in vacuo to afford the title compound (0.269 g, yield 34%).
Rf 0.09 [hexane:ethyl acetate=1:1 (v/v)];
$^1$H-NMR (400MHz, DMSO-$d_6$, TMS): δ 6.85 (2H, d, J=8.8Hz), 7.49 (2H, d, J=8.8Hz), 7.60 (2H, d, J=8.8Hz), 7.75 (2H, d, J=8.8Hz), 7.90 (1H, d, J=8.8Hz), 8.35 (1H, dd, J=2.9, 8.8Hz), 8.48 (1H, d, J=2.9Hz), 9.53 (1H, s), 10.75 (1H, s);
MS(FAB) m/z: 369 $(M + H)^+$;

[Example 136] N-(4-Methanesulfonylaminophenyl)-(2-chloro-5-nitrophenyl)carboxamide

Triethylamine (0.209 ml, 1.5 mmol) and methanesulfonyl chloride (0.116 ml, 1.5 mmol) were added to a solution ofN-(4-aminophenyl)-(2-chloro-5-nitrophenyl)carboxamide mono-hydrochloride (0.291 g, 1.0 mmol) prepared in Example 58 in DMA (5 ml). The mixture was stirred at room temperature for 20 hours. Saturated aqueous sodium bicarbonate solution (1.5 ml) and water (15 ml) were added to the reaction mixture. The insoluble material thus formed was filtered, washed with water and diisopropyl ether and then dried in vacuo to afford the title compound (0.210 g, 57%).
Rf 0.64 [methylene chloride:methanol=7:1 (v/v)];
$^1$H-NMR (400MHz, DMSO-$d_6$, TMS): δ 2.97 (3H, s), 7.23 (2H, d, J=8.8Hz), 7.67 (2H, d, J=8.8Hz), 7.89 (1H, d, J=8.8Hz), 8.34 (1H, dd, J=2.9, 8.8Hz), 8.45 (1H, d, J=2.9Hz), 9.65 (1H, s), 10.71 (1H, s);
MS(FAB) m/z: 370 $(M + H)^+$;

[Example 137] N-(4-n-Hexylaminophenyl)-(2-chloro-5-nitrophenyl)carboxamide

The title compound (0.247 g, yield 33%) was obtained as the more polar compound according to the procedure described in Example 115 using N-(4-aminophenyl)-(2-chloro-5-nitrophenyl)carboxamide mono-hydrochloride (0.583 g, 2.0 mmol) prepared in Example 58, methanol (10 ml), sodium cyanoborohydride (138 mg, 1.1 mmol) and n-hexanal (0.288 ml, 2.4 mmol).
Rf 0.56 [hexane:ethyl acetate=1:1 (v/v)];
$^1$H NMR($CDCl_3$, 400MHz, TMS): δ 0.91 (3H, t, J=7.3Hz), 1.30-1.65 (8H, m), 3.12 (2H, t, J=7.3Hz), 6.62 (2H, d, J=6.6Hz), 7.40 (2H, d, J=6.6Hz), 7.64 (1H, d, J=8.8Hz), 7.66 (1H, br), 8.24 (1H, dd, J=2.2, 8.8Hz), 8.60 (1H, d, J=2.2Hz);

MS(FAB) m/z: 375 $M^+$;

[Example 138] N-[4-(N,N-di-n-Hexylamino)phenyl]-(2-chloro-5-nitrophenyl)carboxamide

The title compound (0.162 g, yield 18%) was obtained as the less polar compound in Example 137.
Rf 0.79 [hexane:ethyl acetate=1:1 (v/v)]
$^1$H NMR($CDCl_3$, 400MHz, TMS): δ 0.90 (6H, m), 1.26-1.38 (12H, m), 1.52-1.62 (4H, m), 3.26 (4H, t, J=7.3Hz), 6.63 (2H, d, J=9.2Hz), 7.42 (2H, d, J=9.2Hz), 7.62 (1H, br), 7.63 (1H, d, J=8.8Hz), 8.24 (1H, dd, J=2.9, 8.8Hz), 8.60 (1H, d, J=2.9Hz);

[Example 139] N-[4-(3,5-di-tert-Butyl-4-hydroxyphenyl)thiazol-2-yl]-(2-chloro-5-nitrophenyl)carboxamide

[139a] 2-Amino-5-(3,5-di-tert-butyl-4-hydroxyphenyl)thiazole

Thiourea (4.56 g, 60 mmol) was added to a solution of 1-(3,5-di-tert-butyl-4-hydroxyphenyl)-2-bromoethan-1-one (9.81 g, 30mmol) in acetone (50 ml). The mixture was stirred overnight. The reaction mixture was concentrated and saturated aqueous sodium bicarbonate solution and hexane were added to the residue. The mixture was stirred and the resulting solid was filtered, washed with water and dried in vacuo to afford the title compound (8.92 g, crude yield 98%).
$^1$H-NMR (400MHz, DMSO-$d_6$, TMS): δ 1.40 (9H, s), 6.71 (1H, s,), 6.96 (2H, s), 6.97 (1H, s,), 7.52 (2H, s,);

[139b] N-[4-(3,5-di-tert-Butyl-4-hydroxyphenyl)-thiazol-2-yl]-(2-chloro-5-nitrophenyl)carboxamide

The title compound (0.446 g, yield 88%) was obtained according to the procedure described in Example 2 using 2-amino-5-(3,5-di-tert-butyl-4-hydroxyphenyl)thiazole (0.32 g, 1.03 mmol) prepared in Example 139a, DMA (5 ml) and 2-chloro-5-nitrobenzoyl chloride (0.25 g, 1.13 mmol).
$^1$H-NMR (400MHz, DMSO-$d_6$, TMS): δ 1.42 (9H, s), 7.11 (1H, s), 7.53 (1H, s), 7.65 (1H, s), 7.90 (1H, d, J=8.9Hz), 8.37 (1H, dd, J=2.8, 8.9Hz), 8.59 (1H, d, J=2.8);

[Example 140] N-(Pyrazin-2-yl)-(2-chloro-5-nitrophenyl)carboxamide

The reaction was carried out according to the procedure described in Example 1 using 2-aminopyrazine (0.279 g, 3.13 mmol), DMA (5 ml), and 2-chloro-5-nitrobenzoyl chloride (0.833 g, 3.79 mmol). Saturated aqueous sodium bicarbonate solution (10 ml), water (10 m1) and ethyl acetate (20 ml) were added to the reaction mixture and the mixture was partitioned. The organic layer was separated, washed with saturated aqueous sodium chloride solution (10 ml×2), dried over anhydrous sodium sulfate and then the solvent was removed under reduced pressure to afford a crude title compound. The obtained crude title compound was purified by chromatography on a silica gel column [methylene chloride: ethyl acetate= 1:5 (v/v)] to afford the title compound (0.27 mg). The compound was solidified with diisopropyl ether and the resulting solid was filtered and then dried to afford the title compound (0.18 g, yield 21%).
$^1$H NMR($CDCl_3$, 400MHz, TMS): δ 7.71 (1H, d, J=8.8Hz), 8.31-8.35 (2H, m), 8.47 (1H, d, J=2.5Hz) δ 8.62 (1H, bs), δ 8.68 (1H, d, J=2.7Hz), δ 9.68 (1H, bs;

[Example 141] N-(6-Methybenzothiazol-2-yl)-(2-chloro-5-nitrophenyl)carboxamide

DPPA (0.44 ml, 2.0 mmol), triethylamine (0.28 ml, 2.0 mmol) and 2-chloro-5-nitrobenzoic acid (0.37 g, 1.82 mmol) were added to a solution of 2-amino-6-methylbenzothiazole (0.30 g, 1.80 mmol) in DMA (5 ml). The mixture was stirred at room temperature overnight. The reaction mixture was treated according to a similar procedure to that described in Example 62 to afford the crude title compound. The obtained crude compound was purified by chromatography on a silica gel column [hexane:ethyl acetate= 5:1 (v/v)] to yield the title compound (0.19 mg). The compound was solidified with diisopropyl ether and the resulting solid was filtered and then dried to afford the title compound (0.14 g, yield 21%).
$^1$H-NMR (400MHz, DMSO-$d_6$, TMS): δ 2.44 (3H, s), δ 7.30 (1H, d, J=8.3Hz), δ 7.69 (1H, d, J=8.3Hz), δ 7.84 (1H, s), δ 7.92 (1H, d, J=8.9Hz), 8.39 (1H, dd, J=2.8, 8.9Hz), 8.64 (1H, d, J=2.8Hz);

[Example 142] N-[3-(4-Tolylaminosulfonyl)phenyl]-(2-chloro-5-nitrophenyl)carboxamide

The title compound (0.65 g, yield 96%) was obtained according to the procedure described in Example 2 using 3-(4-tolylaminosulfonyl)aniline (0.40 g, 1.52 mmol), DMA (5 ml) and 2-chloro-5-nitrobenzoyl chloride (0.39 g).

$^1H$ NMR ($CDCl_3$, 400MHz, TMS): δ 2.78 (3H, s), 6.62 (1H, s), 6.95 (2H, d, J=8.4Hz), 7.05 (2H, d, J=8.4Hz), 7.43-7.50 (2H, m), 7.66 (1H, d, J=8.8Hz), 7.94 (1H, s), 8.02-8.05 (1H, m), 8.26-8.30 (2H, m), 8.58 (1H, d, J=2.7Hz);

[Example 143] Methyl 3-(2-chloro-5-nitrobenzoylamino)benzoate

The title compound (10.89 g, yield 93%) was obtained according to the procedure described in Example 2 using methyl N-[3-(methoxycarbonyl)phenyl]-(2-chloro-5-nitrophenyl)carboxamide-3-aminobenzoate (5.3 g, 35 mmol), DMA (50 ml), and 2-chloro-5-nitrobenzoyl chloride (8.49 g).
$^1H$ NMR ($CDCl_3$, 400MHz, TMS): δ 3.94 (3H, s), 7.51 (1H, t, J=7.9Hz), 7.68 (1H, d, J=8.8Hz), 7.89 (1H, d, J=7.9Hz), 8.04 (1H, d, J=8.8Hz), 8.15 (1H, bs), 8.29 (1H, dd, J=8.8, 2.7Hz), 8.64 (1H, d, J=2.7Hz);

[Example 144] N-[4-(4-Tolylaminosulfonyl)phenyl]-(2-chloro-5-nitrophenyl)carboxamide

The title compound (0.375 g, yield 84%) was obtained according to the procedure described in Example 2 using 4-(4-tolylaminosulfonyl)aniline (0.263 g, 1.00 mmol), DMA (5 ml), and 2-chloro-5-nitrobenzoyl chloride (0.243 g).
$^1H$ NMR ($CDCl_3$, 400MHz, TMS): δ 2.29 (3H, s), 6.36 (1H, s), 6.96 (2H, d, J=8.3Hz), 7.07 (2H, d, J=8.3Hz), 7.67-7.70 (5H, m), 8.09 (1H, m), 8.30 (1H, dd, J=2.7, 8.8Hz), 8.61 (1H, d, J=2.7Hz);

[Example 145] 3-(2-Chloro-5-nitrobenzoylamino)benzoic acid

1N Aqueous sodium hydroxide solution (46 ml) was added to a solution of methyl 3-(2-chloro-5-nitrobenzoylamino)benzoate (10.32 g, 30.8 mmol) in dioxane (100 ml). The mixture was stirred for 24 hours. The reaction mixture was concentrated and 1N hydrochloric acid (50 ml) was added dropwise to the resulting residue with stirring under cooling with an ice-water bath. The resulting crystalline solid was filtered, washed with water and then dried to afford the title compound (9.72 g, yield 98%).
$^1H$-NMR (400MHz, DMSO-$d_6$, TMS): δ 7.51 (1H, t, J=8.0Hz), 7.71-7.74 (1H, m), 7.89-7.92 (2H, m), 8.33-8.42 (2H, m), 8.52 (1H, d, J=2.7Hz), 10.90 (1H, s);

[Example 146] N-(Pyridin-4-yl)-(2-chloro-5-nitrophenyl)carboxamide hydrochloride

(Pyridin-4-yl)-(2-chloro-5-nitrophenyl)carboxamide (0.788 g) was obtained according to the procedure described in Example 1 using 4-aminopyridine (0.421 g), DMA (10 ml) and 2-chloro-5-nitrobenzoyl chloride (1.08 g). 4N Hydrogen chloride/dioxane solution was added dropwise to a solution of (pyridin-4-yl)-(2-chloro-5-nitrophenyl)carboxamide (0.657 g, 2.37 mmol) prepared above in dioxane (10 ml). After 1 hour, ether (10 ml) was added to the reaction mixture. The resulting crystalline solid was filtered and dried to afford the title compound (0.73 g, yield 98%).
$^1H$-NMR (400MHz, DMSO-$d_6$, TMS): δ 7.96 (1H, d, J=8.8Hz), 8.20 (2H, d, J=7.0Hz), 8.42 (1H, dd, J=2.8, 8.8Hz), 8.68 (1H, d, J=2.8Hz), 8.80 (2H, d, J=7.0Hz), 12.25 (1H, s);

[Example 147] N-[4-(Pyridin-2-yl)phenyl]-(2-chloro-5-nitrophenyl)carboxamide hydrochloride

N-[4-(Pyridin-2-yl)phenyl]-(2-chloro-5-nitrophenyl)carboxamide (0.388 g) was obtained as a crystal according to the procedure described in Example 68 using 4-[(pyridin-2-yl)phenyl]aniline hydrochloride (0.320 g), pyridine (5 ml) and 2-chloro-5-nitrobenzoyl chloride (0.347 g). The title compound (0.57 g, 99%) was afforded by a similar procedure to that described in example 68 using N-[4-(pyridin-2-yl)phenyl]-(2-chloro-5-nitrophenyl)carboxamide (0.522 g, 1.474 mmol) obtained above, dioxane (10 ml) and 4N hydrogen chloride/dioxane solution (0.46 ml).
$^1H$-NMR (400MHz, DMSO-$d_6$, TMS): δ 7.6-7.73 (1H, m), 7.92 (2H, d, J=8.8Hz), 7.93 (2H, d, J=8.8), 8.16 (2H, d, J=8.8Hz), 8.23-8.26 (1H, m), 8.30-8.34 (1H, m), 8.37 (1H, dd, J=2.8, 8.8Hz), 8.53 (1H, d, J=2.8Hz) 8.77-8.79 (1H ,m), 11.09 (1H, s);

[Example 148] N-[4-(Pyridin-3-ylaminocarbonyl)phenyl]-(2-chloro-5-nitrophenyl)carboxamide

[148a] N-(4-Ethoxycarbonylphenyl)-(2-chloro-5-nitrophenyl)carboxamide

The title compound (20.33 g) was obtained as a crystalline solid according to the procedure described in Example 2 using ethyl 4-aminobenzoate (9.88 g), DMA (50 ml), and 2-chloro-5-nitrobenzoyl chloride (14.47 g).
$^1H$-NMR (400MHz, DMSO-$d_6$, TMS): δ 1.33 (1H, t, J=7.1Hz), 4.31 (2H, q, J=7.1Hz), 7.85 (2H, d, J=8.7Hz), 7.91 (1H, d, J=8.8Hz), 7.99 (2H, d, 8.7Hz), 8.36 (1H, dd, J=8.9, 2.8Hz), 8.53 (1H, d, J=2.8Hz), 11.05 (1H, s).

[148b] 4-[(2-Chloro-5-nitrophenyl)carbonylamino]benzoic acid

1N Aqueous sodium hydroxide solution (84 ml) was added to a solution of N-(4-ethoxycarbonylphenyl)-(2-chloro-5-nitrophenyl)carboxamide (19.55 g) prepared in Example 148a in dioxane (100 ml). The mixture was allowed to stand at room temperature for 3 days. After concentration of the reaction mixture under reduced pressure, water (300 ml) was added to the residue. 1N Hydrochloric acid (90 ml) was added dropwise to the resulting mixture under cooling with an ice-water bath and the mixture was stirred. The resulting crystalline solid was filtered and dried to afford the title compound (17.59 g).
$^1$H-NMR (400MHz, DMSO-$d_6$, TMS): δ 7.82 (2H, d, J=8.7Hz), 7.91 (1H, d, J=8.8Hz), 7.97 (2H, d, 8.7Hz), 8.36 (1H, dd, J=8.8 and 2.8Hz), 8.52 (1H, d, J=2.8Hz), 11.01 (1H, s).

[148c] N-[4-(Pyridin-3-ylaminocarbonyl)phenyl]-(2-chloro-5-nitrophenyl)carboxamide

WSC (0.39 g) and 3-aminopyridine (0.15 g) were added to a solution of 4-[(2-chloro-5-nitrophenyl)carbonylamino]benzoic acid (0.523 g, 1.63 mmol) in DMF (10 ml). The mixture was stirred at room temperature for 4 hours. Saturated aqueous sodium bicarbonate solution (30 ml) and ethyl acetate (50 ml) were added to the reaction mixture and the mixture was partitioned. The organic layer was separated, washed with saturated aqueous sodium chloride (20 ml×2), dried over anhydrous sodium sulfate and then the solvent was removed under reduced pressure to afford the crude title compound. The obtained crude compound was purified by chromatography on a silica gel column [ethylene chloride:methanol=20:1 (v/v)]. The compound was solidified with ethyl acetate, filtered and dried to afford the title compound (0.30 g, yield 46%).
$^1$H-NMR (400MHz, DMSO-$d_6$, TMS): δ 7.40 (1H, dd, J=4.7, 8.3Hz), 7.87 (1H, d, J=8.7Hz), 7.92 (1H, d, J=8.8Hz), 8.04 (2H, d, J=8.7Hz), 8.18-8.22 (1H, m), 8.32 (1H, dd, J=4.7, 1.4Hz), 8.37 (1H, dd, J=2.7, 8.8Hz), 8.54 (1H, d ,J=2.7Hz), 8.94 (1H, d, J=2.4Hz), 10.39 (1H, s), 11.02 (1H, s);

[Example 149] N-[2-(4-Methylbenzoylamino)benzothiazol-6-yl]-(2-chloro-5-nitrophenyl)carboxamide

[149a] 2-(4-Methylbenzoylamino)-6-nitrobenzothiazole

The title compound (9.88 g, yield 96%) was obtained according to the procedure described in Example 2 using 2-amino-6-nitrobenzothiazole (6.44 g, 33 mmol), DMA (45 ml) and 4-methylbenzoyl chloride (4.80 ml).
$^1$H-NMR (400MHz, DMSO-$d_6$, TMS): δ 2.41 (3H, s), 7.39 (2H, d, J=8.2Hz), 7.90 (1H, d, J=8.9Hz), 8.08 (2H, d, J=8.2Hz), 8.25 (1H, bs), 8.30 (1H, dd, J=2.4, 8.9Hz), 9.06 (1H, d, J=2.4Hz).

[149b] 6-Amino-2-(4-methylbenzoylamino)benzothiazole

Nickel (II) chloride hexahydrate (4.85 g) and sodium borohydride (1.55 g) were added to a solution of 2-(4-methylbenzoylamino)-6-nitrobenzothiazole (3.20 g, 10.2 mmol) prepared in Example 149a in THF (150 ml) under cooling with an ice-water bath. The reaction mixture was stirred for 30 minutes. The reaction mixture was concentrated. Ethyl acetate (200 ml) and saturated aqueous sodium bicarbonate solution (200 ml) were added to the resulting residue. The mixture was stirred for 30 minutes and insoluble material was filtered off. The filtrate was partitioned and the organic layer was separated, washed with saturated aqueous sodium chloride solution (20 ml×2) and dried over anhydrous sodium sulfate. The solvent was removed under reduced pressure to give the crude title compound. The crude compound was solidified with isopropyl ether, filtered and dried to afford the title compound (1.50 g, yield 52%).
$^1$H-NMR (400MHz, DMSO-$d_6$, TMS): δ 2.40 (3H, s), 5.20 (2H, bs), 6.74 (1H, dd, J=2.2, 8.6Hz), 7.04 (1H, d, J=2.2Hz), 7.36 (2H, d, J=8.2Hz), 7.45 (1H, d, J=8.6Hz), 8.01 (2H, d, J=8.2,Hz);

[149c] N-[2-(4-Methylbenzoylamino)benzothiazol-6-yl]-(2-chloro-5-nitrophenyl)carboxamide

The title compound (0.41 g, yield 86%) was obtained according to the procedure described in Example 2 using 6-amino-2-(4-methylbenzoylamino)benzothiazole (0.30 g, 1.04 mmol) prepared in Example 149b, DMA (5 ml) and 2-chloro-5-nitrobenzoyl chloride (0.26 g, 1.20 mmol).
$^1$H-NMR (400MHz, DMSO-$d_6$, TMS): δ 2.40 (3H, s), 7.36 (2H, d, J=8.1Hz), 7.64 (1H, dd, J=2.0, 8.7Hz), 7.75 (1H, d , J=8.7Hz), 7.91 (1H, d, J=8.9Hz), 8.05 (2H, d, J=8.1Hz), 8.36 (1H, dd, J=2.8, 8.9Hz), 8.43 (1H, d, J=2.0Hz), 8.50 (1H, d, J=2.8Hz), 10.86 (1H, s);
MS(FAB) m/z: 000 $(M + H)^+$;

[Example 150] N-[4-(4-Ethylbenzenesulfonylamino)phenyl]-(2-chloro-5-nitrophenyl)carboxamide

The title compound (0.56 g, yield 90%) was obtained according to the procedure described in Example 2 using 4-(4-ethylbenzenesulfonylamino)aniline (0.37 g, 1.35 mmol), DMA (5 ml) and 2-chloro-5-nitrobenzoyl chloride (0.33 g, 1.48 mmol).
Rf 0.00 [hexane:ethyl acetate=9:1 (v/v)];
$^1$H-NMR (400MHz, DMSO-$d_6$, TMS): δ 1.16 (3H, t, J=7.6Hz), 2.65 (2H, q, J=7.6Hz), 7.10 (2H, d, J=8.9Hz), 7.39 (2H, d, J=8.4Hz), 7.55 (2H, d, J=8.9Hz), 7.66 (2H, d, J=8.4Hz), 7.87 (1H, d, J=8.8Hz), 8.32 (1H, dd, J=2.7, 8.8Hz), 8.42 (1H, d, J=2.7Hz), 10.16 (1H, s), 10.63 (1H, s);

[Example 151] N-[4-(Piperidylsulfonyl)phenyl]-(2-chloro-5-nitrophenyl)carboxamide

The title compound (0.32 g, yield 68%) was obtained according to the procedure described in Example 2 using 4-(piperidylsulfonyl)aniline (0.27 g, 1.12 mmol), DMA (5 ml) and 2-chloro-5-nitrobenzoyl chloride (0.27 g, 1.24 mmol).
$^1$H-NMR (400MHz, DMSO-$d_6$, TMS): δ 1.36-1.39 (2H, m), 1.52-1.58 (4H, m), 2.88 (4H ,t, J=5.3Hz), 7.75 (2H, d, J=8.9Hz), 7.92 (1H, d, J=8.8Hz), 7.94 (2H, d, J=8.8Hz), 8.37 (1H, dd, J=2.7, 8.9Hz), 8.54 (1H, d, J=2.7Hz);

[Example 152] N-[4-(Pyrrolidylsulfonyl)phenyl]-(2-chloro-5-nitrophenyl)carboxamide

The title compound (0.46 g, yield 94%) was obtained according to the procedure described in Example 2 using 4-(pyrrolidylsulfonyl)aniline (0.27 g, 1.19 mmol), DMA (4 ml) and 2-chloro-5-nitrobenzoyl chloride (0.31 g, 1.43 mmol).
$^1$H-NMR (400MHz, DMSO-$d_6$, TMS): δ 1.63-1.68 (4H, m), 3.14 (4H, t, J=6.7Hz), 7.83 (2H, d, J=8.8Hz), 7.92 (2H, d, J=8.8Hz), 7.94 (1H, d, J=8.9Hz), 8.37 (1H, dd, J=2.8, 8.9Hz), 8.55 (1H, d, J=2.8Hz), 11.13 (1H, s);

[Example 153] N-[4-(Morpholin-4-yl-sulfonyl)phenyl]-(2-chloro-5-nitrophenyl)carboxamide

The title compound (0.36 g, yield 73%) was obtained according to the procedure described in Example 2 using 4-(morpholin-4-ylsulfonyl)aniline (0.28 g, 1.17 mmol), DMA (4 ml) and 2-chloro-5-nitrobenzoyl chloride (0.31 g, 1.40 mmol).
$^1$H-NMR (400MHz, DMSO-$d_6$, TMS): δ 2.87 (4H, t, J=4.6Hz), 3.64 (4H, t, J=4.6Hz), 7.77 (2H, d, J=8.8Hz), 7.92 (1H, d, J=8,8Hz), 7.98 (2H, d, J=8.8Hz), 8.37 (1H, dd, J=2.7, 8.8Hz), 8.55 (1H, d, J=2.7Hz);

[Example 154] N-[3-(Pyrrolidylsulfonyl)phenyl]-(2-chloro-5-nitrophenyl)carboxamide

The title compound (0.39 g, yield 93%) was obtained according to the procedure described in Example 2 using 3-(pyrrolidylsulfonyl)aniline (0.23 g, 1.02 mmol), DMA (4 ml) and 2-chloro-5-nitrobenzoyl chloride (0.27 g, 1.23 mmol).
$^1$H NMR ($CDCl_3$, 400MHz, TMS): δ 1.77-1.81 (4H, m), 3.24 (4H, t, J=6.8Hz), 7.55-7.62 (2H, m), 7.68 (1H, d, J=8.8Hz), 8.19 (1H, d, J=7.6Hz), 8.29 (1H, dd, J=2.7, 8.8Hz), 8.59 (1H, d, J=2.7Hz);

[Example 155] N-(4-Acetylphenyl)-(2-chloro-5-nitrophenyl)carboxamide

The title compound (16.4 g, yield 99%) was obtained according to the procedure described in Example 2 using 4-acetylaniline (7.04 g, 52.1 mmol), DMA (70 ml) and 2-chloro-5-nitrobenzoyl chloride (13.2 g, 60 mmol).
$^1$H-NMR (400MHz, DMSO-$d_6$, TMS): δ 2.61 (3H, s), 7.68 (1H, d, J=8.8Hz), 7.77 (2H, d, J=8.6Hz), 8.01 (2H, d, J=8.7Hz), 8.14 (1H, bs), 8.29 (1H, dd, J=2.7, 8.8Hz), 8.63 (1H, d, J=2.7Hz);

[Example 156] N-(3-Acetylphenyl)-(2-chloro-5-nitrophenyl)carboxamide

The title compound (17.4 g, yield 99%) was obtained according to the procedure described in Example 2 using 3-acetylaniline (7.44 g, 55.0 mmol), DMA (75 ml) and 2-chloro-5-nitrobenzoyl chloride (13.3 g, 63.3 mmol).
$^1$H-NMR (400MHz, DMSO-$d_6$, TMS): δ 2.63 (3H, s), 7.53 (1H, t, J=7.9Hz), 7.69 (1H, d, J=8.8Hz), 7.80 (1H, d, J=7.9Hz), 8.03-8.06 (1H, m), 8.11 (1H, bs), 8.13 (1H, bs), 8.29 (1H, dd, J=2.7,8.8Hz), 8.64 (1H, d, J=2.7Hz);

[Example 157] N-[4-[(3,5-di-tert-Butyl-4-hydroxybenzoyl)amino]phenyl]-(2-chloro-5-nitrophenyl)carboxamide

The title compound (0.63 g, yield 93%) was obtained according to the procedure described in Example 2 using 4-[(3,5-di-tert-butyl-4-hydroxybenzoyl)amino]aniline (0.44 g, 1.29 mmol), DMA (5 ml) and 2-chloro-5-nitrobenzoyl chloride (0.34 g, 1.54 mmol).
$^1$H-NMR (400MHz, DMSO-$d_6$, TMS): δ 1.44 (18H, s), 7.51 (1H, bs), 7.65-7.73 (6H, m), 7.90 (1H, d, J=8.9Hz), 8.34 (1H, dd, J=2.7, 8.8Hz), 8.47 (1H, d, J=2.7Hz), 10.06 (1H, s), 10.68 (1H, s);
MS(FAB) m/z: 524 $(M + H)^+$;

[Example 158] N-[4-(Morpholin-4-yl-carbonyl)phenyl]-(2-chloro-5-nitrophenyl)carboxamide

The title compound (0.57 mg, yield 97%) was obtained according to the procedure described in Example 2 using 4-(morpholin-4-yl-carbonyl)aniline (0.31 g, 1.50 mmol), DMA (5 ml) and 2-chloro-5-nitrobenzoyl chloride (0.40 g, 1.80 mmol).
$^1$H-NMR (400MHz, DMSO-$d_6$, TMS): δ 3.48-3.61 (8H, m), 7.45 (2H, d, J=8.5Hz), 7.77 (2H, d, J=8.6Hz), 7.91 (1H, d, J=8.8Hz), 8.35 (1H, dd, J=2.7, 8.8Hz), 8.49 (1H, d, J=2.7Hz), 10.90 (1H, s);
MS(FAB) m/z: 390 $(M + H)^+$;

[Example 159] N-[4-(Piperidylcarbonyl)phenyl]-(2-chloro-5-nitrophenyl)carboxamide

The title compound (0.49 g, yield 85%) was obtained according to the procedure described in Example 2 using 4-(piperidylcarbonyl)aniline (0.31 g, 1.50 mmol), DMA (5 ml) and 2-chloro-5-nitrobenzoyl chloride (0.40 g, 1.80 mmol).
$^1$H-NMR (400MHz, DMSO-$d_6$, TMS): δ 1.51 (4H, m), 1.62 (2H, m), 3.50-3.56 (4H, m), 7.41 (2H, d, J=8.4Hz), 7.76 (2H, d, J=8.4Hz), 7.91 (1H, d, J=8.8Hz), 8.35 (1H, dd, J=2.7, 8.8Hz), 8.49 (1H, d, J=2.7Hz), 10.88 (1H, s);
MS(FAB) m/z: 388 $(M + H)^+$;

[Example 160] N-[4-(Pyrrolidinylcarbonyl)phenyl]-(2-chloro-5-nitrophenyl)-carboxamide

The title compound (0.47 g, yield 83%) was obtained according to the procedure described in Example 2 using 4-(pyrrolidinylcarbonyl)aniline (0.29 g, 1.50 mmol), DMA (5 ml) and 2-chloro-5-nitrobenzoyl chloride (0.40 g, 1.80 mmol).
$^1$H-NMR (400MHz, DMSO-$d_6$, TMS): δ 3.31-3.40 (4H, m), 3.40-3.49 (4H, m), 7.56 (2H, d, J=8.5Hz), 7.76 (2H, d, J=8.5Hz), 7.91 (1H, d, J=8.8Hz), 8.35 (1H, dd, J=2.6,8.8Hz), 8.50 (1H, d, J=2.6Hz);
MS(FAB) m/z: 374 $(M + H)^+$;

[Example 161] N-[3-[(3,5-di-tent-Butyl-4-hydroxyphenyl)aminosulfonyl]phenyl]-(2-chloro-5-nitrophenyl)carboxamide

The title compound (0.46 g, yield 87%) was obtained according to the procedure described in Example 2 using 3-[(3,5-di-tert-butyl-4-hydroxyphenyl)aminosulfonyl]aniline (0.36 g, 0.95 mmol), DMA (4 ml) and 2-chloro-5-nitrobenzoyl chloride (0.25 g, 1.14 mmol).
$^1$H NMR ($CDl_3$, 400MHz): δ 1.32 (18H, s), 6.30 (1H, s), 6.76 (2H, s), 7.44-7.48 (2H, m), 7.67 (1H, d, J=8.8Hz), 8.10 (1H, d, J=7.7Hz), 8.29 (1H, dd, J=2.7, 8.8Hz), 8.29 (1H, bs), 8.58 (1H, d, J=2.7Hz);

[Example 162] N-[3-[(3,5-di-tert-Butyl-4-hydroxybenzoyl)amino]phenyl]-(2-chloro-5-nitrophenyl)carboxamide

The title compound (0.66 g, yield 90%) was obtained according to the procedure described in Example 2 using 3-[(3,5-di-tert-butyl-4-hydroxybenzoyl)amino]aniline (0.48 g, 1.40 mmol), DMA (5 ml) and 2-chloro-5-nitrobenzoyl chloride (0.37 g, 1.68 mmol).
$^1$H-NMR (400MHz, DMSO-$d_6$, TMS): δ 1.49 (18H, s), 5.65 (1H, s), 7.38-7.40 (2H, m), 7.53-7.55 (1H, m), 7.60 (1H, d, J=8.8Hz), 7.64 (2H, s), 7.78 (1H, s), 8.08 (1H, s), 8.19 (1H, dd, J=2.7, 8.8Hz), 8.26 (1H, bs), 8.53 (1H, d, J=2.7Hz);

[Example 163] 4-[4-[(2-Chloro-5-nitrophenyl)carbonylamino]phenyl]phenyl-(2-chloro-5-nitrophenyl)carboxamide

The title compound (2.49 g, yield 95%) was obtained according to the procedure described in Example 2 using 4-(4-aminophenyl)aniline (0.875 g, 4.75 mmol), DMA (10 ml) and 2-chloro-5-nitrobenzoyl chloride (2.30 g, 10.5 mmol).
$^1$H-NMR (400MHz, DMSO-$d_6$, TMS): δ 7.72 (4H, d, J=8.7Hz), 7.81 (4H, d, J=8.7Hz), 7.91 (2H, d, J=8.8Hz), 8.36 (2H,

dd, J=2.8, 8.8Hz), 8.49 (2H, d, J=2.8Hz), 10.81 (2H, s);
MS(FAB) m/z: 551 $(M + H)^+$;

[Example 164] N-[4-(4-Methylpiperazin-1-yl-carbonyl)phenyl]-(2-chloro-5-nitrophenyl)carboxamide

The title compound (0.54 g, yield 89%) was obtained according to the procedure described in Example 2 using 4-(4-methylpiperazin-1-yl-carbonyl)aniline (0.33 g, 1.50 mmol), DMA (5 ml) and 2-chloro-5-nitrobenzoyl chloride (0.40 g, 1.80 mmol).
$^1$H-NMR (400MHz, DMSO-$d_6$, TMS): δ 2.32 (4H, m), 3.34-3.59 (4H, m), 7.42 (2H, d, J=8.6Hz), 7.77 (2H, d, J=8.6Hz), 7.91 (1H, d, J=8.8Hz), 8.35 (1H, dd, J=2.7, 8.8Hz), 8.49 (1H, d, J=2.7Hz), 10.89 (1H, s);
MS(FAB) m/z: 403 $(M + H)^+$;

[Example 165] N-[4-(4-Phenylpiperazin-1-yl-carbonyl)phenyl]-(2-chloro-5-nitrophenyl)carboxamide

The title compound (0.66 mg, yield 95%) was obtained according to the procedure described in Example 2 using 4-(4-phenylpiperazin-1-yl-carbonyl)phenyl (0.42 g, 1.50 mmol), DMA (5 ml) and 2-chloro-5-nitrobenzoyl chloride (0.40 g, 1.80 mmol).
$^1$H-NMR (400MHz, DMSO-$d_6$, TMS): δ 3.18 (4H, bs), 3.55-3.74 (4H, m), 6.82 (1H, t, J=7.2Hz), 6.97 (2H, d, J=8.7Hz), 7.21-7.27 (2H, m), 7.49 (2H, d, J=8.5Hz), 7.79 (2H, d, J=8.5Hz), 7.91 (1H, d, J=8.8Hz), 8.36 (1H, dd, J=2.8, 8.8Hz), 8.51 (1H, d, J=2.8Hz), 10.91 (1H, s);
MS(FAB) m/z: 465 $(M + H)^+$;

[Example 166] N-[4-(4-tert-Butoxycarbonylpiperazin- 1-yl-carbonyl)phenyl]-(2-chloro-5-nitrophenyl)carboxamide

The title compound (0.70 g, yield 95%) was obtained according to the procedure described in Example 2 using 4-(4-tert-butoxycarbonylpiperazin-1-yl-carbonyl)aniline (0.46 g, 1.50 mmol), DMA (5 ml) and 2-chloro-5-nitrobenzoyl chloride (0.40 g, 1.80 mmol).
$^1$H-NMR (400MHz, DMSO-$d_6$, TMS): δ 1.41 (9H, s), 3.32-3.55 (8H, m), 7.45 (2H, d, J=8.5Hz), 7.77 (2H, d, J=8.5Hz), 7.91 (1H, d, J=8.8Hz), 8.36 (1H, dd, J=2.7, 8.8Hz), 8.50 (1H, d, J=2.7Hz), 10.90 (1H, s);
MS(FAB) m/z: 489 $(M + H)^+$;

[Example 167] N-[4-(2-tert-Butoxycarbonylamino)ethylphenyl]-(2-chloro-5-nitrophenyl)carboxamide

The title compound (10.0 g, yield 99%) was obtained according to the procedure described in Example 2 using 4-(2-tent-butoxycarbonylaminoethyl)aniline (5.66 g, 24 mmol), DMA (50 ml) and 2-chloro-5-nitrobenzoyl chloride (6.32 g, 28.8 mmol).
$^1$H-NMR (400MHz, DMSO-$d_6$, TMS): δ 1.37 (9H, s), 2.67 (2H, t, J=7.5Hz), 3.10-3.13 (2H, m), 7.19 (2H, d, J=8.4Hz), 7.61 (2H, d, J=8.4Hz), 7.89 (1H, d, J=8.8Hz), 8.34 (1H, dd, J=2.8, 8.8Hz), 8.44 (1H, d, J=2.8Hz), 10.64 (1H, s);
MS(FAB) m/z: 420 $(M + H)^+$;

[Example 168] Ethyl [4-(2-chloro-5-nitrobenzoylamino)phenyl]acetate

The title compound (8.20 g, yield 90%) was obtained according to the procedure described in Example 2 using ethyl 4-aminophenylacetate (4.58 g, 25 mmol), DMA (70 ml) and 2-chloro-5-nitrobenzoyl chloride (6.60 g, 30 mmol).
$^1$H-NMR (400MHz, DMSO-$d_6$, TMS): δ 1.19 (3H, t, J=7.1Hz), 3.64 (2H, s), 4.08 (2H, q, J=7.1Hz), 7.27 (2H, d, J=8.5Hz), 7.65 (2H, d, J=8.5Hz), 7.89 (1H, d, J=8.8Hz), 8.34 (1H, dd, J=2.8, 8.8Hz), 8.46 (1H, d, J=2.8Hz), 10.76 (1H, s);
MS(FAB) m/z: 401 $(M + H)^+$;

[Example 169] N-[4-(2-Aminoethyl)phenyl]-(2-chloro-5-nitrophenyl)carboxamide hydrochloride 0.2 hydrate

The title compound (6.87 g, yield 84%) was obtained according to the procedure described in Example 88 using N-[4-(2-tert-butoxycarbonylaminoethyl)phenyl]-(2-chloro-5-nitrophenyl)carboxamide (8.73 g, 20.8 mmol) prepared in Example 167, dioxane (90 ml) and 4N hydrogen chloride/dioxane solution (100 ml).
$^1$H-NMR (400MHz, DMSO-$d_6$, TMS): δ 2.86 (2H, t, J=7.8Hz), 3.02 (2H, bs), 7.27 (2H, d, J=7.9Hz), 7.67 (2H, d, J=7.9Hz), 7.89 (2H, d, J=8.8Hz), 7.96-8.00 (2H, m), 8.33-8.37 (1H, m), 8.43 (1H, d, J=1.5Hz), 10.74 (1H, s);
MS(FAB) m/z: 320 $(M + H)^+$;

[Example 170] [4-(2-Chloro-5-nitrobenzoylamino)phenyl]acetic acid

Reaction was carried out according to the procedure described in Example 145 using ethyl [4-(2-chloro-5-nitrobenzoylamino)phenyl]acetate (7.02 g, 19.3 mmol) prepared in Example 168, THF (50 ml) and 1N aqueous sodium hydroxide solution (39 ml). After the reaction was completed, the product was treated according to the procedure described in Example 67 using 1N hydrochloric acid (40 ml) to afford the title compound (6.47 g, yield 99%).
$^1$H-NMR (400MHz, DMSO-$d_6$, TMS): δ 3.55 (2H, s), 7.26 (2H, d, J=8.5Hz), 7.64 (2H, d, J=8.5Hz), 7.89 (1H, d, J=8.8Hz), 8.34 (1H, dd, J=2.7, 8.8Hz), 8.45 (1H, d, J=2.7Hz), 10.69 (1H, s), 12.31 (1H, s);
MS(FAB) m/z: 335 $(M + H)^+$;

[Example 171] N-[4-[4-(Pyrrolidin-1-yl-amino)phenyl]phenyl]-(2-chloro-5-nitrophenyl)carboxamide

The title compound (0.45 g, yield 99%) was obtained according to the procedure described in Example 2 using 4-(4-pyrrolidinylphenyl)aniline (0.26 g, 1.1 mmol), DMA (5 ml) and 2-chloro-5-nitrobenzoyl chloride (0.28 g, 1.28 mmol).
$^1$H-NMR (400MHz, DMSO-$d_6$, TMS): δ 1.95-1.99 (4H, m), 3.26-3.29 (4H, m), 6.62 (2H, d, J=8.7Hz), 7.51 (2H, d, J=8.6Hz), 7.59 (2H, d, J=8.6Hz), 7.72 (2H, d, J=8.7Hz), 7.91 (1H, d, J=8.8Hz), 8.35 (1H, dd, J=2.7, 8.8Hz), 8.47 (1H, d, J=2.7Hz), 10.71 (1H, s);
MS(FAB) m/z: 421 $(M + H)^+$;

[Example 172] N-(4-Diethylaminophenyl)-(2-chloro-5-nitrophenyl)carboxamide

The title compound (0.66 g, yield 73%) was obtained according to the procedure described in Example 2 using 4-diethylaminoaniline (0.42 g, 2.58 mmol), DMA (5 ml) and 2-chloro-5-nitrobenzoyl chloride (0.62 g, 2.82 mmol).
$^1$H-NMR (400MHz, DMSO-$d_6$, TMS): δ 1.08 (6H, t, J=7.0Hz), 3.32 (4H, q, J=7.0Hz), 6.67 (2H, d, J=9.1Hz), 7.48 (2H, d, J=9.1Hz), 7.87 (1H, d, J=8.79Hz), 8.31 (1H, dd, J=2.8,8.8Hz), 8.38 (1H, d, J=2.8Hz), 10.33 (1H, s);
MS(FAB) m/z: 347 $(M)^+$, 348$(M + H)^+$;

[Example 173] N-(4-Dimethylaminophenyl)-(2-chloro-5-nitrophenyl)carboxamide

The title compound (0.66 g, yield 85%) was obtained according to the procedure described in Example 2 using 4-dimethylaniline (0.33 g, 2.43 mmol), DMA (5 ml) and 2-chloro-5-nitrobenzoyl chloride (0.59 g, 2.67 mmol).
$^1$H-NMR (400MHz, DMSO-$d_6$, TMS): δ 2.88 (6H, s), 6.74 (2H, d, J=9.1Hz), 7.52 (2H, d, J=9.1Hz), 7.87 (1H, d, J=8.8Hz), 8.32 (1H, dd, J=2.8, 8.8Hz), 8.40 (1H, d, J=2.8Hz), 10.38 (1H, s);
MS(FAB) m/z: 320 $(M)^+$, 319 $(M + H)^+$;

[Example 174] N-[4-(Imidazol-1-yl)phenyl]-(2-chloro-5-nitrophenyl)carboxamide

The title compound (0.69 g, yield 92%) was obtained according to the procedure described in Example 2 using 4-(imidazol-1-yl)aniline (0.35 g, 2.18 mmol), DMA (5 ml) and 2-chloro-5-nitrobenzoyl chloride (0.55 g, 2.50 mmol).
$^1$H-NMR (400MHz, DMSO-$d_6$, TMS): δ 7.12 (1H, bs), 7.67 (2H, d, J=9.0Hz), 7.73 (1H, bs), 7.83 (2H, d, J=8.9Hz), 7.91 (1H, d, J=8.9Hz), 8.24 (1H, bs), 8.36 (1H, dd, J=2.8, 8.9Hz), 8.51 (1H, d, J=2.8Hz), 10.87 (1H, s);
MS(FAB) m/z: 343 $(M + H)^+$;

[Example 175] N-[4-[(3,5-di-tert-Butyl-4-hydroxyphenyl)aminosulfonyl]phenyl]-(2-chloro-5-nitrophenyl)carboxamide

The title compound (0.32 g, yield 80%) was obtained according to the procedure described in Example 2 using 4-[(3,5-di-tert-butyl-4-hydroxyphenyl)aminosulfonyl]aniline (0.27 g, 0.72 mmol), DMA (3 ml) and 2-chloro-5-nitrobenzoyl chloride (0.17 g, 0.80 mmol).
$^1$H-NMR (400MHz, DMSO-$d_6$, TMS): δ 1.27 (18H, s), 6.76 (2H, s), 6.83 (1H, s), 7.66 (2H, d, J=8.6Hz), 7.82 (2H, d, J=8.6Hz), 7.90 (1H, d, J=8.8Hz), 8.33-8.37 (1H, m), 8.49 (1H, d, J=2.7Hz), 9.56-9.59 (1H, m), 11.05 (1H, s);
MS(FAB) m/z: 559 $(M)^+$;

[Example 176] N-(3-Dimethylamino)phenyl-(2-chloro-5-nitrophenyl)carboxamide

Reaction was carried out according to the procedure described in Example 2 using 3-dimethylaminoaniline dihydrochloride (0.511 g, 2.45 mmol), THF (10 ml), pyridine (2 ml) and 2-chloro-5-nitrobenzoyl chloride (0.59 g, 2.70 mmol). After the reaction was completed, saturated aqueous sodium bicarbonate solution (30 ml) and ethyl acetate

(30 ml) were added to the reaction mixture and the mixture was partitioned. The organic layer was separated, washed with saturated aqueous sodium chloride solution (20 ml×2), dried over anhydrous sodium sulfate and then the solvent was removed under reduced pressure to afford a crude title compound. The obtained crude compound was purified by chromatography on a silica gel column [hexane:ethyl acetate=1:1 (v/v)]. The compound was solidified with diisopropyl ether, filtered and dried to afford the title compound (0.38 g, yield 49%).
$^1$H-NMR (400MHz, DMSO-$d_6$, TMS): δ 2.99 (6H, s), 6.58 (1H, dd, J=2.2, 8.3Hz), 6.84 (1H, dd, J=1.7, 7.8Hz), 7.16 (1H, t, J=2.2Hz), 7.23 (1H, t, J=8.3Hz), 7.65 (1H, d, J=8.8Hz), 7.74 (1H, bs), 8.25 (1H, dd, J=2.7, 8.8Hz);
MS(FAB) m/z: 319 $(M)^+$, 320 $(M + H)^+$;

[Example 177] N-[4-[4-(Piperidin-1-yl)phenyl]phenyl]-(2-chloro-5-nitrophenyl)carboxamide

The title compound (0.41 g, yield 94%) was obtained according to the procedure described in Example 2 using 4-(piperidinylphenyl)aniline (0.25 g, 1.0 mmol), DMA (10 ml) and 2-chloro-5-nitrobenzoyl chloride (0.26 g, 1.2 mmol).
$^1$H-NMR (400MHz, DMSO-$d_6$, TMS): δ 1.55-1.58 (2H, m), 1.61-1.66 (4H, m), 3.19 (4H, t, J=5.2Hz), 7.00 (2H, d, J=8.7Hz), 7.52 (2H, d, J=8.7Hz), 7.62 (2H, d, J=8.6Hz), 7.74 (2H, d, J=8.6Hz), 7.91 (1H, d, J=8.8Hz), 8.35 (1H, dd, J=2.7, 8.8Hz), 8.47 (1H, d, J=2.7Hz), 10.74 (1H, s);
MS(FAB) m/z: 436 $(M + H)^+$, 435 $(M)^+$;

[Example 178] N-[4-[(Piperidin-1-yl)aminosulfonyl]phenyl]-(2-chloro-5-nitrophenyl)carboxamide

The title compound (0.30 g, yield 31%) was obtained according to the procedure described in Example 2 using 4-[(piperidin-1-yl)aminosulfonyl]aniline (0.57 g, 2.27 mmol), DMA (5 ml) and 2-chloro-5-nitrobenzoyl chloride (0.26 g, 1.2 mmol).
$^1$H-NMR (400MHz, DMSO-$d_6$, TMS): δ 7.30 (1H, dd, J=4.7, 8.3Hz), 7.50-7.54 (1H, m), 7.78 (2H, d, J=8.8Hz), 7.86 (2H, d, J=8.8Hz), 7.90 (1H, d, J=8.8Hz), 8.26 (1H, d, J=4.7Hz), 8.29 (1H, d, J=2.5Hz), 8.35 (1H, dd, J=2.7, 8.8Hz), 8.52 (1H, d, J=2.7Hz), 10.52 (1H, s), 11.09 (1H, s);

[Example 179] N-[4-[4-(Morpholin-4-yl)phenyl]aminosulfonyl]phenyl-(2-chloro-5-nitrophenyl)carboxamide

The title compound (0.51 g, yield 81%) was obtained according to the procedure described in Example 2 using [4-[4-(morpholin-4-yl)phenyl]aminosulfonyl]aniline (0.40 g, 1.21 mmol), DMA (5 ml) and 2-chloro-5-nitrobenzoyl chloride (0.29 g, 1.33 mmol).
$^1$H-NMR (400MHz, DMSO-$d_6$, TMS): δ 3.01 (4H, t, J=4.8Hz), 3.69 (4H, t, J=4.8Hz), 6.83 (2H, d, J=9.1Hz), 6.94 (2H, d, J=9.1Hz), 7.72(2H,d,J=8.7Hz), 7.82(2H,d,J=8.7Hz), 7.90(1H,d,J=8.8Hz), 8.35 (1H, dd, J=2.7, 8.8Hz), 8.52 (1H, d, J=2.7Hz), 9.79 (1H, s), 11.05 (1H, s);
MS(FAB) m/z: 516 $(M)^+$, 517 $(M + H)^+$;

[Example 180] N-(4-Aminophenyl)-(2-chloro-5-nitrophenyl)carboxamide

N-(4-Aminophenyl)-(2-chloro-5-nitrophenyl)carboxamide mono-hydrochloride (4.89 g, 14.9 mmol) prepared in Example 58 was suspended in saturated aqueous sodium bicarbonate (200 ml) and water (100 ml) for 2 hours with stirring. The resulting crystalline solid was filtered, washed with water and dried to afford the title compound (4.29 g, yield 99%).
$^1$H NMR(DMSO-$d_6$, 400MHz): δ 4.99 (1H, bs), 6.55 (2H, d, J=8.8Hz), 7.34 (2H, d, J=8.8Hz), 7.86 (1H, d, J=8.8Hz), 8.31 (1H, dd, J=8.8, 2.8Hz), 8.37 (1H, d, J=2.8Hz), 10.26 (1H, s);
MS(FAB) m/z: 292 $(M + H)^+$;

[Example 181] N-[4-(N-Propylamino)phenyl]-(2-chloro-5-nitrophenyl)carboxamide

The title compound (0.307 g, yield 46%) was obtained as the more polar compound according to the procedure described in Example 115 using N-(4-aminophenyl)-(2-chloro-5-nitrophenyl)carboxamide (0.583 g, 2.00 mmol) prepared in Example 180, methanol (10 ml), sodium cyanoborohydride (0.151 g, 2.40 mmol) and propionaldehyde (0.289 ml, 4.00 mmol).
Rf 0.20 [hexane:ethyl acetate=2:1 (v/v)];
$^1$H NMR ($CDCl_3$, 400MHz): δ 1.01 (3H, t, J=7.3Hz), 1.66 (2H, tq, J=7.3Hz), 3.10 (2H, t, J=7.3Hz), 6.62 (2H, d, J=8.8Hz), 7.41 (2H, d, J=8.8Hz), 7.63 (1H, br), 7.64 (1H, d, J=8.8Hz), 8.24 (1H, dd, J=8.8, 2.9Hz), 8.61 (1H, d, J=2.9Hz);
MS(FAB) m/z: 333 $M^+$.

[Example 182] N-[4-(N,N-Dipropylamino)phenyl]-(2-chloro-5-nitrophenyl)carboxamide

The title compound (0.099 g, yield 13%) was obtained as the less polar compound in Example 181.
Rf 0.47 [hexane:ethyl acetate=2:1 (v/v)];
$^1$H NMR ($CDCl_3$, 400MHz): δ 0.93 (6H, t, J=7.3Hz), 1.61 (4H, tq, J=7.3Hz), 3.24 (4H, t, J=7.3Hz), 6.64 (2H, d, J=8.8Hz), 7.42 (2H, d, J=8.8Hz), 7.62 (1H, br), 7.63 (1H, d, J=8.8Hz), 8.24 (1H, dd, J=8.8, 2.9Hz), 8.61 (1H, d, J=2.9Hz);
MS(FAB) m/z: 375 $M^+$.

[Example 183] N-[4-(4-Acetylpiperazin-1-yl)phenyl]-(2-chloro-5-nitrophenyl)carboxamide

Acetic anhydride (0.131 ml, 1.18 mmol) was added to a solution of N-[(4-piperazin-1-yl)phenyl]-(2-chloro-5-nitrophenyl)carboxamide di-hydrochloride (0.200 g, 0.461 mmol) prepared in Example 133 in pyridine (2 ml). The mixture was stirred at room temperature for 1 hour. Saturated aqueous sodium bicarbonate solution (2 ml) and water (20 ml) were added to the reaction mixture. The resulting insoluble material was filtered, washed with water and diisopropyl ether and dried in vacuo to afford the title compound (0.164 g, yield 88%).
$^1$H NMR ($CDCl_3$, 400MHz): δ 2.15 (3H, s), 3.14 (2H, t, J=5.5Hz), 3.19 (2H, t, J=5.5Hz), 3.64 (2H, t, J=5.1Hz), 3.78 (2H, t, J=5.1Hz), 6.96 (2H, d, J=8.8Hz), 7.55 (2H, d, J=8.8Hz), 7.66 (1H, d, J=8.8Hz), 7.81 (1H, br), 8.26 (1H, dd, J=8.8, 2.9Hz), 8.61 (1H, d, J=2.9Hz);
MS(FAB) m/z: 403 $(M + H)^+$.

[Example 184] N-[4-(4-Benzoylpiperazin-1-yl)phenyl]-(2-chloro-5-nitrophenyl)carboxamide

The title compound (0.020 g, yield 9%) was obtained according to the procedure described in Example 183 using N-[(4-piperazin-1-yl)phenyl]-(2-chloro-5-nitrophenyl)carboxamide di-hydrochloride (0.200 g, 0.461 mmol) prepared in Example 133, benzoyl chloride (0.107 ml, 0.992 mmol), and pyridine (2 ml).
$^1$H NMR ($CDCl_3$, 400MHz): δ 3.20 (4H, m), 3.62 (2H, m), 3.95 (2H, m), 6.96 (2H, d, J=8.8Hz), 7.44 (5H, m), 7.55 (2H, d, J=8.8Hz), 7.66 (1H, d, J=8.8Hz), 7.77 (1H, br), 8.26 (1H, dd, J=8.8, 2.9Hz), 8.61 (1H, d, J=2.9Hz);
MS(FAB) m/z: 465 $(M + H)^+$.

[Example 185] N-[4-[4-(N,N-Dioctylamino)phenyl]phenyl]-(2-chloro-5-nitrophenyl)carboxamide hydrochloride

The reaction was carried out according to the procedure described in Example 2 using 4-[4-(N,N-dioctylamino) phenyl]aniline (0.31 g, 0.75 mmol), DMA (10 ml) and 2-chloro-5-nitrobenzoyl chloride (0.20 g, 0.91 mmol) to give an oil compound. The obtained oil compound was treated with 4N hydrogen chloride/dioxane solution (0.4 ml) according to a similar procedure to that described in Example 146 to afford the title compound of a hydrochloride (0.47 g, yield 99%).
$^1$H NMR (DMSO-$d_6$, 400MHz): δ 0.83 (6H ,t, J=6.7Hz), 1.12-1.80 (24H, m), 4.7-5.2 (4H, m), 7.7-7.9 (8H, m), 7.91 (2H, d, J=8.8Hz), 8.36 (1H, dd, J=2.7, 8.8Hz), 8.49 (1H, d, J=2.7Hz), 10.88 (1H,s);
MS(FAB) m/z: 592 $(M + H)^+$ 591 $(M)^+$;

[Example 186] N-[4-[4-(Pyrrol-1-yl)phenyl]phenyl]-(2-chloro-5-nitrophenyl)carboxamide

[186a] 4-[4-(Pyrrol-1-yl)phenyl]nitrobenzene

2,5-Dimethoxytetrahydrofuran (10.47 g, 79.2 mmol) was added to a suspension of 4-(4-nitrophenyl)aniline (8.49 g, 39.6 mmol) in acetic acid (40 ml). The mixture was heated under reflux for 5 hours. After cooling the reaction mixture, ether (200 ml) was added to the mixture and the mixture was stirred. The resulting crystalline solid was filtered and dried to afford the title compound (9.65 g, yield 92%).
$^1$H NMR (DMSO-$d_6$, 400MHz): δ 6.32 (2H, t, J=2.2Hz), 7.49 (2H, t, J=2.2Hz), 7.76 (2H, d, J=8.7Hz), 7.90 (2H, d, J=8.7Hz), 8.02 (2H, d, J=8.8Hz), 8.31 (2H, d, J=8.8Hz), 10.82 (1H, s);
MS(FAB) m/z: 000 $(M + H)^+$;

[186b] 4-[4-(Pyrrol-1-yl)phenyl]aniline

After nickel chloride hexahydrate (17.36 g, 73.0 mmol) was added to a solution of 4-[4-(pyrrol-1-yl)phenyl] nitrobenzene (9.65 g, 36.5 mmol) prepared in Example 186a in a mixture of dioxane (200 ml) and methanol (100 ml), sodium cyanoborohydride (5.53 g, 146 mmol) was added portionwise to the mixture under cooling with an ice-water bath. The reaction mixture was concentrated and saturated aqueous sodium bicarbonate solution (400 ml) and ethyl

acetate (400 ml) were added to the residue. After the mixture was stirred for 30 minutes, the resulting insoluble material was filtered off and the filtrate was extracted with ethyl acetate. The organic layer was washed with saturated aqueous sodium chloride solution, dried over anhydrous sodium sulfate, filtered and then the filterate was concentrated to afford the title compound (6.67 g, yield 78%).
$^1$H NMR (DMSO-$d_6$, 400MHz): δ 5.25 (2H, bs), 6.26 (2H, t, J=2.2Hz), 6.65 (2H, d, J=8.6Hz), 7.37 (2H, d, J=2.2Hz), 7.39 (2H, d, J=8.6Hz), 7.56 (2H, d, J=8.8Hz), 7.65 (2H, d, J=8.8Hz);
MS(FAB) m/z: 000 $(M + H)^+$;

[186c] N-[4-[4-(Pyrrol-1-yl)phenyl]phenyl]-(2-chloro-5-nitrophenyl)carboxamide

The title compound (0.75 g, yield 90%) was obtained according to the procedure described in Example 2 using 4-[4-(pyrrol-1-yl)phenyl]aniline (0.47 g, 2.0 mmol) prepared in Example 186b, DMA (10 ml) and 2-chloro-5-nitrobenzoyl chloride (0.53 g, 2.40 mmol).
$^1$H NMR (DMSO-$d_6$, 400MHz): δ 6.30 (2H, t, J=2.0Hz), 7.43 (2H, t, J=2.0Hz), 7.67 (2H, d, J=8.6Hz), 7.7-7.83 (6H, m), 7.92 (1H, d, J=8.5Hz), 8.36 (1H, dd, J=8.5, 2.7Hz), 8.50 (1H, d, J=2.7Hz), 10.82 (1H, s);
MS(FAB) m/z: 418 $(M + H)^+$;

[Example 187] N-[[4-[4-(Imidazol-1-yl)phenyl]aminosulfonyl]phenyl]-(2-chloro-5-nitrophenyl)carboxamide

The title compound (0.30 g, yield 61%) was obtained according to the procedure described in Example 2 using 4-[[4-(imidazol-1-yl)phenyl]aminosulfonyl]aniline (0.31 g, 1.0 mmol), DMA (10 ml) and 2-chloro-5-nitrobenzoyl chloride (0.26 g, 1.2 mmol).
$^1$H NMR (DMSO-$d_6$, 400MHz): δ 7.06 (1H, s), 7.21 (2H, d, J=8.7Hz), 7.53 (2H, d, J=8.7Hz), 7.63 (1H, s), 7.80 (2H, d, J=d, 8.8Hz), 7.85 (2H, d, J=8.8Hz), 7.89 (1H, d, J=8.8Hz), 7.96-8.03 (1H, m), 8.13 (1H, s), 8.35 (1H, dd ,J=8.8, 2.7Hz), 8.51 (1H, d, J=2.7Hz), 10.44 (1H, bs), 11.08 (1H, s);
MS(FAB) m/z: 498 $(M + H)^+$;

[Example 188] N-[4-(N-Ethyl-N-hexylamino)phenyl]-(2-chloro-5-nitrophenyl)carboxamide

The title compound (0.019 g, yield 25%) was obtained according to the procedure described in Example 115 using N-[4-(N-ethylamino)phenyl]-(2-chloro-5-nitrophenyl)carboxamide (0.062 g, 0.195 mmol) prepared in Example 115, methanol (1.2 ml), sodium cyanoborohydride (0.015 g, 0.234 mmol) and hexanal (0.035 ml, 0.293 mmol).
Rf 0.52 [hexane:ethyl acetate=2:1 (v/v)];
$^1$H NMR ($CDCl_3$, 400MHz): δ 0.90 (3H, t, J=7.3Hz), 1.15 (3H, t, J=7.3Hz), 1.32 (6H, m), 1.58 (2H, m), 3.25 (2H, t, J=7.3Hz), 3.37 (2H, q, J=7.3Hz), 6.65 (2H, d, J=8.8Hz), 7.42 (2H, d, J=8.8Hz), 7.62 (1H, d, J=8.8Hz), 7.69 (1H, br), 8.22 (1H, dd, J=8.8, 2.9Hz), 8.58 (1H, m);
MS(FAB) m/z: 403 $M^+$.

[Example 189] N-[4-(N-Ethyl-N-propylamino)phenyl]-(2-chloro-5-nitrophenyl)carboxamide

The title compound (0.029 g, yield 54%) was obtained according to the procedure described in Example 115 using N-[4-(N-propylamino)phenyl]-(2-chloro-5-nitrophenyl)carboxamide (0.056 g, 0.168 mmol) prepared in Example 181, methanol (2 ml), sodium cyanoborohydride (0.021 g, 0.294 mmol) and acetaldehyde (0.016 ml, 0.294 mmol).
Rf 0.26 [hexane:ethyl acetate=9:1 (v/v)];
$^1$H NMR ($CDCl_3$, 400MHz): δ 0.94 (3H, t, J=7.3Hz), 1.16 (3H, t, J=7.3Hz), 1.62 (2H, tq, J=7.3Hz), 3.22 (2H, t, J=7.3Hz), 3.38 (2H, q, J=7.3Hz), 6.65 (2H, d, J=8.8Hz), 7.41 (2H, d, J=8.8Hz), 7.62 (1H, d, J=8.8Hz), 7.69 (1H, br), 8.22 (1H, dd, J=8.8, 2.9Hz), 8.58 (1H, d, J=2.9Hz);
MS(FAB) m/z: 361 $M^+$.

[Example 190] N-[4-(4-tert-Butoxycarbonylaminophenyl)-thiazol-2-yl]-(2-chloro-5-nitrophenyl)carboxamide

[190a] 2-Amino-4-(4-tert-butoxycarbonylaminophenyl)thiazole

tert-Butoxycarboxylic acid anhydride (2.94 g, 13.5 mmol) was added to a solution of 2-amino-4-(4-aminophenyl)thiazole (2.15 g, 11.2 mmol) in methanol (30 ml). The mixture was allowed to stand overnight. The reaction mixture was concentrated and saturated aqueous sodium bicarbonate solution (50 ml) and ethyl acetate (50 ml) were added to the residue. After the mixture was stirred for 1 hour, the mixture was extracted with ethyl acetate. The organic layer was washed with aqueous sodium chloride solution, dried over anhydrous sodium sulfate, filtered, and concentrated.

The residue was crystallized with isopropyl ether (IPE) and the resulting crystalline solid was filtered to afford the title compound (1.80 g, yield 55%).

[190b] N-[4-(4-tert-Butoxycarbonylaminophenyl)thiazol-2-yl]-(2-chloro-5-nitrophenyl)carboxamide

The title compound (0.55 g, yield 69%) was obtained according to the procedure described in Example 2 using 2-amino-4-(4-tent-butoxycarbonylaminophenyl)thiazole (0.49 g, 1.67 mmol) prepared in Example 190a, DMA (5 ml) and 2-chloro-5-nitrobenzoyl chloride (0.40 g, 1.84 mmol).
$^1$H NMR (DMSO-$d_6$, 400MHz): δ 7.52 (2H, d, J=8.7Hz), 7.56 (1H, s), 7.81 (2H, d, J=8.7Hz), 7.88 (1H, d, J=8.8Hz), 8.35 (1H, dd, J=8.8, 2.7Hz), 9.46 (1H, s);
MS(FAB) m/z: 475 $(M + H)^+$;

[Example 191] N-(3-Hydroxyphenyl)-(2-chloro-5-nitrophenyl)carboxamide

The title compound (2.19 g, yield 75%) was obtained according to the procedure described in Example 2 using 3-aminophenol (1.09 g, 10.0 mmol), DMA (20 ml) and 2-chloro-5-nitrobenzoyl chloride (2.31 g, 10.5 mmol).
$^1$H NMR (DMSO-$d_6$, 400MHz): δ 6.52-6.55 (1H, m), 7.04-7.07 (1H, m), 7.14 (1H, t, J=8.0Hz), 7.30 (1H,t, J=2.1Hz), 7.88 (1H, d, J=8.9Hz), 8.33 (1H, dd, J=8.9, 2.8Hz), 8.43 (1H, d, J=2.8Hz), 9.49 (1H, s), 10.57 (1H, s);
MS(FAB) m/z: 292 $(M + H)^+$;

[Example 192] N-[4-(4-Aminophenyl)phenyl]-(2-chloro-5-nitrophenyl)carboxamide

Anisole (1 ml) and trifluoroacetic acid (10 ml) were added to a solution of N-[4-[4-(N-tert-Butoxycarbonyl) aminophenyl]phenyl]-(2-chloro-5-nitrophenyl)carboxamide (4.37 g, 9.34 mmol) prepared in Example 87 in methylene chloride (100 ml). The mixture was stirred for 2 hours. After the reaction mixture was concentrated, saturated aqueous sodium bicarbonate solution (300 ml) and water (200 ml) were added to the residue and the mixture was stirred. After 1 hour, diisopropyl ether (50 ml) was added to the mixture and the mixture was stirred for 30 minutes. The resulting solid was filtered and dried to afford the title compouind (3.38 g, yield 98%).
$^1$H NMR (DMSO-$d_6$, 400MHz): δ 5.19 (1H, d, J=8.2Hz), 6.64 (2H, d, J=8.5Hz), 7.36 (2H, d, J=8.5Hz), 7.55 (2H, d, J=8.7Hz), 7.71 (2H, d, J=8.7Hz), 7.90 (1H, d, J=8.8Hz), 8.34 (1H, dd, J=8.8, 2.8Hz), 8.47 (1H, d, J=2.8Hz), 10.70 (1H, s);
MS(FAB) m/z: 368 $(M + H)^+$, 367 $(M)^+$

[Example 193] N-(4-Hydroxyphenyl)-(2-chloro-5-nitrophenyl)carboxamide

The title compound (6.29 g, yield 74%) was obtained according to the procedure described in Example 2 using 4-aminophenol (3.52 g, 32.3 mmol), DMA (50 ml) and 2-chloro-5-nitrobenzoyl chloride (7.45 g, 33.9 mmol).
$^1$H NMR (DMSO-$d_6$, 400MHz): δ 6.76 (2H, d, J=8.8Hz), 7.49 (2H, d, J=8.8Hz), 7.88 (1H, d, J=8.8Hz), 8.32 (1H, d, J=8.8, 2.7Hz), 8.41 (1H, d, J=2.7Hz), 9.34 (1H, s), 10.45 (1H, s);
MS(FAB) m/z: 292 $(M)^+$;

[Example 194] N-[4-[4-(N,N-Dipropylamino)phenyl]phenyl]-(2-chloro-5-nitrophenyl)carboxamide

The title compound (72 mg, yield 96%) was obtained according to the procedure described in Example 2 using 4-[4-(N,N-dipropylamino)phenyl]aniline (0.045 g, 0.17 mmol), DMA (5 ml) and 2-chloro-5-nitrobenzoyl chloride (0.044 g, 0.2 mmol).
Rf 0.00 [hexane:ethyl acetate=9:1 (v/v)];
$^1$H NMR (DMSO-$d_6$, 400MHz): δ 0.90 (6H, t, J=7.4Hz), 1.50-1.60 (4H, m), 3.27 (4H, t, J=7.4Hz), 6.70 (2H, d, J=8.8Hz), 7.47 (2H, d, J=8.8Hz), 7.58 (2H, d, J=8.7Hz), 7.72 (2H, d, J=8.7Hz), 7.90 (1H, d, J=8.8Hz), 8.35 (1H, dd, J=8.8, 2.8Hz), 8.47 (1H, d, J=2.8Hz), 10.71 (1H, s);
MS(FAB) m/z: 452 $(M + H)^+$;

[Example 195] N-[4-[4-(N-Hexylamino)phenyl]phenyl]-(2-chloro-5-nitrophenyl)carboxamide

Sodium cyanoborohydride (0.698 g, 11.1 mmol) and hexanal (1.33 ml, 11.1 mmol) were added to a suspension of N-[4-(4-aminophenyl)phenyl]-(2-chloro-5-nitrophenyl)carboxamide (2.04 g, 5.55 mmol) prepared in Example 192 in methanol (40 ml). The mixture was stirred at 0°C for 4 hours and then at room temperature for 12 hours. Saturated aqueous sodium bicarbonate solution (200 ml) was added to the reaction mixture. The resulting insoluble material was filtered, washed with water and diisopropyl ether and then dried in vacuo to afford the title compound (2.02 g, yield 82%).

Rf 0.83 [hexane:ethyl acetate=1:1 (v/v)];
$^1$H NMR (DMSO-$d_6$, 400MHz): δ 0.88 (3H, t, J=6.6Hz), 1.26-1.42 (6H, m), 1.56 (2H, m), 3.25 (2H, t, J=7.3Hz), 5.75 (1H, br), 6.63 (2H, d, J=8.8Hz), 7.41 (2H, d, J=8.8Hz), 7.56 (2H, d, J=8.8Hz), 7.71 (2H, d, J=8.8Hz), 7.90 (1H, d, J=8.8Hz), 8.35 (1H, dd, J=8.8, 2.9Hz), 8.47 (1H, d, J=2.9Hz), 10.70 (1H, s);
MS(FAB) m/z: 451 $M^+$.

[Example 196] N-[4-[4-(N-Hexyl-N-methylamino)phenyl]phenyl]-(2-chloro-5-nitrophenyl)carboxamide

Sodium hydride (0.021 g, 0.487 mmol) was added to a solution of N-[4-[4-(N-hexylamino)phenyl]phenyl]-(2-chloro-5-nitrophenyl)carboxamide (0.200 g, 0.433 mmol) prepared in Example 195 in DMF (2ml). The mixture was stirred at 0°C for 30 minutes and then methyl iodide (0.033 ml, 0.531 mmol) was added to the mixture and the resulting mixture was stirred for 15 minutes. Water (20 ml) and saturated aqueous sodium bicarbonate solution (1 ml) were added to the reaction mixture and the mixture was extracted with ethyl acetate. The organic layer was dried over anhydrous sodium sulfate and the solvent was removed. The resulting residue was purified by chromatography on a silica gel [hexane:ethyl acetate=3:1 (v/v)] to afford the title compound (0.185 g, yield 90%).
Rf 0.43 [hexane:ethyl acetate=2:1 (v/v)];
$^1$H NMR ($CDCl_3$, 400MHz): δ 0.89 (3H, t, J=6.6Hz), 1.30-1.45 (6H, m), 1.56 (2H, tt, J=7.3Hz), 3.11 (2H, t, J=7.3Hz), 3.53 (3H, s), 3.76 (1H, br), 6.60 (2H, d, J=8.8Hz), 7.13 (2H, d, J=8.1Hz), 7.31 (2H, d, J=8.1Hz), 7.36 (3H, m), 7.96 (1H, dd, J=8.8, 2.9Hz), 8.06 (1H, d, J=2.9Hz);
MS(FAB) m/z: 465 $M^+$.

[Example 197] N-[4-[4-(N-Ethyl-N-hexylamino)phenyl]phenyl]-(2-chloro-5-nitrophenyl)carboxamide

Sodium cyanoborohydride (0.150 g, 2.39 mmol), acetaldehyde (0.500 ml, 8.94 mmol) and a catalytic amount of sulufuric acid were added to a suspension of N-[4-[4-(N-hexylamino)phenyl]phenyl]-(2-chloro-5-nitrophenyl)carboxamide (0.168 g, 0.352 mmol) prepared in Example 195 in methanol (2 ml). The mixture was stirred at 0°C for 4 hours and then at room temperature for 14 hours. Saturated aqueous sodium bicarbonate solution (2 ml) and water (20 ml) were added to the reaction mixture. The resulting insoluble material was filtered, washed with water and diisopropyl ether and then dried in vacuo to afford the title compound (0.112 g, yield 62%).
Rf 0.19 [hexane:ethyl acetate=2:1 (v/v)];
$^1$H NMR (DMSO-$d_6$, 400MHz): δ 0.88 (3H, t, J=6.2Hz), 1.10 (3H, t, J=7.3Hz), 1.31 (6H, m), 1.55 (2H, m), 3.27 (2H, m), 3.38 (2H, q, J=7.3Hz), 6.71 (2H, d, J=8.8Hz), 7.49 (2H, d, J=8.8Hz), 7.59 (2H, d, J=8.8Hz), 7.72 (2H, d, J=8.8Hz), 7.90 (1H, d, J=8.8Hz), 8.35 (1H, dd, J=8.8, 2.9Hz), 8.47 (1H, d, J=2.9Hz), 10.71 (1H, s);
MS(FAB) m/z: 479 $M^+$.

[Example 198] N-[4-[4-(N-Hexyl-N-propylamino)phenyl]phenyl]-(2-chloro-5-nitrophenyl)carboxamide

Propionaldehyde (0.128 ml, 1.77 mmol), acetic acid (0.203 ml, 3.54 mmol) and sodium triacetoxyborohydride (0.375 g, 1.77 mmol) were added to a solution of N-[4-[4-(N-hexylamino)phenyl]phenyl]-(2-chloro-5-nitrophenyl)carboxamide (0.200 g, 0.433 mmol) prepared in Example 195 in THF (4 ml). The mixture was stirred at 0°C for 1.5 hours. Saturated aqueous sodium bicarbonate solution (10 ml) and water (20 ml) were added to the reaction mixture and the resulting mixture was extracted with ethyl acetate. The organic layer was dried over anhydrous sodium sulfate and the solvent was removed. The residue was dried in vacuo and the obtained residue was dissolved in ethanol (2 ml) and excess water was added to the solution. The resulting insoluble material was filtered, washed with water and diisopropyl ether and dried in vacuo to afford the title compound (0.158 g, yield 72%).
Rf 0.29 [hexane:ethyl acetate=3:1 (v/v)];
$^1$H NMR ($CDCl_3$, 400MHz): δ 0.90 (3H, t, J=6.6Hz), 1.02 (3H, t, J=7.3Hz), 1.28-1.45 (6H, m), 1.60 (2H, m), 1.71 (2H, m), 3.12 (2H, t, J=7.0Hz), 3.92 (2H, t, J=7.3Hz), 6.61 (2H, d, J=8.8Hz), 7.14 (2H, d, J=8.8Hz), 7.32 (2H, d, J=8.8Hz), 7.37 (3H, m), 7.94 (1H, dd, J=8.8, 2.9Hz), 8.01 (1H, d, J=2.9Hz);

[Example 199] N-[4-[4-(N-Butyl-N-hexylamino)phenyl]phenyl]-(2-chloro-5-nitrophenyl)carboxamide

The title compound (0.175 g, yield 78%) was obtained according to the procedure described in Example 198 using N-[4-[4-(N-hexylamino)phenyl]phenyl]-(2-chloro-5-nitrophenyl)carboxamide (0.200 g, 0.433 mmol) prepared in Example 195, butyraldehyde (0.160 ml, 1.77 mmol), acetic acid (0.203 ml, 3.54 mmol), sodium triacetoxyborohydride (0.375 g, 1.77 mmol) and THF (4 ml).
Rf 0.31 [hexane:ethyl acetate=1:1 (v/v)];
$^1$H NMR ($CDCl_3$, 400MHz): δ 0.91 (3H, t, J=6.6Hz), 0.97 (3H, t, J=7.3Hz), 1.30-1.44 (6H, m), 1.54-1.66 (6H, m), 3.31

(4H, m), 6.71 (2H, d, J=8.8Hz), 7.47 (2H, d, J=8.8Hz), 7.57 (2H, d, J=8.8Hz), 7.64 (2H, d, J=8.8Hz), 7.66 (1H, d, J=8.8Hz), 7.84 (1H, br), 8.26 (1H, dd, J=8.8, 2.9Hz), 8.62 (1H, d, J=2.9Hz);
MS(FAB) m/z: 508 $(M + H)^+$.

[Example 200] N-[4-[4-(N-Hexyl-N-pentylamino)phenyl]phenyl]-(2-chloro-5-nitrophenyl)carboxamide

The title compound (0.212 g, yield 92%) was obtained according to the procedure described in Example 198 using N-[4-[4-(N-hexylamino)phenyl]phenyl]-(2-chloro-5-nitrophenyl)carboxamide (0.200 g, 0.433 mmol) prepared in Example 195, valeraldehyde (0.188 ml, 1.77 mmol), acetic acid (203 ml, 3.54 mmol), sodium triacetoxyborohydride (0.375 g, 1.77 mmol) and THF (4 ml).
Rf 0.34 [hexane:ethyl acetate=1:1 (v/v)];
$^1$H NMR($CDCl_3$, 400MHz): δ 0.91 (5H, m), 1.32-1.44 (8H, m), 1.52-1.68 (6H, m), 3.30 (4H, t, J=7.3Hz), 6.71 (2H, d, J=8.8Hz), 7.47 (2H, d, J=8.8Hz), 7.58 (2H, d, J=8.8Hz), 7.65 (2H, d, J=8.8Hz), 7.66 (1H, d, J=8.8Hz), 7.84 (1H, br), 8.27 (1H, dd, J=8.8, 2.9Hz), 8.63 (1H, d, J=2.9Hz);
MS(FAB) m/z: 522 $(M + H)^+$.

[Example 201] N-[4-[4-(N,N-Dihexylamino)phenyl]phenyl]-(2-chloro-5-nitrophenyl)carboxamide

The title compound (0.128 g, yield 54%) was obtained according to the procedure described in Example 198 using N-[4-[4-(N-hexylamino)phenyl]phenyl]-(2-chloro-5-nitrophenyl)carboxamide (0.200 g, 0.433 mmol) prepared in Example 195, hexanal (0.212 ml, 1.77 mmol), acetic acid (203 ml, 3.54 mmol), sodium triacetoxyborohydride (0.375 g, 1.77 mmol) and THF (4 ml).
Rf 0.35 [hexane:ethyl acetate=1:1 (v/v)];
$^1$H NMR ($CDCl_3$, 400MHz): δ 0.91 (6H, t, J=6.6Hz), 1.28-1.42 (12H, m), 1.61 (4H, m), 3.30 (4H, t, J=7.3Hz), 6.71 (2H, d, J=8.8Hz), 7.47 (2H, d, J=8.8Hz), 7.58 (2H, d, J=8.8Hz), 7.65 (2H, d, J=8.8Hz), 7.66 (1H, d, J=8.8Hz), 7.84 (1H, br), 8.27 (1H, dd, J=8.8, 2.9Hz), 8.63 (1H, d, J=2.9Hz);
MS(FAB) m/z: 536 $(M + H)^+$.

[Example 202] N-[4-[4-(N-Pentylamino)phenyl]phenyl]-(2-chloro-5-nitrophenyl)carboxamide

Sodium triacetoxyborohydride (0.950 g, 4.49 mmol) and valeraldehyde (0.477 ml, 4.49 mmol) were added to a suspension of N-[4-(4-aminophenyl)phenyl]-(2-chloro-5-nitrophenyl)carboxamide (1.50 g, 4.08 mmol) prepared in Example 192 in methanol (30 ml). The mixture was stirred at 0°C for 4 hours. Aqueous sodium bicarbonate solution (100 ml) was added to the reaction mixture and the mixture was extracted with ethyl acetate. The organic layer was washed with saturated aqueous sodium chloride solution, dried over anhydrous sodium sulfate and the solvent was removed. The resulting residue was purified by chromatography on a silica gel column [hexane:ethyl acetate= 3:1 (v/v)] to afford the crude title compound (1.15 g). The obtained crude compound was dissolved in ethanol with heating and the insoluble material was filtered off. The filtrate was concentrated and the residue was further purified by chromatography on a silica gel column [hexane:ethyl acetate=3:1 (v/v)] to afford the title compound (0.143 g, yield 8%).
Rf 0.74 [hexane:ethyl acetate=1:1 (v/v)];
$^1$H NMR (DMSO-$d_6$, 400MHz): δ 0.90 (3H, t, J=7.0Hz), 1.35 (4H, m), 1.56 (2H, m), 3.02 (2H, t, J=7.3Hz), 5.72 (1H, br), 6.63 (2H, d, J=8.8Hz), 7.41 (2H, d, J=8.8Hz), 7.56 (2H, d, J=8.8Hz), 7.71 (2H, d, J=8.8Hz), 7.90 (1H,d,J=8.8Hz), 8.34 (1H, dd, J=8.8, 2.9Hz), 8.47 (1H, d, J=2.9Hz), 10.70 (1H, s);
MS(FAB) m/z: 437 $M^+$.

[Example 203] N-[4-[4-(N-Butylamino)phenyl]phenyl]-(2-chloro-5-nitrophenyl)carboxamide

The title compound (1.50 g, yield 86%) was obtained according to the procedure described in Example 195 using N-[4-(4-aminophenyl)phenyl]-(2-chloro-5-nitrophenyl)carboxamide (1.50 g, 4.08 mmol) prepared in Example 192, methanol (30 ml), sodium cyanoborohydride (1.54 g, 24.5 mmol) and butyraldehyde (2.20 ml, 24.5 mmol).
Rf 0.69 [hexane:ethyl acetate=1:1 (v/v)];
$^1$H NMR (DMSO-$d_6$, 400MHz): δ 0.93 (3H, t, J=7.3Hz), 1.40 (2H, tq, J=7.3, 7.3Hz), 1.54 (2H, tt, J=7.0, 7.3Hz), 3.03 (2H, t, J=7.0Hz), 5.74 (1H, br), 6.64 (2H, d, J=8.4Hz), 7.42 (2H, d, J=8.4Hz), 7.56 (2H, d, J=8.4Hz), 7.71 (2H, d, J=8.4Hz), 7.90 (1H, d, J=8.8Hz), 8.35 (1H, dd, J=8.8, 2.9Hz), 8.47 (1H, d, J=2.9Hz), 10.70 (1H, s);
MS(FAB) m/z: 423 $M^+$.

[Example 204] N-[4-[4-(N-Propylamino)phenyl]phenyl]-(2-chloro-5-nitrophenyl)carboxamide

The title compound (0.988 g, yield 89%) was obtained according to the procedure described in Example 195 using N-[4-(4-aminophenyl)phenyl]-(2-chloro-5-nitrophenyl)carboxamide (1.00 g, 2.71 mmol) prepared in Example 192, methanol (20 ml), sodium cyanoborohydride (1.03 g, 16.3 mmol) and propionaldehyde (1.18 ml, 16.3 mmol).
Rf 0.67 [hexane:ethyl acetate=1:1 (v/v)];
$^1$H NMR (DMSO-$d_6$, 400MHz): δ 0.95 (3H, t, J=7.3Hz), 1.58 (2H, tq, J=7.3, 7.3Hz), 3.01 (2H, t, J=7.3Hz), 5.78 (1H, br), 6.64 (2H, d, J=8.8Hz), 7.42 (2H, d, J=8.8Hz), 7.56 (2H, d, J=8.8Hz), 7.71 (2H, d, J=8.8Hz), 7.90 (1H, d, J=8.8Hz), 8.35 (1H, dd, J=8.8, 2.9Hz), 8.47 (1H, d, J=2.9Hz), 10.70 (1H, s);
MS(FAB) m/z: 409 $M^+$.

[Example 205] N-[4-[4-(N-Ethylamino)phenyl]phenyl]-(2-chloro-5-nitrophenyl)carboxamide

The title compound (0.714 g, yield 66%) was obtained according to the procedure described in Example 195 using N-[4-(4-aminophenyl)phenyl]-(2-chloro-5-nitrophenyl)carboxamide (1.00 g, 2.71 mmol) prepared in Example 192, methanol (20 ml), sodium cyanoborohydride (1.03 g, 16.3 mmol) and acetaldehyde (0.92 ml, 16.3 mmol).
Rf 0.65 [hexane:ethyl acetate=1:1 (v/v)];
$^1$H NMR (DMSO-$d_6$, 400MHz): δ 1.18 (3H, t, J=7.3Hz), 3.10 (2H, q, J=7.0Hz), 5.72 (1H, br), 6.63 (2H, d, J=8.8Hz), 7.42 (2H, d, J=8.8Hz), 7.56 (2H, d, J=8.8Hz), 7.71 (2H, d, J=8.8Hz), 7.90 (1H, d, J=8.8Hz), 8.34 (1H, dd, J=8.8, 2.9Hz), 8.47 (1H, d, J=2.9Hz), 10.70 (1H, s);
MS(FAB) m/z: 395 $(M + H)^+$.

[Example 206] N-[4-[4-(N-Butyl-N-pentylamino)phenyl]phenyl]-(2-chloro-5-nitrophenyl)carboxamide

The title compound (0.133 g, yield 57%) was obtained according to the procedure described in Example 198 using N-[4-[4-(N-butylamino)phenyl]phenyl]-(2-chloro-5-nitrophenyl)carboxamide (0.200 g, 0.472 mmol) prepared in Example 203, valeraldehyde (0.201 ml, 1.89 mmol), acetic acid (0.216 ml, 3.77 mmol), sodium triacetoxyborohydride (0.400 g, 1.89 mmol) and THF (4 ml).
Rf 0.36 [hexane:ethyl acetate=3:1 (v/v)];
$^1$H NMR ($CDCl_3$, 400MHz): δ 0.93 (3H, t, J=7.3Hz), 0.97 (3H, t, J=7.3Hz), 1.30-1.42 (6H, m), 1.56-1.68 (4H, m), 3.30 (4H, m), 6.71 (2H, d, J=8.8Hz), 7.47 (2H, d, J=8.8Hz), 7.58 (2H, d, J=8.8Hz), 7.65 (2H, d, J=8.8Hz), 7.66 (1H, d, J=8.8Hz), 7.84 (1H, br), 8.27 (1H, dd, J=8.8, 2.9Hz), 8.63 (1H, d, J=2.9Hz);
MS(FAB) m/z: 493 $M^+$.

[Example 207] N-[4-[4-(N,N-Dibutylamino)phenyl]phenyl]-(2-chloro-5-nitrophenyl)carboxamide

The title compound (0.202 g, yield 89%) was obtained according to the procedure described in Example 198 using N-(4'-butylamino-biphenyl-4-yl)-(2-chloro-5-nitrophenyl)carboxamide (0.200 g, 0.472 mmol) prepared in Example 203, butyraldehyde (0.170 ml, 1.89 mmol), acetic acid (0.216 ml, 3.77 mmol), sodium triacetoxyborohydride (0.400 g, 1.89 mmol) and THF (4 ml).
Rf 0.32 [hexane:ethyl acetate=3:1 (v/v)];
$^1$H NMR ($CDCl_3$, 400MHz): δ 0.97 (6H, t, J=7.3Hz), 1.38 (4H, tq, J=7.3Hz), 1.60 (4H, m), 3.31 (4H, t, J=7.7Hz), 6.71 (2H, d, J=8.8Hz), 7.47 (2H, d, J=8.8Hz), 7.58 (2H, d, J=8.0Hz), 7.65 (2H, d, J=8.0Hz), 7.66 (1H, d, J=8.8Hz), 7.84 (1H, br), 8.26 (1H, dd, J=8.8, 2.9Hz), 8.63 (1H, d, J=2.9Hz);
MS(FAB) m/z: 479 $(M + H)^+$.

[Example 208] N-[4-[4-(N-Butyl-N-propylamino)phenyl]phenyl]-(2-chloro-5-nitrophenyl)carboxamide

The title compound (0.123 g, yield 56%) was obtained according to the procedure described in Example 198 using N-[4-[4-(N-butylamino)phenyl]phenyl]-(2-chloro-5-nitrophenyl)carboxamide (0.200 g, 0.472 mmol) prepared in Example 203, propionaldehyde (0.136 ml, 1.89 mmol), acetic acid (0.216 ml, 3.77 mmol), sodium triacetoxyborohydride (0.400 g, 1.89 mmol) and THF (4 ml).
Rf 0.29 [hexane:ethyl acetate=3:1 (v/v)];
$^1$H NMR($CDCl_3$, 400MHz): δ 0.96 (6H, m), 1.38 (2H, tq, J=7.3, 7.3Hz), 1.62 (4H, m), 3.30 (4H, m), 6.71 (2H, d, J=8.8Hz), 7.47 (2H, d, J=8.8Hz), 7.58 (2H, d, J=8.0Hz), 7.65 (2H, d, J=8.0Hz), 7.66 (1H, d, J=8.8Hz), 7.84 (1H, br), 8.27 (1H, dd, J=8.8, 2.9Hz), 8.64 (1H, d, J=2.9Hz);
MS(FAB) m/z: 465 $M^+$.

[Example 209] N-[4-[4-(N-Butyl-N-ethylamino)phenyl]phenyl]-(2-chloro-5-nitrophenyl)carboxamide

The title compound (0.120 g, yield 56%) was obtained according to the procedure described in Example 198 using N-[4-[4-(N-butylamino)phenyl]phenyl]-(2-chloro-5-nitrophenyl)carboxamide (0.200 g, 0.472 mmol) prepared in Example 203, propionaldehyde (0.106 ml, 1.89 mmol), acetic acid (0.216 ml, 3.77 mmol), sodium triacetoxyborohydride (0.400 g, 1.89 mmol) and THF (4 ml).
Rf 0.27 [hexane:ethyl acetate=3:1 (v/v)];
$^1$HNMR ($CDCl_3$, 400MHz): δ 0.98 (3H, t, J=7.3Hz), 1.19 (3H, t, J=7.3Hz), 1.39 (2H, tq, J=7.3, 7.3Hz), 1.56-1.66 (4H, m), 3.30 (2H, t, J=7.3Hz), 3.41 (2H, q, J=7.3Hz), 6.73 (2H, d, J=8.8Hz), 7.47 (2H, d, J=8.8Hz), 7.58 (2H, d, J=8.0Hz), 7.65 (2H, d, J=8.0Hz), 7.66 (1H, d, J=8.8Hz), 7.84 (1H, br), 8.27 (1H, dd, J=8.8, 2.9Hz), 8.63 (1H, d, J=2.9Hz);
MS(FAB) m/z: 451 $M^+$;

[Example 210] N-[4-[4-(N-Butyl-N-methylamino)phenyl]phenyl]-(2-chloro-5-nitrophenyl)carboxamide

Sodium hydride (0.023 g, 0.519 mmol) was added to a solution of N-[4-[4-(N-butylamino)phenyl]phenyl]-(2-chloro-5-nitrophenyl)carboxamide (0.200 g, 0.472 mmol) prepared in Example 203 in DMF (2 ml). The mixture was stirred at 0°C for 30 minutes and methyl iodide (0.035 ml, 0.566 mmol) was added to the mixture. The reaction mixture was further stirred for 1 hour. Water (20 ml) was added to the reaction mixture and the mixture was extracted with ethyl acetate. The organic layer was washed with water three times and with saturated aqueous sodium chloride solution once, and then dried over anhydrous sodium sulfate. The solvent was removed and the residue was dried in vacuo. The residue was dissolved in ethanol (2 ml) and allowed to stand. The resulting crystalline solid was filtered and dried in vacuo to afford the title compound (0.120 g yield 58%).
Rf 0.41 [hexane:ethyl acetate=2:1 (v/v)];
$^1$H NMR ($CDCl_3$, 400MHz): δ 0.96 (3H, t, J=7.3Hz), 1.43 (2H, tq, J=7.3, 7.3Hz), 1.60 (2H, tt, J=7.3, 7.3Hz), 3.13 (2H, t, J=7.3Hz), 3.53 (3H, s), 6.61 (2H, d, J=8.8Hz), 7.14 (2H, d, J=8.8Hz), 7.32 (2H, d, J=8.0Hz), 7.37 (2H, d, J=8.0Hz), 7.38 (1H, d, J=8.8Hz), 7.97 (1H, dd, J=8.8, 2.9Hz), 8.06 (1H, d, J=2.9Hz);
MS(PAB) m/z: 438 $(M + H)^+$.

[Example 211] N-[4-[4-(N-Pentyl-N-propylamino)phenyl]phenyl]-(2-chloro-5-nitrophenyl)carboxamide

The title compound (0.179 g, yield 77%) was obtained according to the procedure described in Example 198 using N-[4-[4-(N-propylamino)phenyl]phenyl]-(2-chloro-5-nitrophenyl)carboxamide (0.200 g, 0.488 mmol) prepared in Example 204, valeraldehyde (0.208 ml, 1.95 mmol), acetic acid (0.223 ml, 3.90 mmol), sodium triacetoxyborohydride (0.414 g, 1.95 mmol) and THF (4 ml).
Rf 0.60 [hexane:ethyl acetate=2:1 (v/v)];
$^1$H NMR ($CDCl_3$, 400MHz): δ 0.92 (3H, t, J=7.3Hz), 0.95 (3H, t, J=7.3Hz), 1.35 (4H, m), 1.63 (4H, m), 3.29 (4H, m), 6.61 (2H, d, J=8.8Hz), 7.47 (2H, d, J=8.8Hz), 7.57 (2H, d, J=8.8Hz), 7.64 (2H, d, J=8.8Hz), 7.66 (1H, d, J=8.8Hz), 7.83 (1H, br), 8.26 (1H, dd, J=8.8, 2.9Hz), 8.63 (1H, d, J=2.9Hz);

[Example 212] N-[4-[4-(N-Ethyl-N-propylamino)phenyl]phenyl]-(2-chloro-5-nitrophenyl)carboxamide

Acetaldehyde (0.109 ml, 1.95 mmol), acetic acid (0.223 ml, 3.90 mmol), and sodium triacetoxyborohydride (0.414 g, 1.95 mmol) were added to a solution of N-[4-[4-(N-propylamino)phenyl]phenyl]-(2-chloro-5-nitrophenyl)carboxamide (0.200 g, 0.488 mmol) prepared in Example 204 in THF (4 ml). The mixture was stirred at 0°C for 1.5 hours. To the reaction mixture was added saturated aqueous sodium bicarbonate solution (10 ml) and water (20 ml) and the resulting mixture was extracted with ethyl acetate. The organic layer was washed with saturated aqueous sodium chloride solution and then dried over anhydrous sodium sulfate. The solvent was removed and the residue was dried in vacuo. The residue was dissolved in ethanol (2 ml) and allowed to stand. The resulting crystalline solid was filtered and dried in vacuo to afford the title compound (0.124 g yield 58%).
Rf 0.41 [hexane:ethyl acetate=2:1 (v/v)];
$^1$H NMR ($CDCl_3$, 400MHz): δ 0.96 (3H, t, J=7.3Hz), 1.19 (3H, t, J=7.3Hz), 1.66 (2H, tq, J=7.3, 7.3Hz), 3.27 (2H, t, J=7.3Hz), 3.42 (2H, q, J=7.3Hz), 6.73 (2H, d, J=8.8Hz), 7.47 (2H, d, J=8.8Hz), 7.58 (2H, d, J=8.8Hz), 7.65 (2H, d, J=8.8Hz), 7.66 (1H, d, J=8.8Hz), 7.85 (1H, br), 8.26 (1H, dd, J=8.8, 2.9Hz), 8.63 (1H, d, J=2.9Hz);

[Example 213] N-[4-[4-(N-Methyl-N-propylamino)phenyl]phenyl]-(2-chloro-5-nitrophenyl)carboxamide

Sodium hydride (0.023 g, 0.537 mmol) was added to a solution of N-[4-[4-(N-propylamino)phenyl]phenyl]-(2-chloro-5-nitrophenyl)carboxamide (0.200 g, 0.488 mmol) prepared in Example 204 in DMF (2 ml). The mixture was

stirred at 0°C for 30 minutes and methyl iodide (0.037 ml, 0.586 mmol) was added to the mixture. The resulting mixture was further stirred for 1 hour. Water (20 ml) was added to the reaction mixture and the mixture was extracted with ethyl acetate. The organic layer was washed with water three times and with saturated aqueous sodium chloride solution once and then dried over anhydrous sodium sulfate. The solvent was removed and the residue was dried in vacuo. The resulting solid was filtered, washed with ethanol and dried in vacuo to afford the title compound (0.119 g yield 58%).
Rf 0.31 [hexane:ethyl acetate=2:1 (v/v)];
$^1$H NMR ($CDCl_3$, 400MHz): δ 1.00 (3H, t, J=7.3Hz), 1.65 (2H, tq, J=7.3, 7.3Hz), 3.10 (2H, t, J=7.3Hz), 3.53 (3H, s), 3.78 (1H, br), 6.61 (2H, d, J=8.8Hz), 7.13 (2H, d, J=8.8Hz), 7.32 (2H, d, J=8.8Hz), 7.37 (2H, d, J=8.8Hz), 7.38 (1H, d, J=8.8Hz), 7.98 (1H, dd, J=8.8, 2.9Hz), 8.06 (1H, d, J=2.9Hz);

[Example 214] N-[4-[4-(N,N-Dipentylamino)phenyl]phenyl]-(2-chloro-5-nitrophenyl)carboxamide

The title compound (0.034 g, yield 34%) was obtained according to the procedure described in Example 198 using N-[4-[4-(N-pentylamino)phenyl]phenyl]-(2-chloro-5-nitrophenyl)carboxamide (0.086 g, 0.197 mmol) prepared in Example 202, valeraldehyde (0.084 ml, 0.789 mmol), acetic acid (0.090 ml, 1.58 mmol), sodium triacetoxyborohydride (0.167 g, 0.789 mmol) and THF (1.5 ml).
Rf 0.65 [hexane:ethyl acetate=2:1 (v/v)];
$^1$H NMR ($CDCl_3$, 400MHz): δ 0.93 (6H, t, J=7.3Hz), 1.36 (8H, m), 1.62 (4H, m), 3.30 (4H, t, J=7.3Hz), 6.71 (2H, d, J=8.8Hz), 7.47 (2H, d, J=8.8Hz), 7.58 (2H, d, J=8.8Hz), 7.65 (2H, d, J=8.8Hz), 7.66 (1H, d, J=8.8Hz), 8.27 (1H, dd, J=8.8, 2.9Hz), 8.64 (1H, d, J=2.9Hz);
MS(FAB) m/z: 507 $M^+$.

[Example 215] N-[4-[4-(N-Methyl-N-pentylamino)phenyl]phenyl]-(2-chloro-5-nitrophenyl)carboxamide

The title compound (0.142 g, yield 79%) was obtained according to the procedure described in Example 210 using N-[4-[4-(N-pentylamino)phenyl]phenyl]-(2-chloro-5-nitrophenyl)carboxamide (0.175 g, 0.400 mmol) prepared in Example 202, sodium hydride (0.019 g, 0.440 mmol), methyl iodide (0.030 ml, 0.480 mmol) and DMF (2 ml).
Rf 0.42 [hexane:ethyl acetate=2:1 (v/v)];
$^1$H NMR (CDCl3, 400MHz): δ 0.92 (3H, t, J=7.3Hz), 1.37 (4H, m), 1.62 (2H, m), 3.12 (2H, t, J=7.3Hz), 3.54 (3H, s), 6.61 (2H, d, J=8.8Hz), 7.14 (2H, d, J=8.8Hz), 7.32 (2H, d, J=8.0Hz), 7.37 (2H, d, J=8.0Hz), 7.38 (1H, d, J=8.8Hz), 7.97 (1H, dd, J=2.2, 8.8Hz), 8.06 (1H, d, J=2.2Hz);
MS(FAB) m/z: 451 $M^+$.

[Example 216] N-[4-[4-(N-Ethyl-N-pentylamino)phenyl]phenyl]-(2-chloro-5-nitrophenyl)carboxamide

The title compound (0.130 g, yield 79%) was obtained according to the procedure described in Example 198 using N-[4-[4-(N-ethylamino)phenyl]phenyl]-(2-chloro-5-nitrophenyl)carboxamide (0.139 g, 0.351 mmol) prepared in Example 205, valeraldehyde (0.149 ml, 1.40 mmol), acetic acid (0.161 ml, 2.81 mmol), sodium triacetoxyborohydride (0.298 g, 1.40 mmol) and THF (2.8 ml).
Rf 0.38 [hexane:ethyl acetate=2:1 (v/v)];
$^1$H NMR ($CDCl_3$, 400MHz): δ 0.93 (3H, t, J=7.3Hz), 1.19 (3H, t, J=7.3Hz), 1.35 (4H, m), 1.63 (2H, m), 3.29 (2H, t, J=7.3Hz), 3.41 (2H, q, J=7.3Hz), 6.73 (2H, d, J=8.8Hz), 7.47 (2H, d, J=8.8Hz), 7.58 (2H, d, J=8.0Hz), 7.65 (2H, d, J=8.0Hz), 7.66 (1H, d, J=8.8Hz), 8.26 (1H, dd, J=8.8, 2.9Hz), 8.63 (1H, d, J=2.9Hz);
MS(FAB) m/z: 465 $M^+$.

[Example 217] N-[4-[4-(N,N-Diethylamino)phenyl]phenyl]-(2-chloro-5-nitrophenyl)carboxamide

Acetaldehyde (0.113 ml, 2.02 mmol), acetic acid (0.231 ml, 4.04 mmol), and sodium triacetoxyborohydride (0.428 g, 2.02 mmol) were added to a solution ofN-[4-[4-(N-ethylamino)phenyl]phenyl]-(2-chloro-5-nitrophenyl)carboxamide (0.200 g, 0.505 mmol) prepared in Example 205 in THF (4 ml). The mixture was stirred at 0°C for 1.5 hours. To the reaction mixture was added saturated aqueous sodium bicarbonate solution (10 ml) and water (20 ml). The resulting mixture was extracted with ethyl acetate. The organic layer was washed with saturated aqueous sodium chloride solution, and then dried over anhydrous sodium sulfate. The solvent was removed and the residue was dried in vacuo. The resulting solid was filtered, washed with diisopropyl ether and dried in vacuo to afford the title compound (0.186 g yield 87%).
Rf 0.25 [hexane:ethyl acetate=2:1 (v/v)];
$^1$H NMR ($CDCl_3$, 400MHz): δ 1.20 (6H, t, J=7.3Hz), 3.40 (4H, q, J=7.3Hz), 6.75 (2H, d, J=8.8Hz), 7.48 (2H, d, J=8.8Hz), 7.58 (2H, d, J=8.0Hz), 7.65 (2H, d, J=8.0Hz), 7.67 (1H, d, J=8.8Hz), 8.26 (1H, dd, J=8.8, 2.9Hz), 8.63 (1H, d, J=2.9Hz);

MS(FAB) m/z: 423 $M^+$.

[Example 218] N-[4-[4-(N-Ethyl-N-methylamino)phenyl]phenyl]-(2-chloro-5-nitrophenyl)carboxamide

The title compound (0.141 g, yield 68%) was obtained according to the procedure described in Example 210 using N-[4-[4-(N-ethylamino)phenyl]phenyl]-(2-chloro-5-nitrophenyl)carboxamide (0.202 g, 0.505 mmol) prepared in Example 205, sodium hydride (0.024 g, 0.555 mmol), methyl iodide (0.038 ml, 0.606 mmol) and DMF (2 ml).
Rf 0.32 [hexane:ethyl acetate=2:1 (v/v)];
$^1$H NMR (CDCl3, 400MHz): δ 1.26 (3H, t, J=7.3Hz), 3.18 (2H, q, J=7.3Hz), 3.54 (3H, s), 6.62 (2H, d, J=8.8Hz), 7.14 (2H, d, J=8.8Hz), 7.32 (2H, d, J=8.0Hz), 7.38 (3H, m), 7.97 (1H, dd, J=8.8, 2.9Hz), 8.06 (1H, d, J=2.9Hz);
MS(FAB) m/z: 409 $M^+$.

[Example 219] N-[4-[4-(N-Octylamino)phenyl]phenyl]-(2-chloro-5-nitrophenyl)carboxamide

Sodium cyanoborohydride (1.54 g, 14.4 mmol) and octanal (3.82 ml, 14.4 mmol) were added to a suspension of N-[4-(4-aminophenyl)phenyl]-(2-chloro-5-nitrophenyl)carboxamide (1.50 g, 4.08 mmol) in methanol (30 ml). The mixture was stirred at room temperature for 12 hours. The reaction mixture was filtered and the resulting solid was washed sequentially with methanol, diisopropyl ether and water and then dried in vacuo to afford the title compound (1.20 g, yield 61%).
Rf 0.79 [hexane:ethyl acetate=1:1 (v/v)];
$^1$H NMR (DMSO-$d_6$, 400MHz): δ 0.87 (3H, t, J=6.6Hz), 1.20-1.40 (10H, m), 1.55 (2H, m), 3.02 (2H, t, J=7.0Hz), 5.75 (1H, br), 6.63 (2H, d, J=8.8Hz), 7.41 (2H, d, J=8.8Hz), 7.56 (2H, d, J=8.8Hz), 7.71 (2H, d, J=8.8Hz), 7.90 (1H, d, J=8.8Hz), 8.34 (1H, dd, J=8.8, 2.9Hz), 8.47 (1H, d, J=2.9Hz), 10.70 (1H, s);
MS(FAB) m/z: 479 $M^+$.

[Example 220] N-[4-[4-(N-Hexyl-N-octylamino)phenyl]phenyl]-(2-chloro-5-nitrophenyl)carboxamide mono-hydrochloride

Hexanal (0.150 ml, 1.25 mmol), acetic acid (0.143 ml, 2.50 mmol) and sodium triacetoxyborohydride (0.265 g, 1.25 mmol) were added to a solution of N-[4-[4-(N-hexylamino)phenyl]phenyl]-(2-chloro-5-nitrophenyl)carboxamide (0.150g, 0.313 mmol) prepared in Example 219 in THF (3 ml). The mixture was stirred at 0°C for 2 hours. To the reaction mixture were added saturated aqueous sodium bicarbonate solution (10 ml) and water (20 ml). The resulting mixture was extracted with ethyl acetate. The organic layer was dried over anhydrous sodium sulfate. The solvent was removed and the residue was dried in vacuo. The resulting residue was dissolved in ethanol (2 ml) and excess water was added to the mixture. The resulting insoluble material was filtered and purified by chromatography on a silica gel column [hexane: ethyl acetate=4:1 (v/v)]. The obtained compound was dissolved in diethyl ether (4 ml) and 1N hydrogen chloride/diethyl ether solution (0.5 ml) was added to the mixture. The resulting solid was filtered and dried in vacuo to afford the title compound (0.102 g, yield 54%).
$^1$H NMR (DMSO-$d_6$, 400MHz): δ 0.83 (6H, m), 1.21 (18H, m), 1.66 (2H, m), 3.91 (4H, m), 7.70-8.00 (8H, m), 7.91 (1H, d, J=8.8Hz), 8.36 (1H, dd, J=8.8, 2.9Hz), 8.50 (1H, d, J=2.9Hz);
MS(FAB) m/z: 564 $(M - Cl)^+$.

[Example 221] N-[4-[4-(N-Octyl-N-pentylamino)phenyl]phenyl]-(2-chloro-5-nitrophenyl)carboxamide mono-hydrochloride

The title compound (0.060 g yield 33%) was obtained according to the procedure described in Example 220 using N-[4-[4-(N-hexylamino)phenyl]phenyl]-(2-chloro-5-nitrophenyl)carboxamide (0.150 g, 0.313 mmol) prepared in Example 219, valeraldehyde (0.133 ml, 1.25 mmol), acetic acid (0.143 ml, 2.50 mmol) and sodium triacetoxyborohydride (0.265 g, 1.25 mmol) and THF (3 ml).
$^1$H NMR (DMSO-$d_6$, 400MHz): δ 0.82 (6H, m), 1.21 (16H ,m), 1.65 (2H, m), 3.77 (4H, m), 7.70-8.00 (8H, m), 7.91 (1H, d, J=8.8Hz), 8.36 (1H, dd, J=8.8, 2.9Hz), 8.49 (1H, d, J=2.9Hz);
MS(FAB) m/z: 550 $(M - Cl)^+$.

[Example 222] N-[4-[4-(N-Butyl-N-octylamino)phenyl]phenyl]-(2-chloro-5-nitrophenyl)carboxamide

The title compound (0.091 g, yield 54%) was obtained according to the procedure described in Example 198 using N-[4-[4-(N-hexylamino)phenyl]phenyl]-(2-chloro-5-nitrophenyl)carboxamide (0.150 g, 0.313 mmol) prepared in Example 219, butyraldehyde (0.113 ml, 1.25 mmol), acetic acid (0.143 ml, 2.50 mmol), sodium triacetoxyborohydride

(0.265 g, 1.25 mmol) and THF (3 ml).
Rf 0.65 [hexane:ethyl acetate=2:1 (v/v)];
$^1$H NMR ($CDCl_3$, 400MHz): δ 0.90 (3H, m), 0.97 (3H, t, J=7.3Hz), 1.24-1.42 (12H, m), 1.60 (4H, m), 3.30 (4H, m), 6.71 (2H, d, J=8.8Hz), 7.47 (2H, d, J=8.8Hz), 7.58 (2H, d, J=8.8Hz), 7.65 (2H, d, J=8.8Hz), 7.66 (1H, d, J=8.8Hz), 7.84 (1H, br), 8.27 (1H, dd, J=8.8, 2.9Hz), 8.64 (1H, d, J=2.9Hz);
MS(FAB) m/z: 535 $M^+$.

[Example 223] N-[4-[4-(N-Butyl-N-octylamino)phenyl]phenyl]-(2-chloro-5-nitrophenyl)carboxamide mono-hydrochloride

1N Hydrogen chloride/diethyl ether solution (0.2 ml) was added to a solution of N-[4-[4-(N-Butyl-N-octylamino) phenyl]phenyl]-(2-chloro-5-nitrophenyl)carboxamide (0.034 g, 0.063 mmol) prepared in Example 222 in diethyl ether (1 ml). The resulting solid was filtered and dried in vacuo to afford the title compound (0.028 g, yield 77%).
$^1$H NMR (DMSO-$d_6$, 400MHz): δ 0.82 (6H, m), 1.21 (14H, m), 1.64 (2H, m), 3.74 (4H, m), 7.70-8.00 (8H, m), 7.91 (1H, d, J=8.8Hz), 8.36 (1H, dd, J=8.8, 2.9Hz), 8.49 (1H, m);
MS(FAB) m/z: 536 $(M - Cl)^+$.

[Example 224] N-[4-[4-(N-Octyl-N-propylamino)phenyl]phenyl]-(2-chloro-5-nitrophenyl)carboxamide

The title compound (0.103 g, yield 63%) was obtained according to the procedure described in Example 198 using N-[4-[4-(N-hexylamino)phenyl]phenyl]-(2-chloro-5-nitrophenyl)carboxamide (0.150 g, 0.313 mmol) prepared in Example 219, propionaldehyde (0.090 ml, 1.25 mmol), acetic acid (0.143 ml, 2.50 mmol), sodium triacetoxyborohydride (0.265 g, 0.125 mmol) and THF (3 ml).
Rf 0.63 [hexane:ethyl acetate=2:1 (v/v)];
$^1$H NMR ($CDCl_3$, 400MHz): δ 0.89 (3H, t, J=7.3Hz), 0.95 (3H, t, J=7.3Hz), 1.24-1.38 (10H, m), 1.63 (4H, m), 3.28 (4H, m), 6.71 (2H, d, J=8.8Hz), 7.47 (2H, d, J=8.8Hz), 7.57 (2H, d, J=8.8Hz), 7.64 (2H, d, J=8.8Hz), 7.65 (1H, d, J=8.8Hz), 7.86 (1H, br), 8.25 (1H, dd, J=8.8, 2.9Hz), 8.62 (1H, d, J=2.9Hz);
MS(FAB) m/z: 521 $M^+$.

[Example 225] N-[4-[4-(N-Ethyl-N-octylamino)phenyl]phenyl]-(2-chloro-5-nitrophenyl)carboxamide

The title compound (0.097 g, yield 61%) was obtained according to the procedure described in Example 217 using N-[4-[4-(N-hexylamino)phenyl]phenyl]-(2-chloro-5-nitrophenyl)carboxamide (0.150 g, 0.313 mmol) prepared in Example 219, acetaldehyde (0.078 ml, 1.25 mmol), acetic acid (0.143 ml, 2.50 mmol), sodium triacetoxyborohydride (0.265 g, 0.125 mmol) and THF (3 ml).
Rf 0.61 [hexane:ethyl acetate=2:1 (v/v)];
$^1$H NMR ($CDCl_3$, 400MHz): δ 0.89 (3H, t, J=7.3Hz), 1.19 (3H, t, J=7.3Hz), 1.24-1.38 (10H, m), 1.62 (2H, m), 3.29 (2H, t, J=7.3Hz), 3.41 (2H, q, J=7.3Hz), 6.73 (2H, d, J=8.8Hz), 7.48 (2H, d, J=8.8Hz), 7.58 (2H, d, J=8.8Hz), 7.65 (2H, d, J=8.8Hz), 7.66 (1H, d, J=8.8Hz), 7.83 (1H, br), 8.27 (1H, dd, J=8.8, 2.9Hz), 8.64 (1H, d, J=2.9Hz);
MS(FAB) m/z: 507 $(M + H)^+$.

[Example 226] N-[4-[4-(N-Methyl-N-octylamino)phenyl]phenyl]-(2-chloro-5-nitrophenyl)carboxamide

The title compound (0.101 g, yield 66%) was obtained according to the procedure described in Example 210 using N-[4-[4-(N-octylamino)phenyl]phenyl]-(2-chloro-5-nitrophenyl)carboxamide (0.150 g, 0.313 mmol) prepared in Example 219, sodium hydride (0.015 g, 0.345 mmol), methyl iodide (0.023 ml, 0.375 mmol) and DMF (2 ml).
Rf 0.31 [hexane:ethyl acetate=2:1 (v/v)];
$^1$H NMR ($CDCl_3$, 400MHz): δ 0.88 (3H, t, J=7.3Hz), 1.25-1.45 (10H, m), 1.50-1.65 (4H, m), 3.12 (2H, t, J=7.3Hz), 3.53 (3H, s), 6.61 (2H, d, J=8.8Hz), 7.14 (2H, d, J=8.8Hz), 7.32 (2H, d, J=8.0Hz), 7.37 (2H, d, J=8.0Hz), 7.38 (1H, d, J=8.8Hz), 7.97 (1H, dd, J=8.8, 2.2Hz), 8.06 (1H, d, J=2.2Hz);
MS(FAB) m/z: 494 $(M + H)^+$.

[Example 227] N-[4-[4-(Pyridin-3-yl-carbonyl)piperazin-1-yl]phenyl]-2-(chloro-5-nitrophenyl)carboxamide

The title compound (0.140 g, yield 65%) was obtained according to the procedure described in Example 183 using N-[4-(piperazin-1-yl)phenyl]-(2-chloro-5-nitrophenyl)carboxamide di-hydrochloride (0.200 g, 0.416 mmol) prepared in Example 133, nicotinoyl chloride hydrochloride (0.123 g, 0.692 mmol), and pyridine (2 ml).
$^1$H NMR ($CDCl_3$, 400MHz): δ 3.20 (4H, m), 3.62 (2H, m), 3.95 (2H, m), 6.94 (2H, d, J=8.8Hz), 7.39 (1H, dd, J=5.1,

8.0Hz), 7.55 (2H, d, J=8.8Hz), 7.62 (1H, d, J=8.8Hz), 7.79 (1H, m), 8.14 (1H, br), 8.22 (1H, dd, J=8.8, 2.1Hz), 8.55 (1H, d, J=2.1Hz), 8.68 (2H, m);
MS(FAB) m/z: 466 $(M + H)^+$.

[Example 228] N-[4-[4-(Pyridin-4-ylcarbonyl)piperazin-1-yl]phenyl]-(2-chloro-5-nitrophenyl)carboxamide

The title compound (0.205 g, yield 96%) was obtained according to the procedure described in Example 183 using N-[4-(piperazin-1-yl)phenyl]-(2-chloro-5-nitrophenyl)carboxamide. di-hydrochloride (0.200 g, 0.416 mmol) prepared in Example 133, isonicotinoyl chloride hydrochloride (0.123 g, 0.692 mmol), and pyridine (2 ml).
$^1$H NMR ($CDCl_3$, 400MHz): δ 3.12 (2H, m), 3.27 (2H, m), 3.54 (2H, m), 3.95 (2H, m), 6.93 (2H, d, J=8.8Hz), 7.32 (2H, d, J=5.8Hz), 7.55 (2H, d, J=8.8Hz), 7.64 (1H, d, J=8.8Hz), 8.00 (1H, br), 8.24 (1H, dd, J=8.8, 2.1Hz), 8.58 (1H, d, J=2.1Hz), 8.72 (2H, d, J=5.8Hz);
MS(FAB) m/z: 466 $(M + H)^+$.

[Example 229] N-(Ethoxyphenyl)-(2-chloro-5-nitrophenyl)carboxamide

The title compound (0.41 g, yield 54%) was obtained according to the procedure described in Example 2 using 2-ethoxyaniline (0.33 g, 2.40 mmol), DMA (4 ml) and 2-chloro-5-nitrobenzoyl chloride (0.58 g, 2.64 mmol).
$^1$H NMR (DMSO-$d_6$, 400MHz): δ 1.35 (3H, t, J=7.0Hz), 4.10 (2H, q, J=7.0Hz), 6.98 (1H, t, J=7.5Hz), 7.09 (1H, d, J=7.5Hz), 7.18 (1H, t, J=7.5Hz), 7.87 (1H, d, J=7.5Hz), 7.87 (1H, d, J=8.6Hz), 8.33 (1H, dd, J=8.6, 2.7Hz), 8.41 (1H, d, J=2.7Hz), 9.91 (1H, s);
MS(FAB) m/z: 320 $(M + H)^+$;

[Example 230] N-[4-(2-Hydroxyethyl)phenyl]-(2-chloro-5-nitrophenyl)carboxamide

The title compound (9.18 g, yield 78%) was obtained according to the procedure described in Example 2 using 4-(2-hydroxyethyl)aniline (5.02 g, 36.6 mmol), DMA (50 ml) and 2-chloro-5-nitrobenzoyl chloride (8.45 g, 38.4 mmol).
$^1$H NMR (DMSO-$d_6$, 400MHz): δ 2.70 (2H, t, J=7.1Hz), 3.56-3.61 (2H, m), 4.64 (1H, t, J=5.2Hz), 7.22 (2H, d, J=8.4Hz), 7.60 (2H, d, J=8.4Hz), 7.89 (1H, d, J=8.8Hz), 8.33 (1H, dd, J=8.8, 2.8Hz), 8.43 (1H, d, J=2.8Hz), 10.63 (1H, s);
MS(FAB) m/z: 321 $(M + H)^+$;

[Example 231] N-[[3-[4-(Imidazol-1-yl)phenyl]aminocarbonyl]phenyl]-(2-chloro-5-nitrophenyl)carboxamide

The title compound (0.41 g, yield 89%) was obtained according to the procedure described in Example 2 using 3-amino-N-[4-(imidazol-1-yl)phenyl]benzamide (0.28 g, 1.0 mmol), DMA (10 ml) and 2-chloro-5-nitrobenzoyl chloride (0.26 g, 1.20 mmol).
$^1$H NMR (DMSO-$d_6$, 400MHz): δ 7.11 (1H, s), 7.57 (1H, t, J=7.9Hz), 7.65 (2H, d, J=8.9Hz), 7.72 (1H, s), 7.77 (1H, d, J=7.6Hz), 7.90-7.95 (4H, m), 8.22-8.26 (2H, s), 8.51 (1H, d, J=2.7Hz), 10.49 (1H, s), 10.94 (1H, s);
MS(FAB) m/z: 462 $(M + H)^+$;

[Example 232] N-[[4-[4-(N-Ethyl-N-isopropylamino)phenyl]aminosulfonyl]phenyl]-(2-chloro-5-nitrophenyl) carboxamide

The title compound (0.42 g, yield 81%) was obtained according to the procedure described in Example 2 using 4-[4-(N-ethyl-N-isopropylamino)phenylaminosulfonyl]aniline (0.33 g, 1.0 mmol) DMA (10 ml) and 2-chloro-5-nitrobenzoyl chloride (0.26 g, 1.20 mmol).
$^1$H NMR (DMSO-$d_6$, 400MHz): δ 1.03 (3H, t, J=6.9Hz), 1.08 (6H, d, J=3.3Hz), 3.14 (2H, q, J=6.9Hz), 3.8-4.0 (1H, m), 6.57 (2H, d, J=9.1Hz), 6.85 (2H, d, J=9.1Hz), 7.69 (2H, d, J=8.8Hz), 7.82 (2H, d, J=8.8Hz), 7.90 (1H, d, J=8.8Hz), 8.35 (1H, dd, J=8.8, 2.7Hz), 8.52 (1H, d, J=2.7Hz), 11.05 (1H, s);
MS(FAB) m/z: 516 $(M)^+$, 517 $(M + H)^+$;

[Example 233] N-[3-(2-Amino-thiazol-4-yl)phenyl]-(2-chloro-5-nitrophenyl)carboxamide

The title compound (2.06 g, yield 91%) was obtained according to the procedure described in Reference example 2 using N-(3-acetylphenyl)-(2-chloro-5-nitrophenyl)carboxamide (1.91 g, 6.0 mmol) prepared in example 156, thiourea (0.91 g, 12 mmol) and iodine (1.52 g, 6.0 mmol).
$^1$H NMR (DMSO-$d_6$, 400MHz): δ 7.01 (1H, s), 7.40 (1H, t, J=7.9Hz), 7.53-7.58 (2H, m), 7.90 (1H, d), 8.19 (1H, t,

J=1.7Hz), 8.35 (1H, dd, J=8.8, 2.7Hz), 8.47 (1H, d, J=2.7Hz), 10.77 (1H, s);
MS(FAB) m/z: 375 $(M + H)^+$;

[Example 234] N-[4-(3-tert-Butoxycarbonylaminophenyl)thiazol-2-yl]-(2-chloro-5-nitrophenyl)carboxamide

[234a] 2-Amino-4-(3-tent-butoxycarbonylaminophenyl)thiazole

The title compound (2.10 g, yield 97%) was obtained according to the procedure described in Example 190a using 2-amino-(3-aminophenyl)thiazole (1.43 g, 7.47 mmol), methanol (30 ml) and tert-butoxycarboxylic acid anhydride (1.80 g, 8.24 mmol).
$^1$H NMR (DMSO-$d_6$, 400MHz): δ 6.86 (1H, s), 7.03 (2H, s), 7.18-7.26 (2H, m), 7.36-7.38 (1H, m), 8.01 (1H, s), 9.33 (1H, s);

[234b] N-[4-(3-tert-Butoxycarbonylaminophenyl)thiazol-2-yl]-(2-chloro-5-nitrophenyl)carboxamide

The title compound (0.45 g, yield 95%) was obtained according to the procedure described in Example 2 using 2-amino-4-(3-tert-butoxycarbonylaminophenyl)thiazole (0.29 g, 1.0 mmol) prepared in Example 234a, DMA (5 ml) and 2-chloro-5-nitrobenzoyl chloride (0.26 g, 1.2 mmol).
$^1$H NMR (DMSO-$d_6$, 400MHz): δ 7.26-7.33 (2H, m), 7.51 (1H, dd, J=1.7, 6.8Hz), 7.65 (1H, s), 7.91 (1H, d, J=8.8Hz), 8.22 (1H, s), 8.37 (1H, dd, J=8.8, 2.7Hz), 8.60 (1H, d, J=2.7Hz), 9.43 (1H, s);
MS(FAB) m/z: 475 $(M + H)^+$;

[Example 235] N-[4-[4-(N,N-Dimethanesulfonylamino)phenyl]phenyl]-(2-chloro-5-nitrophenyl)carboxamide

Triethylamine (0.55 ml, 4.0 mmol) and methanesulfonyl chloride (0.17 ml, 2.2 mmol) were added to a solution of N-[4-(4-aminophenyl)phenyl]-(2-chloro-5-nitrophenyl)carboxamide (0.37 g, 1.0 mmol) prepared in Example 192 in THE (10 ml). The mixture was stirred for 3 hours. To the reaction mixture were added saturated aqueous sodium bicarbonate solution and ethyl acetate and the resulting mixture was stirred for 1 hour. The mixture was extracted with ethyl acetate. The organic layer was separated, washed sequentially with saturated aqueous potassium bisulfate solution and saturated aqueous sodium chloride solution, dried over anhydrous sodium sulfate, filtered and concentrated. The resulting residue was solidified by addition of diisopropyl ether and hexane and the resulting solid was filtered to afford the title compound (0.44mg, yield 83%).
$^1$H NMR (DMSO-$d_6$, 400MHz): δ 3.56 (6H, s), 7.60 (2H, d, J=8.4Hz), 7.78 (2H, d, J=8.8Hz), 7.78 (2H, d, J=8.4Hz), 7.84 (2H, d, J=8.8Hz), 7.92 (1H, d, J=8.8Hz), 8.36 (1H, dd, J=8.8, 2.7Hz), 8.50 (1H, d, J=2.7Hz), 10.86 (1H, s);
MS(FAB) m/z: 524 $(M + H)^+$;

[Example 236] N-[4-[4-(Methylaminothiocarbonylamino)phenyl]phenyl]-(2-chloro-5-nitrophenyl)carboxamide

Methyl thioisocyanate (0.18 g, 2.5 mmol) was added to a solution of N—[4-(4-aminophenyl)phenyl]-(2-chloro-5-nitrophenyl)carboxamide (0.37 g, 1.0 mmol) prepared in Example 192 in THF (5 ml). The mixture was stirred overnight. After 1 hour of addition of methanol (0.2 ml), the resulting mixture was concentrated. To the residue were added water (10 ml) and hexane (5 ml) and the mixture was stirred. The forming solid was filtered and dried to afford the title compound (042g, yield 96%).
$^1$H NMR (DMSO-$d_6$, 400MHz): δ 7.33 (2H, d, J=8.5Hz), 7.49 (2H, d, J=8.5Hz), 7.54 (2H, d, J=8.7Hz), 7.65 (2H, d, J=8.7Hz), 7.76 (1H, d, J=8.8Hz), 8.21 (1H, dd, J=8.8, 2.8Hz), 8.34 (1H, d, J=2.8Hz), 9.52 (1H, bs), 10.79 (1H, s);
MS(FAB) m/z: 440 $(M)^+$;

[Example 237] N-[4-(2,2,2-Trifluoro-1-hydroxy-1-trifluoromethyl-ethyl)phenyl]-(2-chloro-5-nitrophenyl)carboxamide

The title compound (0.62 g, yield 82%) was obtained according to the procedure described in Example 2 using 4-(2,2,2-trifluoro-1-hydroxy-1-trifluoromethylethyl)aniline (0.44 g, 1.69 mmol), DMA (5 ml) and 2-chloro-5-nitrobenzoyl chloride (0.41 g, 1.86 mmol).
$^1$H NMR (DMSO-$d_6$, 400MHz): δ 5.43 (1H, bs), 7.69 (2H, d, J=8.7Hz), 7.84 (2H, d, J=8.7Hz), 7.91 (1H, d, J=8.8Hz), 8.36 (1H, dd, J=8.8, 2.7Hz), 8.50 (1H, d, J=2.7Hz), 10.94 (1H, s);
MS(FAB) m/z: 443 $(M + H)^+$;

[Example 238] N-[4-(1-Hydroxyethyl)phenyl]-(2-chloro-5-nitrophenyl)carboxamide

Sodium borohydride (0. 04 g, 1.0 mmol) was added to a mixture of N-(4-acetylphenyl)-(2-chloro-5-nitrophenyl) carboxamide (0.32 g, 1.0 mmol) prepared in Example 155 and THF-water [9:1 (v/v), 5 ml]. The resulting mixture was stirred at room temperature for 1 hour. Ethyl acetate and saturated aqueous sodium bicarbonate solution were added to the reaction mixture and the mixture was partitioned. The organic layer was washed with saturated aqueous sodium chloride solution, dried over anhydrous sodium sulfate, filtered and concentrated. Hexane was added to the residue and the resulting solid was filtered and dried to afford the title compound (0.24 g, yield 76%).
$^1$H NMR (DMSO-$d_6$, 400MHz): δ 1.31 (3H, d, J=6.4Hz), 4.67-4.73 (1H, m), 5.13 (1H, d, J=4.7Hz), 7.33 (2H, d, J=8.5Hz), 7.63 (2H, d, J=8.5Hz), 7.89 (1H, d, J=8.8Hz), 8.34 (1H, dd, J=8.8, 2.8Hz), 8.44 (1H, d, J=2.8Hz), 10.65 (1H, s);
MS(FAB) m/z: 321 $(M + H)^+$;

[Example 239] N-[3-(1-Hydroxyethyl)phenyl]-(2-chloro-5-nitrophenyl)carboxamide

The title compound (0.24 g, yield 74%) was obtained according to the procedure described in Example 238 using N-(3-acetylphenyl)-(2-chloro-5-nitrophenyl)carboxamide (0.32 g, 1.0 mmol) prepared in Example 156, THF-water [9:1 (v/v), 5 ml] and sodium borohydride (0.04 g, 1.0 mmol).
Rf 0.00 [hexane:ethyl acetate=9:1 (v/v)];
$^1$H NMR (DMSO-$d_6$, 400MHz): δ 1.31 (3H, d, J=6.4Hz), 4.66-4.73 (1H, m), 5.18 (1H, d, J=4.1Hz), 7.09 (1H, d, J=7.7Hz), 7.29 (1H, t, J=7.7Hz), 7.54-7.57 (1H, m), 7.70 (1H, bs), 7.87 (1H, dd, J=8.8Hz), 8.32 (1H, dd, J=8.8, 2.7Hz), 8.43 (1H, d, J=2.7Hz), 10.67 (1H, s);
MS(FAB) m/z: 320 $(M)^+$;

[Example 240] N-[4-(5,7,7,10,10-Pentamethyl-7,8,9,10-tetrahydro-5H-5,13-diaza-benzo[4,5]cyclohepta[1,2-b]naphthalen-12-yl)phenyl]-(2-chloro-5-nitrophenyl)carboxamide

[240a] 4-(5,7,7,10,10-Pentamethyl-7,8,9,10-tetrahydro-5H-5,13-diaza-benzo[4,5]cyclohepta[1,2-b]naphthalen-12-yl) aniline

The Boc derivative of 4-(5,7,7,10,10-pentamethyl-7,8,9,10-tetrahydro-5H-5,13-diaza-benzo[4,5]cyclohepta [1,2-b]naphthalen-12-yl)aniline (0.59 g, 1.15 mmol), which was prepared from 4-(5,7,7,10,10-pentamethyl-7,8,9,10-tetrahydro-5H-5,13-diaza-benzo[4,5]cyclohepta[1,2-b]naphthalen-12-yl)benzoic acid (0.97 g, 2.21 mmol), tert-butyl alcohol (10 ml), DPPA (0.59 ml, 2.74 mmol) and triethylamine (0.38 ml, 2.74 mmol), was treated with 4N-hydrogen chloride/ dioxane solution (2 ml) according to the procedure described in Example 88 to afford the hydrochloride of the title compound (0.50 g). The hydrochloride was extracted with saturated aqueous sodium bicarbonate solution and ethyl acetate. The organic layer was dried over anhydrous sodium sulfate, filtered and then concentrated to afford the title compound (0.41 g, yield 87%) as an oil.
$^1$H NMR (DMSO-$d_6$, 400MHz): δ 1.05 (3H, s), 1.17 (3H, s), 1.25 (3H, s), 1.29 (3H, s), 1.60-1.65 (4H, m), 3.16 (3H, s), 5.67 (2H, s), 6.57 (2H, d, J=8.7Hz), 6.96 (1H, s), 6.99-7.10 (4H, m), 7.01 (1H, s), 7.42 (2H, d, J=8.7);

[240b] N-[4-(5,7,7,10,10-Pentamethyl-7,8,9,10-tetrahydro-5H-5,13-diaza-benzo[4,5]cyclohepta[1,2-b]naphthalen-12-yl)phenyl]-(2-chloro-5-nitrophenyl)carboxamide

The title compound (0.45 g, yield 76%) was obtained according to the procedure described in Example 2 using 4-(5,7,7,10,10-pentamethyl-7,8,9,10-tetrahydro-5H-5,13-diaza-benzo[4,5]cyclohepta[1,2-b]naphthalen-12-yl) aniline (0.41 g, 1.00 mmol) prepared in Example 240a, DMA (5 ml) and 2-chloro-5-nitrobenzoyl chloride (0.25 g, 1.13 mmol).
$^1$H NMR (DMSO-$d_6$, 400MHz): δ 1.04 (3H, s), 1.16 (3H, s), 1.26 (3H, s), 1.31 (3H, s), 1.57-1.66 (4H, m), 3.21 (3H, s), 6.95 (1H, s), 7.06 (1H, s), 7.06-7.19 (4H, m), 7.73 (2H, d, J=8.8Hz), 7.82 (2H, d, J=8.8Hz), 7.91 (1H, d, J=8.8Hz), 8.36 (1H, dd, J=8.8, 2.7Hz), 8.54 (1H, d, J=2.7Hz), 10.96 (1H, s);
MS(FAB) m/z: 593 $(M + H)^+$, 592 $(M)^+$;

[Example 241] N-[4-(4-Aminophenyl)thiazol-2-yl]-(2-chloro-5-nitrophenyl)carboxamide

The title compound (0.24 g, yield 86%) was obtained according to the procedure described in Example 192 using N-[4-(4-tert-butoxycarbonylaminophenyl)thiazol-2-yl]-(2-chloro-5-nitrophenyl)carboxamide (0.36 g, 0.75 mmol) prepared in Example 190, methylene chloride (10 ml), anisole (0.1 ml) and trifluoroacetic acid (1 ml).
$^1$H NMR (DMSO-$d_6$, 400MHz): δ 5.26-5.27 (2H, m), 6.59 (2H, d, J=8.6Hz), 7.34 (1H, s), 7.59 (2H, d, J=8.6Hz), 7.90

(1H, d, J=8.9Hz), 8.37 (1H, dd, J=8.9, 2.8Hz), 8.58 (1H, d, J=2.8Hz);
MS(FAB) m/z: 375 $(M + H)^+$;

[Example 242] N-[4-[4-(Phenylaminocarbonylamino)phenyl]phenyl]-(2-chloro-5-nitrophenyl)carboxamide

The title compound (0.45 g, yield 93%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and phenyl isocyanate (0.13 ml, 1.2 mmol).
$^1$H NMR (DMSO-$d_6$, 400MHz): δ 6.98 (1H, t, J=7.7Hz), 7.29 (2H, d, J=7.7Hz), 7.47 (2H, d, J=7.7Hz), 7.55 (2H, d, J=8.3Hz), 7.62 (2H, d, J=8.4Hz), 7.67 (2H, d, J=8.4Hz), 7.78 (2H, d, J=8.3Hz), 7.91 (1H, d, J=8.8Hz), 7.35 (1H, d, J=8.8Hz), 8.49 (1H, bs), 8.69 (1H, s), 8.77 (1H, s), 10.78 (1H, s);
MS(FAB) m/z: 487 $(M + H)^+$;

[Example 243] N-[4-[4-(Aminocarbonylamino)phenyl]phenyl]-(2-chloro-5-nitrophenyl)carboxamide

The title compound (0.37 g, yield 90%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and trimethylsilyl isocyanate (0.16 ml, 1.2 mmol).
$^1$H NMR (DMSO-$d_6$, 400MHz): δ 5.87 (1H, s), 7.48 (2H, d, J=8.8Hz), 7.55 (2H, d, J=8.8Hz), 7.64 (2H, d, J=8.7Hz), 7.76 (2H, d, J=8.7Hz), 7.91 (1H, d, J=8.8Hz), 8.35 (1H, dd, J=8.8, 2.8Hz), 8.48 (1H, d, J=2.8Hz), 8.61 (1H, s), 10.77 (1H, s);
MS(FAB) m/z: 411 $(M + H)^+$;

[Example 244] N-[4-[4-(Ethoxycarbonylaminocarbonylamino)phenyl]phenyl]-(2-chloro-5-nitrophenyl)carboxamide

The title compound (0.47 g, yield 97%) was obtained according to the procedure described in Example 236 using N-[4-(4-aminophenyl)phenyl]-(2-chloro-5-nitrophenyl)carboxamide (0.37 g, 1.0 mmol), THF (5 ml) and ethoxycarbonyl isocyanate (0.12 ml, 1.2 mmol).
$^1$H NMR (DMSO-$d_6$, 400MHz): δ 1.14 (3H, t, J=7.1Hz), 4.20 (2H, q, J=7.1Hz), 7.60 (2H, d, J=8.8Hz), 7.65 (2H, d, J=8.8Hz), 7.68 (2H, d, J=8.7Hz), 7.79 (2H, d, J=8.7Hz), 7.91 (1H, d, J=8.8Hz), 8.35 (1H, dd, J=8.8, 2.8Hz), 8.49 (1H, d, J=2.8Hz), 9.93 (1H, s), 10.38 (1H, s), 10.79 (1H, s);
MS(FAB) m/z: 483 $(M + H)^+$; 483 $(M + H)^+$;

[Example 245] N-[4-[4-[(3-Fluorophenyl)aminothiocarbonylamino]phenyl]phenyl]-(2-chloro-5-nitrophenyl) carboxamide

The title compound (0.49 g, yield 95%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and 3-fluorophenyl isocyanate (0.15 ml, 1.2 mmol).
$^1$H NMR (DMSO-$d_6$, 400MHz): δ 6.92-6.98 (1H, m), 7.28 (1H, d, J=8.1Hz), 7.34-7.40 (1H, m), 7.58 (1H, d, J=8.4Hz), 7.59 (1H, s), 7.67 (2H, d, J=8.7Hz), 7.71 (2H, d, J=8.7Hz), 7.80 (2H, d, J=8.7Hz), 7.91 (1H, d, J=8.8Hz), 8.36 (1H, dd, J=8.8, 2.8Hz), 8.49 (1H, d, J=2.8Hz), 10.01 (1H, s), 10.81 (1H, s);
MS(FAB) m/z: 521 $(M + H)^+$;

[Example 246] N-[4-(4-Nitrophenyl)thiazol-2-yl]-(2-chloro-5-nitrophenyl)carboxamide

The title compound (0.71 g, yield 96%) was obtained according to the procedure described in Example 2 using 2-amino-4-(4-nitrophenyl)thiazole (0.40 g, 1.82 mmol), DMA (5 ml) and 2-chloro-5-nitrobenzoyl chloride (0.47 g, 2.13 mmol).
$^1$H NMR (DMSO-$d_6$, 400MHz): δ 7.91 (1H, d, J=8.8Hz), 8.12 (1H, bs), 8.20 (2H, d, J=9.0Hz), 8.32 (2H, d, J=9.0Hz), 8.37 (1H, dd, J=8.8, 2.7Hz), 8.62 (1H, d, J=2.7Hz);
MS(FAB) m/z: 405 $(M + H)^+$;

[Example 247] N-[4-[4-[(3-Methoxyphenyl)aminocarbonylamino]phenyl]phenyl]-(2-chloro-5-nitrophenyl)carboxamide

The title compound (0.50 g, yield 97%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and 3-methoxyphenyl isocyanate (0.16 ml, 1.2 mmol).

$^1H$ NMR (DMSO-$d_6$, 400MHz): δ 3.74 (3H, s), 6.55-6.58 (1H, m), 6.93-6.96 (1H, m), 7.16-7.21 (2H, m), 7.54 (2H, d, J=8.7Hz), 7.61 (1H, d, J=8.7Hz), 7.67 (2H, d, J=8.7Hz), 7.78 (2H, d, J=8.7Hz), 7.91 (1H, d, J=8.8Hz), 8.35 (1H, dd, J=8.8, 2.8Hz), 8.49 (1H, d, J=2.8Hz), 8.71 (1H, s), 8.76 (1H, s), 10.78 (1H, s);
MS(FAB) m/z: 517 $(M + H)^+$;

[Example 248] N-[4-[4-(Benzylaminocarbonylamino)phenyl]phenyl]-(2-chloro-5-nitrophenyl)carboxamide

The title compound (0.49 g, yield 98%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and benzyl isocyanate (0.15 ml, 1.2 mmol).
$^1H$ NMR (DMSO-$d_6$, 400MHz): δ 4.32 (2H, d, J=5.8Hz), 6.65 (1H, t, J=5.8Hz), 7.23-7.27 (1H, m), 7.32-7.37 (4H, m), 7.50 (2H, d, J=8.7Hz), 7.56 (2H, d, J=8.7Hz), 7.65 (2H, d, J=8.7Hz), 7.76 (2H, d, J=8.7Hz), 7.91 (1H, d, J=8.8Hz), 8.35 (1H, dd, J=8.8, 2.7Hz), 8.48 (1H, d, J=2.7Hz), 8.67 (1H, s), 10.76 (1H, s);
MS(FAB) m/z: 501 $(M + H)^+$;

[Example 249] N-[4-[4-[(2,4-Difluorophenyl)aminocarbonylamino]phenyl]phenyl]-(2-chloro-5-nitrophenyl)carboxamide

The title compound (0.50 g, yield 96%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and 2,4-difluorophenyl isocyanate (0.19 g, 1.2 mmol).
$^1H$ NMR (DMSO-$d_6$, 400MHz): δ 7.00-7.06 (1H, m), 7.29 (1H, ddd, J=11.4, 8.9, 2.7Hz), 7.51 (2H, d, J=8.7Hz), 7.60 (2H, d, J=8.7Hz), 7.65 (2H, d, J=8.7Hz), 7.75 (2H, d, J=8.7Hz), 8.04-8.12 (1H, m), 8.32 (1H, dd, J=8.8, 2.7Hz), 8.46 (1H, d, J=2.7Hz), 8.51 (1H, s), 9.10 (1H, bs), 10.75 (1H, s);
MS(FAB) m/z: 523 $(M + H)^+$;

[Example 250] N-[4-[4-(Benzoylaminothiocarbonylamino)phenyl]phenyl]-(2-chloro-5-nitrophenyl)carboxamide

The title compound (0.51 g, yield 96%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and benzoyl isocyanate (0.16 ml, 1.2 mmol).
$^1H$ NMR (DMSO-$d_6$, 400MHz): δ 7.56 (2H, t, J=7.5Hz), 7.68 (1H, t, J=7.5Hz), 7.74-7.76 (4H, m), 7.81-7.83 (4H, m), 7.92 (1H, d, J=8.8Hz), 8.00 (2H, d, J=7.5Hz), 8.36 (1H, dd, J=8.8, 2.8Hz), 8.50 (1H, d, J=2.8Hz), 10.83 (1H, s), 11.60 (1H, s);
MS(FAB) m/z: 531 $(M + H)^+$;

[Example 251] N-[4- [4-(Ethoxycarbonylaminothiocarbonylamino)phenyl]phenyl]-(2-chloro-5-nitrophenyl)carboxamide

The title compound (0.48 g, yield 96%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and ethoxycarbonyl isothiocyanate (0.14 ml, 1.2 mmol).
$^1H$ NMR (DMSO-$d_6$, 400MHz): δ 1.27 (3H, t, J=7.1Hz), 4.23 (2H, q, J=7.1Hz), 7.71 (4H, s), 7.73 (2H, d, J=8.8Hz), 7.81 (2H, d, J=8.8Hz), 7.91 (1H, d, J=8.8Hz), 8.36 (1H, dd, J=8.8, 2.7Hz), 8.50 (1H, d, J=2.7Hz), 10.82 (1H, s), 11.29 (1H, s), 11.61 (1H, s);
MS(FAB) m/z: 499 $(M + H)^+$;

[Example 252] N-[4-[4-(Phenylaminothiocarbonylamino)phenyl]phenyl]-(2-chloro-5-nitrophenyl)carboxamide

The title compound (0.45 g, yield 89%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and phenyl isothiocyanate (0.24 ml, 2.0 mmol).
$^1H$ NMR (DMSO-$d_6$, 400MHz): δ 7.14 (1H, t, J=7.4Hz), 7.35 (2H, t, J=7.9Hz), 7.49-7.53 (2H, m), 7.57-7.61 (2H, m), 7.66 (2H, d, J=8.7Hz), 7.70 (2H, d, J=8.7Hz), 7.80 (2H, d, J=8.7Hz), 7.91 (1H, d, J=8.8Hz), 8.35 (1H, dd, J=8.8, 2.7Hz), 8.49 (1H, d, J=2.7Hz), 9.84 (1H, s), 9.89 (1H, bs), 10.81 (1H, s);
MS(FAB) m/z: 503 $(M + H)^+$;

[Example 253] N-[4-(3-Aminophenyl)thiazol-2-yl]-(2-chloro-5-nitrophenyl)carboxamide

The title compound (0.12 g, yield 78%) was obtained according to the procedure described in Example 192

using N-[4-(3-tert-butoxycarbonylaminophenyl)thiazol-2-yl]-(2-chloro-5-nitrophenyl)carboxamide (0.20 g, 0.43 mmol) prepared in Example 234, methylene chloride (5 ml), anisole (0.05 ml) and trifluoroacetic acid (1 ml).
$^1H$ NMR (DMSO-$d_6$, 400MHz): δ 5.15 (2H, bs), 6.52-6.55 (1H, m), 7.06 (1H, s), 7.06-7.12 (2H, m), 7.54 (1H, s), 7.91 (1H, d, J=8.9Hz), 8.37 (1H, dd, J=8.9, 2.8Hz), 8.59 (1H, d, J=2.8Hz);
MS(FAB) m/z: 375 $(M + H)^+$; 374 $(M)^+$;

[Example 254] N-[4-[4-(2-Nitrophenylaminocarbonylamino)phenyl]phenyl]-(2-chloro-5-nitrophenyl)carboxamide

The title compound (0.48 g, yield 90%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and 2-nitrophenyl isocyanate (0.20 g, 1.2 mmol).
$^1H$ NMR (DMSO-$d_6$, 400MHz): δ 7.20-7.25 (1H, m), 7.59 (2H, d, J=8.8Hz), 7.65 (2H, d, J=8.8Hz), 7.69 (2H, d, J=8.8Hz), 7.70-7.74 (1H, m), 7.79 (2H, d, J=8.8Hz), 7.91 (1H, d, J=8.8Hz), 8.11 (1H, dd, J=8.4, 1.5Hz), 8.32 (1H, dd, J=8.4, 1.5Hz), 8.35 (1H, dd, J=8.8, 2.8Hz), 8.49 (1H, d, J=2.8Hz), 9.64 (1H, bs), 9.95 (1H, bs);
MS(FAB) m/z: 532 $(M + H)^+$;

[Example 255] N-[4-(2-Amino-thiazol-4-yl)phenyl]-(2-chloro-5-nitrophenyl)carboxamide

The title compound (3.49 g, yield 62%) was obtained according to the procedure described in Reference example 2 using N-(4-acetylphenyl)-(2-chloro-5-nitrophenyl)carboxamide (4.78 g, 15.0 mmol) prepared in example 155, thiourea (0.91 g, 12 mmol) and iodine (1.52 g, 6.0 mmol).
$^1H$ NMR (DMSO-$d_6$, 400MHz): δ 6.95 (1H, s), 7.04 (2H, bs), 7.70 (2H, d, J=8.7Hz), 7.80 (2H, d, J=8.7Hz), 7.90 (1H, d, J=8.8Hz), 8.34 (1H, dd, J=8.8, 2.7Hz), 8.48 (1H, d, J=2.7Hz), 10.75 (1H, s);
MS(FAB) m/z: 375 $(M + H)^+$;

[Example 256] N-[4-[4-[(Pyridin-3-yl)aminothiocarbonylamino]phenyl]phenyl]-(2-chloro-5-nitrophenyl)carboxamide

The title compound (0.45 g, yield 89%) was obtained according to the procedure described in Example 236 using N-[4-(4-aminophenyl)phenyl]-(2-chloro-5-nitrophenyl)carboxamide (0.37 g, 1.0 mmol) prepared in Example 192, THF (5 ml) and 3-pyridinyl isothiocyanate (0.16 mg, 1.2 mmol).
$^1H$ NMR (DMSO-$d_6$, 400MHz): δ 7.38 (1H, dd, J=8.5, 4.8Hz), 7.58 (2H, d, J=8.6Hz), 7.68 (2H, d, J=8.6Hz), 7.71 (2H, d, J=8.7Hz), 7.80 (2H, d, J=8.7Hz), 7.91 (1H, d, J=8.9Hz), 7.96-7.98 (1H, m), 8.33 (1H, dd, J=4.8, 1.4Hz), 8.36 (1H, dd, J=8.9, 2.8Hz), 8.49 (1H, d, J=2.8Hz), 8.63 (1H, d, J=2.4Hz), 9.92 (1H, bs), 10.11 (1H, s), 10.81(1H, s);
MS(FAB) m/z: 504 $(M+H)^+$,

[Example 257] N-[4-[(Pyridin-3-yl)aminothiocarbonylamino]phenyl]-(2-chloro-5-nitrophenyl)carboxamide

The title compound (0.39 g, yield 91%) was obtained according to the procedure described in Example 236 using N-(4-aminophenyl)-(2-chloro-5-nitrophenyl)carboxamide (0.29 g, 1.0 mmol) prepared in Example 180, THF (5 ml) and 3-pyridinyl isothiocyanate (0.16 mg, 1.2 mmol).
$^1H$ NMR (DMSO-$d_6$, 400MHz): δ 7.37 (1H, dd, J=8.1, 4.7Hz), 7.46 (2H, d, J=8.6Hz), 7.68 (2H, d, J=8.6Hz), 7.90 (1H, d, J=8.8Hz), 7.95 (1H, d, J=7.9Hz), 8.31-8.36 (2H, m), 8.46 (1H, d, J=2.7Hz), 8.61 (1H, d, J=2.1Hz), 9.80 (1H, bs), 10.00 (1H, s), 10.74 (1H, s);
MS(FAB) m/z: 428 $(M + H)^+$;

[Example 258] N-[4-[4-[(Pyridin-4-yl)aminothiocarbonylamino]phenyl]phenyl]-(2-chloro-5-nitrophenyl)carboxamide

The title compound (0.19 g, yield 94%) was obtained according to the procedure described in Example 236 using N-[4-(4-aminophenyl)phenyl]-(2-chloro-5-nitrophenyl)carboxamide (0.15 g, 0.40 mmol) prepared in Example 192, THF (5 ml) and 4-pyridinyl isothiocyanate (0.06 mg, 1.2 mmol).
$^1H$ NMR (DMSO-$d_6$, 400MHz): δ 7.59 (2H, d, J=8.6Hz), 7.64 (2H, d, J=5.2Hz), 7.68 (2H, d, J=8.6Hz), 7.71 (2H, d, J=8.7Hz), 7.80 (2H, d, J=8.7Hz), 7.91 (1H, d, J=8.8Hz), 8.36 (1H, dd, J=8.8, 2.7Hz), 8.42 (2H, d, J=5.2Hz), 8.49 (1H, d, J=2.7Hz), 10.23 (1H, bs), 10.81 (1H, s);
MS(FAB) m/z: 504 $(M + H)^+$;

[Example 259] N-[4-[(Pyridin-4-yl)aminothiocarbonylamino]phenyl]-(2-chloro-5-nitrophenyl)carboxamide

The title compound (0.16 g, yield 94%) was obtained according to the procedure described in Example 236

using N-(4-aminophenyl)-(2-chloro-5-nitrophenyl)carboxamide (0.12 g, 0.40 mmol) prepared in Example 180, THF (5 ml) and 4-pyridinyl isothiocyanate (0.06 mg, 1.2 mmol).
$^1$H NMR (DMSO-$d_6$, 400MHz): δ 7.47 (2H, d, J=8.8Hz), 7.64 (2H, d, J=6.0Hz), 7.69 (2H, d, J=8.8Hz), 7.90 (1H, d, J=8.8Hz), 8.35 (1H, dd, J=8.8, 2.8Hz), 8.42 (2H, d, J=6.0Hz), 8.46 (1H, d, J=2.8Hz), 10.11 (1H, s), 10.19 (1H, s), 10.75 (1H, s);
MS(FAB) m/z: 428 $(M + H)^+$;

[Example 260] N-[(6-tert-Butoxycarbonylamino)benzothiazol-2-yl]-(2-chloro-5-nitrophenyl)carboxamide

The title compound (6.09 g, yield 85%) was obtained according to the procedure described in Example 2 using 2-amino-6-tent-butoxycarbonylaminobenzothiazole (4.23 g, 15.9 mmol), DMA (40 ml), and 2-chloro-5-nitrobenzoyl chloride (4.21 g, 19.1 mmol).
$^1$H NMR (DMSO-$d_6$, 400MHz): δ 1.50 (9H, s), 7.43 (1H, dd, J=8.7, 2.1Hz), 7.66 (1H, d, J=8.7Hz), 7.90 (1H, d, J=8.9Hz), 8.18 (1H, bs), 8.37 (1H, dd, J=8.9, 2.7Hz), 8.62 (1H, d, J=2.7Hz), 9.54 (1H, bs);
MS(FAB) m/z: 449 $(M + H)^+$;

[Example 261] N-(6-Aminobenzothiazol-2-yl]-(2-chloro-5-nitrophenyl)carboxamide

The title compound (4.14 g, yield 97%) was obtained according to the procedure described in Example 192 using N-[(6-tert-butoxycarbonylamino)benzothiazol-2-yl]-(2-chloro-5-nitrophenyl)carboxamide (5.46 g, 12.2 mmol) prepared in Example 260, anisole (1 ml) and trifluoroacetic acid (11 ml).
$^1$H NMR (DMSO-$d_6$, 400MHz): δ 7.08 (1H, dd, J=8.6, 2.0Hz), 7.55 (1H, bs), 7.68 (1H, d, J=8.6Hz), 7.92 (1H, d, J=8.9Hz), 8.39 (1H, dd, J=8.9, 2.7Hz), 8.62 (1H, d, J=2.7Hz);
MS(FAB) m/z: 349 $(M + H)^+$, 348 $(M)^+$;

[Example 262] N-[4-(4-Methanesulfonylaminophenyl)thiazol-2-yl]-(2-chloro-5-nitrophenyl)carboxamide

The title compound (0.59 g, yield 99%) was obtained according to the procedure described in Example 2 using N-[4-(4-aminophenyl)thiazol-2-yl]-(2-chloro-5-nitrophenyl)carboxamide (0.50 g, 1.32 mmol) prepared in Example 241, methanesulfonyl chloride (0.11 ml, 1.45 mmol) and pyridine (5 ml).
$^1$H NMR (DMSO-$d_6$, 400MHz): δ 3.03 (3H, s), 7.27 (2H, d, J=8.7Hz), 7.69 (1H, s), 7.89 (2H, d, J=8.7Hz), 7.91 (1H, d, J=8.9Hz), 8.38 (1H, dd, J=8.9, 2.7Hz), 8.60 (1H, d, J=2.7Hz), 9.88 (1H, s);
MS(FAB) m/z: 453 $(M + H)^+$;

[Example 263] N-[4-(4-Acetylaminophenyl)thiazol-2-yl]-(2-chloro-5-nitrophenyl)carboxamide

The title compound (0.35 g, yield 96%) was obtained according to the procedure described in Example 2 using N-[4-(4-aminophenyl)thiazol-2-yl]-(2-chloro-5-nitrophenyl)carboxamide (0.33 g, 0.88 mmol), acetyl chloride (0.07 ml, 0.96 mmol) and DMA (4 ml).
$^1$H NMR (DMSO-$d_6$, 400MHz): δ 2.06 (3H, s), 7.64 (2H, d, J=8.7Hz), 7.84 (2H, d, J=8.7Hz), 8.37 (1H, dd, J=8.8, 2.8Hz), 8.59 (1H, d, J=2.8Hz), 10.03 (1H, s);
MS(FAB) m/z: 417 $(M + H)^+$;

[Example 264] N-[4-[(4-Aminocarbonyl)piperazin-1-yl]phenyl]-(2-chloro-5-nitrophenyl)carboxamide

The title compound (0.22 g, yield 84%) was obtained according to the procedure described in Example 236 using N-[4-(piperazin-1-yl)phenyl]-(2-chloro-5-nitrophenyl)carboxamide di-hydrochloride (0.24 g, 0.65 mmol) prepared in Example 133, trimethylsilyl isocyanate (0.11 ml, 0.78 mmol) and THF (5 ml).
$^1$H NMR (DMSO-$d_6$, 400MHz): δ 3.05 (4H, m), 3.43 (4H, m,), 6.05 (2H, bs), 6.98 (2H, d, J=8.4Hz), 7.56 (2H, d, J=8.4Hz), 7.88 (1H, d, J=8.8Hz), 8.33 (1H, d, J=8.8Hz), 8.41 (1H, s), 10.49 (1H, s);
MS(FAB) m/z: 000 $(M + H)^+$;

[Example 265] N-[4-(2-Acetylamino-thiazol-4-yl)phenyl]-(2-chloro-5-nitrophenyl)carboxamide

The title compound (0.16 g, yield 78%) was obtained according to the procedure described in Example 2 using N-[4-(2-amino-thiazol-4-yl)phenyl]-(2-chloro-5-nitrophenyl)carboxamide (0.19 g, 0.50 mmol) prepared in Example 255, acetyl chloride (0.04 ml, 0.55 mmol) and DMA (5 ml).
$^1$H NMR (DMSO-$d_6$, 400MHz): δ 2.17 (3H, s), 7.54 (1H, s), 7.77 (2H, d, J=8.7Hz), 7.79-7.93 (3H, m), 8.35 (1H, dd,

J=8.9, 2.8Hz), 8.49 (1H, d, J=2.8Hz), 10.81 (1H, s);
MS(FAB) m/z: 000 $(M + H)^+$;

[Example 266] N-[3-(2-Acetylamino-thiazol-4-yl)phenyl]-(2-chloro-5-nitrophenyl)carboxamide

The title compound (0.10 mg, yield 70%) was obtained according to the procedure described in Example 2 using N-[3-(2-amino-thiazol-4-yl)phenyl]-(2-chloro-5-nitrophenyl)carboxamide (0.12 g, 0.33 mmol) prepared in Example 233, acetyl chloride (0.03 ml) and DMA (5 ml).
$^1H$ NMR (DMSO-$d_6$, 400MHz): δ 2.17 (3H, s), 7.43 (1H, t, J=7.9Hz), 7.49-7.52 (1H, m), 7.56 (1H, s), 7.65-7.68 (1H, m), 7.91 (1H, d, J=8.8Hz), 8.35 (1H, dd, J=8.8, 2.7Hz), 8.41-8.42 (1H, m), 8.49 (1H, d, J=2.7Hz), 10.78 (1H, s);
MS(FAB) m/z: 000 $(M + H)^+$;

[Example 267] N-[4-(2-Aminoethyl)phenyl]-(2-chloro-5-nitrophenyl)carboxamide

N-[4-(2-Aminoethyl)phenyl]-(2-chloro-5-nitrophenyl)carboxamide hydrochloride 0.2 hydrate (3.74 g, 10 mmol) prepared in Example 169 was added to a mixture of saturated aqueous sodium bicarbonate solution (20 ml), water (20 ml) and hexane (10 ml). The suspension mixture was stirred for 1 hour. After 1 hour, the suspension in the reaction mixture was filtered, washed with water and dried to afford the title compound (3.05 g, yield 96%).
$^1H$ NMR (DMSO-$d_6$, 400MHz): δ 2.63-2.70 (1H, m), 2.79 (27H, t, J=7.0Hz), 3.12-3.18 (1H, m), 7.21 (2H, d, J=8.4Hz), 7.62 (2H, d, J=8.4Hz), 7.89 (1H, d, J=8.8Hz), 8.33 (1H, dd, J=8.8, 2.7Hz), 8.43 (1H, d, J=2.7Hz), 10.65 (1H, s);
MS(FAB) m/z: 320 $(M + H)^+$;

[Example 268] N-[4-(2-Phenylaminocarbonylaminoethyl)phenyl]-(2-chloro-5-nitrophenyl)carboxamide

The title compound (0.35 g, yield 79%) was obtained according to the procedure described in Example 236 using N-[4-(2-aminoethyl)phenyl]-(2-chloro-5-nitrophenyl)carboxamide (0.32 g, 1.0 mmol) prepared in Example 267, THF (5 ml) and phenyl isocyanate (0.13 ml, 1.2 mmol).
$^1H$ NMR (DMSO-$d_6$, 400MHz): δ 2.86 (2H, t, J=7.3Hz), 3.66-3.71 (2H, m), 6.88 (1H, t, J=7.3Hz), 7.21 (2H, t, J=8.3Hz), 7.37 (2H, d, J=7.7Hz), 7.64 (2H, d, J=8.3Hz), 7.89 (1H, d, J=8.9Hz), 8.34 (1H, dd, J=8.9, 2.7Hz), 8.44 (1H, d, J=2.7Hz), 8.46 (1H, s), 10.66 (1H, s);
MS(FAB) m/z: 439 $(M + H)^+$;

[Example 269] N-[4-(2-Phenylaminothiocarbonylaminoethyl)phenyl]-(2-chloro-5-nitrophenyl)carboxamide

The title compound (0.35 g, yield 78%) was obtained according to the procedure described in Example 236 using N-[4-(2-aminoethyl)phenyl]-(2-chloro-5-nitrophenyl)carboxamide (0.32 g, 1.0 mmol) prepared in Example 267, THF (5 ml) and phenyl isocyanate (0.15 ml, 2.0 mmol).
$^1H$ NMR (DMSO-$d_6$, 400MHz): δ 2.87 (2H, t, J=7.3Hz), 3.70-3.72 (2H, m), 7.11 (1H, t, J=6.2Hz), 7.26-7.37 (6H, m), 7.66 (2H, d, J=8.4Hz), 7.72 (1H, bs), 7.90 (1H, d, J=8.8Hz), 8.35 (1H, dd, J=8.8, 2.8Hz), 8.45 (1H, d, J=2.8Hz), 9.57 (1H, bs), 10.67 (1H, s);
MS(FAB) m/z: 455 $(M + H)^+$;

[Example 270] N-[4-(2-Aminocarbonylaminoethyl)phenyl]-(2-chloro-5-nitrophenyl)carboxamide

The title compound (0.28 g, yield 77%) was obtained according to the procedure described in Example 236 using N-[4-(2-aminoethyl)phenyl]-(2-chloro-5-nitrophenyl)carboxamide (0.32 g, 1.0 mmol) prepared in Example 267, THF (5 ml) and trimethylsilyl isocyanate (0.16 ml, 1.2 mmol).
$^1H$ NMR (DMSO-$d_6$, 400MHz): δ 2.65 (2H, t, J=7.2Hz), 3.16-3.22 (2H, m), 5.42 (2H, s), 5.89 (1H, t, J=5.5Hz), 7.21 (2H, d, J=8.3Hz), 7.62 (2H, d, J=8.3Hz), 7.89 (1H, d, J=8.8Hz), 8.34 (1H, dd, J=8.8, 2.7Hz), 8.44 (1H, d, J=2.7Hz), 10.63 (1H, s);
MS(FAB) m/z: 363 $(M + H)^+$;

[Example 271] N-[4-(2-Phenylcarbonylaminothiocarbonylaminoethyl)phenyl]-(2-chloro-5-nitrophenyl)carboxamide

The title compound (0.40 g, yield 84%) was obtained according to the procedure described in Example 236 using N-[4-(2-aminoethyl)phenyl]-(2-chloro-5-nitrophenyl)carboxamide (0.32 g, 1.0 mmol) prepared in Example 267, THF (5 ml) and benzoyl isocyanate (0.16 ml, 1.2 mmol).
$^1H$ NMR (DMSO-$d_6$, 404MHz): δ 2.96 (2H, t, J=7.1Hz), 3.83-3.89 (2H, m), 7.30 (2H, d, J=8.4Hz), 7.51 (2H, t, J=7.7Hz),

7.61-7.67 (3H, m), 7.87-7.93 (3H, m), 8.33 (1H, dd, J=8.8, 2.8Hz), 8.45 (1H, d, J=2.8Hz), 10.68 (1H, s), 10.93 (1H, s), 11.33 (1H, s);
MS(FAB) m/z: 483 $(M + H)^+$;

[Example 272] N-[4-(2-Ethoxycarbonylaminothiocarbonylaminoethyl)phenyl]-(2-chloro-5-nitrophenyl)carboxamide

The title compound (0.41 g, yield 92%) was obtained according to the procedure described in Example 236 using N-[4-(2-aminoethyl)phenyl]-(2-chloro-5-nitrophenyl)carboxamide (0.32 g, 1.0 mmol) prepared in Example 267, THF (5 ml) and ethoxycarbonyl isocyanate (0.14 ml, 1.2 mmol).
$^1$H NMR (DMSO-$d_6$, 400MHz): δ 1.21 (3H, t, J=7.1Hz), 2.89 (2H, t, J=7.2Hz), 3.76-3.81 (2H, m), 4.14 (2H, q, J=7.1Hz), 7.26 (2H, d, J=8.4Hz), 7.64 (2H, d, J=8.4Hz), 7.89 (1H, d, J=8.8Hz), 8.34 (1H, dd, J=8.8, 2.7Hz), 8.45 (1H, d, J=2.7Hz), 9.91 (1H, bs), 10.67 (1H, s), 10.96 (1H, s);
MS(FAB) m/z: 451 $(M + H)^+$;

[Example 273] N-[4-[2-(Pyridin-3-yl)aminothiocarbonylamino)ethyl]phenyl]-(2-chloro-5-nitrophenyl)carboxamide

The title compound (0.41 g, yield 90%) was obtained according to the procedure described in Example 236 using N-[4-(2-aminoethyl)phenyl]-(2-chloro-5-nitrophenyl)carboxamide (032 g, 1.0 mmol) prepared in Example 267, THF (5 ml) and 3-pyridyl isothiocyanate (0.16 g, 1.2 mmol).
$^1$H NMR (DMSO-$d_6$, 400MHz) δ 2.87 (2H, t, J=7.3Hz), 3.66-3.77 (2H, m), 7.27 (2H, d, J=8.3Hz), 7.36 (1H, dd, J=8.1, 4.7Hz), 7.65 (2H, d, J=8.3Hz), 7.89 (1H, d, J=8.8Hz), 7.91-7.98 (2H, m), 8.30 (1H, d, J=4.7Hz), 8.34 (1H, dd, J=8.8, 2.7Hz), 8.44 (1H, d, J=2.7Hz), 8.57 (1H, d, J=2.2Hz), 9.70 (1H, bs), 10.67 (1H, s);
MS(FAB) m/z: 456 $(M + H)^+$;

[Example 274] N-[4-(Imidazo[1,2-a]pyridin-2-yl)phenyl]-(2-chloro-5-nitrophenyl)carboxamide

The title compound (0.39 g, yield 92%) was obtained according to the procedure described in Example 2 using 4-(imidazo[1,2-a]pyridin-2-yl)aniline (0.22 g, 1.07 mmol), DMA (3 ml) and 2-chloro-5-nitrobenzoyl chloride (0.26 g, 1.18 mmol).
$^1$H NMR (DMSO-$d_6$, 400MHz): δ 6.89 (1H ,m), 7.22-7.26 (1H, m), 7.57 (1H, d, J=9.3Hz), 7.79 (2H, d, J=8.6Hz), 7.91 (1H, d, J=8.9Hz), 7.98 (2H, d, J=8.6Hz), 8.35 (1H, dd, J=8.9, 2.8Hz), 8.50 (1H, d, J=2.8Hz), 8.53 (1H, d, J=6.7Hz), 10.80 (1H, s);
MS(FAB) m/z: 000 $(M + H)^+$;

[Example 275] N-[3-(2-Hydroxyethyl)phenyl]-(2-chloro-5-nitrophenyl)carboxamide

The title compound (3.81 g, yield 64%) was obtained according to the procedure described in Example 2 using N-3-(2-hydroxyethyl)aniline (2.56 g, 18.7 mmol), DMA (25 ml) and 2-chloro-5-nitrobenzoyl chloride (4.31 g, 19.6 mmol).
$^1$H NMR (DMSO-$d_6$, 400MHz): δ 2.72 (2H, t, J=7.0Hz), 3.61 (2H, q, J=7.0Hz), 4.66 (1H, t, J=7.0Hz), 7.00 (1H, d, J=7.8Hz), 7.27 (1H, d, J=7.8Hz), 7.54 (1H, d, J=7.8Hz), 7.57 (1H, s), 7.89 (1H, d, J=8.8Hz), 8.34 (1H, dd, J=8.8, 2.8Hz), 8.44 (1H, d, J=2.7Hz), 10.64 (1H, s);
MS(FAB) m/z: 321 $(M + H)^+$;

[Example 276] N-[4-[4-(N,N-Diethanesulfonylamino)phenyl]phenyl]-(2-chloro-5-nitrophenyl)carboxamide

The title compound (0.22 g, yield 40%) was obtained according to the procedure described in Example 235 using N-[4-(4-aminophenyl)phenyl]-(2-chloro-5-nitrophenyl)carboxamide (0.37 g, 1.0 mole), THF (10 ml), triethylamine (0.55 ml, 4.0 mmol) and ethanesulfonyl chloridre (0.31 ml, 2.2 mmol).
$^1$H NMR (DMSO-$d_6$, 400MHz): δ 1.36 (6H, t, J=7.1Hz), 3.68 (4H, q, J=7.1Hz), 7.55 (2H, d, J=7.7Hz), 7.60-7.85 (6H, m), 7.92 (1H, d, J=8.2Hz), 8.66 (1H, d, J=8.2Hz), 8.50 (1H, s), 10.86 (1H, s);
MS(FAB) m/z: 552 $(M + H)^+$;

[Example 277] N-[4-(3-Acetylaminophenyl)thiazol-2-yl]-(2-chloro-5-nitrophenyl)carboxamide

The title compound (0.22 g, yield 86%) was obtained according to the procedure described in Example 2 using N-[4-(3-aminophenyl)thiazol-2-yl]-(2-chloro-5-nitrophenyl)carboxamide (0.22 g, 0.60 mmol) prepared in Example 253, acetyl chloride (0.05 ml, 0.7 mmol) and DMA (5 ml).

$^1H$ NMR (DMSO-$d_6$, 400MHz): δ 2.06 (3H, s), 7.35 (1H, t, J=8.0Hz), 7.44 (1H, d, J=8.0Hz), 7.58 (1H, d, J=8.0Hz), 7.67 (1H, s), 7.91 (1H, d, J=8.8Hz), 8.26 (1H, bs), 8.37 (1H, dd, J=8.8, 2.7Hz), 8.60 (1H, d, J=2.7Hz), 10.01 (1H, s); MS(FAB) m/z: 417 $(M + H)^+$;

[Example 278] N-[4-(3-Methanesulfonylaminophenyl)thioazol-2-yl]-(2-chloro-5-nitrophenyl)carboxamide

The title compound (0.18 g, yield 65%) was obtained according to the procedure described in Example 2 using N-[4-(3-aminophenyl)thiazol-2-yl]-(2-chloro-5-nitrophenyl)carboxamide (0.22 g, 0.60 mmol) prepared in Example 260, methanesulfonyl chloride (0.05 ml, 0.7 mmol) and pyridine (5 ml).
$^1H$ NMR (DMSO-$d_6$, 400MHz): δ 3.01 (3H, s), 7.17 (1H, d, J=7.6Hz), 7.41 (1H, t, J=7.6), 7.66 (1H, d, J=7.6Hz), 7.74 (1H, s), 7.81 (1H, s), 7.91 (1H, d, J=8.4), 8.38 (1H, d, J=8.4Hz), 8.61 (1H, bs), 9.84 (1H, s):
MS(FAB) m/z: 452 $(M + H)^+$;

[Example 279] N-[4-[4-(2,5-Dimethylpyrrol-1-yl)phenyl]phenyl]-(2-chloro-5-nitrophenyl)carboxamide

The title compound (0.17 g, yield 85%) was obtained according to the procedure described in Example 2 using 4-[4-(2,5-dimethylpyrrol-1-yl)phenyl]aniline (0.12 g, 0.45 mmol), DMA (5 ml) and 2-chloro-5-nitrobenzoyl chloride (0.11 g, 0.53 mmol).
$^1H$ NMR (DMSO-$d_6$, 400MHz): δ 2.01 (6H, s), 5.82 (2H, s), 7.35 (2H, d, J=8.4Hz), 7.77-7.85 (6H, m), 7.92 (1H, d, J=8.8Hz), 8.36 (1H, dd, J=8.8, 2.7Hz), 8.51 (1H, d, J=2.7Hz), 10.85 (1H, s);
MS(FAB) m/z: 262 $(M)^+$;

[Example 280] N-(6-Acetylaminobenzothiazol-2-yl)-(2-chloro-5-nitrophenyl)carboxamide

The title compound (0.22 g, yield 69%) was obtained according to the procedure described in Example 2 using N-(6-aminobenzothiazol-2-yl)-(2-chloro-5-nitrophenyl)carboxamide (0.28 g, 0.80 mmol), acetyl chloride (0.06 ml, 0.9 mmol) and DMA (5 ml).
$^1H$ NMR (DMSO-$d_6$, 400MHz): δ 2.09 (3H, s), 7.53 (1H, d, J=8.7Hz), 7.72 (1H, d, J=8.3Hz), 7.92 (1H, dd, J=8.8, 1.7Hz), 8.37-8.40 (2H, m), 8.64 (1H, d, J=1.7Hz);
MS(FAB) m/z: 391 $(M + H)^+$;

[Example 281] N-(6-Aminocarbonylaminobenzothiazol-2-yl)-(2-chloro-5-nitrophenyl)carboxamide

The title compound (0.18 g, yield 77%) was obtained according to the procedure described in Example 236 using N-(6-aminobenzothiazol-2-yl)-(2-chloro-5-nitrophenyl)carboxamide (0.22 g, 0.60 mmol) prepared in Example 261, trimethylsilyl isocyanate (0.01 ml, 0.7 mmol) and THF (5 ml).
$^1H$ NMR (DMSO-$d_6$, 400MHz): δ 5.91 (2H, bs), 7.36 (1H, dd, J=8.8, 2.2Hz), 7.64-7.68 (1H, m), 7.92 (1H, d, J=8.8Hz), 8.18 (1H, bs), 8.38 (1H, dd, J=8.8, 2.5Hz), 8.63 (1H, d, J=2.5Hz), 8.72 (1H, bs);
MS(FAB) m/z: 392 $(M + H)^+$;

[Reference example 1] 2-Amino-4-(3,5-di-t-butyl-4-hydroxyphenyl)thiazole

Thiourea (4.56 g) was added to a solution of 3,5-di-t-butyl-4-hydroxyphenacyl bromide (9.81 g) in acetone (50 ml). The-resulting mixture was stirred at room temperature overnight. The reaction mixture was concentrated and saturated aqueous sodium bicarbonate solution and ethyl acetate were added to the residue. The mixture was stirred and partitioned. The organic layer was washed with saturated aqueous sodium chloride solution, dried over anhydrous sodium sulfate, filtered and then concentrated. The resulting residue was solidified by addition of IPE and hexane. The solid was filtered and dried to afford the title compound (8.92 g).
$^1H$-NMR (400MHz, DMSO-$d_6$, TMS): δ(ppm) 1.44 (9H, s), 6.71 (1H, s), 7.26 (1H, s), 6.96 (2H, s), 6.97 (1H, s), 7.52 (1H, s).

[Reference example 2] 2-Amino-4-(3-pyridyl)thiazole

A mixture of 3-acetylpyridine (4.37 g), thiourea (5.49 g) and iodide (9.16 g) was stirred at 105°C for 30 hours. After the reaction mixture was cooled, water and ether were added to the mixture and the mixture was stirred. The solidified reaction mixture was broken into pieces and filtered. The solid was suspended in a mixture of saturated aqueous sodium bicarbonate solution and ethyl acetate (in which the pH of the aqueous layer was adjusted to less than 8) and the mixture was stirred for 1 hour. The resulting solid was filtered and dried to afford the title compound

(3.69 g).
$^1$H-NMR (400MHz, DMSO-$d_6$, TMS): δ(ppm) 7.16 (2H, s), 7.19 (1H, s), 7.39 (1H, dd, J=8.0, 4.8Hz), 8.12 (1H, dt, J=8.0, 2.0Hz), 8.45 (1H, dd, J=4.8, 1.6Hz), 9.00 (1H, m).

[Test examples]

The following experiments were carried out according to the procedures described in Molecular Cloning (written by Sambrook, J., Fritsch, E.F. and Maniatis, T.: published from Cold Spring Harbor Laboratory Press, 1989), unless otherwise described. Further, when the commercially available reagents or kits were used, they were used according to their instructions.

(Test Example 1) Determination of the PPAR γ modulating activity.

(Procedure 1) Chemical synthesis of DNA oligomers for the primers of the polymerase chain reaction.

For Escherichia coli, as a host, the primers of the polymerase chain reaction (hereinafter "PCR") were designed according to the gene sequence of human PPAR γ 2 (GenBANK accession No. D83233). In order to insert the genes into the BamHI site of the plasmid pSG5 (STRATAGENE CLONING SYSTEMS), Bgl II sites were added upstream and downstream of the genes encoding human PPAR γ 2 protein. For this, 2 kinds of polynucleotides shown in the sequence numbers 1 and 2 in the sequence table described below (hereinafter "S1" and "AS1", respectively) were used as PCR primers.

(Procedure 2) Chemical synthesis of DNA oligomers containing PPAR γ responsive element.

In order to determine transcriptional activity via PPAR γ, 2 kinds of polynucleotides represented in sequence numbers 3 and 4 in the sequence table described below (hereinafter "S2" and "AS2", respectively) were used for construction of the reporter plasmid having PPAR responsive element. The DNA fragment inserted was designed based on the gene sequence in the promoter region of rat acylCo-A oxidase (J.D. Tugwood, EMBO J., 11 (2), pp433-439 (1992)). For insertion of the DNA fragment into the reporter plasmid pGV-P2 (TOYO INK MFG CO., LTD), a NheI site and an XhoI site were added to the S2 and AS2, respectively.

(Procedure 3) Construction of human PPAR γ expression plasmid.

A schematic diagram of the PPAR γ expression plasmid is illustrated in Fig. 2.
Using cDNA library (Clontech) derived from human adipose tissue as a template, DNA oligomers S1 and AS1 that were synthesized in the procedure 1 as primers and Ex-TaqTM (TAKARA BIO INC.) as a thermostable DNA polymerase-PCR was carried out. As a result of the PCR, the c.a. 1500 bps DNA fragments were amplified. Each cycle consisted of incubation at 94°C for 1 min for denaturation of the template, then at 55°C for 30 sec for annealing of DNA oligomers of the PCR primer, and then at 72°C for 30 sec for extension of the chain. The obtained DNA fragments with approximately 1500 base pairs were digested at the Bgl II sites and were inserted into BamHI sites of pSG5. Thus a human PPAR γ expression plasmid named pSG5-hPPAR g was obtained. The nucleotide sequence of the inserted DNA fragment was confirmed to be identical sequence to the human PPAR γ 2 reported by the dideoxy method.

(Procedure 4) Construction of reporter plasmid.

A schematic diagram of the PPAR γ expression plasmid is illustrated in Fig. 3.
Vector pGV-P2 digestion products was prepared by digestion using the restriction enzymes, NheI and XhoI, and purified by 1.0% agarose-gel electrophoresis. The DNA oligomers, S2 and AS2 obtained in Test example 2, were mixed together and were incubated in a hot bath at 94°C for 1 min. After the annealing was partially broken, they were further incubated at 25°C for 1 hr. Thus double-stranded DNA with annealing of S2 and AS2 was formed. Then, the terminus of the double-stranded DNA with annealing of S2 and AS2 was phosphorylated with DNA polynucleotide kinase (TOYOBO CO., LTD). After that, it was ligated to the pGV-P2 digestion products which were prepared previously using the NheI site and XhoI site. Thus the reporter plasmid pGV-P2-PPRE was obtained.

(Procedure 5) Transfection of the gene into the animal cells.

Using the plasmid obtained by the procedures 3 and 4, transformation of Escherichia coli HB-101 strain was carried out by conventional methods. HB-101 strain having the plasmid was incubated in L-broth medium (containing

10 g of trypton (Difco), 5 g of yeast extract (Difco), 5 g of sodium chloride in 1 L of aqueous solution) containing 100 µg/ml of ampicillin at 37°C for 17 hrs. Then, each plasmid was purified by the alkaline-SDS method and employed for the gene transfection to mammalian cells. After mixing the pSG5-hPPARg, PGV-P2-PPRE and LipofectAMINE reagent (GIBCO BRL), the human osteosarcoma MG63 was transiently transfected. Then the cells were recovered. The recovered cells were plated on a 96-well plate so as to be 30,000 cells/well and cultured in a $CO_2$ incubator (NAPCO) at 37°C for 24 hr under the condition of 5% $CO_2$, 95%-RH.

(Procedure 6) Determination of inhibitory activity of the compound against transcription.

Ten (10) nM of the following compound A (although compound A is described here as a representative compound, the compound is not limited to compound A, if the compound exerts a PPAR γ agonistic activity) and various concentrations of the test compound were added to culturing medium of the cells prepared in the Procedure 5. After 24 hr of culturing LT2.0 (TOYO INK MFG CO., LTD), a luciferase substrate, was added to the medium free culture plate. The luciferase activity was measured by a luminometer ARGUS50 (Hamamatsu Photonics CO., LTD) or Analyst (LjL Instruments). Using the data, the dose-response curve of transcriptional inhibitory activity was drawn.

(Compound A and process for preparation of compound A)

Compound A : N-[4-[2-[4-(2,4-dioxothiazolidin-5-ylmethyl)phenoxymethyl]-1-methyl-1H-benzimidazol-6-yloxy]phenyl] benzamide

Triethylamine (0.36 ml) and benzoyl chloride (0.1 ml) were added dropwise to a solution of 5-[4-[6-(4-aminophenoxy)-1-methyl-1H-benzimidazol-2-ylmethoxy]benzyl]thiazoldine-2,4-dione dihydrochloride (400 mg) in anhydrous N, N-dimethylformamide (8 ml). The resulting mixture was stirred at room temperature for 1 hour. The solvent was removed from the reaction mixture under reduced pressure. Water was added to the residue and the mixture was extracted with ethyl acetate. The extract was washed with saturated aqueous sodium chloride solution, dried over anhydrous sodium sulfate and ethyl acetate was removed from the extract. The resulting residue was chromatographed on a silica gel column [ethyl acetate : n-hexane = 1:1, 2:1, 3:1 to 4:1] to afford the title compound (247 mg) as a white powder.
melting point : 200-204°C.

(Procedure 7) Determination of the transcriptional activity.

Various concentrations of the test compound alone were added to culturing medium of the cells prepared in the Procedure 5. After 24 hr of culturing, LT2.0 (TOYO INK MFG CO., LTD), a luciferase substrate, was added to the medium free culture plate. The luciferase activity was measured by a luminometer ARGUS50 (Hamamatsu Photonics CO., LTD) or Analyst (LjL Instruments). Using the data, the dose-response curve of transcriptional activity was drawn.

(Procedure 8) Calculation of $IC_{50}$ and $EC_{50}$ values.

$EC_{50}$ and $IC_{50}$ values of partial agonists or partial antagonists were defined as the following. As demonstrated in Fig. 1, the transcriptional activity in the presence of the agonist was defined as 100%, while that in the vehicle alone was defined as 0%. The maximum transcriptional activity of the test compound alone was defined as Emax (%), while the maximum inhibition of the transcriptional activity in the presence of the agonist was defined as Imax (%). The concentration of the test compound indicating Emax/2 value was defined as the $EC_{50}$ value for the transcriptional activity. Further, the concentration of the test compound indicating 100-Imax/2 value was defined as the $IC_{50}$ value. These calculated $IC_{50}$ and $EC_{50}$ values were used for evaluation of the modulating activity of the compound. The results of the assay are summarized in Table 1.

[Table 1]

| Example No. | EC50 (nM) | IC50 (nM) | Example No. | EC50 (nM) | IC50 (nM) | Example No. | EC50 (nM) | IC50 (nM) | Example No. | EC50 (nM) | IC50 (nM) |
|---|---|---|---|---|---|---|---|---|---|---|---|
| 1 | 220 | 33 | 2 | 840 | 1.9 | 4 | 15 | 120 | 5 | 400 | 82 |
| 6 | 34 | 160 | 7 | 270 | | 8 | 260 | 5.9 | 10 | | 71 |
| 16 | 34 | 7.5 | 20 | | 15 | 26 | 2.1 | 6.3 | 29 | 4.4 | 230 |
| 30 | 1.9 | 12 | 35 | 25 | 30 | 37 | 510 | 15 | 38 | 250 | 45 |
| 39 | 39 | 40 | 40 | | 57 | 41 | 11 | 77 | 44 | | 84 |
| 46 | 810 | 54 | 47 | 420 | 38 | 49 | | 180 | 50 | | 110 |
| 51 | 6.6 | 120 | 52 | 5.6 | 210 | 54 | 4.7 | 340 | 57 | 260 | |
| 58 | 20 | 2.8 | 65 | | 37 | 66 | 120 | | 74 | 5.1 | |
| 76 | 120 | | 78 | | 84 | 79 | 68 | | 81 | 760 | 23 |
| 82 | 260 | 400 | 85 | 28 | 91 | 86 | 11 | 710 | 87 | | 33 |
| 89 | | 400 | 96 | 22 | 650 | 97 | 360 | 790 | 99 | 34 | 680 |
| 101 | | 61 | 102 | 43 | 490 | 103 | 8.8 | | 104 | | 270 |
| 105 | 87 | 710 | 106 | 49 | 360 | 107 | 88 | | 108 | 57 | 59 |
| 110 | 15 | 190 | 111 | 79 | 27 | 112 | | 14 | 116 | 11 | 55 |
| 117 | 8.7 | 120 | 118 | | 8.6 | 119 | 21 | 36 | 120 | | 13 |
| 122 | | 7.6 | 124 | | 8.1 | 125 | 19 | 240 | 126 | | 14 |

| | | | | | | | | | | | |
|---|---|---|---|---|---|---|---|---|---|---|---|
| 127 | | 11 | 128 | | 35 | 129 | | 26 | 130 | | 11 |
| 138 | 130 | 20 | 140 | | 110 | 151 | | 12 | 152 | 8.7 | 42 |
| 153 | | 49 | 154 | | 81 | 155 | | 9.8 | 156 | | 100 |
| 157 | 760 | 23 | 158 | | 82 | 159 | | 11 | 160 | | 110 |
| 164 | | 0.6 | 171 | | 48 | 174 | | 9.4 | 175 | | 99 |
| 176 | 11 | 66 | 177 | | 94 | 178 | 16 | 330 | 179 | 780 | 56 |
| 181 | 5.6 | 20 | 188 | | 33 | 189 | 0.7 | 3.6 | 190 | | 36 |
| 191 | 4.2 | 30 | 193 | 31 | | 194 | | 330 | 195 | | 18 |
| 196 | | 990 | 197 | | 280 | 218 | | | 219 | | 8.3 |
| 220 | | 18 | 221 | | 34 | 222 | | 160 | 223 | | 89 |
| 224 | | 79 | 225 | | 190 | 226 | | | 227 | | 81 |
| 228 | | 130 | 229 | 160 | 46 | 230 | | 19 | 231 | | 60 |
| 232 | 610 | 56 | 233 | | 28 | 234 | | 19 | 235 | | 19 |
| 238 | | 9.3 | 239 | 44 | 52 | 256 | | 12 | 257 | 120 | 130 |
| 258 | | 25 | 259 | 0.7 | 31 | 267 | | 60 | 268 | | 25 |
| 269 | | 30 | 273 | | 13 | | | | | | |

As shown in Table 1, the compounds of the present invention exert PPAR γ modulating activity. Thus the compounds of the present invention are useful as a preventive or a therapeutic agent for diabetes mellitus, retrograde osteoporosis, obesity or cancers.

(Test Example 2) Osteoblastogenesis assay.

In this experiment, α-MEM medium that contained 15% (v/v) of inactivated bovine fetal serum (HyClone Laboratories, Inc., FBS, Lot. AHM0419) and 1% (v/v) of penicillin-streptomycin liquid (GIBCO BRL, Cat. No. 15140-122) were used for a primary culture of murine bone marrow cells. All culturing in this experiment was carried out within a $CO_2$ incubator (37°C, 95%R.H., 5% $CO_2$).

Male BDF1 mice of 7 weeks old were purchased from Charles River Co., Ltd and were used in the following experiment. Under anesthesia with ether, the mice were sacrificed by bleeding by cutting the carotid artery, and the femur and the tibia of both sides were isolated. After the connective tissues surrounding the isolated femur and the tibia were removed, both edges of these bones were transected. A syringe needle containing 1 mL of 15%-FBS-α-MEM was inserted into the cut-end of the bone and the bone marrow was pushed out and collected. The marrow cells collected following filtration through a Cellstrainer (Falcon Co., Ltd.) were plated in a 25 $cm^2$ culture plate (SUMILON Co., Ltd., Cat. No. MS-22050), and were incubated with 5 mL of 15%-FBS-α-MEM for 7 days until they were confluent.

The cells described above were isolated using 1 mL of 0.05% trypsin-EDTA solution (GIBCO BRL, Cat No. 25200-056), and were suspended with 5 mL of 15%-FBS-α-MEM. Then the cells were recovered by centrifugation (25°C, 800 rpm, for 4 min). A cell suspension was prepared with 15%-FBS-α-MEM at a concentration of 80,000 cells/mL. A hundred (100) μL of the cell suspension was plated on each well of a 96-well microplate (SUMILON Co., Ltd.) so as to be 8,000 cells/well and were incubated for 24 hr. Further, 96 μL of 15%-FBS-α-MEM containing the compound A prepared at the final concentration of 10 nM, was added in each well. The wells were divided in the following 3 groups. In the group 1), the test compound was dissolved in DMSO solution at concentrations of 1 mM, 100 μM and 10 μM, which were diluted with 15%-FBS-α-MEM by 20 times. Four (4) μL/well of the solutions dilutedof the test compound (the final concentrations of the test compound: 1 μM, 100 nM and 10 nM) were added in each well; in the group 2), 4 μL/well of DMSO solution diluted by 20 times with 15%-FBS-α-MEM (final concentration: 0.1% (v/v)) (control group I) and in the group 3), 4 μL/well of DMSO solution diluted by 20 times with 15%-FBS-α-MEM (final concentration: 0.1% (v/v)) (control II) were added to each well. After the cells were cultured for a week, the alkaline phosphatase (ALP) activity in each well was determined.

The ALP activity was determined as follows: after all medium was removed from each well of the plate, each well was washed twice with 100 μL of Dulbecco's phosphate buffer solution (GIBCO BRL, Cat No. 14190-144). A cell lysis buffer containing 0.67 M diethanolamine (Wako Pure Chemical Industries, Ltd., Cat. No. 099-03112), 0.67 mM $MgCl_2$ and 0.1% (v/v) Triton X-100 (Sigma-Aldrich Japan K.K.) was prepared. The cell lysis buffer was divided in each well at a volume of 50 μL/well and was stirred for 5 min at room temperature. ALP substrate solution was prepared by mixing Saphirell (trade mark, TROPIX INC.) and CDP-Star (TROPIX INC.) in the cell lysis buffer so as to give final concentrations of 20% (v/v) and 6.7% (v/v), respectively. Fifty (50) μL/well of the ALP substrate solution were added into each well and stirred for 10 min at the room temperature. The luminescent intensity of each well was determined with the microplate multi-reader Analyst (LjL Instruments). The recovery rate of alkaline phosphatase activity in the control group I was defined as 100%, while that in the control group II was defined as 0%. The recovery rate of the alkaline phosphatase activity in the well to which the test compound was applied was calculated. Thus the differentiation rate of the osteoblast was estimated.

As an indicator of the inhibition of adipocyte differentiation, the adipocytes were stained as following. All medium was removed away, and 60 μL of 10% (v/v) formaldehyde solution (for fixation) were added to each well. The cells were left for 20 min at room temperature. After the fixative solution were discarded, 60 μL of 0.2% (v/v) Triton X (Sigma-Aldrich Japan K. K.) solution were added into each well and the microplate was left stationary for 5 min at room temperature. Then, the Triton X solution was discarded and 60 μL of 60% (v/v) isopropanol solution containing dissolved Oil Red O (Sigma-Aldrich Japan K. K.) at a concentration of 0.3%, which was adipose tissue staining solution was added and the microplate was left stationary for 10 min at room temperature. After the adipose tissue staining solution was discarded, the well was washed twice with 60 μL of 60% (v/v) isopropanol solution. From microscopic observation of the plate, the ratio of the stained cells in the test compound treated cells was compared with that in the control group II. And the differentiation of the adipogenesis in cells treated with the test compound was confirmed to be less than that in the cells treated with the vehicle.

Results of the recovery rate of alkaline phosphatase activities are summarized below.

[Table 2]

| Example No. | 1μM | 100nM | 10nM | Example No. | 1μM | 100nM | 10nM | Example No. | 1μM | 100nM | 10nM | Example No. | 1μM | 100nM | 10nM |
|---|---|---|---|---|---|---|---|---|---|---|---|---|---|---|---|
| 2 | 63.0 | 60.5 | 20.7 | 3 | 21.9 | | | 5 | 70.0 | 26.4 | 3.0 | 6 | 24.0 | 15.0 | |
| 7 | 24.8 | 10.6 | | 8 | 43.1 | 14.5 | | 9 | 89.2 | 48.2 | 9.1 | 10 | 29.2 | | |
| 11 | 53.1 | 15.4 | | 12 | 59.7 | 22.3 | 17.7 | 13 | 77.1 | 31.4 | 10.6 | 14 | 46.4 | 20.0 | 13.8 |
| 15 | 50.4 | 36.2 | 18.0 | 16 | 46.3 | 23.6 | | 17 | | 56.4 | 52.7 | 18 | | 55.1 | 48.5 |
| 19 | | 29.8 | | 20 | | 67.0 | 60.3 | 21 | | 38.5 | 14.0 | 22 | 68.1 | 21.5 | |
| 23 | 49.1 | 16.6 | 1.3 | 24 | | 76.6 | 42.0 | 25 | 24.4 | 12.0 | | 26 | 44.3 | 19.5 | |
| 30 | 27.4 | 14.6 | 10.8 | 32 | 38.1 | | | 33 | 22.9 | 7.9 | | 34 | 51.0 | 13.6 | 2.7 |
| 35 | 42.3 | 11.2 | | 36 | 21.8 | 6.8 | | 39 | 38.0 | | | 42 | 66.8 | 27.0 | 5.6 |
| 43 | | 64.6 | 43.7 | 45 | 21.2 | | | 46 | 44.1 | 12.3 | | 47 | 88.0 | 56.3 | |
| 48 | 56.3 | 24.9 | | 51 | 56.4 | 6.0 | | 52 | 71.2 | 18.9 | 13.9 | 53 | 89.0 | 99.2 | 87.2 |
| 54 | | 54.1 | 41.5 | 58 | 27.4 | | | 61 | 28.2 | 14.3 | 10.6 | 62 | 53.7 | 25.9 | 13.2 |
| 63 | 48.3 | | | 64 | 29.6 | | | 65 | | 32.1 | 18.4 | 77 | 36.2 | | |
| 80 | 34.9 | | | 82 | 80.6 | 73.9 | 13.2 | 84 | 57.4 | 5.5 | 9.7 | 87 | 89.5 | 39.3 | 11.0 |
| 88 | 102.4 | 97.9 | 92.7 | 89 | 37.9 | 18.9 | 15.2 | 90 | 82.4 | 59.1 | | 91 | 26.8 | 20.0 | 17.9 |
| 92 | 40.5 | 19.3 | 14.2 | 93 | 105.5 | 98.6 | 58.4 | 94 | 86.4 | 91.4 | 41.5 | 95 | 69.1 | 30.3 | 18.5 |
| 105 | 25.8 | | | 106 | 21.7 | | | 111 | 49.2 | 26.5 | 8.6 | 115 | 28.2 | 21.0 | 19.8 |

| | | | | | | | | | | | | | | | |
|---|---|---|---|---|---|---|---|---|---|---|---|---|---|---|---|
| 116 | 49.8 | 30.4 | | 118 | 87.5 | 81.3 | 45.6 | 120 | 153.7 | 91.3 | 51.4 | 121 | 24.1 | | |
| 122 | 212.4 | 123.7 | 27.8 | 123 | 34.0 | 14.5 | | 124 | 143.5 | 84.8 | 43.7 | 126 | 45.1 | 6.4 | |
| 128 | 58.8 | 65.1 | 74.6 | 130 | 91.7 | 95.6 | 63.1 | 131 | | 23.9 | | 132 | 96.4 | 79.0 | 64.9 |
| 133 | 73.7 | 97.8 | 99.3 | 134 | 72.1 | 97.3 | 114.5 | 135 | 55.8 | 78.2 | 88.6 | 136 | 30.1 | | |
| 137 | 38.7 | 93.8 | 62.9 | 138 | | 119.7 | 70.4 | 139 | 31.8 | 59.6 | 12.4 | 142 | 21.7 | | |
| 143 | 42.3 | 15.5 | | 144 | 25.5 | | | 145 | 38.8 | 47.2 | 16.5 | 146 | 58.1 | 34.6 | 19.4 |
| 147 | 53.6 | 35.9 | 7.4 | 148 | 37.2 | 10.9 | 10.8 | 150 | 50.6 | | | 155 | | 27.8 | |
| 161 | | 24.6 | | 163 | 67.9 | 73.5 | 15.0 | 164 | 76.7 | 69.9 | 25.3 | 165 | | 51.0 | 21.6 |
| 166 | 85.9 | 22.0 | | 167 | 102.8 | 32.1 | 15.0 | 168 | 43.1 | 28.6 | | 169 | 78.3 | | |
| 170 | 78.5 | 7.7 | | 171 | 83.9 | 101.7 | 114.8 | 172 | 21.7 | 20.4 | | 173 | 29.4 | 10.1 | |
| 174 | 22.4 | 46.3 | 29.0 | 176 | 20.6 | | | 177 | | 88.1 | 96.8 | 180 | 55.0 | | |
| 181 | 31.7 | | | 182 | | 48.1 | 40.9 | 183 | 79.0 | 59.0 | 19.7 | 184 | 70.4 | 65.3 | 27.5 |
| 185 | 27.7 | | | 186 | | 88.2 | 98.2 | 187 | 75.1 | 84.2 | 53.8 | 188 | 63.1 | 147.2 | 59.7 |
| 189 | | 31.7 | | 190 | 91.6 | 25.6 | | 192 | 156.7 | 97.6 | 47.0 | 194 | 63.4 | 62.8 | 11.3 |
| 195 | 93.7 | 62.2 | 30.6 | 196 | | 24.9 | 11.3 | 197 | 62.4 | 45.4 | 14.3 | 202 | 56.2 | 27.1 | |
| 203 | 53.7 | | | 204 | 89.3 | 74.5 | 38.0 | 205 | 98.3 | 64.7 | 33.7 | 206 | 46.3 | 55.6 | 32.5 |
| 207 | 47.3 | 66.8 | 28.8 | 208 | 51.9 | 48.8 | | 209 | 45.4 | | | 211 | 23.4 | 16.9 | |
| 212 | 33.7 | 34.8 | | 214 | 39.8 | | | 216 | 50.9 | 90.1 | 15.2 | 217 | 111.7 | 110.2 | 50.5 |
| 219 | 64.0 | 21.9 | | 222 | 40.1 | | | 223 | 47.9 | | | 224 | 34.3 | | |

| | | | | | | | | | | | | | | | |
|---|---|---|---|---|---|---|---|---|---|---|---|---|---|---|---|
| 225 | 55.1 | 18.3 | | 227 | 55.7 | | | 228 | 70.8 | 16.7 | | 230 | 29.1 | 20.5 | |
| 234 | 98.4 | 36.3 | 19.9 | 235 | 86.1 | 83.0 | 34.5 | 236 | 54.5 | 21.1 | 3.3 | 237 | 24.0 | 23.3 | |
| 238 | 32.0 | 9.3 | 9.7 | 243 | 87.4 | 38.7 | | 244 | 57.5 | 9.3 | | 245 | 136.0 | 53.2 | 73.0 |
| 246 | 57.9 | 39.7 | 14.2 | 247 | 84.1 | 39.8 | | 248 | 74.7 | 56.8 | 25.9 | 249 | 55.8 | 29.9 | 32.5 |
| 252 | 36.5 | 13.2 | 31.7 | 253 | 27.1 | 18.1 | | 254 | 34.7 | 28.1 | 35.3 | 255 | 23.9 | | |
| 256 | 72.3 | 14.5 | | 258 | 67.7 | 23.0 | | 268 | 49.5 | 12.2 | 25.1 | 269 | 50.5 | 15.6 | 23.2 |
| 270 | 51.2 | | | 273 | 21.7 | | | 276 | 74.4 | 72.1 | 81.1 | | | | |
| | | | | | | | | | | | | | | | |

As is clearly demonstrated, the example compounds described above of the present invention inhibited adipogenesis in the osteoblasts. Thus the compounds of the present invention are useful as a preventive or a therapeutic agent for osteoporosis.

(Test Example 3) Inhibition of the differentiation of adipocytes.

Rat white adipocytes contained in the white adipocyte culture kit purchased from HOKUDO were used. As the medium for proliferation and for differentiation-inducing, the medium contained in the white adipocyte culture kit purchased from HOKUDO was used. The cells in the present experiment were cultured in a $CO_2$ incubator (37°C, 95% R.H., 5% $CO_2$).

Immediately after arrival of the purchased cells, all medium for transportation was discarded and replaced with 5 ml of proliferation medium (/25 $cm^2$-flask) and the cells were cultured for one day. Then, a cell suspension was prepared with proliferation medium so as to be 83,000 cells/mL, and the cell suspension was plated into a type I 96-well collagen-coated microplate (SUMITOMO BAKELITE CO., ltd.) so as to be 5,000 cells/well (60 µL/well). For the blank, the proliferation medium that did not contain the cells was plated into the well (blank well) in each plate.

On the next day, all the proliferation medium was discarded and replaced with 147µL/well of differentiation-inducing medium. Further, 1) in the group treated with the test compound, 3 µL/well of the solution, in which a test compound (100 µM) was dissolved in DMSO solution and diluted with differentiation-inducing medium by 20 times (final concentration of the test compound: 100 nM, final concentration of DMSO solution: 0.1% (v/v)), were added to the well where the cells were plated. 2) In the control group, 3 µL/well of DMSO solution that was diluted with differentiation-inducing medium by 20 times (final concentration of DMSO: 0.1% (v/v)) were added to the well where the cells were plated. Further, 3) in the blank group, 3 µL/well of DMSO solution that was diluted with differentiation-inducing medium by 20 times (final concentration: 0.1% (v/v)), was added to the blank well.

After culturing for 5 days, all differentiation-inducing medium was discarded and replaced with 60 µL of 10% (v/v) formaldehyde solution (for fixation) in each well and the cells were kept stationary for 20 min at room temperature. After this, the fixative solution was discarded and replaced with 60 µL of 0.2% (v/v) Triton X (Sigma-Aldrich Japan K. K.) solution in each well and kept stationary for 5 min at room temperature. Then all Triton X solution was discarded and replaced with 60 µL of 60% (v/v) isopropanol solution containing dissolved Oil Red O (Sigma-Aldrich Japan K. K.) so as to be 0.3%, which was adipose tissue staining solution, and the well was left stationary for 10 min at room temperature. After the adipose tissue staining solution was discarded, the well was washed twice with 60 µL of 60% isopropanol solution. Then, 100 µL of the DMSO solution were added to each well and stirred for 5 min at room temperature. The absorbance was measured at 550 nm (ABS550) with a multi-plate reader (LabSystems), and the stained amount with Oil Red O was determined. The measured level at ABS550 in the control group was defined as 100%, while the level at ABS550 in the blank group was defined as 0%. The differentiation rate (%) of the adipocytes treated with the test compound was calculated.

The results are summarized is Table 3, as shown below.

[Table 3]

| Example No. | Rate of Differentiation (%) | Example No. | Rate of Differentiation (%) | Example No. | Rate of Differentiation (%) | Example No. | Rate of Differentiation (%) |
|---|---|---|---|---|---|---|---|
| 10 | 51.1 | 20 | 57.7 | 21 | 63.0 | 23 | 57.7 |
| 24 | 59.1 | 25 | 84.0 | 31 | 77.2 | 49 | 71.0 |
| 58 | 51.7 | 62 | 68.5 | 64 | 85.5 | 75 | 88.6 |
| 77 | 68.0 | 87 | 75.2 | 88 | 63.3 | 93 | 62.9 |
| 94 | 68.8 | 118 | 53.6 | 120 | 79.8 | 122 | 78.4 |
| 124 | 84.2 | 126 | 75.2 | 128 | 35.4 | 130 | 64.0 |
| 132 | 26.9 | 143 | 84.9 | 146 | 78.2 | 149 | 80.3 |
| 155 | 82.7 | 163 | 48.0 | 165 | 85.8 | 171 | 43.0 |
| 174 | 68.8 | 177 | 14.0 | 180 | 59.1 | 184 | 46.6 |
| 186 | 56.3 | 190 | 64.2 | 191 | 63.4 | 192 | 17.6 |
| 193 | 62.4 | 194 | 37.1 | 195 | 36.5 | 196 | 53.4 |
| 197 | 45.7 | 198 | 48.2 | 199 | 35.8 | 200 | 44.1 |
| 201 | 46.9 | 202 | 35.4 | 203 | 32.6 | 204 | 38.3 |
| 205 | 39.8 | 206 | 30.6 | 207 | 32.7 | 208 | 35.1 |
| 209 | 40.1 | 210 | 47.6 | 211 | 37.3 | 212 | 44.0 |
| 213 | 47.4 | 214 | 31.7 | 215 | 45.2 | 216 | 32.1 |

| | | | | | | | |
|---|---|---|---|---|---|---|---|
| 217 | 21.0 | 218 | 31.7 | 219 | 36.6 | 220 | 38.4 |
| 221 | 40.2 | 222 | 48.6 | 223 | 35.0 | 224 | 40.9 |
| 225 | 45.9 | 226 | 42.0 | 227 | 39.7 | 228 | 17.5 |
| 229 | 62.7 | 230 | 48.9 | 231 | 20.1 | 232 | 45.4 |
| 233 | 47.4 | 234 | 60.5 | 235 | 3.6 | 236 | 47.1 |
| 237 | 46.9 | 238 | 64.0 | 239 | 52.8 | 240 | 35.2 |
| 241 | 57.9 | 242 | 57.6 | 243 | 41.1 | 244 | 45.0 |
| 245 | 36.1 | 247 | 54.0 | 248 | 64.4 | 249 | 61.9 |
| 250 | 40.8 | 251 | 39.9 | 252 | 36.7 | 253 | 55.7 |
| 254 | 52.8 | 255 | 55.0 | 256 | 38.2 | 257 | 59.8 |
| 258 | 39.0 | 259 | 56.5 | 260 | 84.6 | 265 | 80.8 |
| 266 | 63.5 | 267 | 47.3 | 268 | 54.8 | 269 | 52.9 |
| 270 | 45.9 | 271 | 45.4 | 272 | 41.7 | 273 | 47.9 |
| 275 | 47.9 | 276 | 14.9 | 278 | 68.6 | | |

The example compounds described above of the present invention inhibited the differentiation of the white adipocytes. Thus the compounds of the present invention are useful as a therapeutic agent for obesity.

(Test Example 4) Determination of enhancing activity for leptin production.

Hanks solution was used for collection of adipose tissue. The Hanks solution was prepared by filtration of a mixed solution (pH: 7.5) of 120 mM NaCl, 4.8 mM KCl, 0.74 mM $MgSO_4$, 0.30 mM $Na_2HPO_4$, 0.40 mM $KH_2PO_4$, 20 mM HEPES, 0.05% (w/v) glucose, 2% (w/v) BSA (Sigma-Aldrich Japan K. K., Cat. No. A7888), 0.95 mM $CaCl_2$, 4.17 mM $NaHCO_3$ and 1% (v/v) penicillin-streptomycin liquid (GIBCO BRL, Cat. No. 15140-122). The adipocytes were cultured in Dulbecco's modified Eagle's medium (GIBCO BRL, Cat. No. 11995-040) mixed with DMEM/F-12 (GIBCO BRL, Cat. No. 11320-033) at a ratio of 1:1, and added 0.2% (v/v) Hanks solution, 0.2 μg/L sodium selenite (GIBCO BRL, Cat. No. 13012-018), 0.002% (v/v) ethanolamine, 25 mM HEPES and 1% (v/v) penicillin-streptomycin liquid (GIBCO BRL, Cat. No. 15140-122). As many as possible of the other reagents used were special grade. In this experiment, the cells were cultured in a $CO_2$ incubator (37°C, 95% humidity, 5% $CO_2$).

Male Wistar Imamichi rats of 6 weeks old were sacrificed by bleeding following decapitation, and adipose tissue was removed from the epididymis and sectioned after the vessels were removed in warmed Hanks solution at 37°C and were weighed. One (1) mL of Hanks solution containing 10 mg collagenase (Wako Pure Chemical Industries, Ltd., 034-10533) was added to the adipose tissue prepared. The solution was transferred into a tube having a volume of 50 mL, and the cells were evenly dispersed by incubation under gentle shaking at 37°C, 90 rpm, for 50 min. Then 20 mL of Hanks solution were added and filtrated through Nylon-mesh with a mesh diameter of 200 μm for removal of undigested tissue pieces. The floating adipocytes were separated from sediments and substances located in the lower layer by centrifugation at 1,000 rpm for 1 min at room temperature. The sediment and 22 mL of the low layer were pulled out with a probe, and the floating adipocytes were recovered. This procedure was repeated twice with Hanks solution and for 4 times with the culture medium. After these procedures the adipocytes were suspended in the culture medium at a volume of 60 mL per 1 g of the adipose tissue weighed previously. 100 μL of the suspended solution were plated in each well of a 96-well microplate (ASAHI TECHNO GLASS Corp.) In the group treated with the test compound, 1 μL/well of DMSO solution containing the dissolved the test compound at concentrations of 200 μM, 20 μM, 2 μM and 0.2 μM (final concentrations of the test compound: 1 μM, 100 nM, 10 nM and 1 nM, respectively) was plated in each of the well. In the control group, 1 μL/well of DMSO (final concentration: 0.1% (v/v)) was plated in each well. The plate was placed stationary in a $CO_2$ incubator for 1 hr or longer, and the contents in the solution were equilibrated at 37°C. After they were equilibrated, the solution in each well was stirred with a magnetic stirrer in a water bath and the suspended solution maintained at 37°C was plated into each well of the plate at 100 μL/well. After incubation of the cells for 24 hr, the suspended solution of cells was filtrated through a filter for the microplate, MultiScreen (MILLOPORE, MDV-N65). The concentration of leptin in the filtrated solution was determined with ELISA methods by using a Rat Leptin Determination Kit-IBL (Immuno-Biological Laboratories Co., Ltd., Cat. No. 17195). The facilitating activity of the compound to produce leptin was calculated from the following equation based on the concentration of leptin determined.
Facilitating Activity of the Compound to Produce Leptin (%)=(A/B)×100

A: Concentration of Leptin in the Treated Group with the Test Compound.
B: Concentration of Leptin in the Control Group.

Results of the determination on the facilitatory action of the test compounds to produce leptin are summarized in the following Table 4 (unit: %).

[Table 4]

| Example No. | 1μM | 100nm | 10nm | 1nm | Example No. | 1μM | 100nm | 10nm | 1nm |
|---|---|---|---|---|---|---|---|---|---|
| 2 | 113.3 | | | | 162 | 110.7 | | | |
| 6 | 108.1 | 110.7 | | | 163 | 123.7 | 117.5 | 117.1 | 107.5 |
| 7 | | 113.8 | | | 166 | 113.6 | 114.7 | | |
| 10 | 118.2 | | | | 169 | | 111.6 | | |
| 20 | 125.5 | 154.7 | 135.3 | 130.5 | 170 | | 117.1 | | |
| 21 | 129.7 | 149.7 | 119.9 | 116.2 | 171 | 126.7 | 128.1 | 115.4 | 113.2 |

| | | | | | | | | | |
|---|---|---|---|---|---|---|---|---|---|
| 23 | 109.8 | 158.1 | 138.0 | 133.9 | 173 | 118.8 | | | 112.4 |
| 24 | 144.1 | 142.6 | 121.4 | 112.2 | 174 | | 127.0 | 123.1 | 117.7 |
| 25 | | 156.3 | 117.9 | 112.7 | 176 | | 110.1 | | |
| 26 | | 114.7 | | | 177 | 144.6 | 133.7 | 120.9 | |
| 28 | 107.7 | 120.3 | | | 178 | 117.4 | | | |
| 29 | 141.7 | | | | 182 | | 111.6 | 110.3 | |
| 31 | | 116.9 | | | 183 | | 112.5 | | |
| 36 | | 120.4 | | | 184 | | 122.6 | 122.4 | |
| 39 | 118.8 | 109.2 | | | 185 | 123.4 | 131.7 | 121.9 | |
| 42 | 127.0 | | | | 186 | 133.1 | 116.8 | 117.9 | |
| 43 | 111.3 | 112.0 | | | 187 | | 126.2 | 113.3 | |
| 49 | 120.8 | 132.9 | 130.7 | | 188 | | | | 110.2 |
| 50 | 108.7 | 119.4 | 123.7 | 118.4 | 192 | 112.1 | 121.7 | 121.7 | |
| 52 | | | | 110.6 | 195 | 120.2 | 122.7 | 114.2 | |
| 54 | 113.4 | | | | 198 | 111.3 | 111.3 | | |
| 55 | | 112.8 | 114.9 | 115.6 | 200 | 116.9 | | | |
| 62 | | 132.2 | 115.4 | 119.1 | 201 | 112.2 | 114.2 | | |
| 64 | | 118.7 | 112.0 | 110.3 | 202 | 137.2 | 133.1 | 135.6 | |
| 75 | | 117.5 | | | 203 | 133.9 | 124.8 | 134.6 | |
| 77 | 113.4 | 129.9 | 109.7 | 108.3 | 204 | 133.3 | 143.4 | 128.8 | |
| 78 | 113.8 | 126.2 | | | 205 | 119.5 | 122.6 | 123.2 | |
| 82 | 111.1 | 108.5 | | | 206 | 118.8 | 112.1 | | |
| 83 | | 125.6 | | | 207 | 115.9 | 109.6 | 107.3 | |
| 86 | | 110.5 | | | 208 | | 110.6 | | |
| 87 | 115.2 | 113.4 | | | 209 | 117.2 | 111.0 | | |
| 88 | 114.9 | 118.8 | | | 212 | 111.6 | | | |
| 89 | | 123.2 | | | 214 | 124.7 | 125.1 | 112.4 | |
| 91 | 113.6 | 110.2 | | | 216 | 117.4 | 112.1 | 112.9 | |
| 92 | | 115.6 | | | 217 | 121.2 | 116.7 | 115.9 | |
| 93 | 113.5 | | | | 219 | 117.4 | 119.0 | 112.6 | |
| 94 | 115.9 | 110.0 | | | 220 | 111.3 | | | |
| 98 | 117.0 | 110.1 | | | 222 | 113.5 | 109.4 | 110.5 | |

| | | | | | | | | | |
|---|---|---|---|---|---|---|---|---|---|
| 99 | 113.9 | | | | 223 | 115.9 | 115.2 | 109.0 | |
| 101 | 117.2 | | | | 224 | 113.0 | 112.0 | 109.6 | |
| 109 | 112.4 | | | | 225 | 117.2 | 108.5 | 106.9 | |
| 118 | 134.0 | 126.7 | 114.6 | 114.0 | 226 | | | 111.2 | |
| 120 | 120.7 | 118.9 | 110.4 | 107.5 | 228 | 111.5 | 121.7 | 118.7 | |
| 121 | | 114.0 | | | 230 | | | 110.1 | |
| 122 | 128.5 | 128.5 | | | 231 | | 154.9 | 128.3 | |
| 124 | 123.7 | 145.5 | 113.0 | 112.8 | 233 | | 120.8 | 111.7 | |
| 126 | 141.5 | 120.6 | 109.4 | | 235 | 142.6 | 121.3 | 110.8 | |
| 127 | 114.8 | | | | 236 | | | 112.0 | |
| 128 | 137.3 | 130.8 | 108.8 | | 242 | | 111.1 | | |
| 130 | 116.2 | 123.0 | | | 245 | 110.7 | | 111.0 | |
| 132 | 127.2 | 119.3 | 137.4 | 123.0 | 246 | | 113.3 | 111.1 | |
| 138 | 111.3 | | | | 249 | 110.6 | 112.9 | 113.7 | |
| 140 | | 114.4 | | | 251 | | 107.2 | 110.8 | |
| 146 | 126.5 | 137.9 | 112.2 | | 252 | 107.6 | 112.7 | 110.1 | |
| 149 | 116.9 | 111.0 | | | 254 | 108.9 | 106.0 | 111.4 | |
| 151 | 112.7 | 112.9 | | | 258 | 111.8 | 114.0 | 120.0 | |
| 152 | | 116.4 | | | 259 | | 109.9 | 111.3 | |
| 153 | 112.6 | | | | 267 | | 110.3 | 117.5 | |
| 154 | 116.9 | 109.5 | | | 270 | | | 118.4 | |
| 155 | 114.3 | 129.7 | | | 271 | | 115.2 | 120.6 | |
| 156 | 111.8 | 117.6 | | | 276 | 116.6 | 122.9 | 111.5 | 108.3 |
| 161 | 131.3 | | | | | | | | |

As shown clearly in Table 4, the compounds of the present invention exert remarkable facilitating activity on leptin production. Thus the compounds of the present invention are useful as an agent for obesity.

(Test Example 5) Inhibition of differentiation as an indication of acetic acid uptake.

Mouse preadipocyte strain 3T3-L1 (ATCC CCL-92.1), which was successively cultured under the conditions described below, was used. As the basal medium, Dulbecco's modified Eagle's (DEM) medium (NISSUI PHARMACEUTICAL CO., LTD) was used. The medium was prepared by addition of the following compounds so as to be at the final concentrations indicated in each parenthesis; glucose (final concentration: 4.5 g/L), D-biotin (final concentration: 8 mg/L), pantothenic acid (final concentration: 4 mg/L), streptomycin sulfate (final concentration: 50 mg/L), penicillin G (final concentration: 100,000 units/L), HEPES (pH: 7.2, final concentration: 10 mM) and L-glutamine (final concentration: 0.584 g/L). Then the cells were successively cultured in the medium to which was added 10% (v/v) inactivated bovine fetal serum (FBS) (PAA, Lot. 07347). All culturing in the present Test Example was carried out in a $CO_2$ incubator

at 37°C and 10% $CO_2$ concentration.

The successively cultured 3T3-L1 cells were inoculated in a Biocoat Collagen I 96-well white/clear plate (Becton Dickinson and Co.) at a concentration of 4,900 cells/well, and were cultured in the basal medium containing added 10% (v/v) inactivated bovine fetal serum (FBS) for 10 days.

The medium was replaced with a differentiation-inducing medium in which inactivated FBS (final concentration: 5%), insulin (Sigma-Aldrich Japan K. K., final concentration: 10 μg/mL), isobutyl methyl xanthine (Aldrich K.K., final concentration: 0.5 mM) and dexamethazone (final concentration: 1 μM) were added so as to be at the final concentrations indicated in the parentheses. The cells were divided in the following 2 groups and were cultured for 4 days; 1) in the presence of 0.01% (v/v) dimethylsulfoxide, and 2) in the presence of 0.01% (v/v) dimethylsulfoxide in which was dissolved 1 mM of the test substance (final concentration of 0.1 μM). Then, the medium was further replaced with a maintenance medium in which inactivated FBS and insulin were added to the base medium so as to be at final concentrations of 5% and 100 ng/mL, respectively, and the cells were cultured for 2 days.

The inhibitory activity of the test compound against cell differentiation was assayed by using uptake of [2-$^{14}$C] acetic acid into the cultured cells as an indicator. The supernatant of the culture medium of the adipocytes was discarded and was replaced with 50 μL/well of the base medium to which was added [2-$^{14}$C] acetic acid (Amersham Biosciences K.K., 1.85 Gbq/mmol, final concentration: 7.4 Kbeq/mL), inactivated FBS (final concentration: 5%) and insulin (final concentration: 100 ng/mL). Then the cells were further incubated with the newly prepared medium for 1 hr. After washing the well with PBS (-), the cells were dried. To these wells, 100 μL/well of a scintillation cocktail (Hewlett-Packard Co., MICROSCINT-20 PACKARD) were added and their radioactivities were determined with a Packard TopCont microplate Scintillation Counter (Hewlett-Packard Co., PACKARD) and the uptake level of [2-$^{14}$C] acetic acid into the cultured cell was calculated.

On the basis of the calculated uptake level of [2-$^{14}$C] acetic acid into the cultured cells, the relative uptake level of [2-$^{14}$C] acetic acid in the group treated with the test compound was calculated against the uptake level of [2-$^{14}$C] acetic acid in the presence of 0.01% (v/v) dimethylsulfoxide solution described in 1) (100%).

Value = Uptake Level (cpm) of [2-$^{14}$C] Acetic Acid in the Presence of the Test Substance

Control = Uptake Level (cpm) of [2-$^{14}$C] Acetic Acid in the Control Group (0.01% dimethylsulfoxide)

Uptake Rate (%) of [2-$^{14}$C] Acetic Acid = 100 × [Value]/[Control]

Results of the test compound are summarized in Table 5.

[Table 5]

| Example No. | Rate of Uptake (%) | Example No. | Rate of Uptake (%) | Example No. | Rate of Uptake (%) | Example No. | Rate of Uptake (%) |
|---|---|---|---|---|---|---|---|
| 1 | 64.0 | 2 | 42.7 | 3 | 68.9 | 4 | 92.5 |
| 5 | 43.4 | 6 | 55.8 | 7 | 65.0 | 8 | 47.2 |
| 9 | 59.9 | 10 | 85.3 | 11 | 67.3 | 12 | 66.4 |
| 13 | 93.0 | 14 | 82.1 | 15 | 78.4 | 16 | 83.7 |
| 17 | 91.9 | 18 | 53.8 | 19 | 64.8 | 20 | 65.9 |
| 21 | 70.3 | 22 | 51.5 | 23 | 59.5 | 24 | 66.1 |
| 25 | 66.3 | 26 | 87.5 | 27 | 66.0 | 28 | 87.8 |
| 29 | 68.6 | 30 | 60.1 | 31 | 85.9 | 32 | 60.5 |
| 33 | 86.1 | 34 | 60.8 | 35 | 71.5 | 36 | 29.2 |

[Table 5] (continued)

| Example No. | Rate of Uptake (%) | Example No. | Rate of Uptake (%) | Example No. | Rate of Uptake (%) | Example No. | Rate of Uptake (%) |
|---|---|---|---|---|---|---|---|
| 37 | 38.6 | 38 | 82.0 | 39 | 62.5 | 40 | 45.3 |
| 41 | 53.8 | 42 | 60.0 | 43 | 56.7 | 44 | 67.6 |
| 45 | 68.9 | 46 | 67.0 | 47 | 63.1 | 48 | 70.3 |
| 49 | 69.6 | 50 | 61.4 | 51 | 57.5 | 52 | 52.3 |
| 53 | 76.2 | 54 | 51.3 | 55 | 55.4 | 56 | 32.4 |
| 57 | 58.4 | 59 | 36.3 | 60 | 68.8 | 61 | 74.8 |
| 62 | 82.9 | 63 | 85.5 | 64 | 96.2 | 65 | 63.4 |
| 66 | 70.7 | 67 | 70.2 | 68 | 67.9 | 69 | 57.0 |
| 70 | 70.2 | 71 | 65.8 | 72 | 76.7 | 73 | 81.5 |
| 74 | 70.4 | 75 | 80.1 | 76 | 54.3 | 77 | 62.7 |
| 78 | 68.2 | 79 | 41.5 | 80 | 83.3 | 82 | 83.0 |
| 83 | 75.6 | 84 | 59.5 | 85 | 67.1 | 86 | 51.7 |
| 87 | 57.1 | 88 | 56.4 | 89 | 58.1 | 90 | 60.5 |
| 91 | 85.8 | 92 | 63.0 | 93 | 90.3 | 94 | 80.4 |
| 95 | 86.3 | 96 | 98.7 | 98 | 89.1 | 101 | 78.3 |
| 102 | 76.8 | 103 | 72.1 | 104 | 89.8 | 105 | 89.4 |
| 106 | 82.0 | 107 | 71.3 | 108 | 87.7 | 109 | 64.6 |
| 110 | 84.6 | 111 | 61.2 | 112 | 82.3 | 113 | 84.6 |
| 114 | 79.0 | 116 | 95.1 | 117 | 87.0 | 118 | 79.6 |
| 119 | 65.7 | 120 | 64.6 | 121 | 81.0 | 122 | 75.4 |
| 123 | 96.2 | 124 | 60.0 | 136 | 88.9 | 139 | 59.2 |
| 140 | 72.4 | 141 | 78.7 | 142 | 74.4 | 143 | 60.1 |
| 144 | 82.2 | 145 | 87.6 | 146 | 60.0 | 147 | 76.2 |
| 148 | 75.3 | 149 | 76.5 | 150 | 82.9 | 151 | 62.3 |
| 152 | 69.6 | 153 | 70.2 | 154 | 67.4 | 178 | 66.2 |

The compounds of the present invention showed inhibitory activity against lipid synthesis. Thus the compounds of the present invention are useful as a preventive or a therapeutic agent for obesity.

(Test Example 6) Plasma glucose lowering effects.

Unless otherwise described, mice were bred individually, and food and water were taken ad libitum in the following experiments. For this, F2 food powder (Funahashi Farm) was used. Plasma glucose level in the blood collected from the tail vein of a mouse with a heparin-coated glass tube was determined with the Glucoloader (A&T Corp.).

Male KK mice (purchased from Clea Japan, Inc) were purchased at 6 weeks old and were bred until 19 weeks old through an acclimatisation period and test period. The plasma glucose level of each mouse was determined and the occurrence of hyperglycemia in the mice was confirmed. The animals with a plasma glucose level of 350 mg/dl and higher were screened and the animals for use in the experiment were selected. The selected mice were divided into a control group and a test compound treated group (n=6/group) matched for body weights and plasma glucose levels.

In the control group, conventional food powder was given to each mouse, while in the test compound-treated group, the food comprising the food powder (F2) mixed with the test compound (final concentration: 0.3 weight %) was

given to each mouse. The mice in both groups were bred for 1 week.

One week after the treatment, the blood glucose level was determined. The results are shown in Table 6.

[Table 6]

| Group | plasma glucose on Day7 | | | % of inhibition (vs. control) |
|---|---|---|---|---|
| Control | 568 | ± | 47 | |
| Compound of Example No. 18 | 315 | ± | 14 | 55 |

As shown clearly, the glucose level of the mice treated with the compound of the present invention was remarkably decreased. Thus the compounds of the present invention are useful as a therapeutic agent for diabetes mellitus.

(Test Example 7) Experiments for facilitating activity of the compounds against uptake of lipids.

Rat white adipocytes contained in the white adipocyte culture kit, which was purchased from HOKUDO, were used. Mediums for proliferation and for differentiation-inducing, which were contained in the white adipocyte culture kit purchased from HOKUDO, were used. The cells in the present experiment were cultured in a $CO_2$ incubator (37°C, 95% humidity, 5% $CO_2$).

Immediately after arrival of the purchased cells, all medium for transportation was discarded and replaced with 5 ml of proliferation medium (/25 $cm^2$-flask) and the cells were cultured for one day. Then, a cell suspension was prepared with proliferation medium so as to be 83,000 cells/mL, and the suspension was plated into a type I 96-well collagen-coated microplate (SUMITOMO BAKELITE CO., ltd.) so as to be 5,000 cells/well (60 µL/well). For the blank, the proliferation medium without the cells was plated into the well (blank well) of each plate.

On the next day, all of the proliferation medium was discarded and replaced with 147 µL/well of differentiation-inducing medium. Further, 1) in the group treated with the test compound, 3 µL/well of a solution in which the test compound (100 µM) was dissolved in DMSO solution and diluted with differentiation-inducing medium by 20 times (final concentration of the test compound: 100 nM, final concentration of DMSO: 0.1 % (v/v)) were added to the well where the cells were plated. 2) In the control group, 3 µL/well of DMSO solution that was diluted with differentiation-inducing medium by 20 times (final concentration of DMSO: 0.1% (v/v)) were added to the well where the cells were plated. Further, 3) in the blank group, 3 µL/well of DMSO solution that was diluted with differentiation-inducing medium by 20 times (final concentration: 0.1% (v/v)) were added to the blank well.

After culturing for 5 days, all differentiation-inducing medium was discarded and replaced with 60 µL of 10% (v/v) of formaldehyde solution (for fixation) in each well and the cells were kept stationary for 20 min at room temperature. After this the fixative solution was discarded and replaced with 60 µL of 0.2% (v/v) Triton X (Sigma-Aldrich Japan K. K.) solution in each well and kept stationary for 5 min at the room temperature. Then all Triton X solution was discarded and replaced with 60 µL of 60% (v/v) isopropanol solution containing dissolved Oil Red O (Sigma-Aldrich Japan K. K.) so as to be 0.3%, which was adipose tissue staining solution, and the well was left stationary for 10 min at room temperature. After the adipose tissue staining solution was discarded, the well was washed twice with 60 µL of 60% (v/v) isopropanol solution. Then, 100 µL of the DMSO were added to each well and stirred for 5 min at room temperature. The absorbance was measured at 550 nm (ABS550) with a multi-plate reader (LabsSystems), and the stained amount by Oil Red O was determined. The measured level at ABS550 in the control group was defined as 100%, while the level at ABS550 in the blank group was defined as 0%. The differentiation rate (%) of the adipocytes treated with the test compound was calculated.

The resultant is demonstrated is Table 7, as shown below.

[Table 7]

| Example No. | Rate of Differentiation (%) | Example No. | Rate of Differentiation (%) | Example No. | Rate of Differentiation (%) | Example No. | Rate of Differentiation (%) |
|---|---|---|---|---|---|---|---|
| 1 | 121.0 | 2 | 127.2 | 3 | 125.5 | 4 | 128.4 |
| 7 | 120.5 | 8 | 113.6 | 9 | 116.2 | 12 | 120.0 |
| 13 | 126.6 | 14 | 121.2 | 15 | 123.5 | 16 | 135.7 |
| 17 | 114.4 | 18 | 114.8 | 22 | 127.2 | 28 | 110.9 |
| 29 | 110.6 | 30 | 138.6 | 32 | 114.6 | 33 | 126.8 |
| 34 | 140.0 | 35 | 121.8 | 36 | 111.0 | 37 | 114.2 |
| 38 | 136.7 | 40 | 121.6 | 41 | 118.1 | 43 | 117.5 |
| 46 | 124.1 | 47 | 141.2 | 48 | 131.4 | 51 | 129.0 |
| 52 | 133.2 | 54 | 118.3 | 56 | 113.8 | 59 | 121.3 |
| 60 | 110.5 | 63 | 119.8 | 65 | 111.7 | 66 | 113.0 |
| 68 | 116.0 | 69 | 135.0 | 70 | 122.1 | 72 | 123.8 |
| 73 | 113.0 | 80 | 118.1 | 82 | 114.5 | 83 | 114.5 |
| 84 | 151.4 | 85 | 125.4 | 90 | 131.2 | 95 | 115.4 |
| 96 | 123.3 | 99 | 119.0 | 102 | 113.6 | 104 | 114.8 |
| 106 | 112.6 | 113 | 110.7 | 114 | 118.0 | 115 | 147.2 |
| 116 | 123.6 | 117 | 122.5 | 119 | 134.7 | 121 | 136.3 |

| | | | | | | | |
|---|---|---|---|---|---|---|---|
| 123 | 148.1 | 125 | 132.0 | 127 | 112.3 | 131 | 131.6 |
| 133 | 140.7 | 134 | 120.4 | 135 | 128.0 | 136 | 129.4 |
| 137 | 150.2 | 138 | 126.3 | 141 | 133.2 | 142 | 112.5 |
| 145 | 140.6 | 147 | 115.5 | 148 | 118.6 | 150 | 136.6 |
| 151 | 118.1 | 152 | 112.7 | 153 | 110.1 | 154 | 123.8 |
| 157 | 115.7 | 159 | 123.3 | 160 | 128.2 | 161 | 113.4 |
| 162 | 169.8 | 164 | 125.1 | 166 | 115.3 | 168 | 140.0 |
| 169 | 114.9 | 170 | 114.9 | 172 | 119.5 | 173 | 134.0 |
| 175 | 113.9 | 176 | 117.9 | 179 | 128.5 | 181 | 128.0 |
| 182 | 122.4 | 183 | 117.5 | 187 | 112.7 | 189 | 132.8 |
| 246 | 119.5 | 261 | 128.8 | 262 | 167.3 | 263 | 153.8 |
| 264 | 111.5 | 277 | 123.1 | 280 | 190.3 | 281 | 200.0 |

As shown clearly in the Table, the example compounds described above of the present invention facilitated uptake of adipose lipids. Thus the compounds of the present invention are useful as a therapeutic agent for diabetes mellitus.

(Test Example 8) Inhibition of colony forming activity in human colon cancer cells by the compounds.

For the medium to culture the human colon cancer cells, D-MEM/F-12 medium containing inactivated bovine fetal serum at a concentration of 10% (GIBCO) was used. The human colon cancer cells COL-2-JCK (purchased from Institute for Experimental Animals) grown in mass formation in a plastic dish for the cell culture with an inner diameter of 100 mm were stripped off from the dish by using EDTA (ethylenediaminetetraacetic acid) and 0.05% trypsin solution. Then, a cell dilution solution was prepared at a concentration of 100 cells/mL solution which was diluted with the medium (D-MEM/F-12).

Three (3) mL of the cell solution were inoculated in each well of a 6-well plastic plate (300 cells/well). The test compound that was dissolved in DMSO solution so as to be a final concentration of 1 μM of the test compound and that of DMSO solution of 0.1% was added to each well. DMSO solution alone was added into the well for the control so as to be a final concentration of 0.1%.

The cells in each well were cultured for 10 days at 37°C in the presence of 5% $CO_2$. After the culturing was finalised, each well was washed once with Dulbecco-phosphate-buffer-saline solution (bivalent anion). Then, 1.5 ml of neutral buffered formalin solution containing 0.02% crystal violet was added to each well and the plate was kept stationary for 5 min for fixation and staining the cells. After fixation and staining, the cells were washed with water and were air-dried. Then the sum of the colony area ($mm^2$) of the stained cancer cells was calculated by using an image analyzer PCA-11 (SystemScience). From the calculated values, the inhibition rate (%) of the compound against the colony forming activity was obtained based on the following equation.
Inhibition Rate (%) against the colony forming activity = (1-A/B)$\times$ 100

A: Sum of Colony Areas in the Group Treated with 1 μM of the Test Compound
B: Sum of Colony Areas in the Control Group

The results are shown in Table 8.

[Table 8]

| Example No. | Inhibition Rate (%) | Example No. | Inhibition Rate (%) | Example No. | Inhibition Rate (%) | Example No. | Inhibition Rate (%) |
|---|---|---|---|---|---|---|---|
| 2 | 8.0 | 3 | 27.0 | 4 | 62.0 | 5 | 26.0 |
| 6 | 22.0 | 7 | 24.0 | 9 | 29.0 | 11 | 15.0 |
| 14 | 16.0 | 15 | 14.0 | 16 | 48.0 | 17 | 30.0 |
| 20 | 37.0 | 29 | 41.0 | 30 | 42.0 | 32 | 39.0 |
| 33 | 60.0 | 34 | 41.0 | 35 | 15.0 | 37 | 52.0 |
| 38 | 99.9 | 39 | 48.0 | 40 | 35.0 | 41 | 55.0 |
| 44 | 35.0 | 45 | 32.0 | 47 | 18.0 | 51 | 15.0 |
| 52 | 17.0 | 55 | 8.0 | 56 | 6.0 | 57 | 5.0 |
| 59 | 94.0 | 67 | 31.0 | 69 | 84.0 | 72 | 39.0 |
| 75 | 65.0 | 77 | 1.0 | 88 | 8.0 | 90 | 47.0 |
| 92 | 15.0 | 93 | 44.0 | 95 | 1.0 | 96 | 17.0 |
| 97 | 3.0 | 98 | 25.0 | 99 | 32.0 | 100 | 50.0 |
| 101 | 32.0 | 102 | 26.0 | 104 | 22.0 | 114 | 22.0 |
| 118 | 41.0 | 120 | 44.0 | 141 | 47.0 | 143 | 25.0 |
| 147 | 19.0 | 150 | 41.0 | 152 | 24.0 | | |

The compounds of the present invention showed remarkable inhibitory activity against the colony formation. Thus the compounds of present invention are useful as a preventive or therapeutic agent for cancers.

(Test Example 9) Determination of inhibitory activity of the compounds against cytokine production.

The mouse monocyte/macrophage cell line, RAW264.7 (ATCC TIB71) was purchased from Dainippon Pharmaceutical Co., Ltd. The RAW264.7 cells were cultured in a $CO_2$ incubator (37°C, 95% of humidity, 5% of $CO_2$) by using the Dulbecco's modified Eagle's medium (DMEM medium; GIBCO, Cat. No. 11995-040) containing bovine fetal serum (the final concentration: 10% (v/v), MOREGATE, lot No. 474030), penicillin G (the final concentration: 50 units/mL (v/v), GIBCO) and streptomycin (the final concentration: 20 μg/mL, GIBCO).
Sixty (60) μL of the RAW264.7 cell suspension (83,000 cells/mL) were plated into each well of a 96-well microplate (5,000 cells/well). After the cells were cultured for 4 hr in a $CO_2$ incubator, 40 μL of DMEM medium containing 25 ng/mL of LPS (Sigma-Aldrich Japan K. K.) were added into each well (final concentration of LPS: 10 ng/mL). The test compound dissolved in DMSO at a concentration of 1 mM was diluted with DMEM medium by 20 times. Then 2 μL/well of the diluted solution were added to wells containing the plated cells (final concentration of the test compound: 1 μM). As the control, 2 μL/well of DMSO diluted with DMEM medium by 20 times were added to wells containing the plated cells either with LPS (final concentration: 10 ng/mL) or without LPS. After these cells were cultured in a $CO_2$ incubator for 15 hr, the supernatant of the medium was recovered. The amount of mTNF-α in the medium supernatant was determined with a mouse TNF-α ELISA kit Quantikine M (R&D System), according to the experiment protocol attached with the kit.
The inhibitory activity of the test compound against TNF-α production was calculated according to the following equation.

A: The amount of TNF-α in the presence of test compound
B: The amount of TNF-α in the control without LPS
C: The amount of TNF-α in the control with 10 ng/mL of LPS

$$\text{Inhibitory Activity of the Test Compound against TNF-}\alpha\text{ Production (\%)} = 100-(A-B)/(C-B) \times 100$$

The results are summarized in the Table 9.

[Table 9]

| Example No. | Inhibitory Activity (%) | Example No. | Inhibitory Activity (%) | Example No. | Inhibitory Activity (%) | Example No. | Inhibitory Activity (%) |
|---|---|---|---|---|---|---|---|
| 2 | 24.5 | 3 | 17.0 | 4 | 18.5 | 8 | 18.9 |
| 9 | 34.0 | 12 | 21.8 | 13 | 16.2 | 16 | 19.0 |
| 18 | 16.8 | 23 | 24.4 | 24 | 30.1 | 29 | 22.9 |
| 32 | 19.8 | 34 | 15.2 | 36 | 18.0 | 37 | 16.0 |
| 39 | 29.8 | 40 | 27.1 | 41 | 30.0 | 42 | 18.8 |
| 43 | 17.7 | 44 | 18.0 | 46 | 30.7 | 47 | 16.7 |
| 52 | 16.2 | 53 | 15.6 | 54 | 16.8 | 55 | 15.3 |
| 61 | 22.6 | 62 | 20.5 | 67 | 23.2 | 68 | 15.2 |
| 81 | 32.0 | 88 | 28.0 | 90 | 19.4 | 93 | 15.6 |
| 94 | 34.8 | 111 | 28.7 | 114 | 16.8 | 118 | 16.4 |
| 123 | 15.1 | 128 | 28.8 | 134 | 16.4 | 135 | 16.6 |
| 138 | 17.8 | 139 | 42.7 | 141 | 15.9 | 149 | 18.8 |
| 150 | 16.2 | 157 | 38.6 | 165 | 15.7 | 175 | 15.7 |
| 182 | 32.8 | 184 | 15.0 | 188 | 24.2 | 189 | 16.3 |
| 190 | 33.8 | 192 | 27.9 | 194 | 18.4 | 195 | 16.1 |
| 196 | 15.3 | 197 | 21.6 | 199 | 20.8 | 203 | 27.1 |

| | | | | | | | |
|---|---|---|---|---|---|---|---|
| 204 | 32.7 | 205 | 34.9 | 206 | 27.5 | 207 | 27.1 |
| 208 | 26.3 | 209 | 26.9 | 211 | 27.6 | 212 | 31.2 |
| 214 | 20.1 | 216 | 30.6 | 217 | 28.7 | 218 | 20.3 |
| 219 | 21.7 | 220 | 16.3 | 223 | 28.2 | 224 | 22.4 |
| 234 | 32.5 | 235 | 40.2 | 241 | 28.0 | 244 | 26.5 |
| 245 | 34.6 | 246 | 18.0 | 248 | 19.1 | 251 | 29.0 |
| 252 | 19.3 | 253 | 29.0 | 254 | 44.3 | 271 | 25.2 |
| 273 | 19.9 | 276 | 28.5 | | | | |

As is clearly demonstrated in the above Table, the example compounds of the present invention exert remarkable inhibitory activities against TNF-α production. Thus the compounds of the present invention are useful as an antiinflammatory agent.

(Test Example 10) Studies on prevention of liver injury.

Five (5) Balb/c mice (female, 6-8 weeks old) per group were used. Liver injury was elicited in the mouse by injection of concanavalin A (Con A) dissolved in saline (2.0 mg/mL) into the tail vein at a volume of 10 mL/kg of the solution (Hepatology, 21, 190-198 (1995)).

The test compounds were suspended in distilled water containing 0.5% CMC, at a concentration of 10 mg/mL. This suspended solution was orally administered to the mouse at a volume of 10 mL/kg, 30 min prior to injection of Con A (group A).

Apart from this group, distilled water containing 0.5% CMC powder, instead of the test compound, was orally administered in the second group of mice (group B). In the 3rd group, nothing was given to the mice (group C).

Twenty four (24) hr after the injection of Con A, blood was collected from the abdominal aorta of the mice and the plasma was isolated while mixing with heparin. Activities of glutamic acid oxaloacetic acid transaminase (AST) and glutamic acid pyruvic acid transaminase (ALT) in the plasma were determined according to the methods described by Nagakawa et al. (J. Pharmacol. Exp. Ther., 264, 496-500 (1993)). From the values obtained inhibitory rates of the test compound against AST and ALT levels which had been increased by injection of Con A, were obtained based on the following equation.

Inhibitory Rate (%) = $\{1-[(A-C)/(B-C)]\} \times 100$

A: The average level of AST or ALT in the group A
B: The average level of AST or ALT in the group B
C: The average level of AST or ALT in the group C

The results are summarized in Table 10.

Table 10. Preventing rate of the test compound against liver injury (preventing action).

| Example No. | AST (%) | ALT (%) | Example No. | AST (%) | ALT (%) | Example No. | AST (%) | ALT (%) | Example No. | AST (%) | ALT (%) |
|---|---|---|---|---|---|---|---|---|---|---|---|
| 1 | 19.0 | 40.1 | 2 | 4.1 | 64.5 | 8 | 56.0 | 57.0 | 10 | 49.0 | 55.9 |
| 15 | 54.0 | 39.0 | 16 | 21.4 | 44.4 | 18 | 24.1 | 39.8 | 20 | 77.0 | 67.2 |
| 35 | | 12.4 | 37 | | 20.7 | 49 | 61.0 | 59.0 | 53 | 1.7 | 22.0 |
| 59 | 20.5 | 18.7 | 88 | 57.0 | 42.3 | 105 | 27.5 | 22.4 | 106 | 105.8 | 113.6 |
| 114 | 50.5 | 32.8 | 114 | 47.0 | 40.0 | 118 | 45.0 | 55.8 | 136 | 12.0 | 13.1 |
| 139 | 48.4 | 54.0 | 142 | 63.0 | 54.0 | 156 | 82.6 | 85.5 | 159 | 91.2 | 78.7 |
| 161 | 69.3 | 82.8 | 171 | 102.8 | 90.0 | 178 | 64.5 | 50.5 | | | |

As shown clearly in the Table, the compounds of the present invention exert a remarkable preventing action

against liver injury, and are useful as a preventive or a therapeutic agent for the hepatitis.

(Test Example 11) Assessment of the test compound as a therapeutic agent for cataracts.

The lenses were isolated from Sprague-Dawley rats (male, purchased from SLC Japan), and the lenses were pre-cultured in a medium (Medium-199: GIBCO BRL) for 3 hr and the lenses maintaining transparency were selected and used in the experiment. The crystalline lenses were cultured for 24 hr in the following 3 groups; 1) in Medium-199 (3 mL), 2) in Medium-199 (3 mL) containing hydrogen peroxide (final concentration: 5 mM) and 3) in Medium-199 (3 mL) containing the test compound (final concentration: 100 nM), DMSO (final concentration: 0.1% v/v) and hydrogen peroxide (final concentration: 5 mM). After finalising culturing, the opacity of the lens was scored based on the following criteria: clear: 0, slightly opaque: 1, moderately opaque: 2. Thus the improving effect of the test compound on cataracts was assessed.
The results (average scores in each culture condition) are summarized in the below Table.

[Table 11]

| Example No. | | | | |
|---|---|---|---|---|
| Control | Hydrogen peroxide | 53 | 139 | 92 |
| 0.0 | 1.7 | 1.0 | 1.0 | 1.0 |

As is shown clearly in the Table, the example compounds of the present invention exert an improving effect on cataracts. Thus the compounds of the present invention are useful as an agent for cataracts.

(Test Example 12) Quantitative analysis of ABCA1 mRNA.

The mouse monocyte/macrophage cell line, RAW264.7 (ATCC TIB71) was purchased from Dainippon Pharmaceutical Co., Ltd. As the medium, the Dulbecco's modified Eagle's medium (DMEM medium; GIBCO, Cat. No. 11995-040) containing bovine fetal serum (the final concentration: 10% (v/v), MOREGATE, lot No. 474030), penicillin G (the final concentration: 50 units/mL, GIBCO) and streptomycin (the final concentration: 20 μg/mL, GIBCO) was used. The culture of RAW264.7 cells was carried out in a $CO_2$ incubator (37°C, 95% R.H., 5% of $CO_2$).
A 2 ml volume of RAW264.7 cells suspension (1 ×106 cells/mL) was plated in each well of a 6-well plate (2 × 106 cells/well) and the cells were incubated for 2 hr in a $CO_2$ incubator. The test compounds dissolved in DMSO were diluted with DMEM medium by 20 times, and were added to the wells at a volume of 4 μL/well (final concentration of the test compound: 100 nM). As the control, the DMSO solution was diluted with the DMEM medium by 20 times, and was added to the wells at the volume of 4 μL/well. After the cells were cultured in a $CO_2$ incubator for 24 hr, the medium was removed and the total RNA was purified by using an RNeasy Mini Kit (Qiagen), according to the protocol attached. The concentration of the RNA in the samples obtained was calculated from absorbance at 260 nm (A260=1=40 μg/ml). Six (6) μg of total RNA were diluted with RNAase-free water so as to be 22.6 μL, which was heated at 65°C for 10 min, and then was cooled on ice. The RNA in the solution was used as the template, and the cDNA was synthesized by using a First-strand cDNA Synthesis Kit (Amersham Pharmacia Biotech). In this reaction, a solution was prepared having the following composition and was reacted at 37°C for 1 hr.

| | |
|---|---|
| Bulk first-strand reaction mix | 13.4 μL |
| Primer Not I-d (T)18 (dilution by 25 times) | 2.0 μL |
| DTT solution | 2.0 μL |
| RNA (6 μg) | 22.6 μL |
| | 40.0 μL |

After the reaction, 3 μL of soution were collected from each of the reaction mixtures of the test compound-treated cell samples and that of the control cell samples. The mixed solution was used as the standard solution. The standard solution and each reaction solution were purified with a Chroma Spin-100+DEPC-$H_2O$ Column according to the protocol attached, and they were used as the template for TaqMan PCR (Template). In order to draw the calibration curve, the standard solution was diluted by every 5 times for 5 steps and each solution was used for TaqMan PCR. TaqMan PCR was carried out in ABCA1 and β-actin. As Primers for use in the TaqMan PCR, 4 oligonucleotides (abca1 forward (S3), abcal reverse (AS3), β-actin forward (S4) and β-actin reverse (AS4)) were designed which are shown in sequences No. 5 to 8 in the sequence table described later. The synthesis and purification of these 4 oligomers was ordered from

Amersham Pharmacia Biotech Co.
As FAM-TAMRA labelled TaqMan Probe, 4 kinds of oligonucleotides (abca1 (p1) and β-actin (p2)) were designed and were ordered from Applied Biosystems Co. to synthesize and purify.
The following reaction mixture of ABCA 1 or β-actin was divided into 2 wells of a MicroAmp Optical 96-well Reaction Plate (Applied Biosystem Co.). TaqMan Universal PCR Master Mix was purchased from Applied Biosystem Co.

| | |
|---|---|
| Forward Primer (50 pM/μL) | 0.1 μL |
| Reverse Primer (50 pM/μL) | 0.1 μL |
| TaqMan Probe | 1.5 μL |
| TaqMan Universal PCR Master Mix | 25.0 μL |
| $H_2O$ | 18.3 μL |
| Template | 5.0 μL |
| | 50.0 μL/well |

The plate was covered with a MicroAmp Optical Cap (Applied Biosystems Co.), and TaqMan PCR was carried out by using an ABI PRISM 7,700 Sequence Detection System (Applied Biosystem Co.) under the following conditions:

| | | |
|---|---|---|
| 50°C | 2 min | |
| 95°C | 10 min | |
| 95°C | 15 sec | 40 cycles |
| 60°C | 1 min | |

An analysis was made according to the procedures described in the manual of Sequence Detection Software (Applied Biosystem Co.) and the increased rate of ABCA1 mRNA due to the test compound was calculated based on the following equation.

A: Amount of the ABCA1 mRNA in the presence of the test compound
B: Amount of the β-actin mRNA in the presence of the test compound
C: Amount of the ABCA1 mRNA in the presence of DMSO
D: Amount of the β-actin mRNA in the presence of DMSO

$$\text{Increase Rate (\%) of ABCA1 mRNA} = (A/B)/(C/D) \times 100$$

The results are summarized in the Table below.

[Table 12]

| Example No. | Rate of Increase (%) | Example No. | Rate of Increase (%) | Example No. | Rate of Increase (%) | Example No. | Rate of Increase (%) |
|---|---|---|---|---|---|---|---|
| 135 | 163.0 | 217 | 187.0 | 218 | 190.0 | 223 | 204.0 |
| 224 | 179.0 | 273 | 146.0 | | | | |

As shown clearly in the Table, the compounds of the present invention exert remarkable activity in increasing the amount of ABCA1 mRNA. Thus the compounds of the present invention are useful as a therapeutic agent for arteriosclerosis.

[Possibility of industrial utilization]

Compounds having the general formula (I) and pharmaceutically acceptable salts thereof in the present invention exhibit agonistic-, partial agonistic-, antagonistic- and partial antagonistic-like activities against peroxisome proliferator activated receptor γ (PPAR γ).

Thus compounds having the general formula (I) described above in the present invention and pharmaceutically acceptable salts thereof are useful as excellent preventive and/or therapeutic agents for senile osteoporosis, postmenopausal osteoporosis, disuse osteoporosis, method for treatment of steroid-induced osteoporosis, fracture, osteogenesis imperfecta, rachitis, senile arthrosis, obesity, emaciation, type I and type II diabetes mellitus, sclerosis of pulse, lipid metabolism disorder, pancreatitis, autoimmune diseases, glucose metabolism disorder, diabetic neuropathy, diabetic complications, hyperuricemia, leukemia, functional disorders in retinoid related receptors, liver dysfunction, anemia, cancers, inflammation, Basedow's disease, heart disease, Alzheimer's disease, eating disorders, hypertension and renal diseases.

## Claims

**1.** A compound of the following formula (I) or a pharmacologically acceptable salt thereof:

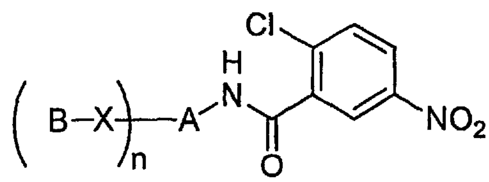

wherein

A represents a phenyl group, a naphthyl group, an acenaphthenyl group, a pyridyl group, a quinolyl group, an isoquinolyl group, a pyrimidinyl group, a furyl group, a benzofuryl group, a pyranyl group, a chromenyl group, a thienyl group, a benzothienyl group, a pyrrolyl group, an indolyl group, an isoindolyl group, an imidazolyl group, a pyrazolyl group, a pyridazinyl group, a pyrazinyl group, an oxazolyl group, an isoxazolyl group, a benzoxazolyl group, a benzisoxazolyl group, a thiazolyl group, an isothiazolyl group, a benzothiazolyl group, benzisothiazolyl or a biphenyl group (said group A is optionally substituted with one, two or more substituents which are the same or different and are selected from the substituent group α described below);
B represents an aryl group, a cycloalkyl group, or a heterocyclic group (said group B is optionally substituted with one, two or more substituents which are the same or different and are selected from the substituent group α and β described below);
X represents a bond, an oxygen atom, a sulfur atom, a $CH_2$ group, a CO group, an NH group, an $SO_2NH$ group, an $NHSO_2$ group, a CONH group, an NHCO group, or a $OCH_2$ group;
n represents 0 or 1;
Substituent group α comprises a $C_1$-$C_{20}$ alkyl group, a nitro group, a cyano group, a carboxyl group, a carboxy-$C_2$-$C_7$ alkyl group, a $C_2$-$C_7$ alkyloxycarbonyl group, a $C_3$-$C_{15}$ alkyloxycarbonylalkyl group, an amino group (said amino group is optionally substituted with one or two $C_1$-$C_6$ alkyl groups which are the same or different, or a $C_3$-$C_6$ alkenyl group), a hydroxyl group (said hydroxyl group is optionally substituted with a $C_1$-$C_6$ alkyl group or a $C_1$-$C_6$ haloalkyl group) and a mercapto group (said mercapto group is optionally substituted with a $C_1$-$C_6$ alkyl group);
Substituent group β comprises a halogen atom, a sulfonamide group, a $C_1$-$C_6$ alkylsulfonamide group, an amidinoaminosulfonyl group or a phenyl group.

**2.** A compound or a pharmacologically acceptable salt thereof wherein A is a thiazolyl group according to Claim 1.

**3.** An agent inhibiting adipocyte differentiation in the marrow containing a compound or a pharmacologically acceptable salt thereof according to Claim 1 or 2.

**4.** An agent enhancing or recovering osteogenetic function containing a compound or a pharmacologically acceptable salt thereof according to Claim 1 or 2

**5.** An agent for treatment or prevention of osteoporosis containing a compound or a pharmacologically acceptable salt thereof according to Claim 1 or 2.

**5.** An agent for treatment or prevention of senile osteoporosis, post-menopausal osteoporosis or disuse osteoporosis containing a compound or a pharmacologically acceptable salt thereof according to Claim I or 2.

**7.** A PPAR γ modulator containing a compound or a pharmacologically acceptable salt thereof according to Claim 1 or 2.

**8.** A blood sugar lowering agent containing a compound or a pharmacologically acceptable salt thereof according to Claim 1 or 2.

**9.** An agent for treatment or prevention of diabetes mellitus containing a compound or a pharmacologically acceptable salt thereof according to Claim 1 or 2.

**10.** An agent for treatment or prevention of type I diabetes mellitus, type II diabetes mellitus, glucose metabolism disorder, diabetes neuropathy or diabetic complications containing a compound or a pharmacologically acceptable salt thereof according to Claim 1 or 2.

**11.** An agent for treatment or prevention of fracture, osteogenesis imperfecta, rachitis, senile arthrosis, obesity, emaciation, sclerosis of pulse, lipid metabolism disorder, pancreatitis, autoimmune diseases, hyperuricemia, leukemia, functional disorders in retinoid related receptors, liver dysfunction, anemia, cancers, inflammation, Basedow's disease, heart disease, Alzheimer's disease, eating disorders, hypertension or renal diseases containing a compound or a pharmacologically acceptable salt thereof according to Claim 1 or 2.

**12.** An agent inhibiting adipocyte differentiation in the marrow containing a PPAR γ modulator.

**13.** An agent enhancing or recovering osteogenetic function containing a PPAR γ modulator:

**14.** An agent for treatment or prevention of osteoporosis containing a PPAR γ modulator.

**15.** An agent for treatment or prevention of senile osteoporosis, post-menopausal osteoporosis or disuse osteoporosis containing a PPAR γ modulator.

**16.** A blood sugar lowering agent containing a PPAR γ modulator.

**17.** An agent for treatment or prevention of diabetes mellitus containing a PPAR γ modulator.

**18.** An agent for treatment or prevention of type I diabetes mellitus, type II diabetes mellitus, glucose metabolism disorder, diabetic neuropathy or diabetic complications containing a PPAR γ modulator.

**19.** An agent for treatment or prevention of fracture, osteogenesis imperfecta, rachitis, senile arthrosis, obesity, emaciation, sclerosis of pulse, lipid metabolism disorder, pancreatitis, autoimmune diseases, hyperuricemia, leukemia, functional disorder in retinoid related receptors, liver dysfunction, anemia, cancers, inflammation, Basedow's disease, heart disease, Alzheimer's disease, eating disorders, hypertension or renal diseases containing a PPAR γ modulator.

**20.** The use of a compound or a pharmacologically acceptable salt thereof according to Claim 1 or 2 in preparation of an agent for treatment or prevention of osteoporosis.

**21.** The use of a PPAR γ modulator in preparation of an agent for treatment or prevention of osteoporosis.

**22.** A partial antagonist of PPAR γ.

**23.** An agent for treatment or prevention according to Claims to 19 wherein the PPAR γ modulator is a partial antagonist of PPAR γ.

**24.** The use of a partial antagonist of PPAR γ in the preparation of an agent for treatment or prevention of osteoporosis.

SEQUENCE LISTING

<110> Sankyo Company, Limited

<120> PPAR-gamma modulator

<130> FP200116WO

<140>

<141>

<150> JP2000-129565

<151> 2000-04-28

<150> JP2001-60366

<151> 2001-03-06

<160> 10

<170> PatentIn Ver. 2.0

<210> 1

<211> 41

<212> DNA

<213> Artificial Sequence

<220>

<223> Description of Artificial Sequence: PCR primer S1

<400> 1

cccagatctc caccatgggt gaaactctgg gagattctcc t 41

<210> 2

<211> 42

<212> DNA

<213> Artificial Sequence

<220>

<223> Description of Artificial Sequence: PCR primer AS1

<400> 2

cccagatctg gatccctagt acaagtcctt gtagatctcc tg 42

<210> 3

<211> 59

<212> DNA

<213> Artificial Sequence

<220>

<223> Description of Artificial Sequence: PCR primer S2

<400> 3

ctagagggga ccaggacaaa ggtcacgttc ggggaccagg acaaaagtca cgttcggga 59

<210> 4

<211> 59

<212> DNA

<213> Artificial Sequence

<220>

<223> Description of Artificial Sequence: PCR primer AS2

<400> 4

tcgatcccga acgtgacctt tgtcctggtc cccgaacgtg acctttgtcc tggtcccct 59

<210> 5

<211> 19

<212> DNA

<213> Artificial Sequence

<220>

<223> Description of Artificial Sequence: PCR primer S3

<400> 5

agttcatcag cggcgtgaa 19

<210> 6

<211> 24

<212> DNA

<213> Artificial Sequence

<220>

<223> Description of Artificial Sequence: PCR primer AS3

<400> 6

ggaccacata attgcacata tccc 24

<210> 7

<211> 21

<212> DNA

<213> Artificial Sequence

<220>

<223> Description of Artificial Sequence: PCR primer S4

<400> 7

ggcgcttttg actcaggatt t 21

<210> 8

<211> 21

<212> DNA

<213> Artificial Sequence

<220>

<223> Description of Artificial Sequencc: PCR primer AS4

<400> 8

ggatgtttgc tccaaccaac t 21

<210> 9

<211> 30

<212> DNA

<213> Artificial Sequence

<220>

<223> Description of Artificial Sequence: Probe P1

<400> 9

cctgtcatct actggctgtc caattttgtc 30

<210> 10

<211> 25

<212> DNA

<213> Artificial Sequence

<220>

<223> Description of Artificial Sequence: Probe P2

<400> 10

aaaactggaa cggtgaaggc gacag 25

【Figure 1】

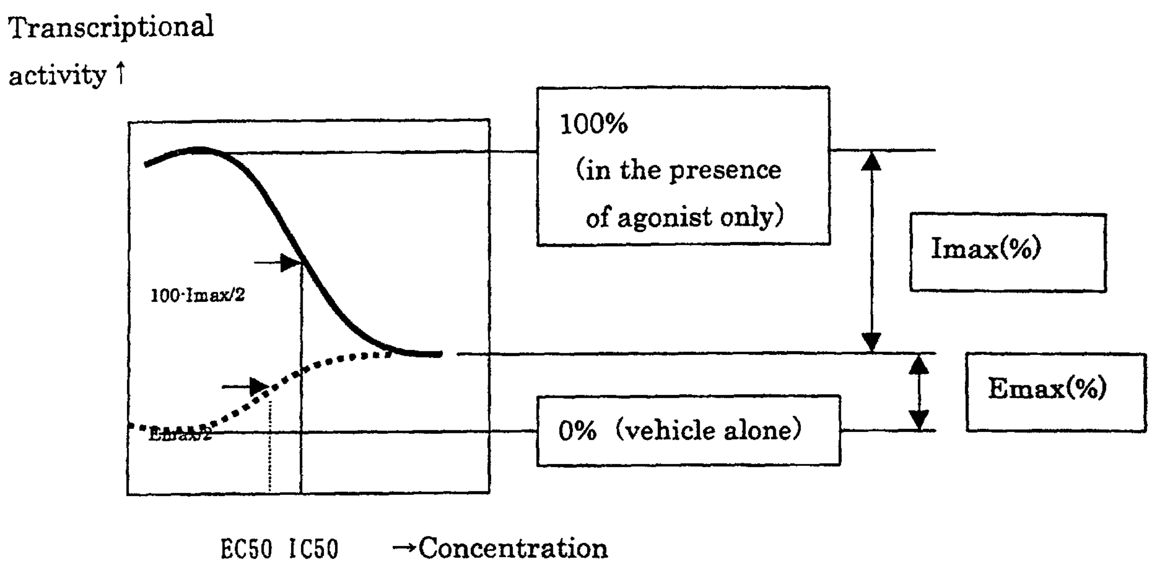
Transcriptional activity ↑
100% (in the presence of agonist only)
100-Imax/2
Imax(%)
Emax(%)
Emax/2
0% (vehicle alone)
EC50 IC50
→Concentration

【Figure 2】

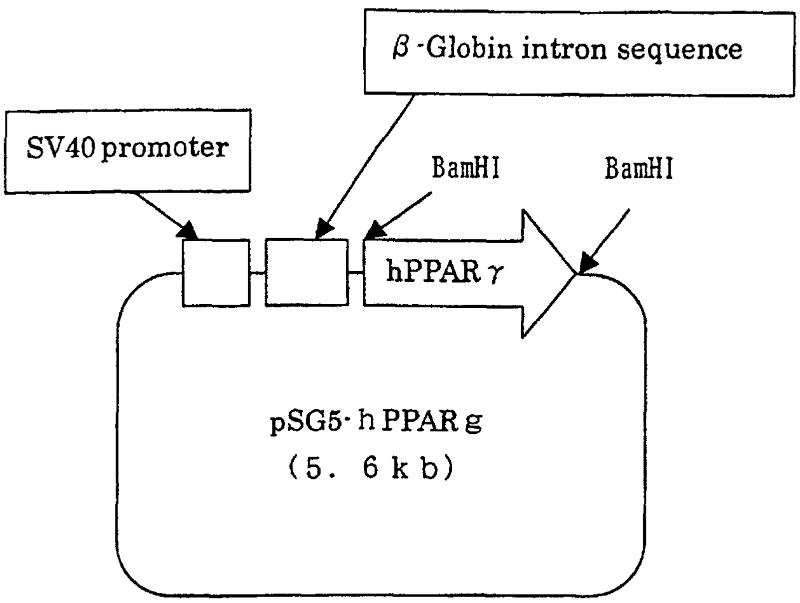
β-Globin intron sequence
SV40 promoter
BamHI
BamHI
hPPARγ
pSG5-hPPARg
(5. 6kb)

【Figure 3】

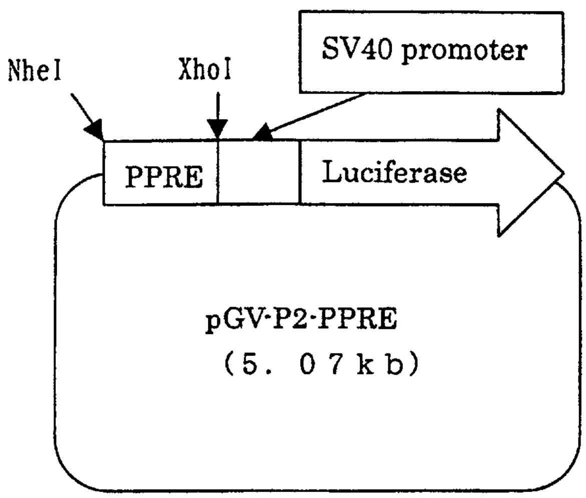

## INTERNATIONAL SEARCH REPORT

International application No.
PCT/JP01/03655

A. CLASSIFICATION OF SUBJECT MATTER
Int.Cl[7] C07C233/65, C07C233/66, C07C233/75, C07C233/80, C07C233/81, C07C271/28, C07C311/08, C07C311/21, C07C311/58, C07C311/64, C07D207/325, C07D213/40, C07D213/75, C07D213/76, C07D213/81, C07D213/82, C07D215/38, C07D215/40, C07D217/22, C07D231/40, C07D231/42,

According to International Patent Classification (IPC) or to both national classification and IPC

B. FIELDS SEARCHED

Minimum documentation searched (classification system followed by classification symbols)
Int.Cl[7] C07C233/65, C07C233/66, C07C233/75, C07C233/80, C07C233/81, C07C271/28, C07C311/08, C07C311/21, C07C311/58, C07C311/64, C07D207/325, C07D213/40, C07D213/75, C07D213/76, C07D213/81, C07D213/82, C07D215/38, C07D215/40, C07D217/22, C07D231/40, C07D231/42,

Documentation searched other than minimum documentation to the extent that such documents are included in the fields searched

Electronic data base consulted during the international search (name of data base and, where practicable, search terms used)
CAPLUS(STN),REGISTRY(STN)

C. DOCUMENTS CONSIDERED TO BE RELEVANT

| Category* | Citation of document, with indication, where appropriate, of the relevant passages | Relevant to claim No. |
|---|---|---|
| PX | WO 00/55118 A1 (GLAXO GROUP LIMITED), 21 September, 2000 (21.09.00) (Family: none) | 1-24 |
| X<br>A | WO 98/34632 A1 (EMISPHERE TECHNOLOGIES, INC.), 13 August, 1998 (13.08.98), & JP 2001-513080 A & EP 1015008 A1 | 1<br>2-11,20,22,24 |
| X<br>A | EP 636625 A2 (AMERICAN CYANAMID COMPANY), 01 February, 1995 (01.02.95), & JP 7-157485 A | 1<br>2-11,20,22,24 |
| X<br>A | NAGARAJAN, Kuppuswamy et al., Piperazinylbenzonaphthoxazepines with CNS depressant properties, Eur. J. Med. Chem.--Chim. Ther., 1986, Vol.21 No.1, pp.21-26 | 1<br>2-11,20,22,24 |
| X<br>A | US 5310760 A (Sterling Winthrop Inc.), 10 May, 1994 (10.05.94) (Family: none) | 1<br>2-11,20,22,24 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 September, 2001 (14.09.01) | 18 September, 2001 (18.09.01) |
| Name and mailing address of the ISA/<br>Japanese Patent Office<br><br>Facsimile No. | Authorized officer<br><br>Telephone No. |

Form PCT/ISA/210 (second sheet) (July 1992)

**INTERNATIONAL SEARCH REPORT**

International application No. PCT/JP01/03655

C (Continuation). DOCUMENTS CONSIDERED TO BE RELEVANT

| Category* | Citation of document, with indication, where appropriate, of the relevant passages | Relevant to claim No. |
|---|---|---|
| X<br>A | NAGARAJAN, K. et al.,<br>Condensed heterotricycles. Synthesis of dibenz[b,f][1,4]oxazepines, dibenz[b,f] [1,4] thiazepines, and dibenz[b,e][1,4]diazepines by cyclization of 2-halo-2'-hydroxy(mercapto or amino)benzanilides,<br>Indian J. Chem., 1974, Vol.12 No.3, pp.227-235 | 1<br>2-11,20,22,24 |
| X<br>A | US 2754209 A (Azoplate Corporation),<br>10 July, 1956 (10.07.56) (Family: none) | 1<br>2-11,20,22,24 |
| PX<br>PA | WO 00/027832 A2 (GLAXO GROUP LIMITED),<br>18 May, 2000 (18.05.00) (Family: none) | 12-15,19,21,23<br>16-18 |
| X<br>A | Jackson, S. M. et al.,<br>Peroxisome proliferator-activated receptor activators modulate the osteoblastic maturation of MC3T3-E1 preosteoblasts,<br>FEBS Lett., 2000, Vol.471 No.1, pp.119-124 | 12-15,19,21,23<br>16-18 |
| X<br>A | WO 99/38845 A1 (TULARIK INC.),<br>05 August, 1999 (05.08.99),<br>& EP 1053227 A1 & US 6200995 B1 | 16-19,21,23<br>12-15 |
| X<br>A | WO 00/00194 A1 (PHOTOGENESIS, INC.),<br>06 January, 2000 (06.01.00),<br>& AU 9947134 A | 16-19,21,23<br>12-15 |
| X<br>A | WO 97/10813 A1 (LIGAND PHARMACEUTICALS INCORPORATED),<br>27 March, 1997 (27.03.97),<br>& EP 788353 A1 & US 5972881 A | 16-19,21,23<br>12-15 |
| X<br>A | WO 96/40128 A2 (THE SALK INSTITUTE FOR BIOLOGICAL STUDIES),<br>19 December, 1996 (19.12.96),<br>& JP 11-506114 A & EP 831818 A2 | 16-19,21,23<br>12-15 |
| X<br>A | WO 96/23884 A2 (LIGAND PHARMACEUTICALS INCORPORATED),<br>08 August, 1996 (08.08.96),<br>& EP 809697 A2 | 16-19,21,23<br>12-15 |
| X<br>A | WO 99/53927 A1 (TRUSTEES OF TUFTS COLLEGE),<br>28 October, 1999 (28.10.99),<br>& EP 1071429 A1 | 16-19,21,23<br>12-15 |
| X<br>A | WO 99/38845 A1 (TULARIK INC.),<br>05 August, 1999 (05.08.99),<br>& EP 1053227 A1 & US 6200995 B1 | 16-19,21,23<br>12-15 |
| X<br>A | WO 98/05331 A2 (LIGAND PHARMACEUTICALS INCORPORATED),<br>12 February, 1998 (12.02.98),<br>& EP 930882 A2 | 16-19,21,23<br>12-15 |
| X<br>A | WO 96/29405 A2 (LIGAND PHARMACEUTICALS INCORPORATED),<br>26 September, 1996 (26.09.96),<br>& EP 815230 A2 | 16-19,21,23<br>12-15 |

Form PCT/ISA/210 (continuation of second sheet) (July 1992)

INTERNATIONAL SEARCH REPORT

International application No.
PCT/JP01/03655

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

(1) claims 1 to 11 and 20: specific compounds and medicinal use thereof; and

(2) claims 12 to 19 and 21 to 24: PPARγ modulators and medicinal use thereof.

Since it does not appear that there is a technical relationship between these groups of inventions involving special technical features, these two groups of inventions are not considered as relating to a group of inventions so linked as to form a single general inventive concept.

Thus, two groups of inventions are described in the claims of this international application.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.

☒ No protest accompanied the payment of additional search fees.

Form PCT/ISA/210 (continuation of first sheet (1)) (July 1992)

**INTERNATIONAL SEARCH REPORT**

International application No.
PCT/JP01/03655

Continuation of A. CLASSIFICATION OF SUBJECT MATTER
(International Patent Classification (IPC))

C07D231/56,C07D233/61,C07D235/32,C07D237/04,C07D237/20,C07D239/20,C07D239/22,
C07D239/26,C07D239/42,C07D239/69,C07D241/20,C07D261/16,C07D263/48,C07D263/58,
C07D277/42,C07D277/44,C07D277/46,C07D277/50,C07D277/52,C07D277/66,C07D277/82,
C07D285/10,C07D285/12,C07D295/12,C07D295/18,C07D295/20,C07D295/22,C07D311/22,
C07D333/38,C07D333/66,C07D417/04,C07D471/04,A61K31/167,A61K31/352,A61K31/381,
A61K31/40,A61K31/415,A61K31/4164,A61K31/4184,A61K31/423,A61K31/426,A61K31/427
,
A61K31/428,A61K31/433,A61K31/437,A61K31/44,A61K31/4402,A61K31/4406,A61K31/440
9,
A61K31/4439,A61K31/4453,A61K31/455,A61K31/47,A61K31/472,A61K31/495,A61K31/496
5,
A61K31/50,A61K31/505,A61K31/5375,A61K31/55,A61K31/635,A61P1/16,A61P1/18,A61P1
3/12,
A61P19/02,A61P19/06,A61P19/08,A61P19/10,A61P25/28,A61P29/00,A61P3/04,A61P3/06
,
A61P3/10,A61P35/00,A61P35/02,A61P37/06,A61P5/14,A61P7/06,A61P9/00,A61P9/10,
A61P9/12

Continuation of B. FIELDS SEARCHED
Minimum documentation searched (International Patent Classification (IPC))

C07D231/56,C07D233/61,C07D235/32,C07D237/04,C07D237/20,C07D239/20,C07D239/22,
C07D239/26,C07D239/42,C07D239/69,C07D241/20,C07D261/16,C07D263/48,C07D263/58,
C07D277/42,C07D277/44,C07D277/46,C07D277/50,C07D277/52,C07D277/66,C07D277/82,
C07D285/10,C07D285/12,C07D295/12,C07D295/18,C07D295/20,C07D295/22,C07D311/22,
C07D333/38,C07D333/66,C07D417/04,C07D471/04,A61K31/167,A61K31/352,A61K31/381,
A61K31/40,A61K31/415,A61K31/4164,A61K31/4184,A61K31/423,A61K31/426,A61K31/427
,
A61K31/428,A61K31/433,A61K31/437,A61K31/44,A61K31/4402,A61K31/4406,A61K31/440
9,
A61K31/4439,A61K31/4453,A61K31/455,A61K31/47,A61K31/472,A61K31/495,A61K31/496
5,
A61K31/50,A61K31/505,A61K31/5375,A61K31/55,A61K31/635,A61P1/16,A61P1/18,A61P1
3/12,
A61P19/02,A61P19/06,A61P19/08,A61P19/10,A61P25/28,A61P29/00,A61P3/04,A61P3/06
,
A61P3/10,A61P35/00,A61P35/02,A61P37/06,A61P5/14,A61P7/06,A61P9/00,A61P9/10,
A61P9/12

Form PCT/ISA/210 (extra sheet) (July 1992)

INTERNATIONAL SEARCH REPORT

International application No.
PCT/JP01/03655

Claims 22 and 24 pertain to compounds having a "partial PPARγ agonist" activity. However, it is recognized that only a part of the claimed compounds are supported by the description under the provision of Article 6 of the PCT and disclosed therein under the provision of Article 5 of the PCT.

Even though the common technical knowledge at the point of the application is taken into consideration, it cannot be recognized that the scope of compounds having the properties as a "partial PPARγ agonist" could be specified. Thus, claim 22 also fails to satisfy the requirement of clearness as defined in Article 6 of the PCT.

Concerning claims 22 and 24, therefore, the search has been practiced on the compounds of claim 1 falling within the scope as particularly disclosed in the description.

Form PCT/ISA/210 (extra sheet) (July 1992)